# EUROPEAN PATENT SPECIFICATION

(11) **EP 4 399 327 B1**
(45) Date of publication and mention of the grant of the patent: **29.10.2025**
(21) Application number: 22785883.4
(22) Date of filing: 07.09.2022
(51) Int. Cl.: C12Q 1/6804, G01N 33/53

(54) **NON-SEQUENCING PCR-BASED METHOD FOR DETECTION OF ANTIBODY-CONJUGATED OLIGONUCLEOTIDES**
NICHT-SEQUENZIERENDES PCR-BASIERTES VERFAHREN ZUM NACHWEIS VON ANTIKÖRPERKONJUGIERTEN OLIGONUKLEOTIDEN
PROCÉDÉ BASÉ SUR PCR SANS SÉQUENÇAGE POUR LA DÉTECTION D'OLIGONUCLÉOTIDES CONJUGUÉS À DES ANTICORPS

(30) Priority: 08.09.2021 US 202163241880 P
(43) Date of publication of application: 17.07.2024
(60) Divisional application: 25196252.8
(73) Proprietor: Becton, Dickinson and Company, Franklin Lakes, NJ 07417-1880 (US)
(72) Inventor: CAMPBELL, Tracy, San Jose, California 95131 (US); PROSEN, Dennis, San Jose, California 95131 (US); SHI, Xiaoshan, San Jose, California 95131 (US)
(74) Representative: Carpmaels & Ransford LLP
(86) International application number: PCT/US2022/076056
(87) International publication number: WO 2023/039433

(56) References cited:
- WO-A1-2020/219721
- WO-A1-2022/256324
- US-A1- 2018 320 241
- US-A1- 2021 214 784
- US-A1- 2021 246 492

## Description

### RELATED APPLICATIONS

This application claims the benefit under 35 U.S.C. §119(e) of U.S. Provisional Patent Application Ser. No. 63/241,880, filed September 8, 2021.

### REFERENCE TO SEQUENCE LISTING

The present application is being filed along with a Sequence Listing in electronic format. The Sequence Listing is provided as a file entitled 68EB-317323-WO, created September 7, 2022, which is 12.0 kilobytes in size. The information in the electronic format of the Sequence Listing is incorporated herein by reference in its entirety.

### BACKGROUND

### Field

The present disclosure relates generally to the field of molecular biology, for example identifying cells of different samples and determining protein expression profiles in cells using molecular barcoding.

### Description of the Related Art

Current technology allows measurement of gene expression of single cells in a massively parallel manner (e.g., >10000 cells) by attaching cell specific oligonucleotide barcodes to poly(A) mRNA molecules from individual cells as each of the cells is co-localized with a barcoded reagent bead in a compartment. Gene expression may affect protein expression. Protein-protein interaction may affect gene expression and protein expression. There is a need for systems and methods that can quantitatively analyze protein expression in cells, and simultaneously measure protein expression and gene expression in cells.

US2018320241 describes methods for identifying multiple epitopes in selected subpopulations of cells. WO2020219721 describes methods and compositions characterizing metastasis. US2021214784 describes methods and compositions for quantitation of proteins and RNA. US2021246492 describes intracellular AbSeq. WO2022256324 describes methods and compositions for analyte detection and probe resolution.

### SUMMARY

The present invention is set out in the appended set of claims. The terminologies "embodiment" and "embodiments" are to be construed as embodiment(s) of the invention only in as far as they fall within the scope of the appended claims. Otherwise, they refer to embodiments of the disclosure only, for reference purposes.

Disclosed herein include methods for measuring cellular component target expression in cells. In some embodiments, the method comprises: contacting a plurality of first cellular component-binding reagents with a first plurality of cells comprising a plurality of cellular component targets, wherein each of the plurality of first cellular component-binding reagents comprises a cellular component-binding reagent specific oligonucleotide comprising a unique identifier sequence for the first cellular component-binding reagent, and wherein the first cellular component-binding reagent is capable of specifically binding to at least one of the plurality of cellular component targets. The method can comprise: generating amplicons of the unique identifier sequence of one or more of the first cellular component-binding reagents. The method can comprise: determining the amount of one or more amplicons as an indication of the amount of the one or more of the first cellular component-binding reagents. The method can comprise: if the amount of one or more amplicons indicates an abundance of a first cellular component-binding reagent above or below a predetermined abundance range, repeating the contacting step with a second plurality of cells in a manner configured to achieve an abundance of said first cellular component-binding reagent within said predetermined abundance range.

In some embodiments, the predetermined abundance range comprises the dynamic range for which acceptable linearity and efficiency of detection of the first cellular component-binding reagent are observed. The method can comprise: contacting the first and/or second plurality of cells with a plurality of third cellular component-binding reagents, wherein a third cellular component-binding reagent is capable of specifically binding to at least one of the plurality of cellular component targets, wherein the third cellular component-binding reagents do not comprise a cellular component-binding reagent specific oligonucleotide, wherein one or more of the first cellular component-binding reagents and one or more of the third cellular component-binding reagents are capable of binding the same cellular component target. In some embodiments, first cellular component-binding reagents and third cellular component-binding reagents capable of binding the same cellular component target have the same clonotype.

In some embodiments, repeating the contacting step with a second plurality of cells in a manner configured to achieve an abundance of said first cellular component-binding reagent within said predetermined abundance range comprises contacting the second plurality of cells with mixture of said first cellular component-binding reagent and a third cellular component-binding reagent capable of binding the same cellular component target as said first cellular component-binding reagent at a titration ratio configured to achieve an abundance of said first cellular component-binding reagent within said predetermined abundance range. In some embodiments, the titration ratio is configured such that sequencing reads comprising the unique identifier sequence of said first cellular component-binding reagent account for less than about 5% of total sequencing reads.

Disclosed herein include methods for measuring cellular component target expression in cells. In some embodiments, the method comprises: contacting a plurality of first cellular component-binding reagents with the first plurality of cells comprising a plurality of cellular component targets, wherein each of the plurality of first cellular component-binding reagents comprises a cellular component-binding reagent specific oligonucleotide comprising a unique identifier sequence for the first cellular component-binding reagent, and wherein the first cellular component-binding reagent is capable of specifically binding to at least one of the plurality of cellular component targets. The method can comprise: isolating one or more cell types of interest from the first plurality of cells, wherein a first cell type of interest is characterized by abundance of: (i) one or more positive cellular component targets above a positive abundance threshold and/or (ii) one or more negative cellular component targets below a negative abundance threshold. The method can comprise: generating amplicons of the unique identifier sequence of one or more of the first cellular component-binding reagents. The method can comprise: determining the amount of one or more amplicons as an indication of the amount of the cellular component targets bound by said one or more of the first cellular component-binding reagents.

In some embodiments, one or more cells suspected of being a first cell type of interest is determined to not be a first cell type of interest if the amount of one or more amplicons indicates an abundance of: (i) one or more positive cellular component targets below a positive abundance threshold and/or (ii) one or more negative cellular component targets above a negative abundance threshold. The method can comprise: if one or more cells suspected of being a first cell type of interest is determined to not be a first cell type of interest, repeating the contacting step and/or isolating step with a second plurality of cells in a manner configured to achieve isolation of the one or more cell types of interest.

The cellular component-binding reagent specific oligonucleotide can comprise a sequence complementary to a capture sequence of an oligonucleotide barcode configured to capture the sequence of the cellular component-binding reagent specific oligonucleotide. In some embodiments, the sequence of the cellular component-binding reagent specific oligonucleotide complementary to the capture sequence comprises a poly(dA) region.

The method can comprise: contacting a plurality of oligonucleotide barcodes with the cellular component-binding reagent specific oligonucleotides for hybridization, wherein the oligonucleotide barcodes each comprise a first molecular label and a first universal sequence; and extending the plurality of oligonucleotide barcodes hybridized to the cellular component-binding reagent specific oligonucleotides to generate a plurality of barcoded cellular component-binding reagent specific oligonucleotides each comprising a sequence complementary to at least a portion of the unique identifier sequence and the first molecular label. The method can comprise: obtaining sequence information of the plurality of barcoded cellular component-binding reagent specific oligonucleotides, or products thereof, to determine the number of copies of at least one cellular component target of the plurality of cellular component targets in one or more of the first plurality of cells and/or second plurality of cells. In some embodiments, obtaining the sequence information comprises attaching sequencing adaptors to the plurality of barcoded cellular component-binding reagent specific oligonucleotides, or products thereof. In some embodiments, the cellular component-binding reagent specific oligonucleotide comprises a second universal sequence.

In some embodiments, generating amplicons comprises: amplifying the plurality of cellular component-binding reagent specific oligonucleotides using a primer capable of hybridizing to the second universal sequence, or a complement thereof, and a primer capable of hybridizing to the capture sequence, or a complement thereof, thereby generating amplicons of the unique identifier sequence of one or more of the first cellular component-binding reagents. In some embodiments, generating amplicons comprises: amplifying the plurality of cellular component-binding reagent specific oligonucleotides using a primer capable of hybridizing to the second universal sequence, or a complement thereof, and a primer capable of hybridizing to the capture sequence, or a complement thereof, thereby generating PCR1 products; and amplifying the PCR1 products using a primer capable of hybridizing to the second universal sequence, or a complement thereof, and a primer capable of hybridizing to the capture sequence, or a complement thereof, thereby generating amplicons of the unique identifier sequence of one or more of the first cellular component-binding reagents.

In some embodiments, generating amplicons comprises: amplifying the plurality of barcoded cellular component-binding reagent specific oligonucleotides using a primer capable of hybridizing to the second universal sequence, or a complement thereof, and a primer capable of hybridizing to the capture sequence, or a complement thereof, thereby generating amplicons of the unique identifier sequence of one or more of the first cellular component-binding reagents. In some embodiments, generating amplicons comprises: amplifying the plurality of barcoded cellular component-binding reagent specific oligonucleotides using a primer capable of hybridizing to the second universal sequence, or a complement thereof, and a primer capable of hybridizing to the capture sequence, or a complement thereof, thereby generating PCR1 products; and amplifying the PCR1 products using a primer capable of hybridizing to the second universal sequence, or a complement thereof, and a primer capable of hybridizing to the capture sequence, or a complement thereof, thereby generating amplicons of the unique identifier sequence of one or more of the first cellular component-binding reagents.

In some embodiments, generating amplicons comprises: amplifying the plurality of barcoded cellular component-binding reagent specific oligonucleotides using a primer capable of hybridizing to the first universal sequence, or a complement thereof, and a primer capable of hybridizing to the second universal sequence, or a complement thereof, thereby generating amplicons of the unique identifier sequence of one or more of the first cellular component-binding reagents. In some embodiments, generating amplicons comprises: amplifying the plurality of barcoded cellular component-binding reagent specific oligonucleotides using a primer capable of hybridizing to the first universal sequence, or a complement thereof, and a primer capable of hybridizing to the second universal sequence, or a complement thereof, thereby generating PCR1 products; and amplifying the PCR1 products using a primer capable of hybridizing to the second universal sequence, or a complement thereof, and a primer capable of hybridizing to the first universal sequence, or a complement thereof, thereby generating amplicons of the unique identifier sequence of one or more of the first cellular component-binding reagents.

The amplification can be carried out using polymerase chain reaction (PCR), ligase chain reaction (LCR), loop-mediated isothermal amplification (LAMP), strand displacement amplification (SDA), replicase-mediated amplification, Immuno-amplification, nucleic acid sequence based amplification (NASBA), self-sustained sequence replication (3SR), rolling circle amplification, and/or transcription-mediated amplification (TMA). In some embodiments, said PCR is real-time PCR. In some embodiments, said PCR is quantitative real-time PCR (QRT-PCR). In some embodiments, said PCR is digital PCR (dPCR), such as, for example, droplet digital PCR (ddPCR). Determining the presence or amount of one or more amplicons can comprise contacting the amplicons with a plurality of oligonucleotide probes, wherein each of the plurality of oligonucleotide probes comprises a sequence configured to bind a unique identifier sequence, or a complement thereof. In some embodiments, each probe is flanked by complementary sequences at the 5' end and 3' end. In some embodiments, one of the complementary sequences comprises a fluorescence emitter moiety and the other complementary sequence comprises a fluorescence quencher moiety. In some embodiments, at least one of the plurality of oligonucleotide probes comprises a fluorescence emitter moiety and a fluorescence quencher moiety. In some embodiments, the oligonucleotide probe comprises a TaqMan detection probe oligonucleotide, a molecular beacon detection probe oligonucleotide, or a molecular torch detection probe oligonucleotide. In some embodiments, the generating amplicons step and determining step are multiplexed. In some embodiments, at least about 4 unique identifier sequences are analyzed simultaneously. In some embodiments, the generating amplicons step and determining step are performed on single cells or multiple cells.

In some embodiments, the isolating step comprises single cell sorting or bulk sorting. In some embodiments, the one or more cell types of interest comprise hemogenic endothelium cells, hematopoietic stem and progenitor cells (HSC), hematopoietic multipotent progenitor cell (MPP), pre-T cell progenitor cells, pre- K cell progenitor cells, T cell progenitor cells, NK cell progenitor cells, T cells, NK cells, NKT cells, B cells, macrophage, neutrophils, CD4 cells, CD8 cells, naive T-cells, memory stem T-cells, central memory T- cells, double negative T-cells, effector memory T-cells, effector T-cells, ThO cells, TcO cells, Thl cells, Tel cells, Th2 cells, Tc2 cells, Thl7 cells, Th22 cells, gamma/delta T-cells, natural killer (NK) cells, natural killer T (NKT) cells, hematopoietic stem cells pluripotent stem cells, or any combination thereof. In some embodiments, of the one or more cell types of interest comprise or more immune cell types (e.g., naive CD4 T cells, effector memory CD4 T cells, naive CD8 T cells, effector memory CD8 T cells, naive CD4 Treg cells, effector memory CD4 Treg cells, naive B cells, memory B cells, CD16 DC, plasmacytoid DC, or any combination thereof).

The method can comprise: contacting a plurality of second cellular component-binding reagents with the first and/or second plurality of cells, wherein each of the plurality of second cellular component-binding reagents comprises a detectable moiety, or a precursor thereof, wherein a second cellular component-binding reagent is capable of specifically binding to at least one of the plurality of cellular component targets, wherein second cellular component-binding reagents capable of binding the same cellular component target comprise the same detectable moiety, or a precursor thereof, and wherein second cellular component-binding reagents capable of binding different cellular component targets comprise different detectable moieties, or precursors thereof. In some embodiments, the first plurality of cells and/or second plurality of cells are contacted with the first cellular component-binding reagents and second cellular component-binding reagents simultaneously. In some embodiments, one or more of the first cellular component-binding reagents and one or more of the second cellular component-binding reagents are capable of binding the same cellular component target. In some embodiments, first cellular component-binding reagents and second cellular component-binding reagents capable of binding the same cellular component target have the same clonotype. The isolating step can comprise fluorescence-activated cell sorting. In some embodiments, fluorescence-activated cell sorting employs the second cellular component-binding reagents. In some embodiments, the isolating step comprises depositing the one or more cells of interest of the first and/or second plurality of cells into one or more microtiter plates. In some embodiments, the isolating step comprises detecting emissions of the detectable moiety of second cellular component-binding reagents with a flow cytometer. In some embodiments, the flow cytometer comprises a conventional flow cytometer, a spectral flow cytometer, a hyperspectral flow cytometer, an imaging flow cytometer, or any combination thereof.

Detectable moiety can comprise an optical moiety, a luminescent moiety, an electrochemically active moiety, a nanoparticle, or a combination thereof. In some embodiments, the nanoparticle comprises a quantum dot. The luminescent moiety can comprise a chemiluminescent moiety, an electroluminescent moiety, a photoluminescent moiety, or a combination thereof. In some embodiments, the photoluminescent moiety comprises a fluorescent moiety, a phosphorescent moiety, or a combination thereof. In some embodiments, the fluorescent moiety comprises a fluorescent dye. The method can comprise: performing a reaction to convert the detectable moiety precursor into the detectable moiety.

In some embodiments, the number of unique first molecular label sequences associated with the unique identifier sequence for the first cellular component-binding reagent capable of specifically binding to the at least one cellular component target in the sequencing data indicates the number of copies of the at least one cellular component target in the one or more of the first and/or second plurality of cells. In some embodiments, the cellular component-binding reagent specific oligonucleotide comprises one or more of: (a) a second molecular label sequence (e.g., 2-20 nucleotides in length); (b) an alignment sequence adjacent to a poly(dA) region, optionally the alignment sequence is one or more nucleotides, or two or more nucleotides, in length; and (c) a linker, wherein the cellular component-binding reagent specific oligonucleotide is associated with the first cellular component-binding reagent through the linker. In some embodiments, the second molecular label sequences of at least two cellular component-binding reagent specific oligonucleotides are different, and wherein the unique identifier sequences of the at least two cellular component-binding reagent specific oligonucleotides are identical. In some embodiments, the second molecular label sequences of at least two cellular component-binding reagent specific oligonucleotides are different, and wherein the unique identifier sequences of the at least two cellular component-binding reagent specific oligonucleotides are different. In some embodiments, the number of unique second molecular label sequences associated with the unique identifier sequence for the first cellular component-binding reagent capable of specifically binding to the at least one cellular component target in the sequencing data indicates the number of copies of the at least one cellular component target in the one or more of the first and/or second plurality of cells. In some embodiments, (a) the alignment sequence comprises a guanine, a cytosine, a thymine, a uracil, or a combination thereof; (b) the alignment sequence comprises a poly(dT) sequence, a poly(dG) sequence, a poly(dC) sequence, a poly(dU) sequence, or a combination thereof; and/or (c) the alignment sequence is 5' to the poly(dA) region. In some embodiments, the linker comprises a carbon chain. In some embodiments, the carbon chain comprises 2-30 carbons (e.g., 12 carbons). The linker can comprise 5' amino modifier C12 (5AmMC12), or a derivative thereof.

In some embodiments, the plurality of cellular component targets comprises a plurality of protein targets, and wherein the first cellular component-binding reagent is capable of specifically binding to at least one of the plurality of protein targets. In some embodiments, the plurality of cellular component targets comprises a cell-surface protein, an intracellular protein, a cell marker, a B-cell receptor, a T-cell receptor, an antibody, a major histocompatibility complex, a tumor antigen, a receptor, or a combination thereof.

In some embodiments, the cellular component-binding reagent specific oligonucleotide is associated with the first cellular component-binding reagent. In some embodiments, the cellular component-binding reagent specific oligonucleotide is covalently attached to the first cellular component-binding reagent and/or non-covalently attached to the first cellular component-binding reagent. In some embodiments, the cellular component-binding reagent specific oligonucleotide is conjugated to the first cellular component-binding reagent. In some embodiments, the cellular component-binding reagent specific oligonucleotide is conjugated to the first cellular component-binding reagent through a chemical group selected from the group consisting of a UV photocleavable group, a streptavidin, a biotin, an amine, and a combination thereof.

The cellular component-binding reagent specific oligonucleotide can be configured to be detachable from the first cellular component-binding reagent. The method can comprise: dissociating the cellular component-binding reagent specific oligonucleotide from the first cellular component-binding reagent. In some embodiments, dissociating the cellular component-binding reagent specific oligonucleotide comprises detaching the cellular component-binding reagent specific oligonucleotide from the first cellular component-binding reagent by UV photocleaving, chemical treatment, heating, enzyme treatment, or any combination thereof. In some embodiments, the dissociating occurs after barcoding the cellular component-binding reagent specific oligonucleotide and/or wherein the dissociating occurs before barcoding the cellular component-binding reagent specific oligonucleotide. In some embodiments, the cellular component-binding reagent specific oligonucleotide is configured to be non-detachable from the first cellular component-binding reagent.

The method can comprise: after contacting the plurality of first cellular component-binding reagents with the first and/or second plurality of cells, removing one or more first cellular component-binding reagents of the plurality of first cellular component-binding reagents that are not contacted with the first and/or second plurality of cells. In some embodiments, removing the one or more first cellular component-binding reagents not contacted with the first and/or second plurality of cells comprises removing the one or more first cellular component-binding reagents not contacted with the respective at least one of the plurality of cellular component targets. In some embodiments, the first and/or second plurality of cells comprises T cells, B cells, tumor cells, myeloid cells, blood cells, normal cells, fetal cells, maternal cells, or a mixture thereof.

In some embodiments, one or more single cells of the first plurality of cells and/or second plurality of cells comprises copies of a nucleic acid target. The method can comprise: contacting a plurality of oligonucleotide barcodes with the copies of the nucleic acid target for hybridization, wherein each oligonucleotide barcode of the plurality of oligonucleotide barcodes comprises a first universal sequence, a target-binding region capable of hybridizing to the copies of the nucleic acid target, and a first molecular label; extending the plurality of oligonucleotide barcodes hybridized to the copies of a nucleic acid target to generate a plurality of barcoded nucleic acid molecules each comprising a sequence complementary to at least a portion of the nucleic acid target; and obtaining sequence information of the plurality of barcoded nucleic acid molecules, or products thereof, to determine the copy number of the nucleic acid target in each of the one or more single cells. In some embodiments, determining the copy number of the nucleic acid target in each of the one or more single cells comprises determining the copy number of the nucleic acid target in each of the one or more single cells based on the number of first molecular labels with distinct sequences, complements thereof, or a combination thereof, associated with the plurality of barcoded nucleic acid molecules, or products thereof. In some embodiments, obtaining sequencing data comprises attaching sequencing adaptors to the plurality of barcoded nucleic acid molecules, or products thereof.

In some embodiments, the plurality of barcoded nucleic acid molecules comprise barcoded deoxyribonucleic acid (DNA) molecules and/or barcoded ribonucleic acid (RNA) molecules. In some embodiments, the nucleic acid target comprises a nucleic acid molecule (e.g., ribonucleic acid (RNA), messenger RNA (mRNA), microRNA, small interfering RNA (siRNA), RNA degradation product, RNA comprising a poly(A) tail, or any combination thereof). In some embodiments, the mRNA encodes an immune receptor).

In some embodiments, at least 10 of the plurality of oligonucleotide barcodes comprise different first molecular label sequences. In some embodiments, each first molecular label of the plurality of oligonucleotide barcodes comprises at least 6 nucleotides. In some embodiments, the plurality of oligonucleotide barcodes are associated with a solid support. In some embodiments, the plurality of oligonucleotide barcodes associated with the same solid support each comprise an identical sample label. In some embodiments, each sample label of the plurality of oligonucleotide barcodes comprises at least 6 nucleotides. In some embodiments, the plurality of oligonucleotide barcodes each comprise a cell label. In some embodiments, each cell label of the plurality of oligonucleotide barcodes comprises at least 6 nucleotides. In some embodiments, oligonucleotide barcodes associated with the same solid support comprise the same cell label. In some embodiments, oligonucleotide barcodes associated with different solid supports comprise different cell labels.

The method can comprise: associating a synthetic particle comprising the plurality of the oligonucleotide barcodes with a single cell in the first and/or second plurality of single cells. The method can comprise: lysing the single cell after associating the synthetic particle with the single cell. In some embodiments, lysing the single cell comprises heating the sample, contacting the sample with a detergent, changing the pH of the sample, or any combination thereof. In some embodiments, the synthetic particle and the single cell are in the same well. In some embodiments, the synthetic particle and the single cell are in the same droplet. In some embodiments, at least one of the plurality of oligonucleotide barcodes is immobilized or partially immobilized on the synthetic particle, or the at least one of the plurality of oligonucleotide barcodes is enclosed or partially enclosed in the synthetic particle. In some embodiments, the synthetic particle is disruptable. In some embodiments, the synthetic particle comprises a bead. In some embodiments, the bead comprises a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, an anti-biotin microbead, an anti-fluorochrome microbead, or any combination thereof. In some embodiments, the bead comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, and any combination thereof. In some embodiments, the bead comprises a disruptable hydrogel particle. In some embodiments, each of the plurality of oligonucleotide barcodes comprises a linker functional group, the synthetic particle comprises a third solid support functional group, and the support functional group and the linker functional group are associated with each other. In some embodiments, the linker functional group and the support functional group are individually selected from the group consisting of C6, biotin, streptavidin, primary amine(s), aldehyde(s), ketone(s), and any combination thereof. In some embodiments, the first plurality of cells and second plurality of cells are derived from the same sample.

Disclosed herein but not part of the invention are kits. In some embodiments, the kit comprises: a plurality of oligonucleotide probes, wherein each of the plurality of oligonucleotide probes comprises a sequence configured to bind a unique identifier sequence, or a complement thereof. The kit can comprise: a plurality of first cellular component-binding reagents, wherein each of the plurality of first cellular component-binding reagents comprises a cellular component-binding reagent specific oligonucleotide comprising a unique identifier sequence for the first cellular component-binding reagent, and wherein the first cellular component-binding reagent is capable of specifically binding to at least one of a plurality of cellular component targets. The kit can comprise: a plurality of second cellular component-binding reagents, wherein each of the plurality of second cellular component-binding reagents comprises a detectable moiety, or a precursor thereof, wherein a second cellular component-binding reagent is capable of specifically binding to at least one of the plurality of cellular component targets, wherein second cellular component-binding reagents capable of binding the same cellular component target comprise the same detectable moiety, or a precursor thereof, and wherein second cellular component-binding reagents capable of binding different cellular component targets comprise different detectable moieties, or precursors thereof. The kit can comprise: a plurality of third cellular component-binding reagents, wherein a third cellular component-binding reagent is capable of specifically binding to at least one of the plurality of cellular component targets, wherein the third cellular component-binding reagents do not comprise a cellular component-binding reagent specific oligonucleotide, wherein one or more of the first cellular component-binding reagents and one or more of the third cellular component-binding reagents are capable of binding the same cellular component target. In some embodiments, first cellular component-binding reagents and third cellular component-binding reagents capable of binding the same cellular component target have the same clonotype.

In some embodiments, one or more of the first cellular component-binding reagents and one or more of the second cellular component-binding reagents are capable of binding the same cellular component target. In some embodiments, first cellular component-binding reagents and second cellular component-binding reagents capable of binding the same cellular component target have the same clonotype In some embodiments, each probe is flanked by complementary sequences at the 5' end and 3' end. In some embodiments, one of the complementary sequences comprises a fluorescence emitter moiety and the other complementary sequence comprises a fluorescence quencher moiety. In some embodiments, at least one of the plurality of oligonucleotide probes comprises a fluorescence emitter moiety and a fluorescence quencher moiety. In some embodiments, the oligonucleotide probe comprises a TaqMan detection probe oligonucleotide, a molecular beacon. The kit can comprise: a primer capable of hybridizing to a first universal sequence, or a complement thereof. The kit can comprise: a primer capable of hybridizing to a second universal sequence, or a complement thereof. The kit can comprise: a primer capable of hybridizing to a capture sequence, or a complement thereof.

### BRIEF DESCRIPTION OF THE DRAWINGS

FIG. 1 illustrates a non-limiting exemplary stochastic barcode.
FIG. 2 shows a non-limiting exemplary workflow of stochastic barcoding and digital counting.
FIG. 3 is a schematic illustration showing a non-limiting exemplary process for generating an indexed library of the stochastically barcoded targets from a plurality of targets.
FIG. 4 shows a schematic illustration of an exemplary protein binding reagent (antibody illustrated here) associated with an oligonucleotide comprising a unique identifier for the protein binding reagent.
FIG. 5 shows a schematic illustration of an exemplary binding reagent (antibody illustrated here) associated with an oligonucleotide comprising a unique identifier for sample indexing to determine cells from the same or different samples.
FIG. 6 shows a schematic illustration of an exemplary workflow of using oligonucleotide-associated antibodies to determine cellular component expression (e.g., protein expression) and gene expression simultaneously in a high throughput manner.
FIG. 7 shows a schematic illustration of an exemplary workflow of using oligonucleotide-associated antibodies for sample indexing.
FIG. 8 shows a schematic illustration of a non-limiting exemplary workflow of barcoding of a binding reagent oligonucleotide (antibody oligonucleotide illustrated here) that is associated with a binding reagent (antibody illustrated here).
FIGS. 9A-9D show non-limiting exemplary designs of oligonucleotides for determining protein expression and gene expression simultaneously and for sample indexing.
FIG. 10 shows a schematic illustration of a non-limiting exemplary oligonucleotide sequence for determining protein expression and gene expression simultaneously and for sample indexing.
FIGS. 11A-11B show non-limiting exemplary designs of oligonucleotides for determining protein expression and gene expression simultaneously and for sample indexing.
FIG. 12 depicts a non-limiting exemplary workflow of AbSeq qPCR in combination with Rhapsody library preparation.
FIG. 13 depicts a non-limiting exemplary qPCR workflow.
FIGS. 14A and 14B depict non-limiting exemplary qPCR workflows.
FIG. 15 depicts a non-limiting exemplary standard curve generated using the disclosed methods and compositions.
FIG. 16 depicts a non-limiting exemplary experimental workflow.
FIG. 17 depicts a non-limiting exemplary PCR plate set-up.
FIG. 18 depicts a non-limiting exemplary standard curve generated using the methods and compositions described herein.
FIGS. 19A-19B depict non-limiting exemplary data generated using the methods and compositions described herein for naive CD4 T cell (FIG. 19A) and effector memory CD4 T cell (FIG. 19B).
FIG. 20 depicts non-limiting exemplary data generated using the methods and compositions described herein.
FIG. 21 depicts non-limiting exemplary data generated using the methods and compositions described herein.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein and made part of the disclosure herein.

Disclosed herein include methods, compositions and kits for measuring cellular component target expression in cells without the use of sequencing. In some embodiments, the method comprises: contacting a plurality of first cellular component-binding reagents with the first plurality of cells comprising a plurality of cellular component targets, wherein each of the plurality of first cellular component-binding reagents comprises a cellular component-binding reagent specific oligonucleotide comprising a unique identifier sequence for the first cellular component-binding reagent, and wherein the first cellular component-binding reagent is capable of specifically binding to at least one of the plurality of cellular component targets. The method can comprise: generating amplicons of the unique identifier sequence of one or more of the first cellular component-binding reagents. The method can comprise: determining the amount of one or more amplicons as an indication of the amount of the cellular component targets bound by said one or more of the first cellular component-binding reagents.

Quantifying small numbers of nucleic acids, for example messenger ribonucleotide acid (mRNA) molecules, is important (e.g., clinically important) for determining, for example, the genes that are expressed in a cell at different stages of development or under different environmental conditions. However, it can also be very challenging to determine the absolute number of nucleic acid molecules (e.g., mRNA molecules), especially when the number of molecules is very small. One method to determine the absolute number of molecules in a sample is digital polymerase chain reaction (PCR). Ideally, PCR produces an identical copy of a molecule at each cycle. However, PCR can have disadvantages such that each molecule replicates with a stochastic probability, and this probability varies by PCR cycle and gene sequence, resulting in amplification bias and inaccurate gene expression measurements. Stochastic barcodes with unique molecular labels (also referred to as molecular indexes (MIs)) can be used to count the number of molecules and correct for amplification bias. Stochastic barcoding such as the Precise^{™} assay (Cellular Research, Inc. San Jose, CA)) can correct for bias induced by PCR and library preparation steps by using molecular labels (MLs) to label mRNAs during reverse transcription (RT).

The Precise^{™} assay can utilize a non-depleting pool of stochastic barcodes with large number, for example 6561 to 65536, unique molecular labels on poly(T) oligonucleotides to hybridize to all poly(A)-mRNAs in a sample during the RT step. A stochastic barcode can comprise a universal PCR priming site. During RT, target gene molecules react randomly with stochastic barcodes. Each target molecule can hybridize to a stochastic barcode resulting to generate stochastically barcoded complementary ribonucleotide acid (cDNA) molecules). After labeling, stochastically barcoded cDNA molecules from microwells of a microwell plate can be pooled into a single tube for PCR amplification and sequencing. Raw sequencing data can be analyzed to produce the number of reads, the number of stochastic barcodes with unique molecular labels, and the numbers of mRNA molecules.

Methods for determining mRNA expression profiles of single cells can be performed in a massively parallel manner. For example, the Precise^{™} assay can be used to determine the mRNA expression profiles of more than 10000 cells simultaneously. The number of single cells (e.g., 100s or 1000s of singles) for analysis per sample can be lower than the capacity of the current single cell technology. Pooling of cells from different samples enables improved utilization of the capacity of the current single technology, thus lowering reagents wasted and the cost of single cell analysis. The disclosure provides methods of sample indexing for distinguishing cells of different samples for cDNA library preparation for cell analysis, such as single cell analysis. Pooling of cells from different samples can minimize the variations in cDNA library preparation of cells of different samples, thus enabling more accurate comparisons of different samples.

Disclosed herein include methods for measuring cellular component target expression in cells. In some embodiments, the method comprises: contacting a plurality of first cellular component-binding reagents with a first plurality of cells comprising a plurality of cellular component targets, wherein each of the plurality of first cellular component-binding reagents comprises a cellular component-binding reagent specific oligonucleotide comprising a unique identifier sequence for the first cellular component-binding reagent, and wherein the first cellular component-binding reagent is capable of specifically binding to at least one of the plurality of cellular component targets. The method can comprise: generating amplicons of the unique identifier sequence of one or more of the first cellular component-binding reagents. The method can comprise: determining the amount of one or more amplicons as an indication of the amount of the one or more of the first cellular component-binding reagents. The method can comprise: if the amount of one or more amplicons indicates an abundance of a first cellular component-binding reagent above or below a predetermined abundance range, repeating the contacting step with a second plurality of cells in a manner configured to achieve an abundance of said first cellular component-binding reagent within said predetermined abundance range.

Disclosed herein include methods for measuring cellular component target expression in cells. In some embodiments, the method comprises: contacting a plurality of first cellular component-binding reagents with the first plurality of cells comprising a plurality of cellular component targets, wherein each of the plurality of first cellular component-binding reagents comprises a cellular component-binding reagent specific oligonucleotide comprising a unique identifier sequence for the first cellular component-binding reagent, and wherein the first cellular component-binding reagent is capable of specifically binding to at least one of the plurality of cellular component targets. The method can comprise: isolating one or more cell types of interest from the first plurality of cells, wherein a first cell type of interest is characterized by abundance of: (i) one or more positive cellular component targets above a positive abundance threshold and/or (ii) one or more negative cellular component targets below a negative abundance threshold. The method can comprise: generating amplicons of the unique identifier sequence of one or more of the first cellular component-binding reagents. The method can comprise: determining the amount of one or more amplicons as an indication of the amount of the cellular component targets bound by said one or more of the first cellular component-binding reagents.

Disclosed herein include kits. In some embodiments, the kit comprises: a plurality of oligonucleotide probes, wherein each of the plurality of oligonucleotide probes comprises a sequence configured to bind a unique identifier sequence, or a complement thereof. The kit can comprise: a plurality of first cellular component-binding reagents, wherein each of the plurality of first cellular component-binding reagents comprises a cellular component-binding reagent specific oligonucleotide comprising a unique identifier sequence for the first cellular component-binding reagent, and wherein the first cellular component-binding reagent is capable of specifically binding to at least one of a plurality of cellular component targets. The kit can comprise: a plurality of second cellular component-binding reagents, wherein each of the plurality of second cellular component-binding reagents comprises a detectable moiety, or a precursor thereof, wherein a second cellular component-binding reagent is capable of specifically binding to at least one of the plurality of cellular component targets, wherein second cellular component-binding reagents capable of binding the same cellular component target comprise the same detectable moiety, or a precursor thereof, and wherein second cellular component-binding reagents capable of binding different cellular component targets comprise different detectable moieties, or precursors thereof. The kit can comprise: a plurality of third cellular component-binding reagents, wherein a third cellular component-binding reagent is capable of specifically binding to at least one of the plurality of cellular component targets, wherein the third cellular component-binding reagents do not comprise a cellular component-binding reagent specific oligonucleotide, wherein one or more of the first cellular component-binding reagents and one or more of the third cellular component-binding reagents are capable of binding the same cellular component target. In some embodiments, first cellular component-binding reagents and third cellular component-binding reagents capable of binding the same cellular component target have the same clonotype.

### Definitions

Unless defined otherwise, technical and scientific terms used herein have the same meaning as commonly understood by one of ordinary skill in the art to which the present disclosure belongs. *See, e.g.,* Singleton et al., Dictionary of Microbiology and Molecular Biology 2nd ed., J. Wiley & Sons (New York, NY 1994); Sambrook et al., Molecular Cloning, A Laboratory Manual, Cold Spring Harbor Press (Cold Spring Harbor, NY 1989). For purposes of the present disclosure, the following terms are defined below.

As used herein, the term "adaptor" can mean a sequence to facilitate amplification or sequencing of associated nucleic acids. The associated nucleic acids can comprise target nucleic acids. The associated nucleic acids can comprise one or more of spatial labels, target labels, sample labels, indexing label, or barcode sequences (e.g., molecular labels). The adapters can be linear. The adaptors can be pre-adenylated adapters. The adaptors can be double- or single-stranded. One or more adaptor can be located on the 5' or 3' end of a nucleic acid. When the adaptors comprise known sequences on the 5' and 3' ends, the known sequences can be the same or different sequences. An adaptor located on the 5' and/or 3' ends of a polynucleotide can be capable of hybridizing to one or more oligonucleotides immobilized on a surface. An adapter can, in some embodiments, comprise a universal sequence. A universal sequence can be a region of nucleotide sequence that is common to two or more nucleic acid molecules. The two or more nucleic acid molecules can also have regions of different sequence. Thus, for example, the 5' adapters can comprise identical and/or universal nucleic acid sequences and the 3' adapters can comprise identical and/or universal sequences. A universal sequence that may be present in different members of a plurality of nucleic acid molecules can allow the replication or amplification of multiple different sequences using a single universal primer that is complementary to the universal sequence. Similarly, at least one, two (e.g., a pair) or more universal sequences that may be present in different members of a collection of nucleic acid molecules can allow the replication or amplification of multiple different sequences using at least one, two (e.g., a pair) or more single universal primers that are complementary to the universal sequences. Thus, a universal primer includes a sequence that can hybridize to such a universal sequence. The target nucleic acid sequence-bearing molecules may be modified to attach universal adapters (e.g., non-target nucleic acid sequences) to one or both ends of the different target nucleic acid sequences. The one or more universal primers attached to the target nucleic acid can provide sites for hybridization of universal primers. The one or more universal primers attached to the target nucleic acid can be the same or different from each other.

As used herein, an antibody can be a full-length (e.g., naturally occurring or formed by normal immunoglobulin gene fragment recombinatorial processes) immunoglobulin molecule (e.g., an IgG antibody) or an immunologically active (i.e., specifically binding) portion of an immunoglobulin molecule, like an antibody fragment.

In some embodiments, an antibody is a functional antibody fragment. For example, an antibody fragment can be a portion of an antibody such as F(ab')2, Fab', Fab, Fv, sFv and the like. An antibody fragment can bind with the same antigen that is recognized by the full-length antibody. An antibody fragment can include isolated fragments consisting of the variable regions of antibodies, such as the "Fv" fragments consisting of the variable regions of the heavy and light chains and recombinant single chain polypeptide molecules in which light and heavy variable regions are connected by a peptide linker ("scFv proteins"). Exemplary antibodies can include, but are not limited to, antibodies for cancer cells, antibodies for viruses, antibodies that bind to cell surface receptors (e.g., CD8, CD34, and CD45), and therapeutic antibodies.

As used herein the term "associated" or "associated with" can mean that two or more species are identifiable as being co-located at a point in time. An association can mean that two or more species are or were within a similar container. An association can be an informatics association. For example, digital information regarding two or more species can be stored and can be used to determine that one or more of the species were co-located at a point in time. An association can also be a physical association. In some embodiments, two or more associated species are "tethered", "attached", or "immobilized" to one another or to a common solid or semisolid surface. An association may refer to covalent or non-covalent means for attaching labels to solid or semi-solid supports such as beads. An association may be a covalent bond between a target and a label. An association can comprise hybridization between two molecules (such as a target molecule and a label).

As used herein, the term "complementary" can refer to the capacity for precise pairing between two nucleotides. For example, if a nucleotide at a given position of a nucleic acid is capable of hydrogen bonding with a nucleotide of another nucleic acid, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. A first nucleotide sequence can be said to be the "complement" of a second sequence if the first nucleotide sequence is complementary to the second nucleotide sequence. A first nucleotide sequence can be said to be the "reverse complement" of a second sequence, if the first nucleotide sequence is complementary to a sequence that is the reverse (i.e., the order of the nucleotides is reversed) of the second sequence. As used herein, the terms "complement", "complementary", and "reverse complement" are used interchangeably. It is understood from the disclosure that if a molecule can hybridize to another molecule it may be the complement of the molecule that is hybridizing.

As used herein, the term "digital counting" can refer to a method for estimating a number of target molecules in a sample. Digital counting can include the step of determining a number of unique labels that have been associated with targets in a sample. This methodology, which can be stochastic in nature, transforms the problem of counting molecules from one of locating and identifying identical molecules to a series of yes/no digital questions regarding detection of a set of predefined labels.

As used herein, the term "label" or "labels" can refer to nucleic acid codes associated with a target within a sample. A label can be, for example, a nucleic acid label. A label can be an entirely or partially amplifiable label. A label can be entirely or partially sequencable label. A label can be a portion of a native nucleic acid that is identifiable as distinct. A label can be a known sequence. A label can comprise a junction of nucleic acid sequences, for example a junction of a native and non-native sequence. As used herein, the term "label" can be used interchangeably with the terms, "index", "tag," or "label-tag." Labels can convey information. For example, in various embodiments, labels can be used to determine an identity of a sample, a source of a sample, an identity of a cell, and/or a target.

As used herein, the term "non-depleting reservoirs" can refer to a pool of barcodes (e.g., stochastic barcodes) made up of many different labels. A non-depleting reservoir can comprise large numbers of different barcodes such that when the non-depleting reservoir is associated with a pool of targets each target is likely to be associated with a unique barcode. The uniqueness of each labeled target molecule can be determined by the statistics of random choice, and depends on the number of copies of identical target molecules in the collection compared to the diversity of labels. The size of the resulting set of labeled target molecules can be determined by the stochastic nature of the barcoding process, and analysis of the number of barcodes detected then allows calculation of the number of target molecules present in the original collection or sample. When the ratio of the number of copies of a target molecule present to the number of unique barcodes is low, the labeled target molecules are highly unique (i.e., there is a very low probability that more than one target molecule will have been labeled with a given label).

As used herein, the term "nucleic acid" refers to a polynucleotide sequence, or fragment thereof. A nucleic acid can comprise nucleotides. A nucleic acid can be exogenous or endogenous to a cell. A nucleic acid can exist in a cell-free environment. A nucleic acid can be a gene or fragment thereof. A nucleic acid can be DNA. A nucleic acid can be RNA. A nucleic acid can comprise one or more analogs (e.g., altered backbone, sugar, or nucleobase). Some non-limiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, fluorophores (e.g., rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudouridine, dihydrouridine, queuosine, and wyosine. "Nucleic acid", "polynucleotide, "target polynucleotide", and "target nucleic acid" can be used interchangeably.

A nucleic acid can comprise one or more modifications (e.g., a base modification, a backbone modification), to provide the nucleic acid with a new or enhanced feature (e.g., improved stability). A nucleic acid can comprise a nucleic acid affinity tag. A nucleoside can be a base-sugar combination. The base portion of the nucleoside can be a heterocyclic base. The two most common classes of such heterocyclic bases are the purines and the pyrimidines. Nucleotides can be nucleosides that further include a phosphate group covalently linked to the sugar portion of the nucleoside. For those nucleosides that include a pentofuranosyl sugar, the phosphate group can be linked to the 2', the 3', or the 5' hydroxyl moiety of the sugar. In forming nucleic acids, the phosphate groups can covalently link adjacent nucleosides to one another to form a linear polymeric compound. In turn, the respective ends of this linear polymeric compound can be further joined to form a circular compound; however, linear compounds are generally suitable. Linear compounds can have internal nucleotide base complementarity and may therefore fold in a manner as to produce a fully or partially double-stranded compound. Within nucleic acids, the phosphate groups can commonly be referred to as forming the internucleoside backbone of the nucleic acid. The linkage or backbone can be a 3' to 5' phosphodiester linkage.

A nucleic acid can comprise a modified backbone and/or modified internucleoside linkages. Modified backbones can include those that retain a phosphorus atom in the backbone and those that do not have a phosphorus atom in the backbone. Suitable modified nucleic acid backbones containing a phosphorus atom therein can include, for example, phosphorothioates, chiral phosphorothioates, phosphorodithioates, phosphotriesters, aminoalkyl phosphotriesters, methyl and other alkyl phosphonate such as 3'-alkylene phosphonates, 5'-alkylene phosphonates, chiral phosphonates, phosphinates, phosphoramidates including 3'-amino phosphoramidate and aminoalkyl phosphoramidates, phosphorodiamidates, thionophosphoramidates, thionoalkylphosphonates, thionoalkylphosphotriesters, selenophosphates, and boranophosphates having normal 3'-5' linkages, 2'-5' linked analogs, and those having inverted polarity wherein one or more internucleotide linkages is a 3' to 3', a 5' to 5' or a 2' to 2' linkage.

A nucleic acid can comprise polynucleotide backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These can include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH₂ component parts.

A nucleic acid can comprise a nucleic acid mimetic. The term "mimetic" can be intended to include polynucleotides wherein only the furanose ring or both the furanose ring and the internucleotide linkage are replaced with non-furanose groups, replacement of only the furanose ring can also be referred as being a sugar surrogate. The heterocyclic base moiety or a modified heterocyclic base moiety can be maintained for hybridization with an appropriate target nucleic acid. One such nucleic acid can be a peptide nucleic acid (PNA). In a PNA, the sugar-backbone of a polynucleotide can be replaced with an amide containing backbone, in particular an aminoethylglycine backbone. The nucleotides can be retained and are bound directly or indirectly to aza nitrogen atoms of the amide portion of the backbone. The backbone in PNA compounds can comprise two or more linked aminoethylglycine units which gives PNA an amide containing backbone. The heterocyclic base moieties can be bound directly or indirectly to aza nitrogen atoms of the amide portion of the backbone.

A nucleic acid can comprise a morpholino backbone structure. For example, a nucleic acid can comprise a 6-membered morpholino ring in place of a ribose ring. In some of these embodiments, a phosphorodiamidate or other non-phosphodiester internucleoside linkage can replace a phosphodiester linkage.

A nucleic acid can comprise linked morpholino units (e.g., morpholino nucleic acid) having heterocyclic bases attached to the morpholino ring. Linking groups can link the morpholino monomeric units in a morpholino nucleic acid. Non-ionic morpholino-based oligomeric compounds can have less undesired interactions with cellular proteins. Morpholino-based polynucleotides can be nonionic mimics of nucleic acids. A variety of compounds within the morpholino class can be joined using different linking groups. A further class of polynucleotide mimetic can be referred to as cyclohexenyl nucleic acids (CeNA). The furanose ring normally present in a nucleic acid molecule can be replaced with a cyclohexenyl ring. CeNA DMT protected phosphoramidite monomers can be prepared and used for oligomeric compound synthesis using phosphoramidite chemistry. The incorporation of CeNA monomers into a nucleic acid chain can increase the stability of a DNA/RNA hybrid. CeNA oligoadenylates can form complexes with nucleic acid complements with similar stability to the native complexes. A further modification can include Locked Nucleic Acids (LNAs) in which the 2'-hydroxyl group is linked to the 4' carbon atom of the sugar ring thereby forming a 2'-C, 4'-C-oxymethylene linkage thereby forming a bicyclic sugar moiety. The linkage can be a methylene (-CH₂), group bridging the 2' oxygen atom and the 4' carbon atom wherein n is 1 or 2. LNA and LNA analogs can display very high duplex thermal stabilities with complementary nucleic acid (Tm=+3 to +10 °C), stability towards 3'-exonucleolytic degradation and good solubility properties.

A nucleic acid can also include nucleobase (also referred to as "base") modifications or substitutions. As used herein, "unmodified" or "natural" nucleobases can include the purine bases, (e.g., adenine (A) and guanine (G)), and the pyrimidine bases, (e.g., thymine (T), cytosine (C) and uracil (U)). Modified nucleobases can include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Modified nucleobases can include tricyclic pyrimidines such as phenoxazine cytidine(1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), carbazole cytidine (2H-pyrimido(4,5-b)indol-2-one), pyridoindole cytidine (H-pyrido(3',2':4,5)pyrrolo[2,3-d]pyrimidin-2-one).

As used herein, the term "sample" can refer to a composition comprising targets. Suitable samples for analysis by the disclosed methods, devices, and systems include cells, tissues, organs, or organisms.

As used herein, the term "sampling device" or "device" can refer to a device which may take a section of a sample and/or place the section on a substrate. A sample device can refer to, for example, a fluorescence activated cell sorting (FACS) machine, a cell sorter machine, a biopsy needle, a biopsy device, a tissue sectioning device, a microfluidic device, a blade grid, and/or a microtome.

As used herein, the term "solid support" can refer to discrete solid or semi-solid surfaces to which a plurality of barcodes (e.g., stochastic barcodes) may be attached. A solid support may encompass any type of solid, porous, or hollow sphere, ball, bearing, cylinder, or other similar configuration composed of plastic, ceramic, metal, or polymeric material (e.g., hydrogel) onto which a nucleic acid may be immobilized (e.g., covalently or non-covalently). A solid support may comprise a discrete particle that may be spherical (e.g., microspheres) or have a non-spherical or irregular shape, such as cubic, cuboid, pyramidal, cylindrical, conical, oblong, or disc-shaped, and the like. A bead can be non-spherical in shape. A plurality of solid supports spaced in an array may not comprise a substrate. A solid support may be used interchangeably with the term "bead."

As used herein, the term "stochastic barcode" can refer to a polynucleotide sequence comprising labels of the present disclosure. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "gene-specific stochastic barcode" can refer to a polynucleotide sequence comprising labels and a target-binding region that is gene-specific. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "stochastic barcoding" can refer to the random labeling (e.g., barcoding) of nucleic acids. Stochastic barcoding can utilize a recursive Poisson strategy to associate and quantify labels associated with targets. As used herein, the term "stochastic barcoding" can be used interchangeably with "stochastic labeling."

As used here, the term "target" can refer to a composition which can be associated with a barcode (e.g., a stochastic barcode). Exemplary suitable targets for analysis by the disclosed methods, devices, and systems include oligonucleotides, DNA, RNA, mRNA, microRNA, tRNA, and the like. Targets can be single or double stranded. In some embodiments, targets can be proteins, peptides, or polypeptides. In some embodiments, targets are lipids. As used herein, "target" can be used interchangeably with "species."

As used herein, the term "reverse transcriptases" can refer to a group of enzymes having reverse transcriptase activity (i.e., that catalyze synthesis of DNA from an RNA template). In general, such enzymes include, but are not limited to, retroviral reverse transcriptase, retrotransposon reverse transcriptase, retroplasmid reverse transcriptases, retron reverse transcriptases, bacterial reverse transcriptases, group II intron-derived reverse transcriptase, and mutants, variants or derivatives thereof. Non-retroviral reverse transcriptases include non-LTR retrotransposon reverse transcriptases, retroplasmid reverse transcriptases, retron reverse transcriptases, and group II intron reverse transcriptases. Examples of group II intron reverse transcriptases include the *Lactococcus lactis* LI.LtrB intron reverse transcriptase, the *Thermosynechococcus* elongatus TeI4c intron reverse transcriptase, or the *Geobacillus stearothermophilus* GsI-IIC intron reverse transcriptase. Other classes of reverse transcriptases can include many classes of non-retroviral reverse transcriptases (i.e., retrons, group II introns, and diversity-generating retroelements among others).

The terms "universal adaptor primer," "universal primer adaptor" or "universal adaptor sequence" are used interchangeably to refer to a nucleotide sequence that can be used to hybridize to barcodes (e.g., stochastic barcodes) to generate gene-specific barcodes. A universal adaptor sequence can, for example, be a known sequence that is universal across all barcodes used in methods of the disclosure. For example, when multiple targets are being labeled using the methods disclosed herein, each of the target-specific sequences may be linked to the same universal adaptor sequence. In some embodiments, more than one universal adaptor sequences may be used in the methods disclosed herein. For example, when multiple targets are being labeled using the methods disclosed herein, at least two of the target-specific sequences are linked to different universal adaptor sequences. A universal adaptor primer and its complement may be included in two oligonucleotides, one of which comprises a target-specific sequence and the other comprises a barcode. For example, a universal adaptor sequence may be part of an oligonucleotide comprising a target-specific sequence to generate a nucleotide sequence that is complementary to a target nucleic acid. A second oligonucleotide comprising a barcode and a complementary sequence of the universal adaptor sequence may hybridize with the nucleotide sequence and generate a target-specific barcode (e.g., a target-specific stochastic barcode). In some embodiments, a universal adaptor primer has a sequence that is different from a universal PCR primer used in the methods of this disclosure.

### Barcodes

Barcoding, such as stochastic barcoding, has been described in, for example, Fu et al., Proc Natl Acad Sci U.S.A., 2011 May 31,108(22):9026-31; US2011/0160078; Fan et al., Science, 2015, 347(6222):1258367; US2015/0299784; and WO2015/031691. In some embodiments, the barcode disclosed herein can be a stochastic barcode which can be a polynucleotide sequence that may be used to stochastically label (e.g., barcode, tag) a target. Barcodes can be referred to stochastic barcodes if the ratio of the number of different barcode sequences of the stochastic barcodes and the number of occurrence of any of the targets to be labeled can be, or be about, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences. Barcodes can be referred to as stochastic barcodes if the ratio of the number of different barcode sequences of the stochastic barcodes and the number of occurrence of any of the targets to be labeled is at least, or is at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1. Barcode sequences of stochastic barcodes can be referred to as molecular labels.

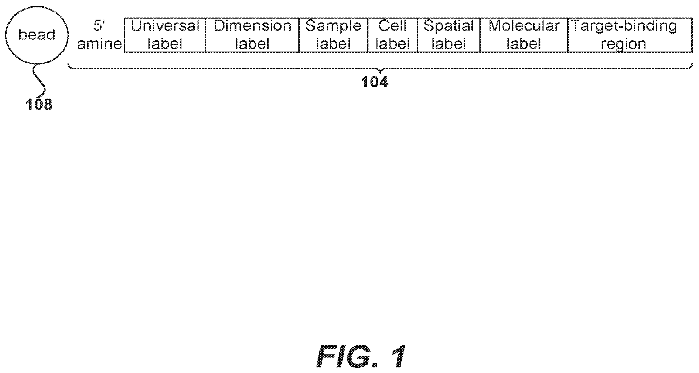

A barcode, for example a stochastic barcode, can comprise one or more labels. Exemplary labels can include a universal label, a cell label, a barcode sequence (e.g., a molecular label), a sample label, a plate label, a spatial label, and/or a pre-spatial label. FIG. 1 illustrates an exemplary barcode 104 with a spatial label. The barcode 104 can comprise a 5'amine that may link the barcode to a solid support 108. The barcode can comprise a universal label, a dimension label, a spatial label, a cell label, and/or a molecular label. The order of different labels (including but not limited to the universal label, the dimension label, the spatial label, the cell label, and the molecule label) in the barcode can vary. For example, as shown in FIG. 1, the universal label may be the 5'-most label, and the molecular label may be the 3'-most label. The spatial label, dimension label, and the cell label can be in any order. In some embodiments, the universal label, the spatial label, the dimension label, the cell label, and the molecular label are in any order. The barcode can comprise a target-binding region. The target-binding region can interact with a target (e.g., target nucleic acid, RNA, mRNA, DNA) in a sample. For example, a target-binding region can comprise an oligo(dT) sequence which can interact with poly(A) tails of mRNAs. In some embodiments, the labels of the barcode (e.g., universal label, dimension label, spatial label, cell label, and barcode sequence) are separated by 1, 2, 3, 4, 5, 6, 7, 8, 9, 10, 11, 12, 13, 14, 15, 16, 17, 18, 19, 20, or more nucleotides.

A label, for example the cell label, can comprise a unique set of nucleic acid sub-sequences of defined length, e.g., seven nucleotides each (equivalent to the number of bits used in some Hamming error correction codes), which can be designed to provide error correction capability. The set of error correction sub-sequences comprise seven nucleotide sequences can be designed such that any pairwise combination of sequences in the set exhibits a defined "genetic distance" (or number of mismatched bases), for example, a set of error correction sub-sequences can be designed to exhibit a genetic distance of three nucleotides. In this case, review of the error correction sequences in the set of sequence data for labeled target nucleic acid molecules (described more fully below) can allow one to detect or correct amplification or sequencing errors. In some embodiments, the length of the nucleic acid sub-sequences used for creating error correction codes can vary, for example, they can be, or be about 1, 2, 3, 4, 5, 6, 7, 8, 9, 10, 15, 20, 30, 31, 40, 50, or a number or a range between any two of these values, nucleotides in length. In some embodiments, nucleic acid sub-sequences of other lengths can be used for creating error correction codes.

The barcode can comprise a target-binding region. The target-binding region can interact with a target in a sample. The target can be, or comprise, ribonucleic acids (RNAs), messenger RNAs (mRNAs), microRNAs, small interfering RNAs (siRNAs), RNA degradation products, RNAs each comprising a poly(A) tail, or any combination thereof. In some embodiments, the plurality of targets can include deoxyribonucleic acids (DNAs).

In some embodiments, a target-binding region can comprise an oligo(dT) sequence which can interact with poly(A) tails of mRNAs. One or more of the labels of the barcode (e.g., the universal label, the dimension label, the spatial label, the cell label, and the barcode sequences (e.g., molecular label)) can be separated by a spacer from another one or two of the remaining labels of the barcode. The spacer can be, for example, 1, 2, 3, 4, 5, 6, 7, 8, 9, 10, 11, 12, 13, 14, 15, 16, 17, 18, 19, or 20, or more nucleotides. In some embodiments, none of the labels of the barcode is separated by spacer.

### Universal Labels

A barcode can comprise one or more universal labels. In some embodiments, the one or more universal labels can be the same for all barcodes in the set of barcodes attached to a given solid support. In some embodiments, the one or more universal labels can be the same for all barcodes attached to a plurality of beads. In some embodiments, a universal label can comprise a nucleic acid sequence that is capable of hybridizing to a sequencing primer. Sequencing primers can be used for sequencing barcodes comprising a universal label. Sequencing primers (e.g., universal sequencing primers) can comprise sequencing primers associated with high-throughput sequencing platforms. In some embodiments, a universal label can comprise a nucleic acid sequence that is capable of hybridizing to a PCR primer. In some embodiments, the universal label can comprise a nucleic acid sequence that is capable of hybridizing to a sequencing primer and a PCR primer. The nucleic acid sequence of the universal label that is capable of hybridizing to a sequencing or PCR primer can be referred to as a primer binding site. A universal label can comprise a sequence that can be used to initiate transcription of the barcode. A universal label can comprise a sequence that can be used for extension of the barcode or a region within the barcode. A universal label can be, be about, be at least, or be at most, 1, 2, 3, 4, 5, 10, 15, 20, 25, 30, 35, 40, 45, 50, 100, 200, 300, or a number or a range between any two of these values, nucleotides in length. In some embodiments, a cleavable linker or modified nucleotide can be part of the universal label sequence to enable the barcode to be cleaved off from the support.

### Dimension Labels

A barcode can comprise one or more dimension labels. In some embodiments, a dimension label can comprise a nucleic acid sequence that provides information about a dimension in which the labeling (e.g., stochastic labeling) occurred. For example, a dimension label can provide information about the time at which a target was barcoded. A dimension label can be associated with a time of barcoding (e.g., stochastic barcoding) in a sample. A dimension label can be activated at the time of labeling. Different dimension labels can be activated at different times. The dimension label provides information about the order in which targets, groups of targets, and/or samples were barcoded. For example, a population of cells can be barcoded at the G0 phase of the cell cycle. The cells can be pulsed again with barcodes (e.g., stochastic barcodes) at the G1 phase of the cell cycle. The cells can be pulsed again with barcodes at the S phase of the cell cycle, and so on. Barcodes at each pulse (e.g., each phase of the cell cycle), can comprise different dimension labels. In this way, the dimension label provides information about which targets were labelled at which phase of the cell cycle. Dimension labels can interrogate many different biological times. Exemplary biological times can include, but are not limited to, the cell cycle, transcription (e.g., transcription initiation), and transcript degradation. In another example, a sample (e.g., a cell, a population of cells) can be labeled before and/or after treatment with a drug and/or therapy. The changes in the number of copies of distinct targets can be indicative of the sample's response to the drug and/or therapy.

A dimension label can be activatable. An activatable dimension label can be activated at a specific time point. The activatable label can be, for example, constitutively activated (e.g., not turned off). The activatable dimension label can be, for example, reversibly activated (e.g., the activatable dimension label can be turned on and turned off). The dimension label can be, for example, reversibly activatable at least 1, 2, 3, 4, 5, 6, 7, 8, 9, 10, or more times. In some embodiments, the dimension label can be activated with fluorescence, light, a chemical event (e.g., cleavage, ligation of another molecule, addition of modifications (e.g., pegylated, sumoylated, acetylated, methylated, deacetylated, demethylated), a photochemical event (e.g., photocaging), and introduction of a non-natural nucleotide.

The dimension label can, in some embodiments, be identical for all barcodes (e.g., stochastic barcodes) attached to a given solid support (e.g., a bead), but different for different solid supports (e.g., beads). In some embodiments, at least 60%, 70%, 80%, 85%, 90%, 95%, 97%, 99% or 100%, of barcodes on the same solid support can comprise the same dimension label.

There can be as many as 10⁶ or more unique dimension label sequences represented in a plurality of solid supports (e.g., beads). A dimension label can be, be about, be at least, or be at most, 1, 2, 3, 4, 5, 10, 15, 20, 25, 30, 35, 40, 45, 50, 75, 100, 125, 150, 200, or a number or a range between any two of these values, nucleotides in length.

### Spatial Labels

A barcode can comprise one or more spatial labels. In some embodiments, a spatial label can comprise a nucleic acid sequence that provides information about the spatial orientation of a target molecule which is associated with the barcode. A spatial label can be associated with a coordinate in a sample. The coordinate can be a fixed coordinate. For example, a coordinate can be fixed in reference to a substrate. A spatial label can be in reference to a two or three-dimensional grid. A coordinate can be fixed in reference to a landmark. The landmark can be identifiable in space. A landmark can be a structure which can be imaged. A landmark can be a biological structure, for example an anatomical landmark. A landmark can be a cellular landmark, for instance an organelle. A landmark can be a non-natural landmark such as a structure with an identifiable identifier such as a color code, bar code, magnetic property, fluorescents, radioactivity, or a unique size or shape. A spatial label can be associated with a physical partition (e.g., a well, a container, or a droplet). In some embodiments, multiple spatial labels are used together to encode one or more positions in space.

The spatial label can be identical for all barcodes attached to a given solid support (e.g., a bead), but different for different solid supports (e.g., beads). In some embodiments, the percentage of barcodes on the same solid support comprising the same spatial label can be, be about, be at least, or be at most, 60%, 70%, 80%, 85%, 90%, 95%, 97%, 99%, 100%, or a number or a range between any two of these values.

There can be as many as 10⁶ or more unique spatial label sequences represented in a plurality of solid supports (e.g., beads). A spatial label can be, be about, at least or at most, 1, 2, 3, 4, 5, 10, 15, 20, 25, 30, 35, 40, 45, 50, 100, 125, 150, 175, 200, 250, or 300 or a number or a range between any two of these values, nucleotides in length.

### Cell labels

A barcode (e.g., a stochastic barcode) can comprise one or more cell labels. In some embodiments, a cell label can comprise a nucleic acid sequence that provides information for determining which target nucleic acid originated from which cell. In some embodiments, the cell label is identical for all barcodes attached to a given solid support (e.g., a bead), but different for different solid supports (e.g., beads). In some embodiments, the percentage of barcodes on the same solid support comprising the same cell label can be, be about, at most, or be at least, 60%, 70%, 80%, 85%, 90%, 95%, 97%, 99%, 100%, or a number or a range between any two of these values.

There can be as many as 10⁶ or more unique cell label sequences represented in a plurality of solid supports (e.g., beads). A cell label can be, be about, at least, or be at most, 1, 2, 3, 4, 5, 10, 15, 20, 25, 30, 35, 40, 45, 50, 100, 125, 150, 175, 200, or a number or a range between any two of these values, nucleotides in length.

### Barcode Sequences

A barcode can comprise one or more barcode sequences. In some embodiments, a barcode sequence can comprise a nucleic acid sequence that provides identifying information for the specific type of target nucleic acid species hybridized to the barcode. A barcode sequence can comprise a nucleic acid sequence that provides a counter (e.g., that provides a rough approximation) for the specific occurrence of the target nucleic acid species hybridized to the barcode (e.g., target-binding region).

In some embodiments, a diverse set of barcode sequences are attached to a given solid support (e.g., a bead). In some embodiments, there can be, or be about, 10², 10³, 10⁴, 10⁵, 10⁶, 10⁷, 10⁸, 10⁹, or a number or a range between any two of these values, unique molecular label sequences. For example, a plurality of barcodes can comprise about 6561 barcodes sequences with distinct sequences. As another example, a plurality of barcodes can comprise about 65536 barcode sequences with distinct sequences. In some embodiments, there can be at least, or be at most, 10², 10³, 10⁴, 10⁵, 10⁶, 10⁷, 10⁸, or 10⁹, unique barcode sequences. The unique molecular label sequences can be attached to a given solid support (e.g., a bead).

The length of a barcode can be different in different implementations. For example, a barcode can be, be about, be at least, or be at most, 1, 2, 3, 4, 5, 10, 15, 20, 25, 30, 35, 40, 45, 50, or a number or a range between any two of these values, nucleotides in length.

### Molecular Labels

A barcode (e.g., a stochastic barcode) can comprise one or more molecular labels. Molecular labels can include barcode sequences. In some embodiments, a molecular label can comprise a nucleic acid sequence that provides identifying information for the specific type of target nucleic acid species hybridized to the barcode. A molecular label can comprise a nucleic acid sequence that provides a counter for the specific occurrence of the target nucleic acid species hybridized to the barcode (e.g., target-binding region).

In some embodiments, a diverse set of molecular labels are attached to a given solid support (e.g., a bead). In some embodiments, there can be, or be about, 10², 10³, 10⁴, 10⁵, 10⁶, 10⁷, 10⁸, 10⁹, or a number or a range between any two of these values, of unique molecular label sequences. For example, a plurality of barcodes can comprise about 6561 molecular labels with distinct sequences. As another example, a plurality of barcodes can comprise about 65536 molecular labels with distinct sequences. In some embodiments, there can be at least, or be at most, 10², 10³, 10⁴, 10⁵, 10⁶, 10⁷, 10⁸, or 10⁹, unique molecular label sequences. Barcodes with unique molecular label sequences can be attached to a given solid support (e.g., a bead).

For stochastic barcoding using a plurality of stochastic barcodes, the ratio of the number of different molecular label sequences and the number of occurrence of any of the targets can be, be about, at least, or is at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences.

A molecular label can be, be about, be at least, or be at most, 1, 2, 3, 4, 5, 10, 15, 20, 25, 30, 35, 40, 45, 50, or a number or a range between any two of these values, nucleotides in length.

### Target-Binding Region

A barcode can comprise one or more target binding regions, such as capture probes. In some embodiments, a target-binding region can hybridize with a target of interest. In some embodiments, the target binding regions can comprise a nucleic acid sequence that hybridizes specifically to a target (e.g., a target nucleic acid, target molecule, cellular nucleic acid to be analyzed), for example to a specific gene sequence. In some embodiments, a target binding region can comprise a nucleic acid sequence that can attach (e.g., hybridize) to a specific location of a specific target nucleic acid. In some embodiments, the target binding region can comprise a nucleic acid sequence that is capable of specific hybridization to a restriction enzyme site overhang (e.g., an EcoRI sticky-end overhang). The barcode can then ligate to any nucleic acid molecule comprising a sequence complementary to the restriction site overhang.

In some embodiments, a target binding region can comprise a non-specific target nucleic acid sequence. A non-specific target nucleic acid sequence can refer to a sequence that can bind to multiple target nucleic acids, independent of the specific sequence of the target nucleic acid. For example, target binding region can comprise a random multimer sequence, or an oligo(dT) sequence that hybridizes to the poly(A) tail on mRNA molecules. A random multimer sequence can be, for example, a random dimer, trimer, quatramer, pentamer, hexamer, septamer, octamer, nonamer, decamer, or higher multimer sequence of any length. In some embodiments, the target binding region is the same for all barcodes attached to a given bead. In some embodiments, the target binding regions for the plurality of barcodes attached to a given bead can comprise two or more different target binding sequences. A target binding region can be, be about, be at least about, or be at most about, 5, 10, 15, 20, 25, 30, 35, 40, 45, 50, or a number or a range between any two of these values, nucleotides in length.

In some embodiments, a target-binding region can comprise an oligo(dT) which can hybridize with mRNAs comprising polyadenylated ends. A target-binding region can be gene-specific. For example, a target-binding region can be configured to hybridize to a specific region of a target. A target-binding region can be, be about, be at least, or be at most, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these values, nucleotides in length. When a barcode comprises a gene-specific target-binding region, the barcode can be referred to herein as a gene-specific barcode.

### Universal Adaptor Primer

A barcode can comprise one or more universal adaptor primers. For example, a gene-specific barcode, such as a gene-specific stochastic barcode, can comprise a universal adaptor primer. A universal adaptor primer can refer to a nucleotide sequence that is universal across all barcodes. A universal adaptor primer can be used for building gene-specific barcodes. A universal adaptor primer can be, be about, at least, or be at most, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these nucleotides in length. A universal adaptor primer can be from 5-30 nucleotides in length.

### Linker

When a barcode comprises more than one of a type of label *(e.g.,* more than one cell label or more than one barcode sequence, such as one molecular label), the labels may be interspersed with a linker label sequence. A linker label sequence can be, be about, be at least about, or be at most about, 5, 10, 11, 12, 13, 14, 15, 16, 17, 18, 19, 20, 25, 30, 35, 40, 45, 50 or more nucleotides in length. A linker label sequence can be used to facilitate the synthesis of the barcode. The linker label can comprise an error-correcting (e.g., Hamming) code.

### Solid Supports

Barcodes, such as stochastic barcodes, disclosed herein can, in some embodiments, be associated with a solid support. The solid support can be, for example, a synthetic particle. In some embodiments, some or all of the barcode sequences, such as molecular labels for stochastic barcodes (e.g., the first barcode sequences) of a plurality of barcodes (e.g., the first plurality of barcodes) on a solid support differ by at least one nucleotide. The cell labels of the barcodes on the same solid support can be the same. The cell labels of the barcodes on different solid supports can differ by at least one nucleotide. For example, first cell labels of a first plurality of barcodes on a first solid support can have the same sequence, and second cell labels of a second plurality of barcodes on a second solid support can have the same sequence. The first cell labels of the first plurality of barcodes on the first solid support and the second cell labels of the second plurality of barcodes on the second solid support can differ by at least one nucleotide. A cell label can be, for example, about 5-20 nucleotides long. A barcode sequence can be, for example, about 5-20 nucleotides long. The synthetic particle can be, for example, a bead.

The bead can be, for example, a silica gel bead, a controlled pore glass bead, a magnetic bead, a Dynabead, a Sephadex/Sepharose bead, a cellulose bead, a polystyrene bead, or any combination thereof. The bead can comprise a material such as polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, or any combination thereof.

In some embodiments, the bead can be a polymeric bead, for example a deformable bead or a gel bead, functionalized with barcodes or stochastic barcodes (such as gel beads from 10X Genomics (San Francisco, CA). In some implementation, a gel bead can comprise a polymer based gels. Gel beads can be generated, for example, by encapsulating one or more polymeric precursors into droplets. Upon exposure of the polymeric precursors to an accelerator (e.g., tetramethylethylenediamine (TEMED)), a gel bead may be generated.

In some embodiments, the particle can be degradable. For example, the polymeric bead can dissolve, melt, or degrade, for example, under a desired condition. The desired condition can include an environmental condition. The desired condition may result in the polymeric bead dissolving, melting, or degrading in a controlled manner. A gel bead may dissolve, melt, or degrade due to a chemical stimulus, a physical stimulus, a biological stimulus, a thermal stimulus, a magnetic stimulus, an electric stimulus, a light stimulus, or any combination thereof.

Analytes and/or reagents, such as oligonucleotide barcodes, for example, may be coupled/immobilized to the interior surface of a gel bead (e.g., the interior accessible via diffusion of an oligonucleotide barcode and/or materials used to generate an oligonucleotide barcode) and/or the outer surface of a gel bead or any other microcapsule described herein. Coupling/immobilization may be via any form of chemical bonding (e.g., covalent bond, ionic bond) or physical phenomena (e.g., Van der Waals forces, dipole-dipole interactions, etc.). In some embodiments, coupling/immobilization of a reagent to a gel bead or any other microcapsule described herein may be reversible, such as, for example, via a labile moiety (e.g., via a chemical cross-linker, including chemical cross-linkers described herein). Upon application of a stimulus, the labile moiety may be cleaved and the immobilized reagent set free. In some embodiments, the labile moiety is a disulfide bond. For example, in the case where an oligonucleotide barcode is immobilized to a gel bead via a disulfide bond, exposure of the disulfide bond to a reducing agent can cleave the disulfide bond and free the oligonucleotide barcode from the bead. The labile moiety may be included as part of a gel bead or microcapsule, as part of a chemical linker that links a reagent or analyte to a gel bead or microcapsule, and/or as part of a reagent or analyte. In some embodiments, at least one barcode of the plurality of barcodes can be immobilized on the particle, partially immobilized on the particle, enclosed in the particle, partially enclosed in the particle, or any combination thereof.

In some embodiments, a gel bead can comprise a wide range of different polymers including but not limited to: polymers, heat sensitive polymers, photosensitive polymers, magnetic polymers, pH sensitive polymers, salt-sensitive polymers, chemically sensitive polymers, polyelectrolytes, polysaccharides, peptides, proteins, and/or plastics. Polymers may include but are not limited to materials such as poly(N-isopropylacrylamide) (PNIPAAm), poly(styrene sulfonate) (PSS), poly(allyl amine) (PAAm), poly(acrylic acid) (PAA), poly(ethylene imine) (PEI), poly(diallyldimethyl-ammonium chloride) (PDADMAC), poly(pyrolle) (PPy), poly(vinylpyrrolidone) (PVPON), poly(vinyl pyridine) (PVP), poly(methacrylic acid) (PMAA), poly(methyl methacrylate) (PMMA), polystyrene (PS), poly(tetrahydrofuran) (PTHF), poly(phthaladehyde) (PTHF), poly(hexyl viologen) (PHV), poly(L-lysine) (PLL), poly(L-arginine) (PARG), poly(lactic-co-glycolic acid) (PLGA).

Numerous chemical stimuli can be used to trigger the disruption, dissolution, or degradation of the beads. Examples of these chemical changes may include, but are not limited to pH-mediated changes to the bead wall, disintegration of the bead wall via chemical cleavage of crosslink bonds, triggered depolymerization of the bead wall, and bead wall switching reactions. Bulk changes may also be used to trigger disruption of the beads.

Bulk or physical changes to the microcapsule through various stimuli also offer many advantages in designing capsules to release reagents. Bulk or physical changes occur on a macroscopic scale, in which bead rupture is the result of mechano-physical forces induced by a stimulus. These processes may include, but are not limited to pressure induced rupture, bead wall melting, or changes in the porosity of the bead wall.

Biological stimuli may also be used to trigger disruption, dissolution, or degradation of beads. Generally, biological triggers resemble chemical triggers, but many examples use biomolecules, or molecules commonly found in living systems such as enzymes, peptides, saccharides, fatty acids, nucleic acids and the like. For example, beads may comprise polymers with peptide cross-links that are sensitive to cleavage by specific proteases. More specifically, one example may comprise a microcapsule comprising GFLGK peptide cross links. Upon addition of a biological trigger such as the protease Cathepsin B, the peptide cross links of the shell well are cleaved and the contents of the beads are released. In other cases, the proteases may be heat-activated. In another example, beads comprise a shell wall comprising cellulose. Addition of the hydrolytic enzyme chitosan serves as biologic trigger for cleavage of cellulosic bonds, depolymerization of the shell wall, and release of its inner contents.

The beads may also be induced to release their contents upon the application of a thermal stimulus. A change in temperature can cause a variety changes to the beads. A change in heat may cause melting of a bead such that the bead wall disintegrates. In other cases, the heat may increase the internal pressure of the inner components of the bead such that the bead ruptures or explodes. In still other cases, the heat may transform the bead into a shrunken dehydrated state. The heat may also act upon heat-sensitive polymers within the wall of a bead to cause disruption of the bead.

Inclusion of magnetic nanoparticles to the bead wall of microcapsules may allow triggered rupture of the beads as well as guide the beads in an array. A device of this disclosure may comprise magnetic beads for either purpose. For example, incorporation of Fe₃O₄ nanoparticles into polyelectrolyte containing beads triggers rupture in the presence of an oscillating magnetic field stimulus.

A bead may also be disrupted, dissolved, or degraded as the result of electrical stimulation. Similar to magnetic particles described in the previous section, electrically sensitive beads can allow for both triggered rupture of the beads as well as other functions such as alignment in an electric field, electrical conductivity or redox reactions. In one example, beads containing electrically sensitive material are aligned in an electric field such that release of inner reagents can be controlled. In other examples, electrical fields may induce redox reactions within the bead wall itself that may increase porosity.

A light stimulus may also be used to disrupt the beads. Numerous light triggers are possible and may include systems that use various molecules such as nanoparticles and chromophores capable of absorbing photons of specific ranges of wavelengths. For example, metal oxide coatings can be used as capsule triggers. UV irradiation of polyelectrolyte capsules coated with SiO₂ may result in disintegration of the bead wall. In yet another example, photo switchable materials such as azobenzene groups may be incorporated in the bead wall. Upon the application of UV or visible light, chemicals such as these undergo a reversible cis-to-trans isomerization upon absorption of photons. In this aspect, incorporation of photon switches can result in a bead wall that may disintegrate or become more porous upon the application of a light trigger.

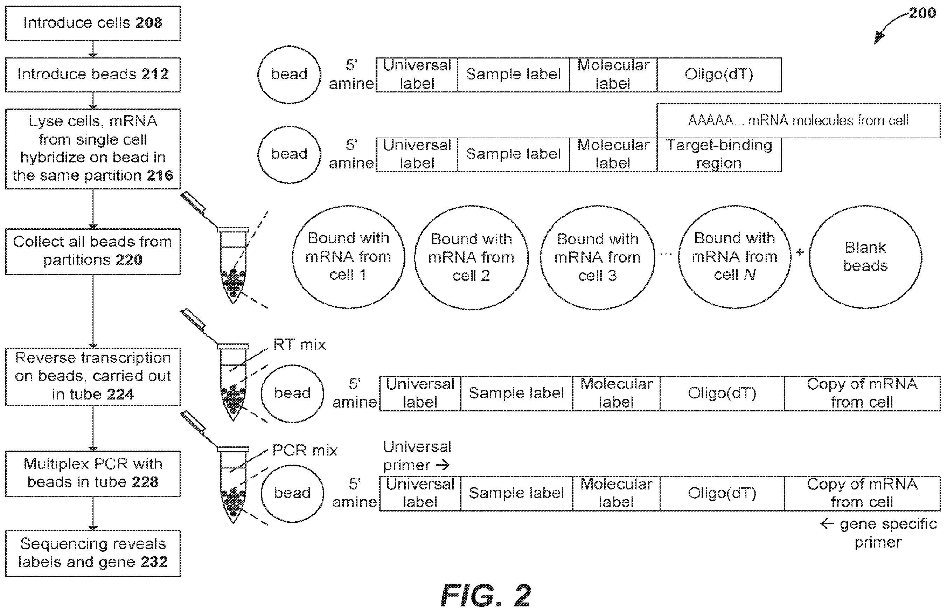

For example, in a non-limiting example of barcoding (e.g., stochastic barcoding) illustrated in FIG. 2, after introducing cells such as single cells onto a plurality of microwells of a microwell array at block 208, beads can be introduced onto the plurality of microwells of the microwell array at block 212. Each microwell can comprise one bead. The beads can comprise a plurality of barcodes. A barcode can comprise a 5' amine region attached to a bead. The barcode can comprise a universal label, a barcode sequence (e.g., a molecular label), a target-binding region, or any combination thereof.

The barcodes disclosed herein can be associated with (e.g., attached to) a solid support (e.g., a bead). The barcodes associated with a solid support can each comprise a barcode sequence selected from a group comprising at least 100 or 1000 barcode sequences with unique sequences. In some embodiments, different barcodes associated with a solid support can comprise barcode with different sequences. In some embodiments, a percentage of barcodes associated with a solid support comprises the same cell label. For example, the percentage can be, or be about 60%, 70%, 80%, 85%, 90%, 95%, 97%, 99%, 100%, or a number or a range between any two of these values. As another example, the percentage can be at least, or be at most 60%, 70%, 80%, 85%, 90%, 95%, 97%, 99%, or 100%. In some embodiments, barcodes associated with a solid support can have the same cell label. The barcodes associated with different solid supports can have different cell labels selected from a group comprising at least 100 or 1000 cell labels with unique sequences.

The barcodes disclosed herein can be associated to (e.g., attached to) a solid support (e.g., a bead). In some embodiments, barcoding the plurality of targets in the sample can be performed with a solid support including a plurality of synthetic particles associated with the plurality of barcodes. In some embodiments, the solid support can include a plurality of synthetic particles associated with the plurality of barcodes. The spatial labels of the plurality of barcodes on different solid supports can differ by at least one nucleotide. The solid support can, for example, include the plurality of barcodes in two dimensions or three dimensions. The synthetic particles can be beads. The beads can be silica gel beads, controlled pore glass beads, magnetic beads, Dynabeads, Sephadex/Sepharose beads, cellulose beads, polystyrene beads, or any combination thereof. The solid support can include a polymer, a matrix, a hydrogel, a needle array device, an antibody, or any combination thereof. In some embodiments, the solid supports can be free floating. In some embodiments, the solid supports can be embedded in a semi-solid or solid array. The barcodes may not be associated with solid supports. The barcodes can be individual nucleotides. The barcodes can be associated with a substrate.

As used herein, the terms "tethered," "attached," and "immobilized," are used interchangeably, and can refer to covalent or non-covalent means for attaching barcodes to a solid support. Any of a variety of different solid supports can be used as solid supports for attaching pre-synthesized barcodes or for *in situ* solid-phase synthesis of barcode.

The solid support can be a bead. The bead can comprise one or more types of solid, porous, or hollow sphere, ball, bearing, cylinder, or other similar configuration which a nucleic acid can be immobilized (e.g., covalently or non-covalently). The bead can be, for example, composed of plastic, ceramic, metal, polymeric material, or any combination thereof. A bead can be, or comprise, a discrete particle that is spherical (e.g., microspheres) or have a non-spherical or irregular shape, such as cubic, cuboid, pyramidal, cylindrical, conical, oblong, or disc-shaped, and the like. In some embodiments, a bead can be non-spherical in shape.

Beads can comprise a variety of materials including, but not limited to, paramagnetic materials (e.g., magnesium, molybdenum, lithium, and tantalum), superparamagnetic materials (e.g., ferrite (Fe₃O₄; magnetite) nanoparticles), ferromagnetic materials (e.g., iron, nickel, cobalt, some alloys thereof, and some rare earth metal compounds), ceramic, plastic, glass, polystyrene, silica, methylstyrene, acrylic polymers, titanium, latex, Sepharose, agarose, hydrogel, polymer, cellulose, nylon, or any combination thereof. In some embodiments, the bead (e.g., the bead to which the labels are attached) is a hydrogel bead. In some embodiments, the bead comprises hydrogel.

Some embodiments disclosed herein include one or more particles (e.g., beads). Each of the particles can comprise a plurality of oligonucleotides (e.g., barcodes). Each of the plurality of oligonucleotides can comprise a barcode sequence (e.g., a molecular label sequence), a cell label, and a target-binding region (e.g., an oligo(dT) sequence, a gene-specific sequence, a random multimer, or a combination thereof). The cell label sequence of each of the plurality of oligonucleotides can be the same. The cell label sequences of oligonucleotides on different particles can be different such that the oligonucleotides on different particles can be identified. The number of different cell label sequences can be different in different implementations. In some embodiments, the number of cell label sequences can be, be about, be at least, or be at most, 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸ 10⁹, a number or a range between any two of these values, or more. In some embodiments, no more than 1, 2, 3, 4, 5, 6, 7, 8, 9, 10, 20, 30, 40, 50, 60, 70, 80, 90, 100, 200, 300, 400, 500, 600, 700, 800, 900, 1000, or more of the plurality of the particles include oligonucleotides with the same cell sequence. In some embodiment, the plurality of particles that include oligonucleotides with the same cell sequence can be at most 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, or more. In some embodiments, none of the plurality of the particles has the same cell label sequence.

The plurality of oligonucleotides on each particle can comprise different barcode sequences (e.g., molecular labels). In some embodiments, the number of barcode sequences can be, be about, be at least, or be at most 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, 10⁹, or a number or a range between any two of these values. For example, at least 100 of the plurality of oligonucleotides comprise different barcode sequences. As another example, in a single particle, at least 100, 500, 1000, 5000, 10000, 15000, 20000, 50000, a number or a range between any two of these values, or more of the plurality of oligonucleotides comprise different barcode sequences. Some embodiments provide a plurality of the particles comprising barcodes. In some embodiments, the ratio of an occurrence (or a copy or a number) of a target to be labeled and the different barcode sequences can be at least 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:30, 1:40, 1:50, 1:60, 1:70, 1:80, 1:90, or more. In some embodiments, each of the plurality of oligonucleotides further comprises a sample label, a universal label, or both. The particle can be, for example, a nanoparticle or microparticle.

The size of the beads can vary. For example, the diameter of the bead can range from 0.1 micrometer to 50 micrometers. In some embodiments, the diameter of the bead can be, or be about, 0.1, 0.5, 1, 2, 3, 4, 5, 6, 7, 8, 9, 10, 20, 30, 40, or 50 micrometers, or a number or a range between any two of these values.

The diameter of the bead can be related to the diameter of the wells of the substrate. In some embodiments, the diameter of the bead can be, or be about, 10%, 20%, 30%, 40%, 50%, 60%, 70%, 80%, 90%, 100%, or a number or a range between any two of these values, longer or shorter than the diameter of the well. The diameter of the beads can be related to the diameter of a cell (e.g., a single cell entrapped by a well of the substrate). In some embodiments, the diameter of the bead can be at least, or be at most, 10%, 20%, 30%, 40%, 50%, 60%, 70%, 80%, 90%, or 100% longer or shorter than the diameter of the well. The diameter of the beads can be related to the diameter of a cell (e.g., a single cell entrapped by a well of the substrate). In some embodiments, the diameter of the bead can be, be about, be at least, or be at most, 10%, 20%, 30%, 40%, 50%, 60%, 70%, 80%, 90%, 100%, 150%, 200%, 250%, 300%, or a number or a range between any two of these values, longer or shorter than the diameter of the cell.

A bead can be attached to and/or embedded in a substrate. A bead can be attached to and/or embedded in a gel, hydrogel, polymer and/or matrix. The spatial position of a bead within a substrate (e.g., gel, matrix, scaffold, or polymer) can be identified using the spatial label present on the barcode on the bead which can serve as a location address.

Examples of beads can include, but are not limited to, streptavidin beads, agarose beads, magnetic beads, Dynabeads^{®}, MACS^{®} microbeads, antibody conjugated beads (e.g., anti-immunoglobulin microbeads), protein A conjugated beads, protein G conjugated beads, protein A/G conjugated beads, protein L conjugated beads, oligo(dT) conjugated beads, silica beads, silica-like beads, anti-biotin microbeads, anti-fluorochrome microbeads, and BcMag^{™} Carboxyl-Terminated Magnetic Beads.

A bead can be associated with (e.g., impregnated with) quantum dots or fluorescent dyes to make it fluorescent in one fluorescence optical channel or multiple optical channels. A bead can be associated with iron oxide or chromium oxide to make it paramagnetic or ferromagnetic. Beads can be identifiable. For example, a bead can be imaged using a camera. A bead can have a detectable code associated with the bead. For example, a bead can comprise a barcode. A bead can change size, for example, due to swelling in an organic or inorganic solution. A bead can be hydrophobic. A bead can be hydrophilic. A bead can be biocompatible.

A solid support (e.g., a bead) can be visualized. The solid support can comprise a visualizing tag (e.g., fluorescent dye). A solid support (e.g., a bead) can be etched with an identifier (e.g., a number). The identifier can be visualized through imaging the beads.

A solid support can comprise an insoluble, semi-soluble, or insoluble material. A solid support can be referred to as "functionalized" when it includes a linker, a scaffold, a building block, or other reactive moiety attached thereto, whereas a solid support may be "nonfunctionalized" when it lacks such a reactive moiety attached thereto. The solid support can be employed free in solution, such as in a microtiter well format; in a flow-through format, such as in a column; or in a dipstick.

The solid support can comprise a membrane, paper, plastic, coated surface, flat surface, glass, slide, chip, or any combination thereof. A solid support can take the form of resins, gels, microspheres, or other geometric configurations. A solid support can comprise silica chips, microparticles, nanoparticles, plates, arrays, capillaries, flat supports such as glass fiber filters, glass surfaces, metal surfaces (steel, gold silver, aluminum, silicon and copper), glass supports, plastic supports, silicon supports, chips, filters, membranes, microwell plates, slides, plastic materials including multiwell plates or membranes (e.g., formed of polyethylene, polypropylene, polyamide, polyvinylidenedifluoride), and/or wafers, combs, pins or needles (e.g., arrays of pins suitable for combinatorial synthesis or analysis) or beads in an array of pits or nanoliter wells of flat surfaces such as wafers (e.g., silicon wafers), wafers with pits with or without filter bottoms.

The solid support can comprise a polymer matrix (e.g., gel, hydrogel). The polymer matrix may be able to permeate intracellular space (e.g., around organelles). The polymer matrix may able to be pumped throughout the circulatory system.

As used herein, a substrate can refer to a type of solid support. A substrate can refer to a solid support that can comprise barcodes or stochastic barcodes of the disclosure. A substrate can, for example, comprise a plurality of microwells. For example, a substrate can be a well array comprising two or more microwells. In some embodiments, a microwell can comprise a small reaction chamber of defined volume. In some embodiments, a microwell can entrap one or more cells. In some embodiments, a microwell can entrap only one cell. In some embodiments, a microwell can entrap one or more solid supports. In some embodiments, a microwell can entrap only one solid support. In some embodiments, a microwell entraps a single cell and a single solid support (e.g., a bead). A microwell can comprise barcode reagents of the disclosure.

### Methods of Barcoding

The disclosure provides for methods for estimating the number of distinct targets at distinct locations in a physical sample (e.g., tissue, organ, tumor, cell). The methods can comprise placing barcodes (e.g., stochastic barcodes) in close proximity with the sample, lysing the sample, associating distinct targets with the barcodes, amplifying the targets and/or digitally counting the targets. The method can further comprise analyzing and/or visualizing the information obtained from the spatial labels on the barcodes. In some embodiments, a method comprises visualizing the plurality of targets in the sample. Mapping the plurality of targets onto the map of the sample can include generating a two dimensional map or a three dimensional map of the sample. The two dimensional map and the three dimensional map can be generated prior to or after barcoding (e.g., stochastically barcoding) the plurality of targets in the sample. Visualizing the plurality of targets in the sample can include mapping the plurality of targets onto a map of the sample. Mapping the plurality of targets onto the map of the sample can include generating a two dimensional map or a three dimensional map of the sample. The two dimensional map and the three dimensional map can be generated prior to or after barcoding the plurality of targets in the sample. in some embodiments, the two dimensional map and the three dimensional map can be generated before or after lysing the sample. Lysing the sample before or after generating the two dimensional map or the three dimensional map can include heating the sample, contacting the sample with a detergent, changing the pH of the sample, or any combination thereof.

Barcoding the plurality of targets can comprise hybridizing a plurality of barcodes with a plurality of targets to create barcoded targets (e.g., stochastically barcoded targets). Barcoding the plurality of targets can comprise generating an indexed library of the barcoded targets. Generating an indexed library of the barcoded targets can be performed with a solid support comprising the plurality of barcodes (e.g., stochastic barcodes).

### Contacting a Sample and a Barcode

The disclosure provides for methods for contacting a sample (e.g., cells) to a substrate of the disclosure. A sample comprising, for example, a cell, organ, or tissue thin section, can be contacted to barcodes (e.g., stochastic barcodes). The cells can be contacted, for example, by gravity flow wherein the cells can settle and create a monolayer. The sample can be a tissue thin section. The thin section can be placed on the substrate. The sample can be one-dimensional (e.g., forms a planar surface). The sample (e.g., cells) can be spread across the substrate, for example, by growing/culturing the cells on the substrate.

When barcodes are in close proximity to targets, the targets can hybridize to the barcode. The barcodes can be contacted at a non-depletable ratio such that each distinct target can associate with a distinct barcode of the disclosure. To ensure efficient association between the target and the barcode, the targets can be cross-linked to barcode.

### Cell Lysis

Following the distribution of cells and barcodes, the cells can be lysed to liberate the target molecules. Cell lysis can be accomplished by any of a variety of means, for example, by chemical or biochemical means, by osmotic shock, or by means of thermal lysis, mechanical lysis, or optical lysis. Cells can be lysed by addition of a cell lysis buffer comprising a detergent (e.g., SDS, Li dodecyl sulfate, Triton X-100, Tween-20, or NP-40), an organic solvent (e.g., methanol or acetone), or digestive enzymes (e.g., proteinase K, pepsin, or trypsin), or any combination thereof. To increase the association of a target and a barcode, the rate of the diffusion of the target molecules can be altered by for example, reducing the temperature and/or increasing the viscosity of the lysate.

In some embodiments, the sample can be lysed using a filter paper. The filter paper can be soaked with a lysis buffer on top of the filter paper. The filter paper can be applied to the sample with pressure which can facilitate lysis of the sample and hybridization of the targets of the sample to the substrate.

In some embodiments, lysis can be performed by mechanical lysis, heat lysis, optical lysis, and/or chemical lysis. Chemical lysis can include the use of digestive enzymes such as proteinase K, pepsin, and trypsin. Lysis can be performed by the addition of a lysis buffer to the substrate. A lysis buffer can comprise Tris HCl. A lysis buffer can comprise at least about, or at most about, 0.01, 0.05, 0.1, 0.5, or 1 M or more Tris HCL. A lysis buffer can comprise about 0.1 M Tris HCl. The pH of the lysis buffer can be, be about, be at least about, or be at most about 1, 2, 3, 4, 5, 5.5, 6, 6.5, 7, 7.5, 8, 8.5, 9, 9.5, 10, or a number or a range between any two of these values. The lysis buffer can comprise a salt (e.g., LiCl). The concentration of salt in the lysis buffer can be at least about 0.1, 0.5, or 1 M or more. The concentration of salt in the lysis buffer can be at most about 0.1, 0.5, or 1 M or more. In some embodiments, the concentration of salt in the lysis buffer is about 0.5M. The lysis buffer can comprise a detergent (e.g., SDS, Li dodecyl sulfate, triton X, tween, NP-40). The concentration of the detergent in the lysis buffer can be at least about, or at most about, 0.0001%, 0.0005%, 0.001%, 0.005%, 0.01%, 0.05%, 0.1%, 0.5%, 1%, 2%, 3%, 4%, 5%, 6%, or 7%, or more. In some embodiments, the concentration of the detergent in the lysis buffer is about 1% Li dodecyl sulfate. The time used in the method for lysis can be dependent on the amount of detergent used. In some embodiments, the more detergent used, the less time needed for lysis. The lysis buffer can comprise a chelating agent (e.g., EDTA, EGTA). The concentration of a chelating agent in the lysis buffer can be at least about 1, 5, 10, 15, 20, 25, or 30 mM or more. The concentration of a chelating agent in the lysis buffer can be at most about 1, 5, 10, 15, 20, 25, or 30mM or more. In some embodiments, the concentration of chelating agent in the lysis buffer is about 10 mM. The lysis buffer can comprise a reducing reagent (e.g., beta-mercaptoethanol, DTT). The concentration of the reducing reagent in the lysis buffer can be at least about, at most about, 1, 5, 10, 15, or 20 mM or more. In some embodiments, a lysis buffer can comprise about 0.1M TrisHCl, about pH 7.5, about 0.5M LiCl, about 1% lithium dodecyl sulfate, about 10mM EDTA, and about 5mM DTT.

Lysis can be performed at a temperature of about 4, 10, 15, 20, 25, or 30 °C. Lysis can be performed for about 1, 5, 10, 15, or 20 or more minutes. A lysed cell can comprise at least about, or at most about, 100000, 200000, 300000, 400000, 500000, 600000, or 700000 or more target nucleic acid molecules.

### Attachment of Barcodes to Target Nucleic Acid Molecules

Following lysis of the cells and release of nucleic acid molecules therefrom, the nucleic acid molecules can randomly associate with the barcodes of the co-localized solid support. Association can comprise hybridization of a barcode's target recognition region to a complementary portion of the target nucleic acid molecule (e.g., oligo(dT) of the barcode can interact with a poly(A) tail of a target). The assay conditions used for hybridization (e.g., buffer pH, ionic strength, temperature) can be chosen to promote formation of specific, stable hybrids. In some embodiments, the nucleic acid molecules released from the lysed cells can associate with the plurality of probes on the substrate (e.g., hybridize with the probes on the substrate). When the probes comprise oligo(dT), mRNA molecules can hybridize to the probes and be reverse transcribed. The oligo(dT) portion of the oligonucleotide can act as a primer for first strand synthesis of the cDNA molecule. For example, in a non-limiting example of barcoding illustrated in FIG. 2, at block 216, mRNA molecules can hybridize to barcodes on beads. For example, single-stranded nucleotide fragments can hybridize to the target-binding regions of barcodes.

Attachment can further comprise ligation of a barcode's target recognition region and a portion of the target nucleic acid molecule. For example, the target binding region can comprise a nucleic acid sequence that can be capable of specific hybridization to a restriction site overhang (e.g., an EcoRI sticky-end overhang). The assay procedure can further comprise treating the target nucleic acids with a restriction enzyme (e.g., EcoRI) to create a restriction site overhang. The barcode can then be ligated to any nucleic acid molecule comprising a sequence complementary to the restriction site overhang. A ligase (e.g., T4 DNA ligase) can be used to join the two fragments.

For example, in a non-limiting example of barcoding illustrated in FIG. 2, at block 220, the labeled targets from a plurality of cells (or a plurality of samples) (e.g., target-barcode molecules) can be subsequently pooled, for example, into a tube. The labeled targets can be pooled by, for example, retrieving the barcodes and/or the beads to which the target-barcode molecules are attached.

The retrieval of solid support-based collections of attached target-barcode molecules can be implemented by use of magnetic beads and an externally-applied magnetic field. Once the target-barcode molecules have been pooled, all further processing can proceed in a single reaction vessel. Further processing can include, for example, reverse transcription reactions, amplification reactions, cleavage reactions, dissociation reactions, and/or nucleic acid extension reactions. Further processing reactions can be performed within the microwells, that is, without first pooling the labeled target nucleic acid molecules from a plurality of cells.

### Reverse Transcription

The disclosure provides for a method to create a target-barcode conjugate using reverse transcription (e.g., at block 224 of FIG. 2). The target-barcode conjugate can comprise the barcode and a complementary sequence of all or a portion of the target nucleic acid (i.e., a barcoded cDNA molecule, such as a stochastically barcoded cDNA molecule). Reverse transcription of the associated RNA molecule can occur by the addition of a reverse transcription primer along with the reverse transcriptase. The reverse transcription primer can be an oligo(dT) primer, a random hexanucleotide primer, or a target-specific oligonucleotide primer. Oligo(dT) primers can be, or can be about, 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

In some embodiments, reverse transcription of the labeled-RNA molecule can occur by the addition of a reverse transcription primer. In some embodiments, the reverse transcription primer is an oligo(dT) primer, random hexanucleotide primer, or a target-specific oligonucleotide primer. Generally, oligo(dT) primers are 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

Reverse transcription can occur repeatedly to produce multiple labeled-cDNA molecules. The methods disclosed herein can comprise conducting at least about, or at most about, 1, 2, 3, 4, 5, 6, 7, 8, 9, 10, 11, 12, 13, 14, 15, 16, 17, 18, 19, or 20 reverse transcription reactions.

### Amplification

One or more nucleic acid amplification reactions (e.g., at block 228 of FIG. 2) can be performed to create multiple copies of the labeled target nucleic acid molecules. Amplification can be performed in a multiplexed manner, wherein multiple target nucleic acid sequences are amplified simultaneously. The amplification reaction can be used to add sequencing adaptors to the nucleic acid molecules. The amplification reactions can comprise amplifying at least a portion of a sample label, if present. The amplification reactions can comprise amplifying at least a portion of the cellular label and/or barcode sequence (e.g., a molecular label). The amplification reactions can comprise amplifying at least a portion of a sample tag, a cell label, a spatial label, a barcode sequence (e.g., a molecular label), a target nucleic acid, or a combination thereof. The amplification reactions can comprise amplifying 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 100%, or a range or a number between any two of these values, of the plurality of nucleic acids. The method can further comprise conducting one or more cDNA synthesis reactions to produce one or more cDNA copies of target-barcode molecules comprising a sample label, a cell label, a spatial label, and/or a barcode sequence (e.g., a molecular label).

In some embodiments, amplification can be performed using a polymerase chain reaction (PCR). As used herein, PCR can refer to a reaction for the in vitro amplification of specific DNA sequences by the simultaneous primer extension of complementary strands of DNA. As used herein, PCR can encompass derivative forms of the reaction, including but not limited to, RT-PCR, real-time PCR, nested PCR, quantitative PCR, multiplexed PCR, digital PCR, and assembly PCR.

Amplification of the labeled nucleic acids can comprise non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), and a Qβ replicase (Qβ) method, use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and ramification extension amplification (RAM). In some embodiments, the amplification does not produce circularized transcripts.

In some embodiments, the methods disclosed herein further comprise conducting a polymerase chain reaction on the labeled nucleic acid (e.g., labeled-RNA, labeled-DNA, labeled-cDNA) to produce a labeled amplicon (e.g., a stochastically labeled amplicon). The labeled amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample label, a spatial label, a cell label, and/or a barcode sequence (e.g., a molecular label). The labeled amplicon can be a single-stranded molecule. The single-stranded molecule can comprise DNA, RNA, or a combination thereof. The nucleic acids of the disclosure can comprise synthetic or altered nucleic acids.

Amplification can comprise use of one or more non-natural nucleotides. Non-natural nucleotides can comprise photolabile or triggerable nucleotides. Examples of non-natural nucleotides can include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise, for example, 1, 2, 3, 4, 5, 6, 7, 8, 9, 10, 11, 12, 13, 14, or 15 or more nucleotides. The one or more primers can comprise at least 1, 2, 3, 4, 5, 6, 7, 8, 9, 10, 11, 12, 13, 14, or 15 or more nucleotides. The one or more primers can anneal to at least a portion of the plurality of labeled targets (e.g., stochastically labeled targets). The one or more primers can anneal to the 3' end or 5' end of the plurality of labeled targets. The one or more primers can anneal to an internal region of the plurality of labeled targets. The internal region can be at least about, or at most about, 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled targets. The one or more primers can comprise a fixed panel of primers. The one or more primers can comprise at least one or more custom primers. The one or more primers can comprise at least one or more control primers. The one or more primers can comprise at least one or more gene-specific primers.

The one or more primers can comprise a universal primer. The universal primer can anneal to a universal primer binding site. The one or more custom primers can anneal to a first sample label, a second sample label, a spatial label, a cell label, a barcode sequence (e.g., a molecular label), a target, or any combination thereof. The one or more primers can comprise a universal primer and a custom primer. The custom primer can be designed to amplify one or more targets. The targets can comprise a subset of the total nucleic acids in one or more samples. The targets can comprise a subset of the total labeled targets in one or more samples. The one or more primers can comprise at least 96 or more custom primers, at least 960 or more custom primers, or at least 9600 or more custom primers. The one or more custom primers can anneal to two or more different labeled nucleic acids. The two or more different labeled nucleic acids can correspond to one or more genes.

Any amplification scheme can be used in the methods of the present disclosure. For example, in one scheme, the first round PCR can amplify molecules attached to the bead using a gene specific primer and a primer against the universal Illumina sequencing primer 1 sequence. The second round of PCR can amplify the first PCR products using a nested gene specific primer flanked by Illumina sequencing primer 2 sequence, and a primer against the universal Illumina sequencing primer 1 sequence. The third round of PCR adds P5 and P7 and sample index to turn PCR products into an Illumina sequencing library. Sequencing using 150 bp x 2 sequencing can reveal the cell label and barcode sequence (e.g., molecular label) on read 1, the gene on read 2, and the sample index on index 1 read.

In some embodiments, nucleic acids can be removed from the substrate using chemical cleavage. For example, a chemical group or a modified base present in a nucleic acid can be used to facilitate its removal from a solid support. For example, an enzyme can be used to remove a nucleic acid from a substrate. For example, a nucleic acid can be removed from a substrate through a restriction endonuclease digestion. For example, treatment of a nucleic acid containing a dUTP or ddUTP with uracil-d-glycosylase (UDG) can be used to remove a nucleic acid from a substrate. For example, a nucleic acid can be removed from a substrate using an enzyme that performs nucleotide excision, such as a base excision repair enzyme, such as an apurinic/apyrimidinic (AP) endonuclease. In some embodiments, a nucleic acid can be removed from a substrate using a photocleavable group and light. In some embodiments, a cleavable linker can be used to remove a nucleic acid from the substrate. For example, the cleavable linker can comprise at least one of biotin/avidin, biotin/streptavidin, biotin/neutravidin, Ig-protein A, a photo-labile linker, acid or base labile linker group, or an aptamer.

When the probes are gene-specific, the molecules can hybridize to the probes and be reverse transcribed and/or amplified. In some embodiments, after the nucleic acid has been synthesized (e.g., reverse transcribed), it can be amplified. Amplification can be performed in a multiplex manner, wherein multiple target nucleic acid sequences are amplified simultaneously. Amplification can add sequencing adaptors to the nucleic acid.

In some embodiments, amplification can be performed on the substrate, for example, with bridge amplification. cDNAs can be homopolymer tailed in order to generate a compatible end for bridge amplification using oligo(dT) probes on the substrate. In bridge amplification, the primer that is complementary to the 3' end of the template nucleic acid can be the first primer of each pair that is covalently attached to the solid particle. When a sample containing the template nucleic acid is contacted with the particle and a single thermal cycle is performed, the template molecule can be annealed to the first primer and the first primer is elongated in the forward direction by addition of nucleotides to form a duplex molecule consisting of the template molecule and a newly formed DNA strand that is complementary to the template. In the heating step of the next cycle, the duplex molecule can be denatured, releasing the template molecule from the particle and leaving the complementary DNA strand attached to the particle through the first primer. In the annealing stage of the annealing and elongation step that follows, the complementary strand can hybridize to the second primer, which is complementary to a segment of the complementary strand at a location removed from the first primer. This hybridization can cause the complementary strand to form a bridge between the first and second primers secured to the first primer by a covalent bond and to the second primer by hybridization. In the elongation stage, the second primer can be elongated in the reverse direction by the addition of nucleotides in the same reaction mixture, thereby converting the bridge to a double-stranded bridge. The next cycle then begins, and the double-stranded bridge can be denatured to yield two single-stranded nucleic acid molecules, each having one end attached to the particle surface via the first and second primers, respectively, with the other end of each unattached. In the annealing and elongation step of this second cycle, each strand can hybridize to a further complementary primer, previously unused, on the same particle, to form new single-strand bridges. The two previously unused primers that are now hybridized elongate to convert the two new bridges to double-strand bridges.

The amplification reactions can comprise amplifying at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, or 100% of the plurality of nucleic acids.

Amplification of the labeled nucleic acids can comprise PCR-based methods or non-PCR based methods. Amplification of the labeled nucleic acids can comprise exponential amplification of the labeled nucleic acids. Amplification of the labeled nucleic acids can comprise linear amplification of the labeled nucleic acids. Amplification can be performed by polymerase chain reaction (PCR). PCR can refer to a reaction for the in vitro amplification of specific DNA sequences by the simultaneous primer extension of complementary strands of DNA. PCR can encompass derivative forms of the reaction, including but not limited to, RT-PCR, real-time PCR, nested PCR, quantitative PCR, multiplexed PCR, digital PCR, suppression PCR, semi-suppressive PCR and assembly PCR.

Amplification of the labeled nucleic acids can comprise non-PCR based methods, including but not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), a Qβ replicase (Qβ), use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and/or ramification extension amplification (RAM).

In some embodiments, the methods disclosed herein further comprise conducting a nested polymerase chain reaction on the amplified amplicon (e.g., target). The amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample tag or molecular identifier label. Alternatively, the amplicon can be a single-stranded molecule. The single-stranded molecule can comprise DNA, RNA, or a combination thereof. The nucleic acids can comprise synthetic or altered nucleic acids.

In some embodiments, the method comprises repeatedly amplifying the labeled nucleic acid to produce multiple amplicons. The methods disclosed herein can comprise conducting at least about, at most about, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, or 100 amplification reactions.

Amplification can further comprise adding one or more control nucleic acids to one or more samples comprising a plurality of nucleic acids. Amplification can further comprise adding one or more control nucleic acids to a plurality of nucleic acids. The control nucleic acids can comprise a control label.

Amplification can comprise use of one or more non-natural nucleotides. Non-natural nucleotides can comprise photolabile and/or triggerable nucleotides. Examples of non-natural nucleotides include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise one or more oligonucleotides. The one or more oligonucleotides can comprise at least about 7-9 nucleotides. The one or more oligonucleotides can comprise less than 12-15 nucleotides. The one or more primers can anneal to at least a portion of the plurality of labeled nucleic acids. The one or more primers can anneal to the 3' end and/or 5' end of the plurality of labeled nucleic acids. The one or more primers can anneal to an internal region of the plurality of labeled nucleic acids. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled nucleic acids. The one or more primers can comprise a fixed panel of primers. The one or more primers can comprise at least one or more custom primers. The one or more primers can comprise at least one or more control primers. The one or more primers can comprise at least one or more housekeeping gene primers. The one or more primers can comprise a universal primer. The universal primer can anneal to a universal primer binding site. The one or more custom primers can anneal to the first sample tag, the second sample tag, the molecular identifier label, the nucleic acid or a product thereof. The one or more primers can comprise a universal primer and a custom primer. The custom primer can be designed to amplify one or more target nucleic acids. The target nucleic acids can comprise a subset of the total nucleic acids in one or more samples. In some embodiments, the primers are the probes attached to the array of the disclosure.

In some embodiments, barcoding (e.g., stochastically barcoding) the plurality of targets in the sample further comprises generating an indexed library of the barcoded targets (e.g., stochastically barcoded targets) or barcoded fragments of the targets. The barcode sequences of different barcodes (e.g., the molecular labels of different stochastic barcodes) can be different from one another. Generating an indexed library of the barcoded targets includes generating a plurality of indexed polynucleotides from the plurality of targets in the sample. For example, for an indexed library of the barcoded targets comprising a first indexed target and a second indexed target, the label region of the first indexed polynucleotide can differ from the label region of the second indexed polynucleotide by, by about, by at least, or by at most, 1, 2, 3, 4, 5, 6, 7, 8, 9, 10, 20, 30, 40, 50, or a number or a range between any two of these values, nucleotides. In some embodiments, generating an indexed library of the barcoded targets includes contacting a plurality of targets, for example mRNA molecules, with a plurality of oligonucleotides including a poly(T) region and a label region; and conducting a first strand synthesis using a reverse transcriptase to produce single-strand labeled cDNA molecules each comprising a cDNA region and a label region, wherein the plurality of targets includes at least two mRNA molecules of different sequences and the plurality of oligonucleotides includes at least two oligonucleotides of different sequences. Generating an indexed library of the barcoded targets can further comprise amplifying the single-strand labeled cDNA molecules to produce double-strand labeled cDNA molecules; and conducting nested PCR on the double-strand labeled cDNA molecules to produce labeled amplicons. In some embodiments, the method can include generating an adaptor-labeled amplicon.

Barcoding (e.g., stochastic barcoding) can include using nucleic acid barcodes or tags to label individual nucleic acid (e.g., DNA or RNA) molecules. In some embodiments, it includes adding DNA barcodes or tags to cDNA molecules as they are generated from mRNA. Nested PCR can be performed to minimize PCR amplification bias. Adaptors can be added for sequencing using, for example, next generation sequencing (NGS). The sequencing results can be used to determine cell labels, molecular labels, and sequences of nucleotide fragments of the one or more copies of the targets, for example at block 232 of FIG. 2.

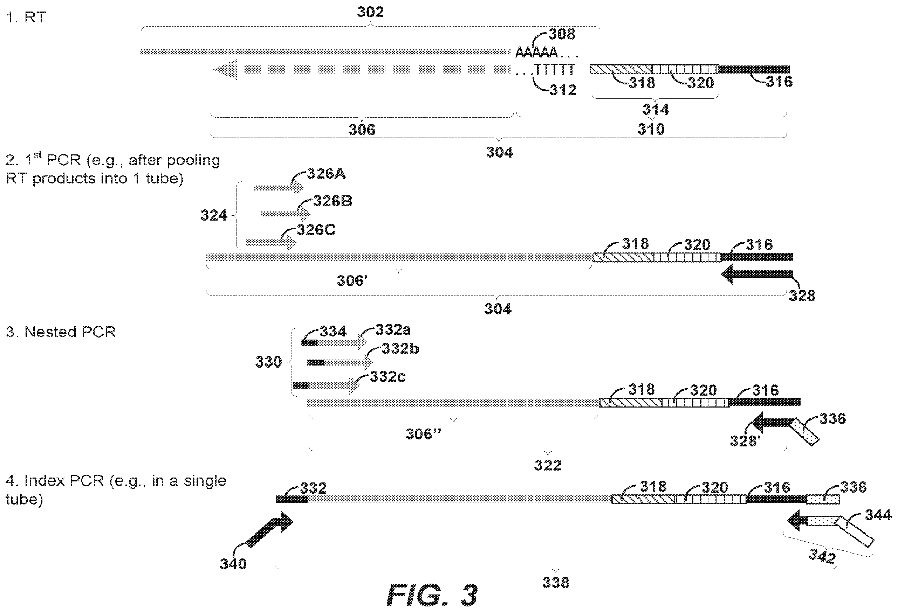

FIG. 3 is a schematic illustration showing a non-limiting exemplary process of generating an indexed library of the barcoded targets (e.g., stochastically barcoded targets), such as barcoded mRNAs or fragments thereof. As shown in step 1, the reverse transcription process can encode each mRNA molecule with a unique molecular label, a cell label, and a universal PCR site. In particular, RNA molecules 302 can be reverse transcribed to produce labeled cDNA molecules 304, including a cDNA region 306, by hybridization (e.g., stochastic hybridization) of a set of barcodes (e.g., stochastic barcodes) 310 to the poly(A) tail region 308 of the RNA molecules 302. Each of the barcodes 310 can comprise a target-binding region, for example a poly(dT) region 312, a label region 314 (e.g., a barcode sequence or a molecule), and a universal PCR region 316.

In some embodiments, the cell label can include 3 to 20 nucleotides. In some embodiments, the molecular label can include 3 to 20 nucleotides. In some embodiments, each of the plurality of stochastic barcodes further comprises one or more of a universal label and a cell label, wherein universal labels are the same for the plurality of stochastic barcodes on the solid support and cell labels are the same for the plurality of stochastic barcodes on the solid support. In some embodiments, the universal label can include 3 to 20 nucleotides. In some embodiments, the cell label comprises 3 to 20 nucleotides.

In some embodiments, the label region 314 can include a barcode sequence or a molecular label 318 and a cell label 320. In some embodiments, the label region 314 can include one or more of a universal label, a dimension label, and a cell label. The barcode sequence or molecular label 318 can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. The cell label 320 can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. The universal label can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. Universal labels can be the same for the plurality of stochastic barcodes on the solid support and cell labels are the same for the plurality of stochastic barcodes on the solid support. The dimension label can be, can be about, can be at least, or can be at most 1, 2, 3, 4, 5, 6, 7, 8, 9, 10, 20, 30, 40, 50, 60, 70, 80, 90, 100, or a number or a range between any of these values, of nucleotides in length.

In some embodiments, the label region 314 can comprise, comprise about, comprise at least, or comprise at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any of these values, different labels, such as a barcode sequence or a molecular label 318 and a cell label 320. Each label can be, can be about, can be at least, or can be at most 1, 2, 3, 4, 5, 6, 7, 8, 9, 10, 20, 30, 40, 50, 60, 70, 80, 90, 100, or a number or a range between any of these values, of nucleotides in length. A set of barcodes or stochastic barcodes 310 can contain, contain about, contain at least, or can be at most, 10, 20, 40, 50, 70, 80, 90, 10², 10³, 10⁴, 10⁵, 10⁶, 10⁷, 10⁸, 10⁹, 10¹⁰, 10¹¹, 10¹², 10¹¹, 10¹⁴, 10¹⁵, 10²⁰, or a number or a range between any of these values, barcodes or stochastic barcodes 310. And the set of barcodes or stochastic barcodes 310 can, for example, each contain a unique label region 314. The labeled cDNA molecules 304 can be purified to remove excess barcodes or stochastic barcodes 310. Purification can comprise Ampure bead purification.

As shown in step 2, products from the reverse transcription process in step 1 can be pooled into 1 tube and PCR amplified with a 1^{st} PCR primer pool and a 1^{st} universal PCR primer. Pooling is possible because of the unique label region 314. In particular, the labeled cDNA molecules 304 can be amplified to produce nested PCR labeled amplicons 322. Amplification can comprise multiplex PCR amplification. Amplification can comprise a multiplex PCR amplification with 96 multiplex primers in a single reaction volume. In some embodiments, multiplex PCR amplification can utilize, utilize about, utilize at least, or utilize at most, 10, 20, 40, 50, 70, 80, 90, 10², 10³, 10⁴, 10⁵, 10⁶, 10⁷, 10¹, 10⁹, 10¹⁰, 10¹¹, 10¹², 10¹¹, 10¹⁴, 10¹⁵, 10²⁰, or a number or a range between any of these values, multiplex primers in a single reaction volume. Amplification can comprise using a 1^{st} PCR primer pool 324 comprising custom primers 326A-C targeting specific genes and a universal primer 328. The custom primers 326 can hybridize to a region within the cDNA portion 306' of the labeled cDNA molecule 304. The universal primer 328 can hybridize to the universal PCR region 316 of the labeled cDNA molecule 304.

As shown in step 3 of FIG. 3, products from PCR amplification in step 2 can be amplified with a nested PCR primers pool and a 2^{nd} universal PCR primer. Nested PCR can minimize PCR amplification bias. In particular, the nested PCR labeled amplicons 322 can be further amplified by nested PCR. The nested PCR can comprise multiplex PCR with nested PCR primers pool 330 of nested PCR primers 332a-c and a 2^{nd} universal PCR primer 328' in a single reaction volume. The nested PCR primer pool 328 can contain, contain about, contain at least, or contain at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any of these values, different nested PCR primers 330. The nested PCR primers 332 can contain an adaptor 334 and hybridize to a region within the cDNA portion 306'' of the labeled amplicon 322. The universal primer 328' can contain an adaptor 336 and hybridize to the universal PCR region 316 of the labeled amplicon 322. Thus, step 3 produces adaptor-labeled amplicon 338. In some embodiments, nested PCR primers 332 and the 2^{nd} universal PCR primer 328' may not contain the adaptors 334 and 336. The adaptors 334 and 336 can instead be ligated to the products of nested PCR to produce adaptor-labeled amplicon 338.

As shown in step 4, PCR products from step 3 can be PCR amplified for sequencing using library amplification primers. In particular, the adaptors 334 and 336 can be used to conduct one or more additional assays on the adaptor-labeled amplicon 338. The adaptors 334 and 336 can be hybridized to primers 340 and 342. The one or more primers 340 and 342 can be PCR amplification primers. The one or more primers 340 and 342 can be sequencing primers. The one or more adaptors 334 and 336 can be used for further amplification of the adaptor-labeled amplicons 338. The one or more adaptors 334 and 336 can be used for sequencing the adaptor-labeled amplicon 338. The primer 342 can contain a plate index 344 so that amplicons generated using the same set of barcodes or stochastic barcodes 310 can be sequenced in one sequencing reaction using next generation sequencing (NGS).

### Compositions Comprising Cellular Component Binding Reagents Associated with Oligonucleotides

Some embodiments disclosed herein provide a plurality of compositions each comprising a cellular component binding reagent (such as a protein binding reagent) that is conjugated with an oligonucleotide, wherein the oligonucleotide comprises a unique identifier for the cellular component binding reagent that it is conjugated with. Cellular component binding reagents (such as barcoded antibodies) and their uses (such as sample indexing of cells) have been described in U.S. Patent Application Publication Nos. US2018/0088112 and US2018/0346970. A cellular component binding reagent can comprise an intracellular target-binding reagent, a cell surface target-binding reagent, and/or a nuclear target-binding reagent. A binding reagent (e.g., a cellular component binding reagent) can be associated with a binding reagent oligonucleotide. A binding reagent oligonucleotide can comprise an intracellular target-binding reagent specific oligonucleotide, a cell surface target-binding reagent specific oligonucleotide, and/or a nuclear target-binding reagent specific oligonucleotide.

The cellular component binding reagent can be capable of specifically binding to a cellular component target (e.g., intracellular target, nuclear target, cell surface target). For example, a binding target of the cellular component binding reagent can be, or comprise, a carbohydrate, a lipid, a protein, an extracellular protein, a cell-surface protein, a cell marker, a B-cell receptor, a T-cell receptor, a major histocompatibility complex, a tumor antigen, a receptor, an integrin, an intracellular protein, or any combination thereof. In some embodiments, the cellular component binding reagent (e.g., a protein binding reagent) is capable of specifically binding to an antigen target or a protein target. In some embodiments, each of the oligonucleotides can comprise a barcode, such as a stochastic barcode. A barcode can comprise a barcode sequence (e.g., a molecular label), a cell label, a sample label, or any combination thereof. In some embodiments, each of the oligonucleotides can comprise a linker. In some embodiments, each of the oligonucleotides can comprise a binding site for an oligonucleotide probe, such as a poly(A) tail. For example, the poly(A) tail can be, e.g., unanchored to a solid support or anchored to a solid support. The poly(A) tail can be from about 10 to 50 nucleotides in length, e.g, 18 nucleotides in length. The oligonucleotides can comprise deoxyribonucleotides, ribonucleotides, or both.

The unique identifiers can be, for example, a nucleotide sequence having any suitable length, for example, from about 4 nucleotides to about 200 nucleotides. In some embodiments, the unique identifier is a nucleotide sequence of 25 nucleotides to about 45 nucleotides in length. In some embodiments, the unique identifier can have a length that is, is about, is less than, is greater than, 4, 5, 6, 7, 8, 9, 10, 15, 20, 25, 30, 35, 40, 45, 50, 55, 60, 70, 80, 90, 100, 200 nucleotides, or a range that is between any two of the above values.

In some embodiments, the unique identifiers are selected from a diverse set of unique identifiers. The diverse set of unique identifiers can comprise, or comprise about, 20, 30, 40, 50, 60, 70, 80, 90, 100, 200, 300, 400, 500, 600, 700, 800, 900, 1000, 2000, 5000, or a number or a range between any two of these values, different unique identifiers. The diverse set of unique identifiers can comprise at least, or comprise at most, 20, 30, 40, 50, 60, 70, 80, 90, 100, 200, 300, 400, 500, 600, 700, 800, 900, 1000, 2000, or 5000, different unique identifiers. In some embodiments, the set of unique identifiers is designed to have minimal sequence homology to the DNA or RNA sequences of the sample to be analyzed. In some embodiments, the sequences of the set of unique identifiers are different from each other, or the complement thereof, by, or by about, 1, 2, 3, 4, 5, 6, 7, 8, 9, 10, or a number or a range between any two of these values, nucleotides. In some embodiments, the sequences of the set of unique identifiers are different from each other, or the complement thereof, by at least, or by at most, 1, 2, 3, 4, 5, 6, 7, 8, 9, or 10 nucleotides. In some embodiments, the sequences of the set of unique identifiers are different from each other, or the complement thereof, by at least 3%, 5%, 8%, 10%, %, 20%, or more.

In some embodiments, the unique identifiers can comprise a binding site for a primer, such as universal primer. In some embodiments, the unique identifiers can comprise at least two binding sites for a primer, such as a universal primer. In some embodiments, the unique identifiers can comprise at least three binding sites for a primer, such as a universal primer. The primers can be used for amplification of the unique identifiers, for example, by PCR amplification. In some embodiments, the primers can be used for nested PCR reactions.

Any suitable cellular component binding reagents are contemplated in this disclosure, such as protein binding reagents, antibodies or fragments thereof, aptamers, small molecules, ligands, peptides, oligonucleotides, or any combination thereof. In some embodiments, the cellular component binding reagents can be polyclonal antibodies, monoclonal antibodies, recombinant antibodies, single chain antibody (sc-Ab), or fragments thereof, such as Fab, Fv. In some embodiments, the plurality of cellular component binding reagents can comprise, comprise about, comprise at least, or comprise at most, 20, 30, 40, 50, 60, 70, 80, 90, 100, 200, 300, 400, 500, 600, 700, 800, 900, 1000, 2000, 5000, or a number or a range between any two of these values, different cellular component reagents.

The oligonucleotide can be conjugated with the cellular component binding reagent through various mechanism. In some embodiments, the oligonucleotide can be conjugated with the cellular component binding reagent covalently. In some embodiment, the oligonucleotide can be conjugated with the cellular component binding reagent non-covalently. In some embodiments, the oligonucleotide is conjugated with the cellular component binding reagent through a linker. The linker can be, for example, cleavable or detachable from the cellular component binding reagent and/or the oligonucleotide. In some embodiments, the linker can comprise a chemical group that reversibly attaches the oligonucleotide to the cellular component binding reagents. The chemical group can be conjugated to the linker, for example, through an amine group. In some embodiments, the linker can comprise a chemical group that forms a stable bond with another chemical group conjugated to the cellular component binding reagent. For example, the chemical group can be a UV photocleavable group, a disulfide bond, a streptavidin, a biotin, or an amine. In some embodiments, the chemical group can be conjugated to the cellular component binding reagent through a primary amine on an amino acid, such as lysine, or the N-terminus. Commercially available conjugation kits, such as the Protein-Oligo Conjugation Kit (Solulink, Inc., San Diego, CA), the Thunder-Link^{®} oligo conjugation system (Innova Biosciences, Cambridge, UK), can be used to conjugate the oligonucleotide to the cellular component binding reagent.

The oligonucleotide can be conjugated to any suitable site of the cellular component binding reagent (e.g., a protein binding reagent), as long as it does not interfere with the specific binding between the cellular component binding reagent and its cellular component target. In some embodiments, the cellular component binding reagent is a protein, such as an antibody. In some embodiments, the cellular component binding reagent is not an antibody. In some embodiments, the oligonucleotide can be conjugated to the antibody anywhere other than the antigen-binding site, for example, the Fc region, C_{H}1 domain, C_{H}2 domain, C_{H}3 domain, and C_{L} domain. Methods of conjugating oligonucleotides to cellular component binding reagents (e.g., antibodies) have been previously disclosed, for example, in U.S. Patent. No. 6,531,283. Stoichiometry of oligonucleotide to cellular component binding reagent can be varied. To increase the sensitivity of detecting the cellular component binding reagent specific oligonucleotide in sequencing, it may be advantageous to increase the ratio of oligonucleotide to cellular component binding reagent during conjugation. In some embodiments, each cellular component binding reagent can be conjugated with a single oligonucleotide molecule. In some embodiments, each cellular component binding reagent can be conjugated with more than one oligonucleotide molecule, for example, at least, or at most, 2, 3, 4, 5, 10, 20, 30, 40, 50, 100, 1000, or a number or a range between any two of these values, oligonucleotide molecules, wherein each of the oligonucleotide molecule comprises the same, or different, unique identifiers.

In some embodiments, the plurality of cellular component binding reagents is capable of specifically binding to a plurality of cellular component targets in a sample, such as a single cell, a plurality of cells, a tissue sample, a tumor sample, a blood sample, or the like. In some embodiments, the plurality of cellular component targets comprises a cell-surface protein, a cell marker, a B-cell receptor, a T-cell receptor, an antibody, a major histocompatibility complex, a tumor antigen, a receptor, or any combination thereof. In some embodiments, the plurality of cellular component targets can comprise intracellular cellular components. In some embodiments, the plurality of cellular components can be, be about, at least, or be at most, 1%, 2%, 3%, 4%, 5%, 6%, 7%, 8%, 9%, 10%, 20%, 30%, 40%, 50%, 60%, 70%, 80%, 90%, 95%, 98%, 99%, or a number or a range between any two of these values, of all the cellular components (e.g., proteins) in a cell or an organism. In some embodiments, the plurality of cellular component targets can comprise, comprise about, comprise at least, or comprise at most, 2, 3, 4, 5, 10, 20, 30, 40, 50, 100, 1000, 10000, or a number or a range between any two of these values, different cellular component targets.

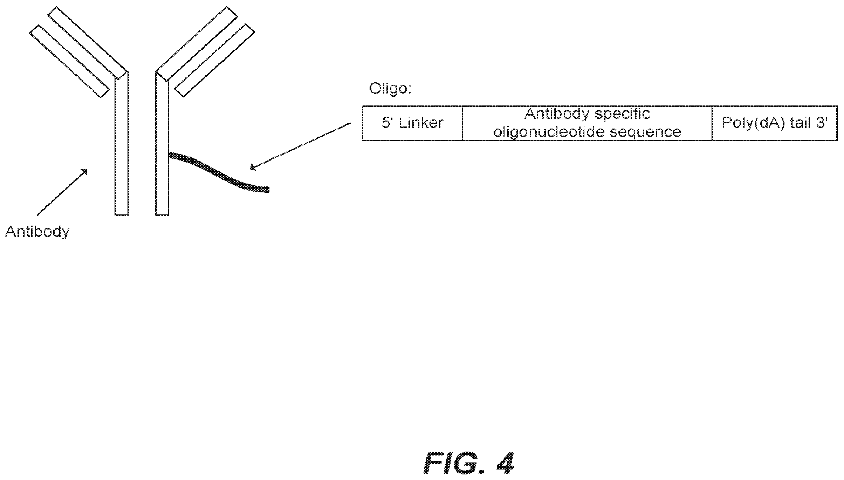

FIG. 4 shows a schematic illustration of an exemplary cellular component binding reagent (e.g., an antibody) that is associated (e.g., conjugated) with an oligonucleotide comprising a unique identifier sequence for the antibody. An oligonucleotide-conjugated with a cellular component binding reagent, an oligonucleotide for conjugation with a cellular component binding reagent, or an oligonucleotide previously conjugated with a cellular component binding reagent can be referred to herein as an antibody oligonucleotide (abbreviated as a binding reagent oligonucleotide). An oligonucleotide-conjugated with an antibody, an oligonucleotide for conjugation with an antibody, or an oligonucleotide previously conjugated with an antibody can be referred to herein as an antibody oligonucleotide (abbreviated as an "AbOligo" or "AbO"). The oligonucleotide can also comprise additional components, including but not limited to, one or more linker, one or more unique identifier for the antibody, optionally one or more barcode sequences (e.g., molecular labels), and a poly(A) tail. In some embodiments, the oligonucleotide can comprise, from 5' to 3', a linker, a unique identifier, a barcode sequence (e.g., a molecular label), and a poly(A) tail. An antibody oligonucleotide can be an mRNA mimic.

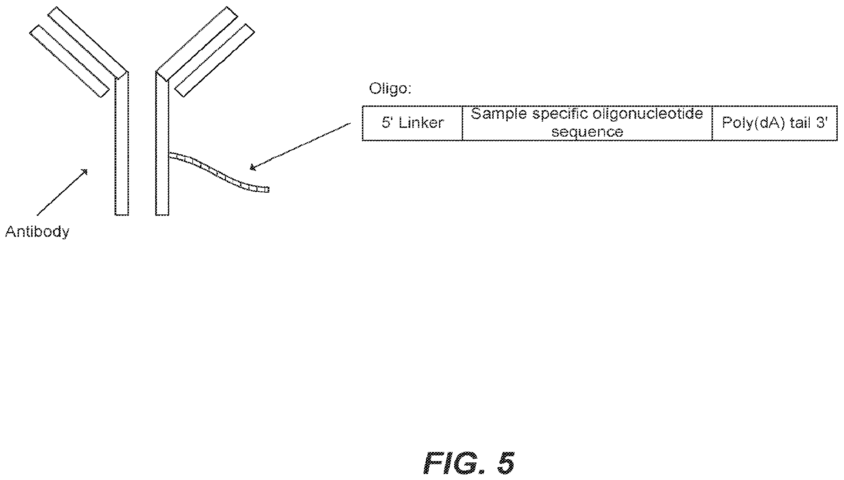

FIG. 5 shows a schematic illustration of an exemplary cellular component binding reagent (e.g., an antibody) that is associated (e.g., conjugated) with an oligonucleotide comprising a unique identifier sequence for the antibody. The cellular component binding reagent can be capable of specifically binding to at least one cellular component target, such as an antigen target or a protein target. A binding reagent oligonucleotide (e.g., a sample indexing oligonucleotide, or an antibody oligonucleotide) can comprise a sequence (e.g., a sample indexing sequence) for performing the methods of the disclosure. For example, a sample indexing oligonucleotide can comprise a sample indexing sequence for identifying sample origin of one or more cells of a sample. Indexing sequences (e.g., sample indexing sequences) of at least two compositions comprising two cellular component binding reagents (e.g., sample indexing compositions) of the plurality of compositions comprising cellular component binding reagents can comprise different sequences. In some embodiments, the binding reagent oligonucleotide is not homologous to genomic sequences of a species. The binding reagent oligonucleotide can be configured to be (or can be) detachable or non-detachable from the cellular component binding reagent.

The oligonucleotide conjugated to a cellular component binding reagent can, for example, comprise a barcode sequence (e.g., a molecular label sequence), a poly(A) tail, or a combination thereof. An oligonucleotide conjugated to a cellular component binding reagent can be an mRNA mimic. In some embodiments, the sample indexing oligonucleotide comprises a sequence complementary to a capture sequence of at least one barcode of the plurality of barcodes. A target binding region of the barcode can comprise the capture sequence. The target binding region can, for example, comprise a poly(dT) region. In some embodiments, the sequence of the sample indexing oligonucleotide complementary to the capture sequence of the barcode can comprise a poly(A) tail. The sample indexing oligonucleotide can comprise a molecular label.

In some embodiments, the binding reagent oligonucleotide (e.g., the sample oligonucleotide, intracellular target-binding reagent specific oligonucleotide, cell surface target-binding reagent specific oligonucleotide, nuclear target-binding reagent specific oligonucleotide) comprises a nucleotide sequence of, or a nucleotide sequence of, or of about, at least, or of at most, 6, 7, 8, 9, 10, 11, 12, 13, 14, 15, 16, 17, 18, 19, 20, 25, 30, 35, 40, 45, 50, 60, 70, 80, 90, 100, 110, 120, 128, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or a number or a range between any two of these values, nucleotides in length.

In some embodiments, the cellular component binding reagent (e.g., intracellular target-binding reagent, cell surface target-binding reagent, or nuclear target-binding reagent) comprises an antibody, a tetramer, an aptamer, a protein scaffold, or a combination thereof. The cellular component binding reagent can be a binding reagent for protein. The binding reagent oligonucleotide can be conjugated to the cellular component binding reagent, for example, through a linker. The binding reagent oligonucleotide can comprise the linker. The linker can comprise a chemical group. The chemical group can be reversibly, or irreversibly, attached to the molecule of the cellular component binding reagent. The chemical group can be selected from the group consisting of a UV photocleavable group, a disulfide bond, a streptavidin, a biotin, an amine, and any combination thereof.

The cellular component binding reagent can bind to ADAM10, CD156c, ANO6, ATP1B2, ATP1B3, BSG, CD147, CD109, CD230, CD29, CD298, ATP1B3, CD44, CD45, CD47, CD51, CD59, CD63, CD97, CD98, SLC3A2, CLDND1, HLA-ABC, ICAM1, ITFG3, MPZL1, NA K ATPase alpha1, ATP1A1, NPTN, PMCA ATPase, ATP2B1, SLC1A5, SLC29A1, SLC2A1, SLC44A2, or any combination thereof.

The protein target can be, or can comprise, an extracellular protein, an intracellular protein, or any combination thereof. In some embodiments, the antigen or protein target is, or comprises, a cell-surface protein, a cell marker, a B-cell receptor, a T-cell receptor, a major histocompatibility complex, a tumor antigen, a receptor, an integrin, or any combination thereof. The antigen or protein target can be, or comprise, a lipid, a carbohydrate, or any combination thereof. The protein target can be selected from a group comprising a number of protein targets. The number of antigen targets or protein targets can be, be about, at least, or be at most, 1, 2, 3, 4, 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, or a number or a range between any two of these values.

The cellular component binding reagent (e.g., a protein binding reagent) can be associated with two or more binding reagent oligonucleotide (e.g., sample indexing oligonucleotides, intracellular target-binding reagent specific oligonucleotides, cell surface target-binding reagent specific oligonucleotides, nuclear target-binding reagent specific oligonucleotides) with an identical sequence. The cellular component binding reagent can be associated with two or more binding reagent oligonucleotides with different sequences. The number of binding reagent oligonucleotides associated with the cellular component binding reagent can be different in different implementations. The number of binding reagent oligonucleotides, whether having an identical sequence or different sequences, can be, or can be about, at least, or be at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any two of these values.

The plurality of compositions comprising cellular component binding reagents (e.g., the plurality of sample indexing compositions) can comprise one or more additional cellular component binding reagents not conjugated with the binding reagent oligonucleotide (such as sample indexing oligonucleotide), which is also referred to herein as the binding reagent oligonucleotide-free cellular component binding reagent (such as sample indexing oligonucleotide-free cellular component binding reagent). The number of additional cellular component binding reagents in the plurality of compositions can be different in different implementations. In some embodiments, the number of additional cellular component binding reagents can be, be about, be at least, or be at most, 1, 2, 3, 4, 5, 6, 7, 8, 9, 10, 20, 30, 40, 50, 60, 70, 80, 90, 100, or a number or a range between any two of these values. The cellular component binding reagent and any of the additional cellular component binding reagents can be identical, in some embodiments.

In some embodiments, a mixture comprising cellular component binding reagent(s) that is conjugated with one or more binding reagent oligonucleotides (e.g., sample indexing oligonucleotides, intracellular target-binding reagent specific oligonucleotides, cell surface target-binding reagent specific oligonucleotides, nuclear target-binding reagent specific oligonucleotides) and cellular component binding reagent(s) that is not conjugated with binding reagent oligonucleotides are provided. The mixture can be used in some embodiments of the methods disclosed herein, for example, to contact the sample(s) and/or cell(s). The ratio of (1) the number of a cellular component binding reagent conjugated with a binding reagent oligonucleotide and (2) the number of another cellular component binding reagent (e.g., the same cellular component binding reagent) not conjugated with the binding reagent oligonucleotide (e.g., sample indexing oligonucleotide) or other binding reagent oligonucleotide(s) in the mixture can be different in different implementations. In some embodiments, the ratio can be, be about, be at least, or be at most, 1:1, 1:1.1, 1:1.2, 1:1.3, 1:1.4, 1:1.5, 1:1.6, 1:1.7, 1:1.8, 1:1.9, 1:2, 1:2.5, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:21, 1:22, 1:23, 1:24, 1:25, 1:26, 1:27, 1:28, 1:29, 1:30, 1:31, 1:32, 1:33, 1:34, 1:35, 1:36, 1:37, 1:38, 1:39, 1:40, 1:41, 1:42, 1:43, 1:44, 1:45, 1:46, 1:47, 1:48, 1:49, 1:50, 1:51, 1:52, 1:53, 1:54, 1:55, 1:56, 1:57, 1:58, 1:59, 1:60, 1:61, 1:62, 1:63, 1:64, 1:65, 1:66, 1:67, 1:68, 1:69, 1:70, 1:71, 1:72, 1:73, 1:74, 1:75, 1:76, 1:77, 1:78, 1:79, 1:80, 1:81, 1:82, 1:83, 1:84, 1:85, 1:86, 1:87, 1:88, 1:89, 1:90, 1:91, 1:92, 1:93, 1:94, 1:95, 1:96, 1:97, 1:98, 1:99, 1:100, 1:200, 1:300, 1:400, 1:500, 1:600, 1:700, 1:800, 1:900, 1:1000, 1:2000, 1:3000, 1:4000, 1:5000, 1:6000, 1:7000, 1:8000, 1:9000, 1:10000, or a number or a range between any two of the values. In some embodiments, the ratio can be, be about, be at least, or be at most, 1:1, 1.1:1, 1.2:1, 1.3:1, 1.4:1, 1.5:1, 1.6:1, 1.7:1, 1.8:1, 1.9:1, 2:1, 2.5:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1, 44:1, 45:1, 46:1, 47:1, 48:1, 49:1, 50:1, 51:1, 52:1, 53:1, 54:1, 55:1, 56:1, 57:1, 58:1, 59:1, 60:1, 61:1, 62:1, 63:1, 64:1, 65:1, 66:1, 67:1, 68:1, 69:1, 70:1, 71:1, 72:1, 73:1, 74:1, 75:1, 76:1, 77:1, 78:1, 79:1, 80:1, 81:1, 82:1, 83:1, 84:1, 85:1, 86:1, 87:1, 88:1, 89:1, 90:1, 91:1, 92:1, 93:1, 94:1, 95:1, 96:1, 97:1, 98:1, 99:1, 100:1, 200:1, 300:1, 400:1, 500:1, 600:1, 700:1, 800:1, 900:1, 1000:1, 2000:1, 3000:1, 4000:1, 5000:1, 6000:1, 7000:1, 8000:1, 9000:1, 10000:1, or a number or a range between any two of the values.

A cellular component binding reagent can be conjugated with a binding reagent oligonucleotide (e.g., a sample indexing oligonucleotide, intracellular target-binding reagent specific oligonucleotide, cell surface target-binding reagent specific oligonucleotide, nuclear target-binding reagent specific oligonucleotide), or not. In some embodiments, the percentage of the cellular component binding reagent conjugated with a binding reagent oligonucleotide (e.g., a sample indexing oligonucleotide) in a mixture comprising the cellular component binding reagent that is conjugated with the binding reagent oligonucleotide and the cellular component binding reagent(s) that is not conjugated with the binding reagent oligonucleotide can be, be about, be at least, or be at most, 0.000000001%, 0.00000001%, 0.0000001%, 0.000001%, 0.00001%, 0.0001%, 0.001%, 0.01%, 0.1%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or a number or a range between any two of these values.

In some embodiments, the percentage of the cellular component binding reagent not conjugated with a binding reagent oligonucleotide (e.g., a sample indexing oligonucleotide, intracellular target-binding reagent specific oligonucleotide, cell surface target-binding reagent specific oligonucleotide, nuclear target-binding reagent specific oligonucleotide) in a mixture comprising a cellular component binding reagent conjugated with a binding reagent oligonucleotide (e.g., a sample indexing oligonucleotide, intracellular target-binding reagent specific oligonucleotide, cell surface target-binding reagent specific oligonucleotide, nuclear target-binding reagent specific oligonucleotide) and the cellular component binding reagent that is not conjugated with the sample indexing oligonucleotide can be, be about, be at least, or be at most 0.000000001%, 0.00000001%, 0.0000001%, 0.000001%, 0.00001%, 0.0001%, 0.001%, 0.01%, 0.1%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or a number or a range between any two of these values.

### Cellular Component Cocktails

A cocktail of cellular component binding reagents (e.g., an antibody cocktail) can be used to increase labeling sensitivity in the methods disclosed herein. Without being bound by any particular theory, it is believed that this may be because cellular component expression or protein expression can vary between cell types and cell states, making finding a universal cellular component binding reagent or antibody that labels all cell types challenging. For example, cocktail of cellular component binding reagents can be used to allow for more sensitive and efficient labeling of more sample types. The cocktail of cellular component binding reagents can include two or more different types of cellular component binding reagents, for example a wider range of cellular component binding reagents or antibodies. Cellular component binding reagents that label different cellular component targets can be pooled together to create a cocktail that sufficiently labels all cell types, or one or more cell types of interest.

In some embodiments, each of the plurality of compositions (e.g., sample indexing compositions) comprises a cellular component binding reagent. In some embodiments, a composition of the plurality of compositions comprises two or more cellular component binding reagents, wherein each of the two or more cellular component binding reagents is associated with a binding reagent oligonucleotide (e.g., a sample indexing oligonucleotide), wherein at least one of the two or more cellular component binding reagents is capable of specifically binding to at least one of the one or more cellular component targets. The sequences of the binding reagent oligonucleotides associated with the two or more cellular component binding reagents can be identical. The sequences of the binding reagent oligonucleotides associated with the two or more cellular component binding reagents can comprise different sequences. Each of the plurality of compositions can comprise the two or more cellular component binding reagents.

The number of different types of cellular component binding reagents (e.g., a CD147 antibody and a CD47 antibody) in a composition can be different in different implementations. A composition with two or more different types of cellular component binding reagents can be referred to herein as a cellular component binding reagent cocktail (e.g., a sample indexing composition cocktail). The number of different types of cellular component binding reagents in a cocktail can vary. In some embodiments, the number of different types of cellular component binding reagents in cocktail can be, be about, be at least, or be at most, 2, 3, 4, 5, 6, 7, 8, 9, 10, 20, 30, 40, 50, 60, 70, 80, 90, 100, 200, 300, 400, 500, 600, 700, 800, 900, 1000, 10000, 100000, or a number or a range between any two of these values. The different types of cellular component binding reagents can be conjugated to binding reagent oligonucleotides with the same or different sequences (e.g., sample indexing sequences).

### Methods of Quantitative Analysis of Cellular Component Targets

Described herein include methods for quantitative analysis of a plurality of cellular component targets (e.g., protein targets) in a sample using oligonucleotide probes that can associate a barcode sequence (e.g., a molecular label sequence) to the oligonucleotides of the cellular component binding reagents (e.g., protein binding reagents). The cellular component binding reagent can comprise an intracellular target-binding reagent, a cell surface target-binding reagent, and/or a nuclear target-binding reagent. The oligonucleotides of the cellular component binding reagents can be, or comprise, an antibody oligonucleotide, a sample indexing oligonucleotide, a cell identification oligonucleotide, a control particle oligonucleotide, a control oligonucleotide, an interaction determination oligonucleotide, intracellular target-binding reagent specific oligonucleotide, cell surface target-binding reagent specific oligonucleotide, and/or nuclear target-binding reagent specific oligonucleotide. In some embodiments, the sample can be a single cell, a plurality of cells, a tissue sample, a tumor sample, a blood sample, or the like. The sample can comprise a mixture of cell types, such as normal cells, tumor cells, blood cells, B cells, T cells, maternal cells, fetal cells, or a mixture of cells from different subjects. In some embodiments, the sample can comprise a plurality of single cells separated into individual compartments, such as microwells in a microwell array.

The binding target of the plurality of cellular component target (i.e., the cellular component target) can be, or can comprise, a carbohydrate, a lipid, a protein, an extracellular protein, a cell-surface protein, a cell marker, a B-cell receptor, a T-cell receptor, a major histocompatibility complex, a tumor antigen, a receptor, an integrin, an intracellular protein, or any combination thereof. In some embodiments, the cellular component target is a protein target. In some embodiments, the plurality of cellular component targets comprises a cell-surface protein, a cell marker, a B-cell receptor, a T-cell receptor, an antibody, a major histocompatibility complex, a tumor antigen, a receptor, or any combination thereof. In some embodiments, the plurality of cellular component targets can comprise intracellular cellular components. In some embodiments, the plurality of cellular components can be, be about, be at least, or be at most, 1%, 2%, 3%, 4%, 5%, 6%, 7%, 8%, 9%, 10%, 20%, 30%, 40%, 50%, 60%, 70%, 80%, 90%, 95%, 98%, 99%, 100%, or a number or a range between any two of these values, of all the encoded cellular components in an organism. In some embodiments, the plurality of cellular component targets can comprise at least 2, 3, 4, 5, 10, 20, 30, 40, 50, 100, 1000, 10000, or more different cellular component targets.

In some embodiments, the plurality of cellular component binding reagents is contacted with the sample for specific binding with the plurality of cellular component targets. Unbound cellular component binding reagents can be removed, for example, by washing. In embodiments where the sample comprises cells, any cellular component binding reagents not specifically bound to the cells can be removed.

In some instances, cells from a population of cells can be separated (e.g., isolated) into wells of a substrate of the disclosure. The population of cells can be diluted prior to separating. The population of cells can be diluted such that at least, or at most, 1%, 5%, 10%, 15%, 20%, 25%, 30%, 35%, 40%, 45%, 50%, 55%, 60%, 65%, 70%, 75%, 80%, 85%, 90%, 95% or 100%, of wells of the substrate receive a single cell. The population of cells can be diluted such that the number of cells in the diluted population is, is at least, or is at most, 1%, 5%, 10%, 15%, 20%, 25%, 30%, 35%, 40%, 45%, 50%, 55%, 60%, 65%, 70%, 75%, 80%, 85%, 90%, 95%, or 100%, of the number of wells on the substrate. In some instances, the population of cells is diluted such that the number of cell is about 10% of the number of wells in the substrate.

Distribution of single cells into wells of the substrate can follow a Poisson distribution. For example, there can be at least, or at most, a 0.1%, 0.5%, 1%, 2%, 3%, 4%, 5%, 6%, 7%, 8%, 9%, or 10%, or more probability that a well of the substrate has more than one cell. Distribution of single cells into wells of the substrate can be random. Distribution of single cells into wells of the substrate can be non-random. The cells can be separated such that a well of the substrate receives only one cell. In some embodiments, the cellular component binding reagents can be conjugated with fluorescent molecules to enable flow sorting of cells into individual compartments.

In some embodiments, the methods disclosed herein provide contacting a plurality of compositions with the sample for specific binding with the plurality of cellular component targets. It would be appreciated that the conditions used may allow specific binding of the cellular component binding reagents, e.g., antibodies, to the cellular component targets. Following the contacting step, unbound compositions can be removed. For example, in embodiments where the sample comprises cells, and the compositions specifically bind to cellular component targets are cell-surface cellular components, such as cell-surface proteins, unbound compositions can be removed by washing the cells with buffer such that only compositions that specifically bind to the cellular component targets remain with the cells.

In some embodiments, the methods disclosed herein can comprise associating an oligonucleotide (e.g., a barcode, or a stochastic barcode), including a barcode sequence (e.g., a molecular label), a cell label, a sample label, or any combination thereof, to the plurality of oligonucleotides associated with the cellular component binding reagents. For example, a plurality of oligonucleotide probes comprising a barcode can be used to hybridize to the plurality of oligonucleotides of the compositions.

The plurality of oligonucleotide probes can be immobilized on solid supports. The solid supports can be free floating, e.g., beads in a solution. The solid supports can be embedded in a semi-solid or solid array. In some embodiments, the plurality of oligonucleotide probes may not be immobilized on solid supports. When the plurality of oligonucleotide probes is in close proximity to the plurality associated with oligonucleotides of the cellular component binding reagents, the plurality of oligonucleotides of the cellular component binding reagents can hybridize to the oligonucleotide probes. The oligonucleotide probes can be contacted at a non-depletable ratio such that each distinct oligonucleotide of the cellular component binding reagents can associate with oligonucleotide probes having different barcode sequences (e.g., molecular labels) of the disclosure.

The methods disclosed herein can comprise detaching the oligonucleotides from the cellular component binding reagents that are specifically bound to the cellular component targets. Detachment can be performed in a variety of ways to separate the chemical group from the cellular component binding reagent, such as UV photocleaving, chemical treatment (e.g., dithiothreitol treatment), heating, enzyme treatment, or any combination thereof. Detaching the oligonucleotide from the cellular component binding reagent can be performed either before, after, or during the step of hybridizing the plurality of oligonucleotide probes to the plurality of oligonucleotides of the compositions.

### Methods of Simultaneous Quantitative Analysis of Cellular Component and Nucleic Acid Targets

The methods disclosed herein can be used for simultaneous quantitative analysis of a plurality of cellular component targets (e.g., protein targets, cell surface targets, an intracellular targets, a nuclear targets) and a plurality of nucleic acid target molecules in a sample using the compositions disclosed herein and oligonucleotide probes that can associate a barcode sequence (e.g., a molecular label sequence) to both the oligonucleotides of the cellular component binding reagents and nucleic acid target molecules. Other methods of simultaneous quantitative analysis of a plurality of cellular component targets and a plurality of nucleic acid target molecules are described in US2018/0088112 and US2018/0346970. In some embodiments, the sample can be a single cell, a plurality of cells, a tissue sample, a tumor sample, a blood sample, or the like. In some embodiments, the sample can comprise a mixture of cell types, such as normal cells, tumor cells, blood cells, B cells, T cells, maternal cells, fetal cells, or a mixture of cells from different subjects. In some embodiments, the sample can comprise a plurality of single cells separated into individual compartments, such as microwells in a microwell array.

The plurality of cellular component targets can comprise a cell surface target, an intracellular target, a nuclear target, a cell-surface protein, a cell marker, a B-cell receptor, a T-cell receptor, an antibody, a major histocompatibility complex, a tumor antigen, a receptor, or any combination thereof. In some embodiments, the plurality of cellular component targets can comprise intracellular cellular components. In some embodiments, the plurality of cellular components can be, be about, be at least, or be at most, 1%, 2%, 3%, 4%, 5%, 6%, 7%, 8%, 9%, 10%, 20%, 30%, 40%, 50%, 60%, 70%, 80%, 90%, 95%, 98%, 99%, or a number or a range between any two of these values, of all the cellular components, such as expressed proteins, in an organism, or one or more cells of the organism. In some embodiments, the plurality of cellular component targets can comprise, comprise about, comprise at least, or comprise at most, 2, 3, 4, 5, 10, 20, 30, 40, 50, 100, 1000, 10000, or a number or a range between any two of these values, different cellular component targets.

In some embodiments, the plurality of cellular component binding reagents is contacted with the sample for specific binding with the plurality of cellular component targets. Unbound cellular component binding reagents can be removed, for example, by washing. In embodiments where the sample comprises cells, any cellular component binding reagents not specifically bound to the cells can be removed.

In some instances, cells from a population of cells can be separated (e.g., isolated) into wells of a substrate of the disclosure. The population of cells can be diluted prior to separating. The population of cells can be diluted such that at least, or at most, 1%, 5%, 10%, 15%, 20%, 25%, 30%, 35%, 40%, 45%, 50%, 55%, 60%, 65%, 70%, 75%, 80%, 85%, 90%, 95%, or 100% of wells of the substrate receive a single cell. The population of cells can be diluted such that the number of cells in the diluted population is, or is at least, 1%, 5%, 10%, 15%, 20%, 25%, 30%, 35%, 40%, 45%, 50%, 55%, 60%, 65%, 70%, 75%, 80%, 85%, 90%, 95%, or 100% of the number of wells on the substrate. In some instances, the population of cells is diluted such that the number of cells is about 10% of the number of wells in the substrate.

Distribution of single cells into wells of the substrate can follow a Poisson distribution. For example, there can be at least a 0.1%, 0.5%, 1%, 2%, 3%, 4%, 5%, 6%, 7%, 8%, 9%, or 10%, or more probability that a well of the substrate has more than one cell. Distribution of single cells into wells of the substrate can be random. Distribution of single cells into wells of the substrate can be non-random. The cells can be separated such that a well of the substrate receives only one cell.

In some embodiments, the methods disclosed herein provide contacting a plurality of compositions with the sample for specific binding with the plurality of cellular component targets. It would be appreciated that the conditions used may allow specific binding of the cellular component binding reagents, e.g., antibodies, to the cellular component targets. Following the contacting step, unbound compositions can be removed. For example, in embodiments where the sample comprises cells, and the compositions specifically bind to cellular component targets are on the cell surface, such as cell-surface proteins, unbound compositions can be removed by washing the cells with buffer such that only compositions that specifically bind to the cellular component targets remain with the cells.

In some embodiments, the methods disclosed herein can provide releasing the plurality of nucleic acid target molecules from the sample, e.g., cells. For example, the cells can be lysed to release the plurality of nucleic acid target molecules. Cell lysis may be accomplished by any of a variety of means, for example, by chemical treatment, osmotic shock, thermal treatment, mechanical treatment, optical treatment, or any combination thereof. Cells may be lysed by addition of a cell lysis buffer comprising a detergent (e.g., SDS, Li dodecyl sulfate, Triton X-100, Tween-20, or NP-40), an organic solvent (e.g., methanol or acetone), or digestive enzymes (e.g., proteinase K, pepsin, or trypsin), or any combination thereof.

The plurality of nucleic acid molecules can comprise a variety of nucleic acid molecules. In some embodiments, the plurality of nucleic acid molecules can comprise, DNA molecules, RNA molecules, genomic DNA molecules, mRNA molecules, rRNA molecules, siRNA molecules, or a combination thereof, and can be double-stranded or single-stranded. In some embodiments, the plurality of nucleic acid molecules comprises, comprises about, comprises at least, or comprise at most, 100, 1000, 10000, 20000, 30000, 40000, 50000, 100000, 1000000, or a number or a range between any two of these values, species. The plurality of nucleic acid molecules can be, or be from a sample, such as a single cell, or a plurality of cells. In some embodiments, the plurality of nucleic acid molecules can be pooled from a plurality of samples, such as a plurality of single cells.

The methods disclosed herein can comprise associating a barcode (e.g., a stochastic barcode), which can include a barcode sequence (such as a molecular label), a cell label, a sample label, or any combination thereof, to the plurality of nucleic acid target molecules and the plurality of oligonucleotides of the cellular component binding reagents. For example, a plurality of oligonucleotide probes comprising a stochastic barcode can be used to hybridize to the plurality of nucleic acid target molecules and the plurality of oligonucleotides of the compositions.

The plurality of oligonucleotide probes can be immobilized on solid supports. The solid supports can be free floating, e.g., beads in a solution. The solid supports can be embedded in a semi-solid or solid array. In some embodiments, the plurality of oligonucleotide probes may not be immobilized on solid supports. When the plurality of oligonucleotide probes are in close proximity to the plurality of nucleic acid target molecules and the plurality of oligonucleotides of the cellular component binding reagents, the plurality of nucleic acid target molecules and the plurality of oligonucleotides of the cellular component binding reagents can hybridize to the oligonucleotide probes. The oligonucleotide probes can be contacted at a non-depletable ratio such that each distinct nucleic acid target molecules and oligonucleotides of the cellular component binding reagents can associate with oligonucleotide probes having different barcode sequences (e.g., molecular labels) of the disclosure.

In some embodiments, the methods disclosed herein provide detaching the oligonucleotides from the cellular component binding reagents that are specifically bound to the cellular component targets. Detachment can be performed in a variety of ways to separate the chemical group from the cellular component binding reagent, such as UV photocleaving, chemical treatment (e.g., dithiothreitol treatment), heating, enzyme treatment, or any combination thereof. Detaching the oligonucleotide from the cellular component binding reagent can be performed either before, after, or during the step of hybridizing the plurality of oligonucleotide probes to the plurality of nucleic acid target molecules and the plurality of oligonucleotides of the compositions.

### Simultaneous Quantitative Analysis of Protein and Nucleic Acid Targets

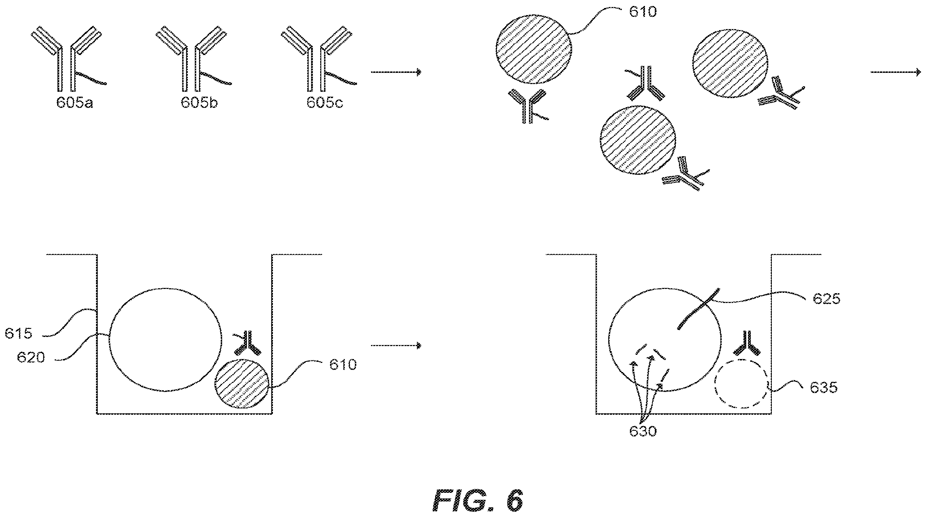

The methods disclosed herein also can be used for simultaneous quantitative analysis of multiple types of target molecules, for example protein and nucleic acid targets. For example, the target molecules can be, or comprise, cellular components. FIG. 6 shows a schematic illustration of an exemplary method of simultaneous quantitative analysis of both nucleic acid targets and other cellular component targets (e.g., proteins) in single cells. In some embodiments, a plurality of compositions 605, 605b, 605c, etc., each comprising a cellular component binding reagent, such as an antibody, is provided. Different cellular component binding reagents, such as antibodies, which bind to different cellular component targets are conjugated with different unique identifiers. Next, the cellular component binding reagents can be incubated with a sample containing a plurality of cells 610. The different cellular component binding reagents can specifically bind to cellular components on the cell surface, such as a cell marker, a B-cell receptor, a T-cell receptor, an antibody, a major histocompatibility complex, a tumor antigen, a receptor, or any combination thereof. Unbound cellular component binding reagents can be removed, e.g., by washing the cells with a buffer. The cells with the cellular component binding reagents can be then separated into a plurality of compartments, such as a microwell array, wherein a single compartment 615 is sized to fit a single cell and a single bead 620. Each bead can comprise a plurality of oligonucleotide probes, which can comprise a cell label that is common to all oligonucleotide probes on a bead, and barcode sequences (e.g., molecular label sequences). In some embodiments, each oligonucleotide probe can comprise a target binding region, for example, a poly(dT) sequence. The oligonucleotides 625 conjugated to the cellular component binding reagent can be detached from the cellular component binding reagent using chemical, optical or other means. The cell can be lysed 635 to release nucleic acids within the cell, such as genomic DNA or cellular mRNA 630. Cellular mRNA 630, oligonucleotides 625 or both can be captured by the oligonucleotide probes on bead 620, for example, by hybridizing to the poly(dT) sequence. A reverse transcriptase can be used to extend the oligonucleotide probes hybridized to the cellular mRNA 630 and the oligonucleotides 625 using the cellular mRNA 630 and the oligonucleotides 625 as templates. The extension products produced by the reverse transcriptase can be subject to amplification and sequencing. Sequencing reads can be subject to demultiplexing of sequences or identifies of cell labels, barcodes (e.g., molecular labels), genes, cellular component binding reagent specific oligonucleotides (e.g., antibody specific oligonucleotides), which can give rise to a digital representation of cellular components and gene expression of each single cell in the sample.

### Association of Barcodes

The oligonucleotides associated with the cellular component binding reagents (e.g., antigen binding reagents or protein binding reagents) and/or the nucleic acid molecules may randomly associate with the oligonucleotide probes (e.g., barcodes, such as stochastic barcodes). The oligonucleotides associated with the cellular component binding reagents, referred to herein as binding reagent oligonucleotides, can be, or comprise oligonucleotides of the disclosure, such as an antibody oligonucleotide, a sample indexing oligonucleotide, a cell identification oligonucleotide, a control particle oligonucleotide, a control oligonucleotide, and an interaction determination oligonucleotide. Association can, for example, comprise hybridization of an oligonucleotide probe's target binding region to a complementary portion of the target nucleic acid molecule and/or the oligonucleotides of the protein binding reagents. For example, a oligo(dT) region of a barcode (e.g., a stochastic barcode) can interact with a poly(A) tail of a target nucleic acid molecule and/or a poly(A) tail of an oligonucleotide of a protein binding reagent. The assay conditions used for hybridization (e.g., buffer pH, ionic strength, temperature, etc.) can be chosen to promote formation of specific, stable hybrids.

The disclosure provides for methods of associating a molecular label with a target nucleic acid and/or an oligonucleotide associated with a cellular component binding reagent using reverse transcription. As a reverse transcriptase can use both RNA and DNA as template. For example, the oligonucleotide originally conjugated on the cellular component binding reagent can be either RNA or DNA bases, or both. A binding reagent oligonucleotide can be copied and linked (e.g., covalently linked) to a cell label and a barcode sequence (e.g., a molecular label) in addition to the sequence, or a portion thereof, of the binding reagent sequence. As another example, an mRNA molecule can be copied and linked (e.g., covalently linked) to a cell label and a barcode sequence (e.g., a molecular label) in addition to the sequence of the mRNA molecule, or a portion thereof.

In some embodiments, molecular labels can be added by ligation of an oligonucleotide probe target binding region and a portion of the target nucleic acid molecule and/or the oligonucleotides associated with (e.g., currently, or previously, associated with) with cellular component binding reagents. For example, the target binding region may comprise a nucleic acid sequence that can be capable of specific hybridization to a restriction site overhang (e.g., an EcoRI sticky-end overhang). The methods can further comprise treating the target nucleic acids and/or the oligonucleotides associated with cellular component binding reagents with a restriction enzyme (e.g., EcoRI) to create a restriction site overhang. A ligase (e.g., T4 DNA ligase) may be used to join the two fragments.

### Determining the Number or Presence of Unique Molecular Label Sequences

The methods disclosed herein can comprise determining the number or presence of unique molecular label sequences for each unique identifier, each nucleic acid target molecule, and/or each binding reagent oligonucleotides (e.g., antibody oligonucleotides). For example, the sequencing reads can be used to determine the number of unique molecular label sequences for each unique identifier, each nucleic acid target molecule, and/or each binding reagent oligonucleotide. As another example, the sequencing reads can be used to determine the presence or absence of a molecular label sequence (e.g., a molecular label sequence associated with a target, a binding reagent oligonucleotide, an intracellular target-binding reagent specific oligonucleotide, a cell surface target-binding reagent specific oligonucleotide, and/or a nuclear target-binding reagent specific oligonucleotide, an antibody oligonucleotide, a sample indexing oligonucleotide, a cell identification oligonucleotide, a control particle oligonucleotide, a control oligonucleotide, and an interaction determination oligonucleotide, e.g., in the sequencing reads).

In some embodiments, the number of unique molecular label sequences for each unique identifier, each nucleic acid target molecule, and/or each binding reagent oligonucleotide indicates the quantity of each cellular component target (e.g., an antigen target, a protein target, a cell surface target, an intracellular target, a nuclear target) and/or each nucleic acid target molecule in the sample. In some embodiments, the quantity of a cellular component target and the quantity of its corresponding nucleic acid target molecules, e.g., mRNA molecules, can be compared to each other. In some embodiments, the ratio of the quantity of a cellular component target and the quantity of its corresponding nucleic acid target molecules, e.g., mRNA molecules, can be calculated. The cellular component targets can be, for example, cell surface protein markers. In some embodiments, the ratio between the protein level of a cell surface protein marker and the level of the mRNA of the cell surface protein marker is low.

The methods disclosed herein can be used for a variety of applications. For example, the methods disclosed herein can be used for proteome and/or transcriptome analysis of a sample. In some embodiments, the methods disclosed herein can be used to identify a cellular component target and/or a nucleic acid target, i.e., a biomarker, in a sample. In some embodiments, the cellular component target and the nucleic acid target correspond to each other, i.e., the nucleic acid target encodes the cellular component target. In some embodiments, the methods disclosed herein can be used to identify cellular component targets that have a desired ratio between the quantity of the cellular component target and the quantity of its corresponding nucleic acid target molecule in a sample, e.g., mRNA molecule. In some embodiments, the ratio is, or is about, 0.001, 0.01, 0.1, 1, 10, 100, 1000, or a number or a range between any two of the above values. In some embodiments, the ratio is at least, or is at most, 0.001, 0.01, 0.1, 1, 10, 100, or 1000. In some embodiments, the methods disclosed herein can be used to identify cellular component targets in a sample that the quantity of its corresponding nucleic acid target molecule in the sample is, or is about, 1000, 100, 10, 5, 2 1, 0, or a number or a range between any two of these values. In some embodiments, the methods disclosed herein can be used to identify cellular component targets in a sample that the quantity of its corresponding nucleic acid target molecule in the sample is more than, or less than, 1000, 100, 10, 5, 2 1, or 0.

### Compositions and Kits (are not part of the invention)

Some embodiments disclosed herein provide kits and compositions for simultaneous quantitative analysis of a plurality of cellular components (e.g., proteins, cell surface targets, an intracellular target, a nuclear targets) and/or a plurality of nucleic acid target molecules in a sample. The kits and compositions can, in some embodiments, comprise a plurality of cellular component binding reagents (e.g., a plurality of protein binding reagents) each conjugated with an oligonucleotide, wherein the oligonucleotide comprises a unique identifier for the cellular component binding reagent, and a plurality of oligonucleotide probes, wherein each of the plurality of oligonucleotide probes comprises a target binding region, a barcode sequence (e.g., a molecular label sequence), wherein the barcode sequence is from a diverse set of unique barcode sequences. In some embodiments, each of the oligonucleotides can comprise a molecular label, a cell label, a sample label, or any combination thereof. In some embodiments, each of the oligonucleotides can comprise a linker. In some embodiments, each of the oligonucleotides can comprise a binding site for an oligonucleotide probe, such as a poly(A) tail. For example, the poly(A) tail can be, e.g., oligodA₁₈ (unanchored to a solid support) or oligoA₁₈V (anchored to a solid support). The oligonucleotides can comprise DNA residues, RNA residues, or both.

Disclosed herein include a plurality of sample indexing compositions. Each of the plurality of sample indexing compositions can comprise two or more cellular component binding reagents. Each of the two or more cellular component binding reagents can be associated with a sample indexing oligonucleotide. At least one of the two or more cellular component binding reagents can be capable of specifically binding to at least one cellular component target. The sample indexing oligonucleotide can comprise a sample indexing sequence for identifying sample origin of one or more cells of a sample. Sample indexing sequences of at least two sample indexing compositions of the plurality of sample indexing compositions can comprise different sequences.

Disclosed herein include methods and kits for sample indexing. In some embodiments, each of two sample indexing compositions comprises a cellular component binding reagent (e.g., a protein binding reagent) associated with a sample indexing oligonucleotide, wherein the cellular component binding reagent is capable of specifically binding to at least one of one or more cellu.ar component targets (e.g., one or more protein targets), wherein the sample indexing oligonucleotide comprises a sample indexing sequence, and wherein sample indexing sequences of the two sample indexing compositions comprise different sequences. In some embodiments, the sample indexing oligonucleotide comprises a molecular label sequence, a binding site for a universal primer, or a combination thereof.

The unique identifiers (or oligonucleotides associated with cellular component binding reagents, such as binding reagent oligonucleotides, intracellular target-binding reagent specific oligonucleotides, cell surface target-binding reagent specific oligonucleotides, nuclear target-binding reagent specific oligonucleotides, antibody oligonucleotides, sample indexing oligonucleotides, cell identification oligonucleotides, control particle oligonucleotides, control oligonucleotides, or interaction determination oligonucleotides) can have any suitable length, for example, from about 25 nucleotides to about 45 nucleotides long. In some embodiments, the unique identifier can have a length that is, is about, is less than, is greater than, 4, 5, 6, 7, 8, 9, 10, 15, 20, 25, 30, 35, 40, 45, 50, 55, 60, 70, 80, 90, 100, 200 nucleotides, or a range that is between any two of the above values.

In some embodiments, the unique identifiers are selected from a diverse set of unique identifiers. The diverse set of unique identifiers can comprise, comprise about, comprise at least, or comprise at most, 20, 30, 40, 50, 60, 70, 80, 90, 100, 200, 300, 400, 500, 600, 700, 800, 900, 1000, 2000, 5000, or a number or a range between any two of these values, different unique identifiers. In some embodiments, the set of unique identifiers is designed to have minimal sequence homology to the DNA or RNA sequences of the sample to be analyzed. In some embodiments, the sequences of the set of unique identifiers are different from each other, or the complement thereof, by, or by about, 1, 2, 3, 4, 5, 6, 7, 8, 9, 10 nucleotides, or a number or a range between any two of these values. In some embodiments, the sequences of the set of unique identifiers are different from each other, or the complement thereof, by at least, or by at most, 1, 2, 3, 4, 5, 6, 7, 8, 9, or 10 nucleotides.

In some embodiments, the unique identifiers can comprise a binding site for a primer, such as universal primer. In some embodiments, the unique identifiers can comprise at least two binding sites for a primer, such as a universal primer. In some embodiments, the unique identifiers can comprise at least three binding sites for a primer, such as a universal primer. The primers can be used for amplification of the unique identifiers, for example, by PCR amplification. In some embodiments, the primers can be used for nested PCR reactions.

Any suitable cellular component binding reagents are contemplated in this disclosure, such as any protein binding reagents (e.g., antibodies or fragments thereof, aptamers, small molecules, ligands, peptides, oligonucleotides, etc., or any combination thereof). In some embodiments, the cellular component binding reagents can be polyclonal antibodies, monoclonal antibodies, recombinant antibodies, single-chain antibody (scAb), or fragments thereof, such as Fab and Fv. In some embodiments, the plurality of protein binding reagents can comprise, comprise about, comprise at least, or comprise at most, 20, 30, 40, 50, 60, 70, 80, 90, 100, 200, 300, 400, 500, 600, 700, 800, 900, 1000, 2000, 5000, or a number or a range between any two of these values, different protein binding reagents.

In some embodiments, the oligonucleotide is conjugated with the cellular component binding reagent through a linker. In some embodiments, the oligonucleotide can be conjugated with the protein binding reagent covalently. In some embodiment, the oligonucleotide can be conjugated with the protein binding reagent non-covalently. In some embodiments, the linker can comprise a chemical group that reversibly or irreversibly attached the oligonucleotide to the protein binding reagents. The chemical group can be conjugated to the linker, for example, through an amine group. In some embodiments, the linker can comprise a chemical group that forms a stable bond with another chemical group conjugated to the protein binding reagent. For example, the chemical group can be a UV photocleavable group, a disulfide bond, a streptavidin, a biotin, an amine, etc. In some embodiments, the chemical group can be conjugated to the protein binding reagent through a primary amine on an amino acid, such as lysine, or the N-terminus. The oligonucleotide can be conjugated to any suitable site of the protein binding reagent, as long as it does not interfere with the specific binding between the protein binding reagent and its protein target. In embodiments where the protein binding reagent is an antibody, the oligonucleotide can be conjugated to the antibody anywhere other than the antigen-binding site, for example, the Fc region, C_{H}1 domain, C_{H}2 domain, C_{H}3 domain, C_{L} domain. In some embodiments, each protein binding reagent can be conjugated with a single oligonucleotide molecule. In some embodiments, each protein binding reagent can be conjugated with, or with about, 2, 3, 4, 5, 10, 20, 30, 40, 50, 100, 1000, or a number or a range between any two of these values, oligonucleotide molecules, wherein each of the oligonucleotide molecule comprises the same unique identifier. In some embodiments, each protein binding reagent can be conjugated with more than one oligonucleotide molecule, for example, at least, or at most, 2, 3, 4, 5, 10, 20, 30, 40, 50, 100, or 1000, oligonucleotide molecules, wherein each of the oligonucleotide molecule comprises the same unique identifier.

In some embodiments, the plurality of cellular component binding reagents (e.g., protein binding reagents) are capable of specifically binding to a plurality of cellular component targets (e.g., protein targets) in a sample. The sample can be, comprise, can be obtained from, or can be derived from, a single cell, a plurality of cells, a tissue sample, a tumor sample, a blood sample, or the like. In some embodiments, the plurality of cellular component targets comprises a cell-surface protein, a cell marker, a B-cell receptor, a T-cell receptor, an antibody, a major histocompatibility complex, a tumor antigen, a receptor, or any combination thereof. In some embodiments, the plurality of cellular component targets can comprise intracellular proteins. In some embodiments, the plurality of cellular component targets can comprise intracellular proteins. In some embodiments, the plurality of cellular component targets can be, be about, be at least, or be at most, 1%, 2%, 3%, 4%, 5%, 6%, 7%, 8%, 9%, 10%, 20%, 30%, 40%, 50%, 60%, 70%, 80%, 90%, 95%, 98%, 99%, or a number or a range between any two of these values of all cellular component targets (e.g., proteins expressed or could be expressed) in an organism. In some embodiments, the plurality of cellular component targets can comprise, comprise about, comprise at least, or comprise at most, 2, 3, 4, 5, 10, 20, 30, 40, 50, 100, 1000, 10000, or a number or a range between any two of these values, different cellular component targets.

### Sample Indexing Using Oligonucleotide-Conjugated Cellular Component Binding Reagent

Disclosed herein include methods for sample identification. In some embodiments, the method comprises: contacting one or more cells from each of a plurality of samples with a sample indexing composition of a plurality of sample indexing compositions, wherein each of the one or more cells comprises one or more cellular component targets, wherein each of the plurality of sample indexing compositions comprises a cellular component binding reagent associated with a sample indexing oligonucleotide, wherein the cellular component binding reagent is capable of specifically binding to at least one of the one or more cellular component targets, wherein the sample indexing oligonucleotide comprises a sample indexing sequence, and wherein sample indexing sequences of at least two sample indexing compositions of the plurality of sample indexing compositions comprise different sequences; removing unbound sample indexing compositions of the plurality of sample indexing compositions; barcoding (e.g., stochastically barcoding) the sample indexing oligonucleotides using a plurality of barcodes (e.g., stochastic barcodes) to create a plurality of barcoded sample indexing oligonucleotides; obtaining sequencing data of the plurality of barcoded sample indexing oligonucleotides; and identifying sample origin of at least one cell of the one or more cells based on the sample indexing sequence of at least one barcoded sample indexing oligonucleotide of the plurality of barcoded sample indexing oligonucleotides.

In some embodiments, barcoding the sample indexing oligonucleotides using the plurality of barcodes comprises: contacting the plurality of barcodes with the sample indexing oligonucleotides to generate barcodes hybridized to the sample indexing oligonucleotides; and extending the barcodes hybridized to the sample indexing oligonucleotides to generate the plurality of barcoded sample indexing oligonucleotides. Extending the barcodes can comprise extending the barcodes using a DNA polymerase to generate the plurality of barcoded sample indexing oligonucleotides. Extending the barcodes can comprise extending the barcodes using a reverse transcriptase to generate the plurality of barcoded sample indexing oligonucleotides.

An oligonucleotide-conjugated with an antibody, an oligonucleotide for conjugation with an antibody, or an oligonucleotide previously conjugated with an antibody is referred to herein as an antibody oligonucleotide ("AbOligo"). Antibody oligonucleotides in the context of sample indexing are referred to herein as sample indexing oligonucleotides. An antibody conjugated with an antibody oligonucleotide is referred to herein as a hot antibody or an oligonucleotide antibody. An antibody not conjugated with an antibody oligonucleotide is referred to herein as a cold antibody or an oligonucleotide free antibody. An oligonucleotide-conjugated with a binding reagent (e.g., a protein binding reagent), an oligonucleotide for conjugation with a binding reagent, or an oligonucleotide previously conjugated with a binding reagent is referred to herein as a reagent oligonucleotide. Reagent oligonucleotides in the context of sample indexing are referred to herein as sample indexing oligonucleotides. A binding reagent conjugated with an antibody oligonucleotide is referred to herein as a hot binding reagent or an oligonucleotide binding reagent. A binding reagent not conjugated with an antibody oligonucleotide is referred to herein as a cold binding reagent or an oligonucleotide free binding reagent.

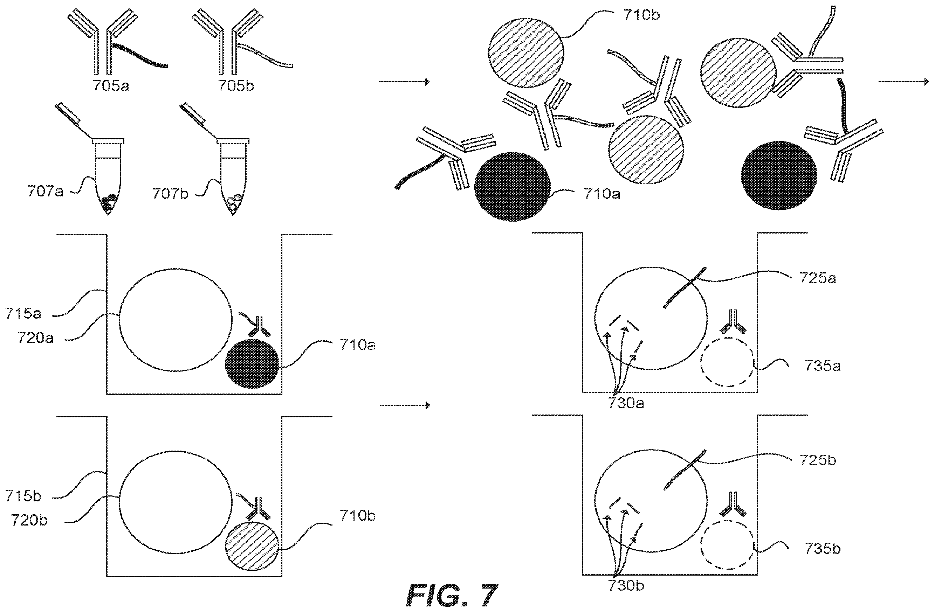

FIG. 7 shows a schematic illustration of an exemplary workflow using oligonucleotide-associated cellular component binding reagents for sample indexing. In some embodiments, a plurality of compositions 705a, 705b, etc., each comprising a binding reagent is provided. The binding reagent can be a protein binding reagent, such as an antibody. The cellular component binding reagent can comprise an antibody, a tetramer, an aptamer, a protein scaffold, or a combination thereof. The binding reagents of the plurality of compositions 705a, 705b can bind to an identical cellular component target. For example, the binding reagents of the plurality of compositions 705, 705b can be identical (except for the sample indexing oligonucleotides associated with the binding reagents).

Different compositions can include binding reagents conjugated with sample indexing oligonucleotides with different sample indexing sequences. The number of different compositions can be different in different implementations. In some embodiments, the number of different compositions can be, be about, be at least, or be at most, 1, 2, 3, 4, 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, or a number or a range between any two of these values.

In some embodiments, the sample indexing oligonucleotides of binding reagents in one composition can include an identical sample indexing sequence. The sample indexing oligonucleotides of binding reagents in one composition may not be identical. In some embodiments, the percentage of sample indexing oligonucleotides of binding reagents in one composition with an identical sample indexing sequence can be, be about, at least, or be at most, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.9%, or a number or a range between any two of these values.

The compositions 705a and 705b can be used to label samples of different samples. For example, the sample indexing oligonucleotides of the cellular component binding reagent in the composition 705a can have one sample indexing sequence and can be used to label cells 710a, shown as black circles, in a sample 707a, such as a sample of a patient. The sample indexing oligonucleotides of the cellular component binding reagents in the composition 705b can have another sample indexing sequence and can be used to label cells 710b, shown as hatched circles, in a sample 707b, such as a sample of another patient or another sample of the same patient. The cellular component binding reagents can specifically bind to cellular component targets or proteins on the cell surface, such as a cell marker, a B-cell receptor, a T-cell receptor, an antibody, a major histocompatibility complex, a tumor antigen, a receptor, or any combination thereof. Unbound cellular component binding reagents can be removed, e.g., by washing the cells with a buffer.

The cells with the cellular component binding reagents can be then separated into a plurality of compartments, such as a microwell array, wherein a single compartment 715a, 715b is sized to fit a single cell 710a and a single bead 720a or a single cell 710b and a single bead 720b. Each bead 720a, 720b can comprise a plurality of oligonucleotide probes, which can comprise a cell label that is common to all oligonucleotide probes on a bead, and molecular label sequences. In some embodiments, each oligonucleotide probe can comprise a target binding region, for example, a poly(dT) sequence. The sample indexing oligonucleotides 725a conjugated to the cellular component binding reagent of the composition 705a can be configured to be (or can be) detachable or non-detachable from the cellular component binding reagent. The sample indexing oligonucleotides 725a conjugated to the cellular component binding reagent of the composition 705a can be detached from the cellular component binding reagent using chemical, optical or other means. The sample indexing oligonucleotides 725b conjugated to the cellular component binding reagent of the composition 705b can be configured to be (or can be) detachable or non-detachable from the cellular component binding reagent. The sample indexing oligonucleotides 725b conjugated to the cellular component binding reagent of the composition 705b can be detached from the cellular component binding reagent using chemical, optical or other means.

The cell 710a can be lysed to release nucleic acids within the cell 710a, such as genomic DNA or cellular mRNA 730a. The lysed cell 735a is shown as a dotted circle. Cellular mRNA 730a, sample indexing oligonucleotides 725a, or both can be captured by the oligonucleotide probes on bead 720a, for example, by hybridizing to the poly(dT) sequence. A reverse transcriptase can be used to extend the oligonucleotide probes hybridized to the cellular mRNA 730a and the oligonucleotides 725a using the cellular mRNA 730a and the oligonucleotides 725a as templates. The extension products produced by the reverse transcriptase can be subject to amplification and sequencing.

Similarly, the cell 710b can be lysed to release nucleic acids within the cell 710b, such as genomic DNA or cellular mRNA 730b. The lysed cell 735b is shown as a dotted circle. Cellular mRNA 730b, sample indexing oligonucleotides 725b, or both can be captured by the oligonucleotide probes on bead 720b, for example, by hybridizing to the poly(dT) sequence. A reverse transcriptase can be used to extend the oligonucleotide probes hybridized to the cellular mRNA 730b and the oligonucleotides 725b using the cellular mRNA 730b and the oligonucleotides 725b as templates. The extension products produced by the reverse transcriptase can be subject to amplification and sequencing.

Sequencing reads can be subject to demultiplexing of cell labels, molecular labels, gene identities, and sample identities (e.g., in terms of sample indexing sequences of sample indexing oligonucleotides 725a and 725b). Demultiplexing of cell labels, molecular labels, and gene identities can give rise to a digital representation of gene expression of each single cell in the sample. Demultiplexing of cell labels, molecular labels, and sample identities, using sample indexing sequences of sample indexing oligonucleotides, can be used to determine a sample origin.

Cellular component binding reagents against cellular component binding reagents on the cell surface can be conjugated to a library of unique sample indexing oligonucleotides to allow cells to retain sample identity. For example, antibodies against cell surface markers can be conjugated to a library of unique sample indexing oligonucleotides to allow cells to retain sample identity. This will enable multiple samples to be loaded onto the same Rhapsody^{™} cartridge as information pertaining sample source is retained throughout library preparation and sequencing. Sample indexing can allow multiple samples to be run together in a single experiment, simplifying and shortening experiment time, and eliminating batch effect.

Disclosed herein include methods for sample identification. In some embodiments, the method comprise: contacting one or more cells from each of a plurality of samples with a sample indexing composition of a plurality of sample indexing compositions, wherein each of the one or more cells comprises one or more cellular component targets, wherein each of the plurality of sample indexing compositions comprises a cellular component binding reagent associated with a sample indexing oligonucleotide, wherein the cellular component binding reagent is capable of specifically binding to at least one of the one or more cellular component targets, wherein the sample indexing oligonucleotide comprises a sample indexing sequence, and wherein sample indexing sequences of at least two sample indexing compositions of the plurality of sample indexing compositions comprise different sequences; removing unbound sample indexing compositions of the plurality of sample indexing compositions. The method can include barcoding (e.g., stochastically barcoding) the sample indexing oligonucleotides using a plurality of barcodes (e.g., stochastic barcodes) to create a plurality of barcoded sample indexing oligonucleotides; obtaining sequencing data of the plurality of barcoded sample indexing oligonucleotides; and identifying sample origin of at least one cell of the one or more cells based on the sample indexing sequence of at least one barcoded sample indexing oligonucleotide of the plurality of barcoded sample indexing oligonucleotides.

In some embodiments, the method for sample identification comprises: contacting one or more cells from each of a plurality of samples with a sample indexing composition of a plurality of sample indexing compositions, wherein each of the one or more cells comprises one or more cellular component targets, wherein each of the plurality of sample indexing compositions comprises a cellular component binding reagent associated with a sample indexing oligonucleotide, wherein the cellular component binding reagent is capable of specifically binding to at least one of the one or more cellular component targets, wherein the sample indexing oligonucleotide comprises a sample indexing sequence, and wherein sample indexing sequences of at least two sample indexing compositions of the plurality of sample indexing compositions comprise different sequences; removing unbound sample indexing compositions of the plurality of sample indexing compositions; and identifying sample origin of at least one cell of the one or more cells based on the sample indexing sequence of at least one sample indexing oligonucleotide of the plurality of sample indexing compositions.

In some embodiments, identifying the sample origin of the at least one cell comprises: barcoding (e.g., stochastically barcoding) sample indexing oligonucleotides of the plurality of sample indexing compositions using a plurality of barcodes (e.g., stochastic barcodes) to create a plurality of barcoded sample indexing oligonucleotides; obtaining sequencing data of the plurality of barcoded sample indexing oligonucleotides; and identifying the sample origin of the cell based on the sample indexing sequence of at least one barcoded sample indexing oligonucleotide of the plurality of barcoded sample indexing oligonucleotides. In some embodiments, barcoding the sample indexing oligonucleotides using the plurality of barcodes to create the plurality of barcoded sample indexing oligonucleotides comprises stochastically barcoding the sample indexing oligonucleotides using a plurality of stochastic barcodes to create a plurality of stochastically barcoded sample indexing oligonucleotides.

In some embodiments, identifying the sample origin of the at least one cell can comprise identifying the presence or absence of the sample indexing sequence of at least one sample indexing oligonucleotide of the plurality of sample indexing compositions. Identifying the presence or absence of the sample indexing sequence can comprise: replicating the at least one sample indexing oligonucleotide to generate a plurality of replicated sample indexing oligonucleotides; obtaining sequencing data of the plurality of replicated sample indexing oligonucleotides; and identifying the sample origin of the cell based on the sample indexing sequence of a replicated sample indexing oligonucleotide of the plurality of sample indexing oligonucleotides that correspond to the least one barcoded sample indexing oligonucleotide in the sequencing data.

In some embodiments, replicating the at least one sample indexing oligonucleotide to generate the plurality of replicated sample indexing oligonucleotides comprises: prior to replicating the at least one barcoded sample indexing oligonucleotide, ligating a replicating adaptor to the at least one barcoded sample indexing oligonucleotide. Replicating the at least one barcoded sample indexing oligonucleotide can comprise replicating the at least one barcoded sample indexing oligonucleotide using the replicating adaptor ligated to the at least one barcoded sample indexing oligonucleotide to generate the plurality of replicated sample indexing oligonucleotides.

In some embodiments, replicating the at least one sample indexing oligonucleotide to generate the plurality of replicated sample indexing oligonucleotides comprises: prior to replicating the at least one barcoded sample indexing oligonucleotide, contacting a capture probe with the at least one sample indexing oligonucleotide to generate a capture probe hybridized to the sample indexing oligonucleotide; and extending the capture probe hybridized to the sample indexing oligonucleotide to generate a sample indexing oligonucleotide associated with the capture probe. Replicating the at least one sample indexing oligonucleotide can comprise replicating the sample indexing oligonucleotide associated with the capture probe to generate the plurality of replicated sample indexing oligonucleotides.

Also disclosed herein include methods for sample identification, which allows for example identifying cell overloading and multiplet. The method can comprise: contacting a first plurality of cells and a second plurality of cells with two sample indexing compositions respectively, wherein each of the first plurality of cells and each of the second plurality of cells comprise one or more cellular components, wherein each of the two sample indexing compositions comprises a cellular component binding reagent associated with a sample indexing oligonucleotide, wherein the cellular component binding reagent is capable of specifically binding to at least one of the one or more cellular components, wherein the sample indexing oligonucleotide comprises a sample indexing sequence, and wherein sample indexing sequences of the two sample indexing compositions comprise different sequences; barcoding the sample indexing oligonucleotides using a plurality of barcodes to create a plurality of barcoded sample indexing oligonucleotides, wherein each of the plurality of barcodes comprises a cell label sequence, a barcode sequence (e.g., a molecular label sequence), and a target-binding region, wherein the barcode sequences of at least two barcodes of the plurality of barcodes comprise different sequences, and wherein at least two barcodes of the plurality of barcodes comprise an identical cell label sequence; obtaining sequencing data of the plurality of barcoded sample indexing oligonucleotides; and identifying one or more cell label sequences that is each associated with two or more sample indexing sequences in the sequencing data obtained; and removing the sequencing data associated with the one or more cell label sequences that is each associated with two or more sample indexing sequences from the sequencing data obtained and/or excluding the sequencing data associated with the one or more cell label sequences that is each associated with two or more sample indexing sequences from subsequent analysis (e.g., single cell mRNA profiling, or whole transcriptome analysis). In some embodiments, the sample indexing oligonucleotide comprises a barcode sequence (e.g., a molecular label sequence), a binding site for a universal primer, or a combination thereof. The method for sample identification can be used in the method

For example, the method can be used to load 50000 or more cells (compared to 10000-20000 cells) using sample indexing. Sample indexing can use oligonucleotide-conjugated cellular component binding reagents (e.g., antibodies) or cellular component binding reagents against a cellular component (e.g., a universal protein marker) to label cells from different samples with a unique sample index. When two or more cells from different samples, two or more cells from different populations of cells of a sample, or two or more cells of a sample, are captured in the same microwell or droplet, the combined "cell" (or contents of the two or more cells) can be associated with sample indexing oligonucleotides with different sample indexing sequences (or cell identification oligonucleotides with different cell identification sequences). The number of different populations of cells can be different in different implementations. In some embodiments, the number of different populations can be, be about, at least, or be at most, 2, 3, 4, 5, 6, 7, 8, 9, 10, 20, 30, 40, 50, 60, 70, 80, 90, 100, or a number or a range between any two of these values. The number, or the average number, of cells in each population can be different in different implementations. In some embodiments, the number, or the average number, of cells in each population can be, be about, at least, or be at most, 1, 2, 3, 4, 5, 6, 7, 8, 9, 10, 20, 30, 40, 50, 60, 70, 80, 90, 100, or a number or a range between any two of these values. When the number, or the average number, of cells in each population is sufficiently small (e.g., equal to, or fewer than, 50, 25, 10, 5, 4, 3, 2, or 1 cells per population), the sample indexing composition for cell overloading and multiplet identification can be referred to as cell identification compositions.

### Oligonucleotide-Conjugated Antibodies

### Unique molecular label sequence

The oligonucleotide associated with a cellular component-binding reagent (e.g., antibody oligonucleotide ("AbOligo" or "AbO"), binding reagent oligonucleotide, cellular component-binding reagent specific oligonucleotides, sample indexing oligonucleotides) can comprises a unique molecular label sequence (also referred to as a molecular index (MI), "molecular barcode," or Unique Molecular Identifier (UMI)). A cellular component binding reagent can comprise an intracellular target-binding reagent, a cell surface target-binding reagent, and/or a nuclear target-binding reagent. A binding reagent oligonucleotide can comprise an intracellular target-binding reagent specific oligonucleotide, a cell surface target-binding reagent specific oligonucleotide, and/or a nuclear target-binding reagent specific oligonucleotide. In some embodiments, binding reagent oligonucleotide species comprising molecule barcodes as described herein reduce bias by increasing sensitivity, decreasing relative standard error, or increasing sensitivity and/or reducing standard error. The molecule barcode can comprise a unique sequence, so that when multiple sample nucleic acids (which can be the same and/or different from each other) are associated one-to-one with molecule barcodes, different sample nucleic acids can be differentiated from each other by the molecule barcodes. As such, even if a sample comprises two nucleic acids having the same sequence, each of these two nucleic acids can be labeled with a different molecule barcode, so that nucleic acids in the population can be quantified, even after amplification. The molecule barcode can comprise a nucleic acid sequence of at least 5 nucleotides, for example at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides, including a number or a range between any two of these values, nucleotides. In some embodiments, the nucleic acid sequence of the molecule barcode comprises a unique sequence, for example, so that each unique oligonucleotide species in a composition comprises a different molecule barcode. In some embodiments, two or more unique oligonucleotide species can comprise the same molecule barcode, but still differ from each other. For example, if the unique oligonucleotide species include sample barcodes, each unique oligonucleotide species with a particular sample barcode can comprise a different molecule barcode. In some embodiments, a composition comprising unique oligonucleotide species comprises a molecule barcode diversity of at least 1000 different molecule barcodes, and thus at least 1000 unique oligonucleotide species. In some embodiments, a composition comprising unique oligonucleotide species comprises a molecule barcode diversity of at least 6,500 different molecule barcodes, and thus at least 6,500 unique oligonucleotide species. In some embodiments, a composition comprising unique oligonucleotide species comprises a molecule barcode diversity of at least 65,000 different molecule barcodes, and thus at least 65,000 unique oligonucleotide species.

The unique molecular label sequence can be positioned 5' of the unique identifier sequence without any intervening sequences between the unique molecular label sequence and the unique identifier sequence. In some embodiments, the unique molecular label sequence is positioned 5' of a spacer, which is positioned 5' of the unique identifier sequence, so that a spacer is between the unique molecular label sequence and the unique identifier sequence. In some embodiments, the unique identifier sequence is positioned 5' of the unique molecular label sequence without any intervening sequences between the unique identifier sequence and the unique molecular label sequence. In some embodiments, the unique identifier sequence is positioned 5' of a spacer, which is positioned 5' of the unique molecular label sequence, so that a spacer is between the unique identifier sequence and the unique molecular label sequence.

The unique molecular label sequence can comprise a nucleic acid sequence of at least 2 nucleotides, for example at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 nucleotides, a number or a range between any two of these values, nucleotides.

In some embodiments, the unique molecular label sequence of the binding reagent oligonucleotide comprises the sequence of at least three repeats of the doublets "VN" and/or "NV" (in which each "V" is any of A, C, or G, and in which "N" is any of A, G, C, or T), for example at least 3, 4, 5, 6, 7, 8, 9, 10, 11, 12, 13, 14, 15, 16, 17, 18, 19, or 20 repeats, including ranges between any two of the listed values. Examples of multiple repeats of the doublet "VN" include VN, VNVN, VNVNVN, and VNVNVNVN. It is noted that while the formulas "VN" and "NV" describe constraints on the base content, not every V or every N has to be the same or different. For example, if the molecule barcodes of unique oligonucleotide species in a composition comprised VNVNVN, one molecule barcode can comprise the sequence ACGGCA, while another molecule barcode can comprise the sequence ATACAT, while another molecule barcode could comprise the sequence ATACAC. It is noted that any number of repeats of the doublet "VN" would have a T content of no more than 50%. In some embodiments, at least 95% of the unique oligonucleotide species of a composition comprising at least 1000 unique oligonucleotide species comprise molecule barcodes comprising at least three repeats of the doublets "VN" and/or "NV," for example at least 3, 4, 5, 6, 7, 8, 9, 10, 11, 12, 13, 14, 15, 16, 17, 18, 19, or 20 repeats, including ranges between any two of the listed values. In some embodiments, at least 99% of the unique oligonucleotide species of a composition comprising at least 1000 unique oligonucleotide species comprise molecule barcodes comprising at least three repeats of the doublets "VN" and/or "NV," for example at least 3, 4, 5, 6, 7, 8, 9, 10, 11, 12, 13, 14, 15, 16, 17, 18, 19, or 20 repeats, including ranges between any two of the listed values. In some embodiments, at least 99.9% of the unique oligonucleotide species of a composition comprising at least 1000 unique oligonucleotide species comprise molecule barcodes comprising at least three repeats of the doublets "VN" and/or "NV," for example at least 3, 4, 5, 6, 7, 8, 9, 10, 11, 12, 13, 14, 15, 16, 17, 18, 19, or 20 repeats, including ranges between any two of the listed values. In some embodiments, at least 95% of the unique oligonucleotide species of a composition comprising at least 6500 unique oligonucleotide species comprise molecule barcodes comprising at least three repeats of the doublets "VN" and/or "NV," for example at least 3, 4, 5, 6, 7, 8, 9, 10, 11, 12, 13, 14, 15, 16, 17, 18, 19, or 20 repeats, including ranges between any two of the listed values. In some embodiments, at least 99% of the unique oligonucleotide species of a composition comprising at least 6500 unique oligonucleotide species comprise molecule barcodes comprising at least three repeats of the doublets "VN" and/or "NV," for example at least 3, 4, 5, 6, 7, 8, 9, 10, 11, 12, 13, 14, 15, 16, 17, 18, 19, or 20 repeats, including ranges between any two of the listed values. In some embodiments, at least 99.9% of the unique oligonucleotide species of a composition comprising at least 6500 unique oligonucleotide species comprise molecule barcodes comprising at least three repeats of the doublets "VN" and/or "NV," for example at least 3, 4, 5, 6, 7, 8, 9, 10, 11, 12, 13, 14, 15, 16, 17, 18, 19, or 20 repeats, including ranges between any two of the listed values. In some embodiments, at least 95% of the unique oligonucleotide species of a composition comprising at least 65,000 unique oligonucleotide species comprise molecule barcodes comprising at least three repeats of the doublets "VN" and/or "NV," for example at least 3, 4, 5, 6, 7, 8, 9, 10, 11, 12, 13, 14, 15, 16, 17, 18, 19, or 20 repeats, including ranges between any two of the listed values. In some embodiments, at least 99% of the unique oligonucleotide species of a of composition comprising at least 65,000 unique oligonucleotide species comprise molecule barcodes comprising at least three repeats of the doublets "VN" and/or "NV," for example at least 3, 4, 5, 6, 7, 8, 9, 10, 11, 12, 13, 14, 15, 16, 17, 18, 19, or 20 repeats, including ranges between any two of the listed values. In some embodiments, at least 99.9% of the unique oligonucleotide species of a composition comprising at least 65,000 unique oligonucleotide species comprise molecule barcodes comprising at least three repeats of the doublets "VN" and/or "NV," for example at least 3, 4, 5, 6, 7, 8, 9, 10, 11, 12, 13, 14, 15, 16, 17, 18, 19, or 20 repeats, including ranges between any two of the listed values. In some embodiments, the composition consists of or consists essentially of at least 1000, 6500, or 65,000 unique oligonucleotide species that each have a molecule barcode comprising the sequence VNVNVN. In some embodiments, the composition consists of or consists essentially of at least 1000, 6500, or 65,000 unique oligonucleotide species that each has a molecule barcode comprising the sequence VNVNVNVN. In some embodiments, at least 95%, 99%, or 99.9% of the barcode regions of the composition as described herein comprise at least three repeats of the doublets "VN" and/or "NV," as described herein. In some embodiments, unique molecular label sequences comprising repeated "doublets "VN" and/or "NV" can yield low bias, while providing a compromise between reducing bias and maintaining a relatively large quantity of available nucleotide sequences, so that relatively high diversity can be obtained in a relatively short sequence, while still minimizing bias. In some embodiments, unique molecular label sequences comprising repeated "doublets "VN" and/or "NV" can reduce bias by increasing sensitivity, decreasing relative standard error, or increasing sensitivity and reducing standard error. In some embodiments, unique molecular label sequences comprising repeated "doublets "VN" and/or "NV" improve informatics analysis by serving as a geomarker. In some embodiments, the repeated doublets "VN" and/or "NV" described herein reduce the incidence of homopolymers within the unique molecular label sequences. In some embodiments, the repeated doublets "VN" and/or "NV" described herein break up homopolymers.

In some embodiments, the sample indexing oligonucleotide comprises a first molecular label sequence. In some embodiments, the first molecular label sequences of at least two sample indexing oligonucleotides are different, and the sample indexing sequences of the at least two sample indexing oligonucleotides are identical. In some embodiments, the first molecular label sequences of at least two sample indexing oligonucleotides are different, and the sample indexing sequences of the at least two sample indexing oligonucleotides are different. In some embodiments, the cellular component-binding reagent specific oligonucleotide comprises a second molecular label sequence. In some embodiments, the second molecular label sequences of at least two cellular component-binding reagent specific oligonucleotides are different, and the unique identifier sequences of the at least two cellular component-binding reagent specific oligonucleotides are identical. In some embodiments, the second molecular label sequences of at least two cellular component-binding reagent specific oligonucleotides are different, and the unique identifier sequences of the at least two cellular component-binding reagent specific oligonucleotides are different. In some embodiments, the number of unique second molecular label sequences associated with the unique identifier sequence for the cellular component-binding reagent capable of specifically binding to the at least one cellular component target in the sequencing data indicates the number of copies of the at least one cellular component target in the one or more of the plurality of cells. In some embodiment, a combination (e.g., minimum, average, and maximum) of (1) the number of unique first molecular label sequences associated with the unique identifier sequence for the cellular component-binding reagent capable of specifically binding to the at least one cellular component target in the sequencing data and (2) the number of unique second molecular label sequences associated with the unique identifier sequence for the cellular component-binding reagent capable of specifically binding to the at least one cellular component target in the sequencing data indicates the number of copies of the at least one cellular component target in the one or more of the plurality of cells.

### Alignment sequence

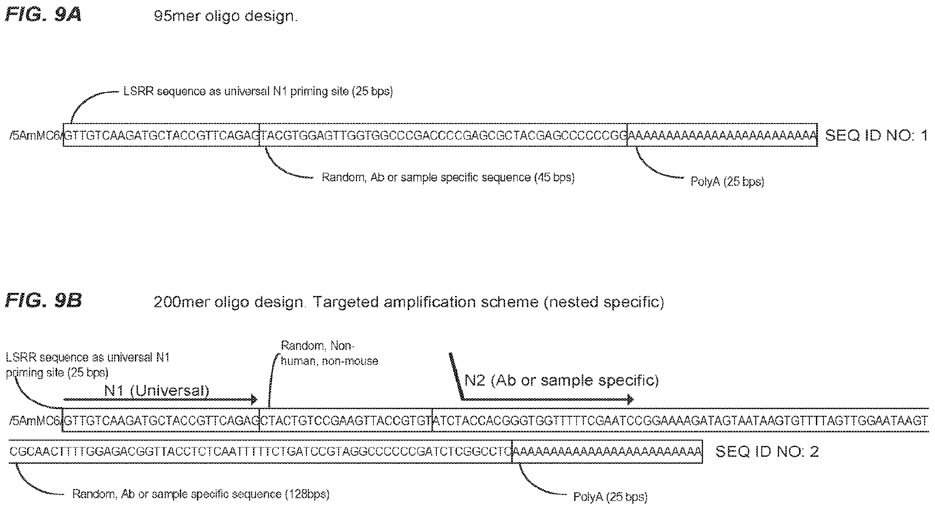

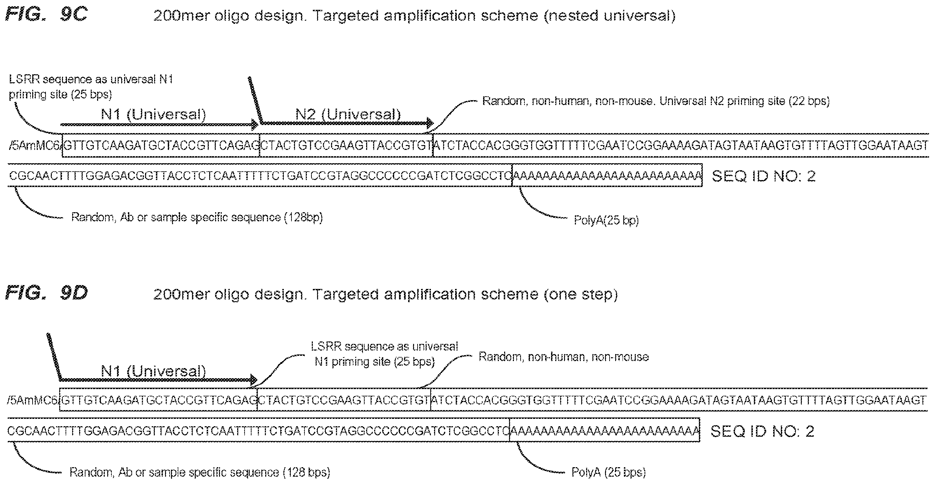

In some embodiments, the binding reagent oligonucleotide (e.g., intracellular target-binding reagent specific oligonucleotide, cell surface target-binding reagent specific oligonucleotide, nuclear target-binding reagent specific oligonucleotide) comprises an alignment sequence (e.g., the alignment sequence 825bb described with reference to FIG. 9) adjacent to the poly(dA) region. The alignment sequence can be 1 or more nucleotides in length. The alignment sequence can be 2 nucleotides in length. The alignment sequence can comprise a guanine, a cytosine, a thymine, a uracil, or a combination thereof. The alignment sequence can comprise a poly(dT) region, a poly(dG) region, a poly(dC) region, a poly(dU) region, or a combination thereof. In some embodiments, the alignment sequence is 5' to the poly(dA) region. Advantageously, in some embodiments, the presence of the alignment sequence enables the poly(A) tail of each of the binding reagent oligonucleotides to have the same length, leading to greater uniformity of performance. In some embodiments, the percentage of binding reagent oligonucleotides with an identical poly(dA) region length within a plurality of binding reagent oligonucleotides, each of which comprise an alignment sequence, can be, or be about, 80%, 90%, 91%, 93%, 95%, 97%, 99.9%, 99.9%, 99.99%, or 100%, or a number or a range between any two of these values. In some embodiments, the percentage of binding reagent oligonucleotides with an identical poly(dA) region length within the plurality of binding reagent oligonucleotides, each of which comprise an alignment sequence, can be at least, or be at most, 80%, 90%, 91%, 93%, 95%, 97%, 99.9%, 99.9%, 99.99%, or 100%.

The length of the alignment sequence can be different in different implementations. In some embodiments, the length of the alignment sequence can be, or can be about, be at least, or can be at most, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values. The number of guanine(s), cytosine(s), thymine(s), or uracil(s) in the alignment sequence can be different in different implementations. The number of guanine(s), cytosine(s), thymine(s), or uracil(s) can be, or can be about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values. The number of guanine(s), cytosine(s), thymine(s), or uracil(s) can be at least, or can be at most, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100. In some embodiments, the sample indexing oligonucleotide comprises an alignment sequence. In some embodiments, the cellular component-binding reagent specific oligonucleotide comprises an alignment sequence.

### Linker

The binding reagent oligonucleotide (e.g., intracellular target-binding reagent specific oligonucleotide, cell surface target-binding reagent specific oligonucleotide, nuclear target-binding reagent specific oligonucleotide) can be conjugated with the cellular component binding reagent (e.g., intracellular target-binding reagent, cell surface target-binding reagent, nuclear target-binding reagent) through various mechanisms. In some embodiments, the binding reagent oligonucleotide can be conjugated with the cellular component binding reagent covalently. In some embodiments, the binding reagent oligonucleotide can be conjugated with the cellular component binding reagent non-covalently. In some embodiments, the binding reagent oligonucleotide is conjugated with the cellular component binding reagent through a linker. In some embodiments, the binding reagent oligonucleotide can comprise the linker. The linker can comprise a chemical group. The chemical group can be reversibly, or irreversibly, attached to the molecule of the cellular component binding reagent. The chemical group can be selected from the group consisting of a UV photocleavable group, a disulfide bond, a streptavidin, a biotin, an amine, and any combination thereof. The linker can comprise a carbon chain. The carbon chain can comprise, for example, 5-50 carbon atoms. The carbon chain can have different numbers of carbon atoms in different embodiments. In some embodiments, the number of carbon atoms in the carbon chain can be, can be about, at least, or can be at most, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or a number or a range between any two of these values. In some embodiments, the carbon chain comprises 2-30 carbons. In some embodiments, the carbon chain comprises 12 carbons. In some embodiments, amino modifiers employed for binding reagent oligonucleotide can be conjugated to the cellular component binding reagent. In some embodiments, the linker comprises 5' amino modifier C6 (5AmMC6) or a derivative thereof. In some embodiments, the linker comprises 5' amino modifier C12 (5AmMC12), or a derivative thereof. In some embodiments, a longer linker achieves a higher efficiency of conjugation. In some embodiments, a longer linker achieves a higher efficiency of modification prior to conjugation. In some embodiments, increasing the distance between the functional amine and the DNA sequence yields a higher efficiency of conjugation. In some embodiments, increasing the distance between the functional amine and the DNA sequence yields a higher efficiency of modification prior to conjugation. In some embodiments, the use of 5AmMC12 as a linker yields a higher efficiency of modification (prior to conjugation) than the use of 5AmMC6 as a linker. In some embodiments the use of 5AmMC12 as a linker yields a higher efficiency of conjugation than the use of 5AmMC6 as a linker. In some embodiments, the sample indexing oligonucleotide is associated with the cellular component-binding reagent through a linker. In some embodiments, the cellular component-binding reagent specific oligonucleotide is associated with the cellular component-binding reagent through a linker.

### Antibody-specific barcode sequence

Disclosed herein, in several embodiments, are improvements to the design of the unique identifier sequence (e.g., antibody-specific barcode sequence) of a binding reagent oligonucleotide (e.g., intracellular target-binding reagent specific oligonucleotide, cell surface target-binding reagent specific oligonucleotide, nuclear target-binding reagent specific oligonucleotide). An intracellular target-binding reagent specific oligonucleotide can comprise a unique intracellular target identifier for the intracellular target-binding reagent specific oligonucleotide. A cell surface target-binding reagent specific oligonucleotide can comprise a unique cell surface target identifier for the cell surface target-binding reagent specific oligonucleotide. A nuclear target-binding reagent specific oligonucleotide can comprise a unique nuclear target identifier for the nuclear target-binding reagent specific oligonucleotide. In some embodiments the unique identifier sequence (e.g., sample indexing sequence, cellular component-binding reagent specific oligonucleotide) is designed to have a Hamming distance greater than 3. In some embodiments, the Hamming distance of the unique identifier sequence can be, or be about, 1, 2, 3, 4, 5, 6, 7, 8, 9, 10, or a number or a range between any two of these values. In some embodiments, the unique identifier sequences has a GC content in the range of 40% to 60% and does not have a predicted secondary structure (e.g., hairpin). In some embodiments, the unique identifier sequence does not comprise any sequences predicted *in silico* to bind to the mouse and/or human transcripts. In some embodiments, the unique identifier sequence does not comprise any sequences predicted *in silico* to bind to Rhapsody and/or SCMK system primers. In some embodiments, the unique identifier sequence does not comprise homopolymers.

### Primer Adapter

In some embodiments, the binding reagent oligonucleotide (e.g., intracellular target-binding reagent specific oligonucleotide, cell surface target-binding reagent specific oligonucleotide, nuclear target-binding reagent specific oligonucleotide) comprises a primer adapter. In some embodiments, the primer adapter comprises the sequence of a first universal primer, a complimentary sequence thereof, a partial sequence thereof, or a combination thereof. In some embodiments, the first universal primer comprises an amplification primer, a complimentary sequence thereof, a partial sequence thereof, or a combination thereof. In some embodiments, the first universal primer comprises a sequencing primer, a complimentary sequence thereof, a partial sequence thereof, or a combination thereof. In some embodiments, the sequencing primer comprises an Illumina sequencing primer. In some embodiments, the sequencing primer comprises a portion of an Illumina sequencing primer. In some embodiments, the sequencing primer comprises a P7 sequencing primer. In some embodiments, the sequencing primer comprises a portion of P7 sequencing primer. In some embodiments, the primer adapter comprises an adapter for Illumina P7. In some embodiments, the primer adapter comprises a partial adapter for Illumina P7. In some embodiments, the amplification primer is an Illumina P7 sequence or a subsequence thereof. In some embodiments, the sequencing primer is an Illumina R2 sequence or a subsequence thereof. In some embodiments, the first universal primer is 5-50 nucleotides in length. In some embodiments, The primer adapter can comprise a nucleic acid sequence of at least 5 nucleotides, for example at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides, a number or a range between any two of these values, nucleotides. The primer adapter can comprise a nucleic acid sequence of at least 5 nucleotides of the sequence of a first universal primer, an amplification primer, a sequencing primer, a complimentary sequence thereof, a partial sequence thereof, or a combination thereof, for example at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides, including a number or a range between any two of these values, nucleotides of the sequence of a first universal primer, an amplification primer, a sequencing primer, a complimentary sequence thereof, a partial sequence thereof, or a combination thereof.

A conventional amplification workflow for sequencing library preparation can employ three rounds of PCR, such as, for example: a first round ("PCR 1") employing a target-specific primer and a primer against the universal Illumina sequencing primer 1 sequence; a second round ("PCR 2") using a nested target-specific primer flanked by Illumina sequencing primer 2 sequence, and a primer against the universal Illumina sequencing primer 1 sequence; and a third round ("PCR 3") adding Illumina P5 and P7 and sample index. Advantageously, in some embodiments, the primer adapter disclosed herein enables a shorter and simpler workflow in library preparation as compared to if the starting template (e.g., a sample indexing oligonucleotide attached to a bead) does not have a primer adapter. In some embodiments, the primer adapter reduces pre-sequencing PCR amplification of a template by one round (as compared to if the template does not comprise a primer adapter). In some embodiments, the primer adapter reduces pre-sequencing PCR amplification of the template to one round (as compared to if the template does not comprise a primer adapter). In some embodiments, a template comprising the primer adapter does not require a PCR amplification step for attachment of Illumina sequencing adapters that would be required pre-sequencing if the template did not comprise a primer adapter. In some embodiments, the primer adapter sequence (or a subsequence thereof) is not part of the sequencing readout of a sequencing template comprising a primer adapter sequence and therefore does not affect read quality of a template comprising a primer adapter. A template comprising the primer adapter can have decreased sequencing diversity as compared to if the template does not comprise a primer adapter.

In some embodiments, the sample indexing oligonucleotide comprises a primer adapter. In some embodiments, replicating a sample indexing oligonucleotide, a barcoded sample indexing oligonucleotide, or a product thereof, comprises using a first universal primer, a first primer comprising the sequence of the first universal primer, or a combination thereof, to generate a plurality of replicated sample indexing oligonucleotides. In some embodiments, replicating a one sample indexing oligonucleotide, a barcoded sample indexing oligonucleotide, or a product thereof, comprises using a first universal primer, a first primer comprising the sequence of the first universal primer, a second universal primer, a second primer comprising the sequence of the second universal primer, or a combination thereof, to generate the plurality of replicated sample indexing oligonucleotides. In some embodiments, the cellular component-binding reagent specific oligonucleotide comprises a primer adapter. In some embodiments, the cellular component-binding reagent specific oligonucleotide comprises the sequence of a first universal primer, a complementary sequence thereof, a partial sequence thereof, or a combination thereof.

### Binding reagent oligonucleotide barcoding

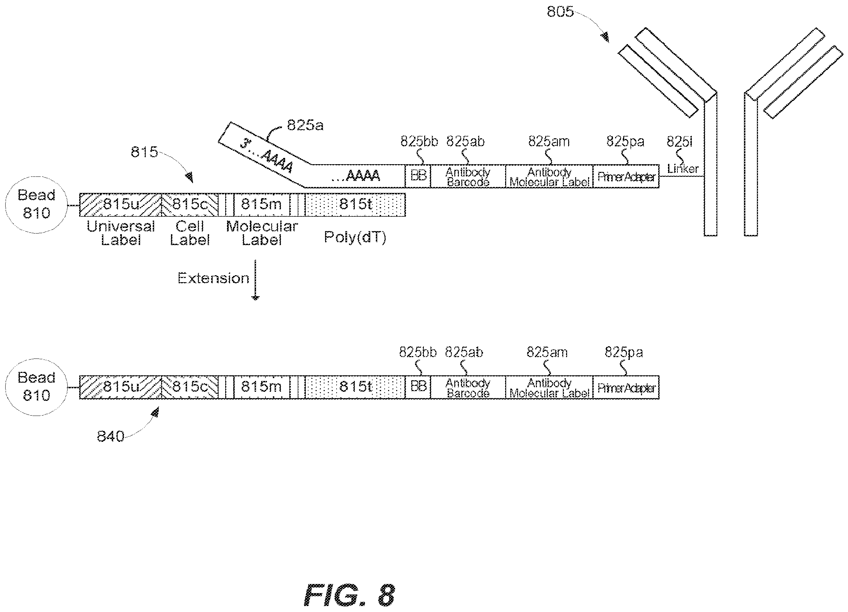

FIG. 8 shows a schematic illustration of a non-limiting exemplary workflow of barcoding of a binding reagent oligonucleotide 825 (antibody oligonucleotide illustrated here) that is associated with a binding reagent 805 (antibody illustrated here). The binding reagent oligonucleotide 825 can be associated with binding reagent 805 through linker 825l. The binding reagent oligonucleotide 825 can be detached from the binding reagent using chemical, optical or other means. The binding reagent oligonucleotide 825 can be an mRNA mimic. The binding reagent oligonucleotide 825 can include a primer adapter 825pa, an antibody molecular label 825am (e.g., a unique molecular label sequence), an antibody barcode 825ab (e.g., a unique identifier sequence), an alignment sequence 825bb, and a poly(A) tail 825a. In some embodiments, the primer adapter 825pa comprises the sequence of a first universal primer, a complimentary sequence thereof, a partial sequence thereof, or a combination thereof. In some embodiments, the primer adapter 825pa can be the same for all or some of binding reagent oligonucleotides 825. In some embodiments, the antibody barcode 825ab can be the same for all or some of binding reagent oligonucleotides 825. In some embodiments, the antibody barcode 825ab of different binding reagent oligonucleotides 825 are different. In some embodiments, the antibody molecular label 825am of different binding reagent oligonucleotides 825 are different.

The binding reagent oligonucleotides 825 can be barcoded using a plurality of barcodes 815 (e.g., barcodes 815 associated with a particle, such as a bead 810) to create a plurality of barcoded binding reagent oligonucleotides 840. In some embodiments, a barcode 815 can include a poly(dT) region 815t for binding to a binding reagent oligonucleotide 825, optionally a molecular label 815m (e.g., for determining the number of occurrences of the binding reagent oligonucleotides), a cell label 815c, and a universal label 815u. In some embodiments the barcode 815 is hybridized to the poly(dT) region 815t of binding reagent oligonucleotides 825. In some embodiments barcoded binding reagent oligonucleotides 840 are generated by extending (e.g., by reverse transcription) the barcode 815 hybridized to the binding reagent oligonucleotide 825. In some embodiments, barcoded binding reagent oligonucleotides 840 comprise primer adapter 825pa, an antibody molecular label 825am (e.g., a unique molecular label sequence), an antibody barcode 825ab (e.g., a unique identifier sequence), an alignment sequence 825bb, poly(dT) region 815t, molecular label 815m, cell label 815c, and universal label 815u.

In some embodiments, the barcoded binding reagent oligonucleotides disclosed herein comprises two unique molecular label sequences: a molecular label sequence derived from the barcode (e.g., molecular label 815m) and a molecular label sequence derived from a binding reagent oligonucleotide (e.g., antibody molecular label 825am, the first molecular label sequence of a sample indexing oligonucleotide, the second molecular label sequence of a cellular component-binding reagent specific oligonucleotide). As used herein, "dual molecular indexing" refers to methods and compositions disclosed herein employing barcoded binding reagent oligonucleotides (or products thereof) that comprise a first unique molecular label sequence and second unique molecular label sequence (or complementary sequences thereof). In some embodiments, the methods of sample identification and of quantitative analysis of cellular component targets disclosed herein can comprise obtaining the sequence of information of the barcode molecular label sequence and/or the binding reagent oligonucleotide molecular label sequence. In some embodiments, the number of barcode molecular label sequences associated with the unique identifier sequence for the cellular component-binding reagent capable of specifically binding to the at least one cellular component target in the sequencing data indicates the number of copies of the at least one cellular component target in the one or more of the plurality of cells. In some embodiments, the number of binding reagent oligonucleotide molecular label sequences associated with the unique identifier sequence for the cellular component-binding reagent capable of specifically binding to the at least one cellular component target in the sequencing data indicates the number of copies of the at least one cellular component target in the one or more of the plurality of cells. In some embodiments, the number of both the binding reagent oligonucleotide molecular label sequences and barcode molecular label sequences associated with the unique identifier sequence for the cellular component-binding reagent capable of specifically binding to the at least one cellular component target in the sequencing data indicates the number of copies of the at least one cellular component target in the one or more of the plurality of cells

The use of PCR to amplify the amount of material before starting the sequencing protocol adds the potential for artifacts, such as artifactual recombination during amplification occurs when premature termination products prime a subsequent round of synthesis). In some embodiments, the methods of dual molecular indexing provided herein allow the identification of PCR chimeras given sufficient sequencing depth. Additionally, in some embodiments, the addition of the unique molecular label sequence to the binding reagent oligonucleotide increases stochastic labelling complexity. Thus, in some embodiments, the presence of the unique molecular label sequence in the binding reagent oligonucleotide can overcome UMI diversity limitations. In some embodiments the methods of dual molecular indexing provided herein decrease the number of cellular component targets flagged as "Saturated" during post-sequencing molecular coverage calculations by at least, or at least about, 2% (e.g., 2%, 3%, 4%, 5%, 6%, 7%, 8%, 9%, 10%, 11%, 12%, 13%, 14%, 15%, 16%, 17%, 18%, 19%, 20%, 25%, 30%, 40%, 50%, 75%, 100%, 150%, 200%, 250%, 500%, 1000%, or higher and overlapping ranges therein) compared to if the methods and compositions are not used.

### Quantitative PCR detection of AbSeq

There are provided, in some embodiments provided herein, systems, methods, compositions, and kits for detecting antibody-conjugated oligonucleotides that do not comprise sequencing. There are provided, in some embodiments, compositions and methods for quantitative PCR detection of AbSeq (e.g., detection of AbSeq on cells). In some embodiments, AbSeq (e.g., first cellular component-binding reagents) comprises an antibody conjugated to an oligo with a unique barcode specific to that antibody. In some embodiments, during single-cell workflows, cells are lysed and AbSeq are captured via their poly dA tail and sequenced downstream. The AbSeq molecule can transcribed with a cell label and unique molecular identifier and the molecules can be sequenced, allowing for in-silico pairing of the antibody and cell combinations, and allowing for cell type identification. However, not all applications require the costly and time-consuming sequencing step. For some purposes, it is sufficient to know the relative abundance of different markers on a cell or group of cells without knowing the individual cell from which it was derived. Accordingly, there are provided, in some embodiments, PCR-based methods of AbSeq detection. Disclosed herein include methods for measuring cellular component target expression in cells without the use of sequencing. In some embodiments, the method comprises: contacting a plurality of first cellular component-binding reagents with the first plurality of cells comprising a plurality of cellular component targets, wherein each of the plurality of first cellular component-binding reagents comprises a cellular component-binding reagent specific oligonucleotide comprising a unique identifier sequence for the first cellular component-binding reagent, and wherein the first cellular component-binding reagent is capable of specifically binding to at least one of the plurality of cellular component targets. The method can comprise: generating amplicons of the unique identifier sequence of one or more of the first cellular component-binding reagents. The method can comprise: determining the amount of one or more amplicons as an indication of the amount of the cellular component targets bound by said one or more of the first cellular component-binding reagents.

There is provided, in some embodiments, a suite of qPCR probes and primers specific to the AbSeq oligos and tested with qPCR. As described herein, the expected profile of AbSeq marker expression from the cells was successfully detected. In some embodiments, the compositions and methods provided herein are absolutely quantitative. In some embodiments, the compositions and methods provided herein are not absolutely quantitative (e.g., providing an identification based on the relative expression profile). However, in some embodiments, quantitation of antigens by this method is provided. Some embodiments of the disclosed methods comprise digital droplet PCR. Most qPCR and ddPCR machines have between 4-5 color channels available, so each reaction can be limited to a small number of AbSeq probes; however, there are provided, in some embodiments, multiple probe sets which can be used across multiple reactions to get a qualitative view of the antigen profile. In some embodiments of this method employ Rhapsody bead amplified product.

Currently available protein qPCR assays (which is a qPCR assay of an oligo conjugated to an antibody) suffer from various deficiencies. The benefits of doing qPCR on AbSeq directly (versus using the currently available protein qPCR assay, which involves different antibody clones and different DNA oligo targets) is that the disclosed methods can directly measure the dynamic range of AbSeq expression, allowing users to adjust AbSeq staining concentrations (e.g., by diluting high expressors with un-conjugated oligos) before undertaking the high cost of sequencing. Users can use it as a QC step before committing to the high sequencing cost. Sequencing costs can be 10-1000x more expensive than qPCR.

Using qPCR on AbSeq directly can allow users to obtain an accurate reference for their AbSeq antibody expression as a QC step before sequencing. This is not possible using currently available assays because they employ different antibody clones, have different oligo sequences, and cannot be used in the workflows provided herein (e.g., Rhapsody workflow) because they do not have a poly dA tail. Thus, currently available assays can suffer from a lack of compatibility with the workflows (e.g., Rhapsody workflow) provided herein, which can make QC for Rhapsody AbSeq using the currently available assay impossible.

The currently available assays can be incompatible with the workflows provided herein (e.g., Rhapsody workflows) due to lack of a poly dA tail, different antibody clonotypes, and different oligo sequences. The disclosed method of qPCR directly on AbSeq can solve these issues. The alternative to the methods provided herein is to spend the full cost for sequencing AbSeq samples (which users currently do). But when users are looking at proteins with highly varied expression, the ability to do a relative quantitation on them before sequencing can be very valuable. A user may find that 60% of their reads are taken up by consistently highexpressing molecules and rework their experiment to provide more meaningful data about rare phenotypes, before spending money on expensive sequencing runs.

Some embodiments of the methods and compositions provided herein rely on AbSeq (oligo-conjugated antibody) and polymerase chain reaction combined with probes specific to the AbSeq barcode. The AbSeq oligo can be amplified directly from the cell or cell lysate. As probes specific to the AbSeq barcode are incorporated into the synthesized strand, the coupled fluorophore will show fluorescence. The fluorescence is typically, though not limited to, made possible by the separation from a quenching molecule. Quantitation of starting molecules is done based on the detection method being employed, for example, a qPCR or ddPCR platform.

There are provided, in some embodiments, methods and compositions enabling users to check the purity of a sorted population or individual cell before doing single cell or bulk analysis. Because the AbSeq antibodies (e.g., first cellular component-binding reagents) are made using the same clones as other antibodies provided herein (e.g., second cellular component-binding reagents), they can be co-stained. In some embodiments, without being bound by any particular theory, co-staining with the same clones will mean they have more similar specificity and sensitivity, meaning no bias from different clonotypes. Optimization between conjugated and non-conjugated clones can be performed for quantitative analysis, but demonstrated herein is cell identification with the workflow disclosed herein, which is a relative quantitation.

There are provided, in some embodiments, methods and compositions enabling users to quality control cell sorts from FACS cytometers. Users can co-stain fluorescent cells and AbSeq antibodies of the same clonotypes and can use qPCR as described herein to verify the purity and specificity of their sort, for either single cell or bulk sorting. The use of AbSeq can be beneficial in such embodiments because there is an existing panel of the same clonotypes as other antibody reagents described and because of the large number of compatible offerings currently provided for users. In some embodiments, optimization is performed to control for competition between the oligo and fluorescent conjugates.

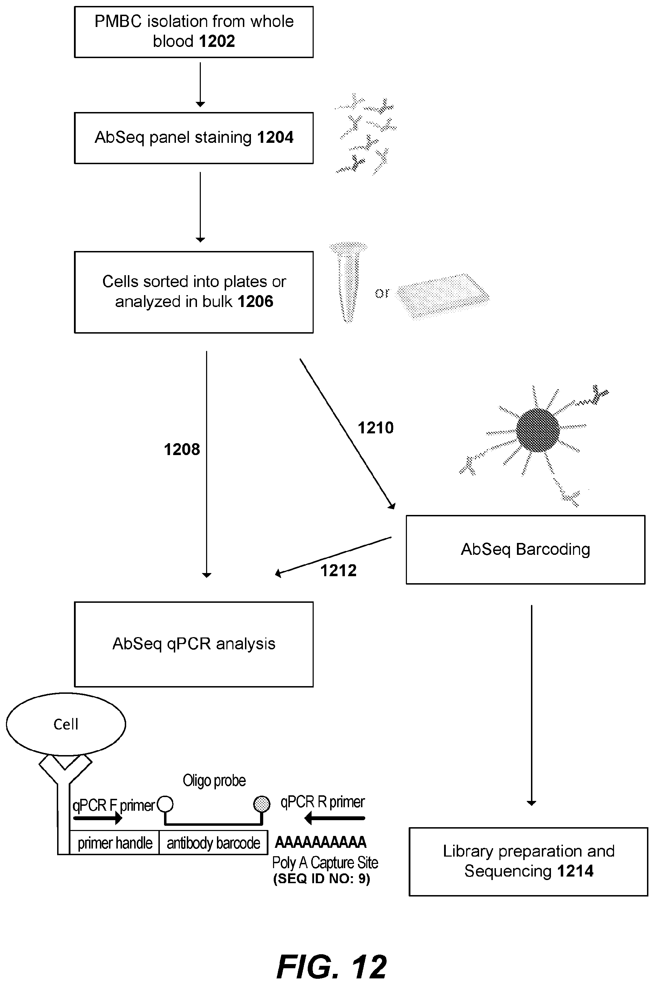

FIG. 12 depicts a non-limiting exemplary workflow of AbSeq qPCR in combination with Rhapsody library preparation. The workflow can comprise PBMC isolation from whole blood (step **1202**). The workflow can comprise staining cells with an AbSeq panel (step **1204**). The workflow can comprise the cells either sorted into plates or analyzed in bulk (e.g., tube) (step **1206**). From this point there are two possible workflows, depending on objectives. The workflow can comprise users quantifying relative proportions of AbSeq molecules on cells before they begin the Rhapsody workflow, allowing them to see relative expression of antigens before they spend time and money on the Rhapsody workflow (step **1208**). In some embodiments, users exhaust the cell material used in the qPCR during this step, so Rhapsody capture would have to be done on a separate aliquot of cells. Alternatively, and unlike currently available methods, AbSeq conjugated oligonucleotides are compatible with the Rhapsody workflow and the workflow can comprise Rhapsody cell capture and cDNA synthesis (step **1210**). The workflow can comprise users quantifying the relative expression from Rhapsody bead PCR product as a check on relative expression before sequencing library prep (step **1212**). This workflow is unique from the currently available methods. The workflow can comprise, after seeing acceptable relative expression of their molecules of interest, Rhapsody Library preparation and sequencing as described herein (step **1214**). While qPCR is depicted in FIG. 12, any oligo probe-based method could be employed here, such as ddPCR.

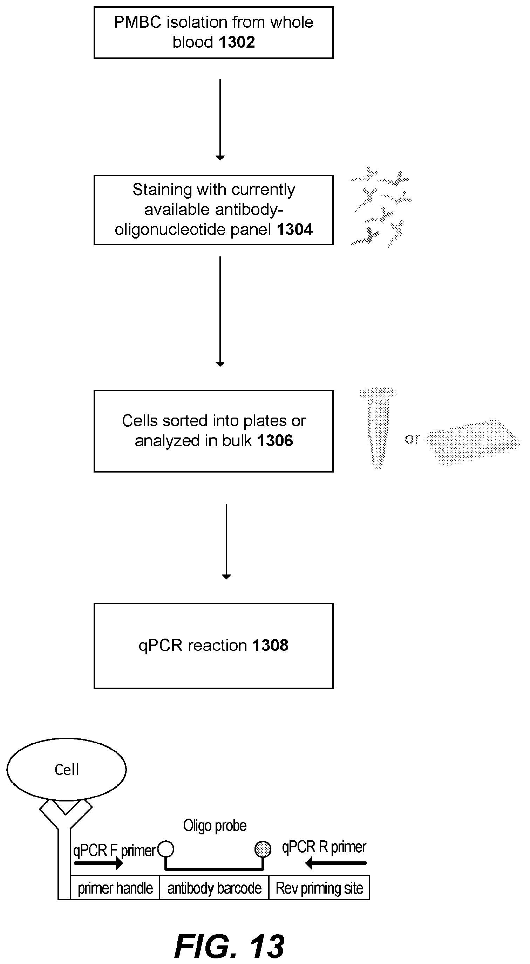

FIG. 13 depicts a non-limiting exemplary qPCR workflow. Currently available workflows, such as those shown in FIG. 13, are not compatible with AbSeq and Rhapsody workflows provided herein, so there can be no way to use this method to QC AbSeq library proportions. The workflow can comprise PBMC isolation from whole blood (step **1302**). The workflow can comprise staining with a currently available antibody-oligonucleotide panel (step **1304**). The workflow can comprise cells sorted into plates or analyzed in bulk (step **1306**). The workflow can comprise a qPCR reaction (step **1308**).

Disclosed herein include methods for measuring cellular component target expression in cells. In some embodiments, the method comprises: contacting a plurality of first cellular component-binding reagents with a first plurality of cells comprising a plurality of cellular component targets, wherein each of the plurality of first cellular component-binding reagents comprises a cellular component-binding reagent specific oligonucleotide comprising a unique identifier sequence for the first cellular component-binding reagent, and wherein the first cellular component-binding reagent is capable of specifically binding to at least one of the plurality of cellular component targets. The method can comprise: generating amplicons of the unique identifier sequence of one or more of the first cellular component-binding reagents. The method can comprise: determining the amount of one or more amplicons as an indication of the amount of the one or more of the first cellular component-binding reagents. The method can comprise: if the amount of one or more amplicons indicates an abundance of a first cellular component-binding reagent above or below a predetermined abundance range, repeating the contacting step with a second plurality of cells in a manner configured to achieve an abundance of said first cellular component-binding reagent within said predetermined abundance range.

In some embodiments, the predetermined abundance range comprises the dynamic range for which acceptable linearity and efficiency of detection of the first cellular component-binding reagent are observed. The method can comprise: contacting the first and/or second plurality of cells with a plurality of third cellular component-binding reagents, wherein a third cellular component-binding reagent is capable of specifically binding to at least one of the plurality of cellular component targets, wherein the third cellular component-binding reagents do not comprise a cellular component-binding reagent specific oligonucleotide, wherein one or more of the first cellular component-binding reagents and one or more of the third cellular component-binding reagents are capable of binding the same cellular component target. In some embodiments, first cellular component-binding reagents and third cellular component-binding reagents capable of binding the same cellular component target have the same clonotype.

In some embodiments, repeating the contacting step with a second plurality of cells in a manner configured to achieve an abundance of said first cellular component-binding reagent within said predetermined abundance range comprises contacting the second plurality of cells with mixture of said first cellular component-binding reagent and a third cellular component-binding reagent capable of binding the same cellular component target as said first cellular component-binding reagent at a titration ratio configured to achieve an abundance of said first cellular component-binding reagent within said predetermined abundance range. In some embodiments, the titration ratio is configured such that sequencing reads comprising the unique identifier sequence of said first cellular component-binding reagent account for less than about 5% of total sequencing reads.

Disclosed herein include methods for measuring cellular component target expression in cells. In some embodiments, the method comprises: contacting a plurality of first cellular component-binding reagents with the first plurality of cells comprising a plurality of cellular component targets, wherein each of the plurality of first cellular component-binding reagents comprises a cellular component-binding reagent specific oligonucleotide comprising a unique identifier sequence for the first cellular component-binding reagent, and wherein the first cellular component-binding reagent is capable of specifically binding to at least one of the plurality of cellular component targets. The method can comprise: isolating one or more cell types of interest from the first plurality of cells, wherein a first cell type of interest is characterized by abundance of: (i) one or more positive cellular component targets above a positive abundance threshold and/or (ii) one or more negative cellular component targets below a negative abundance threshold. The positive abundance threshold and negative abundance threshold can be predetermined and can vary depending on the cellular component target and the needs of the user. The method can comprise: generating amplicons of the unique identifier sequence of one or more of the first cellular component-binding reagents. The method can comprise: determining the amount of one or more amplicons as an indication of the amount of the cellular component targets bound by said one or more of the first cellular component-binding reagents.

In some embodiments, one or more cells suspected of being a first cell type of interest is determined to not be a first cell type of interest if the amount of one or more amplicons indicates an abundance of: (i) one or more positive cellular component targets below a positive abundance threshold and/or (ii) one or more negative cellular component targets above a negative abundance threshold. The method can comprise: if one or more cells suspected of being a first cell type of interest is determined to not be a first cell type of interest, repeating the contacting step and/or isolating step with a second plurality of cells in a manner configured to achieve isolation of the one or more cell types of interest.

In some embodiments, the cellular component-binding reagent specific oligonucleotide comprises a sequence complementary to a capture sequence of an oligonucleotide barcode configured to capture the sequence of the cellular component-binding reagent specific oligonucleotide. In some embodiments, the sequence of the cellular component-binding reagent specific oligonucleotide complementary to the capture sequence comprises a poly(dA) region.

The method can comprise: contacting a plurality of oligonucleotide barcodes with the cellular component-binding reagent specific oligonucleotides for hybridization, wherein the oligonucleotide barcodes each comprise a first molecular label and a first universal sequence; and extending the plurality of oligonucleotide barcodes hybridized to the cellular component-binding reagent specific oligonucleotides to generate a plurality of barcoded cellular component-binding reagent specific oligonucleotides each comprising a sequence complementary to at least a portion of the unique identifier sequence and the first molecular label. The method can comprise: obtaining sequence information of the plurality of barcoded cellular component-binding reagent specific oligonucleotides, or products thereof, to determine the number of copies of at least one cellular component target of the plurality of cellular component targets in one or more of the first plurality of cells and/or second plurality of cells. In some embodiments, obtaining the sequence information comprises attaching sequencing adaptors to the plurality of barcoded cellular component-binding reagent specific oligonucleotides, or products thereof. In some embodiments, the cellular component-binding reagent specific oligonucleotide comprises a second universal sequence.

In some embodiments, the isolating step comprises single cell sorting or bulk sorting. In some embodiments, the one or more cell types of interest comprise hemogenic endothelium cells, hematopoietic stem and progenitor cells (HSC), hematopoietic multipotent progenitor cell (MPP), pre-T cell progenitor cells, pre- K cell progenitor cells, T cell progenitor cells, NK cell progenitor cells, T cells, NK cells, NKT cells, B cells, macrophage, neutrophils, CD4 cells, CD8 cells, naive T-cells, memory stem T-cells, central memory T- cells, double negative T-cells, effector memory T-cells, effector T-cells, ThO cells, TcO cells, Thl cells, Tel cells, Th2 cells, Tc2 cells, Thl7 cells, Th22 cells, gamma/delta T-cells, natural killer (NK) cells, natural killer T (NKT) cells, hematopoietic stem cells pluripotent stem cells, or any combination thereof. In some embodiments, of the one or more cell types of interest comprise or more immune cell types (e.g., naive CD4 T cells, effector memory CD4 T cells, naive CD8 T cells, effector memory CD8 T cells, naive CD4 Treg cells, effector memory CD4 Treg cells, naive B cells, memory B cells, CD16 DC, plasmacytoid DC, or any combination thereof).

The method can comprise: contacting a plurality of second cellular component-binding reagents with the first and/or second plurality of cells, wherein each of the plurality of second cellular component-binding reagents comprises a detectable moiety, or a precursor thereof, wherein a second cellular component-binding reagent is capable of specifically binding to at least one of the plurality of cellular component targets, wherein second cellular component-binding reagents capable of binding the same cellular component target comprise the same detectable moiety, or a precursor thereof, and wherein second cellular component-binding reagents capable of binding different cellular component targets comprise different detectable moieties, or precursors thereof. In some embodiments, the first plurality of cells and/or second plurality of cells are contacted with the first cellular component-binding reagents and second cellular component-binding reagents simultaneously. In some embodiments, one or more of the first cellular component-binding reagents and one or more of the second cellular component-binding reagents are capable of binding the same cellular component target. In some embodiments, first cellular component-binding reagents and second cellular component-binding reagents capable of binding the same cellular component target have the same clonotype. In some embodiments, the isolating step comprises fluorescence-activated cell sorting. In some embodiments, fluorescence-activated cell sorting employs the second cellular component-binding reagents. In some embodiments, the isolating step comprises depositing the one or more cells of interest of the first and/or second plurality of cells into one or more microtiter plates. In some embodiments, the isolating step comprises detecting emissions of the detectable moiety of second cellular component-binding reagents with a flow cytometer. In some embodiments, the flow cytometer comprises a conventional flow cytometer, a spectral flow cytometer, a hyperspectral flow cytometer, an imaging flow cytometer, or any combination thereof.

In some embodiments, the one or more positive cellular component targets and/or one or more negative cellular component targets comprises CD4, CD8, CCR7, CD45RO, CD25, HLA-DR, CD45RA, CD19, CD3, CD27, IgD, CD11c, CD16, or any combination thereof. In some embodiments, the one or more positive cellular component targets and/or one or more negative cellular component targets is selected from the group comprising CD2, CD2R, CD3, CD3gd, CD3e, CD4, CD5, CD6, CD7, CD8, CD8a, CD8b, CD9, CD10, CD11a, CD11b, CD11c, CD12, CD12w, CD13, CD14, CD15, CD15s, CD15u, CD16, CD16a, CD16b, CD17, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD42a, CD42b, CD42c, CD42d, CD43, CD44, CD44R, CD45, CD45RA, CD45RB, CD45RO, CD46, CD47, CD47R, CD48, CD49a, CD49b, CD49c, CD49e, CD49f, CD50, CD51, CD52, CD53, CD54, CD55, CD56, CD57, CD58, CD59, CD60, CD60a, CD60b, CD60c, CD61, CD62E, CD62L, CD62P, CD63, CD64, CD65, CD65s, CD66, CD66a, CD66b, CD66c, CD66d, CD66e, CD66f, CD67, CD68, CD69, CD70, CD71, CD72, CD73, CD74, CD74, CD75, CD75s, CD76, CD77, CD78, CD79, CD79a, CD79b, CD80, CD81, CD82, CD83, CD84, CDw84, CD85, CD86, CD87, CD88, CD89, CD90, CD91, CD92, CDw92, CD93, CD94, CD95, CD96, CD97, CD98, CD99, CD99R, CD100, CD101, CD102, CD103, CD104, CD105, CD106, CD107, CD107a, CD107b, CD108, CD109, CD110, CD111, CD112, CD113, CDw113, CD114, CD115, CD116, CD117, CD118, CD119, CDw119, CD120a, CD120b, CD121a, CD121b, CDw121b, CD122, CD123, CDw123, CD124, CD125, CDw125, CD126, CD127, CD128, CDw128, CD129, CD130, CD131, CDw131, CD132, CD133, CD134, CD135, CD136, CDw136, CD137, CDw137, CD138, CD139, CD140a, CD140b, CD141, CD142, CD143, CD144, CD145, CDw145, CD146, CD147, CD148, CD149, CD150, CD151, CD152, CD153, CD154, CD155, CD156a, CD156b, CDw156c, CD157, CD158a, CD158b, CD159a, CD159b, CD159c, CD160, CD161, CD162, CD162R, CD163, CD164, CD165, CD166, CD167, CD167a, CD168, CD169, CD170, CD171, CD172a, CD172b, CD172g, CD173, CD174, CD175, CD175s, CD176, CD177, CD178, CD179, CD180, CD181, CD182, CD183, CD184, CD185, CD186, CDw186, CD187, CD188, CD189, CD190, Cd191, CD192, CD193, CD194, CD195, CD196, CD197, CD198, CDw198, CD199, CDw199, CD200, CD200a, CD200b, CD201, CD202, CD202b, CD203, CD203c, CD204, CD205, CD206, CD207, CD208, CD209, CD210, CDw210, CD212, CD213a1, CD213a2, CDw217, CDw218a, CDw218b, CD220, CD221, CD222, CD223, CD224, CD225, CD226, CD227, CD228, CD229, CD230, CD231, CD232, CD233, CD234, CD235a, CD235ab, CD235b, CD236, CD236R, CD238, CD239, CD240, CD240CE, CD240D, CD241, CD242, CD243, CD244, CD245, CD246, CD247, CD248, CD249, CD252, CD253, CD254, CD256, CD257, CD258, CD261, CD262, CD263, CD265, CD266, CD267, CD268, CD269, CD271, CD273, CD274, CD275, CD276 (B7-H3), CD277, CD278, CD279, CD280, CD281, CD282, CD283, CD284, CD289, CD292, CDw293, CD294, CD295, CD296, CD297, CD298, CD299, CD300a, CD300c, CD300e, CD301, CD302, CD303, CD304, CD305, CD306, CD309, CD312, CD314, CD315, CD316, CD317, CD318, CD319, CD320, CD321, CD322, CD324, CDw325, CD326, CDw327, CDw328, CDw329, CD331, CD332, CD333, CD334, CD335, CD336, CD337, CDw338, CD339, 4-1BB, 5AC, 5T4 (Trophoblast glycoprotein, TPBG, 5T4, Wnt-Activated Inhibitory Factor 1 or WAIF 1), Adenocarcinomaantigen, AGS-5, AGS-22M6, Activin receptor-like kinase 1, AFP, AKAP-4, ALK, Alpha intergrin, Alpha v beta6, Amino-peptidase N, Amyloid beta, Androgen receptor, Angiopoietin 2, Angiopoietin 3, Annexin A1, Anthrax toxin-protective antigen, Anti-transferrin receptor, AOC3 (VAP-1), B7-H3, Bacillus anthracis anthrax, BAFF (B-cell activating factor), B-lymphoma cell, bcr-abl, Bombesin, BORIS, C5, C242 antigen, CA125 (carbohydrate antigen 125, MUC16), CA-IX (or CAIX, carbonic anhydrase 9), CALLA, CanAg, Canis lupus familiaris IL31, Carbonic anhydrase IX, Cardiac myosin, CCL11 (C-C motif chemokine 11), CCR4 (C-C chemokine receptor type 4, CD194), CCR5, CD3E (epsilon), CEA (Carcinoembryonic antigen), CEACAM3, CEACAM5 (carcinoembryonic antigen), CFD (Factor D), Ch4D5, Cholecystokinin 2 (CCK2R), CLDN18 (Claudin-18), Clumping factor A, CRIPTO, FCSF1R (Colony stimulating factor 1 receptor, CD115), CSF2 (colony stimulating factor 2, Granulocytemacrophage colony-stimulating factor (GM-CSF)), CTLA4 (cytotoxic T-lymphocyte associated protein 4), CTAA16.88 tumor antigen, CXCR4 (CD184), C-X-C chemokine receptor type 4, cyclic ADP ribose hydrolase, Cyclin B1, CYP1B1, Cytomegalovirus, Cytomegalovirus glycoprotein B, Dabigatran, DLL3 (delta-like-ligand 3), DLL4 (delta-like-ligand 4), DPP4 (Dipeptidyl-peptidase 4), DR5 (Death receptor 5), E. coli shiga toxintype-1, E. coli shiga toxintype-2, ED-B, EGFL7 (EGF-like domain-containing protein 7), EGFR, EGFRII, EGFRvIII, Endoglin (CD105), Endothelin B receptor, Endotoxin, EpCAM (epithelial cell adhesion molecule), EphA2, Episialin, ERBB2 (Epidermal Growth Factor Receptor 2), ERBB3, ERG (TMPRSS2 ETS fusion gene), Escherichia coli, ETV6-AML, FAP (Fibroblast activation proteinalpha), FCGR1, alpha-Fetoprotein, Fibrin II, beta chain, Fibronectin extra domain-B, FOLR (folate receptor), Folate receptor alpha, Folate hydrolase, Fos-related antigen 1, F protein of respiratory syncytial virus, Frizzled receptor, Fucosyl GM1, GD2 ganglioside, G-28 (a cell surface antigen glyvolipid), GD3 idiotype, GloboH, Glypican 3, N-glycolylneuraminic acid, GM3, GMCSF receptor α-chain, Growth differentiation factor 8, GP100, GPNMB (Transmembrane glycoprotein NMB), GUCY2C (Guanylate cyclase 2C, guanylyl cyclase C (GC-C), intestinal Guanylate cyclase, Guanylate cyclase-C receptor, Heat-stable enterotoxin receptor (hSTAR)), Heat shock proteins, Hemagglutinin, Hepatitis B surface antigen, Hepatitis B virus, HER1 (human epidermal growth factor receptor 1), HER2, HER2/neu, HER3 (ERBB-3), IgG4, HGF/SF (Hepatocyte growth factor/scatter factor), HHGFR, HIV-1, Histone complex, HLA-DR (human leukocyte antigen), HLA-DR10, HLA-DRB, HMWMAA, Human chorionic gonadotropin, HNGF, Human scatter factor receptor kinase, HPV E6/E7, Hsp90, hTERT, ICAM-1 (Intercellular Adhesion Molecule 1), Idiotype, IGF1R (IGF-1, insulin-like growth factor 1 receptor), IGHE, IFN-γ, Influeza hemag-glutinin, IgE, IgE Fc region, IGHE, interleukins (e.g. IL-1, IL-2, IL-3, IL-4, IL-5, IL-6, IL-6R, IL-7, IL-8, IL-9, IL-10, IL-11, IL-12, IL-13, IL-15, IL-17, IL-17A, IL-18, IL-19, IL-20, IL-21, IL-22, IL-23, IL-27, or IL-28), IL31RA, ILGF2 (Insulin-like growth factor 2), Integrins (α4, αIIbβ3, αvβ3, α4β7, α5β1, α6β4, α7β7, allβ3, α5β5, αvβ5), Interferon gamma-induced protein, ITGA2, ITGB2, KIR2D, LCK, Le, Legumain, Lewis-Y antigen, LFA-1 (Lymphocyte function-associated antigen 1, CD11a), LHRH, LINGO-1, Lipoteichoic acid, LIV1A, LMP2, LTA, MAD-CT-1, MAD-CT-2, MAGE-1, MAGE-2, MAGE-3, MAGE A1, MAGE A3, MAGE 4, MART1, MCP-1, MIF (Macrophage migration inhibitory factor, or glycosylation-inhibiting factor (GIF)), MS4A1 (membrane-spanning 4-domains subfamily A member 1), MSLN (mesothelin), MUC1 (Mucin 1, cell surface associated (MUC1) orpolymorphic epithelial mucin (PEM)), MUC1-KLH, MUC16 (CA125), MCP1 (monocyte chemotactic protein 1), MelanA/MART1, ML-IAP, MPG, MS4A1 (membrane-spanning 4-domains subfamily A), MYCN, Myelin-associated glycoprotein, Myostatin, NA17, NARP-1, NCA-90 (granulocyte antigen), Nectin-4 (ASG-22ME), NGF, Neural apoptosis-regulated proteinase 1, NOGO-A, Notch receptor, Nucleolin, Neu oncogene product, NY-BR-1, NY-ESO-1, OX-40, OxLDL (Oxidized low-density lipoprotein), OY-TES1, P21, p53 nonmutant, P97, Page4, PAP, Paratope of anti-(N-glycolylneuraminic acid), PAX3, PAX5, PCSK9, PDCD1 (PD-1, Programmed cell death protein 1, CD279), PDGF-Rα (Alphatype platelet-derived growth factor receptor), PDGFR-β, PDL-1, PLAC1, PLAP-like testicular alkaline phosphatase, Platelet-derived growth factor receptor beta, Phosphate-sodium cotransporter, PMEL 17, Polysialic acid, Proteinase3 (PR1), Prostatic carcinoma, PS (Phosphatidylserine), Prostatic carcinoma cells, Pseudomonas aeruginosa, PSMA, PSA, PSCA, Rabies virus glycoprotein, RHD (Rh polypeptide 1 (RhPI), CD240), Rhesus factor, RANKL, RANTES receptors (CCR1, CCR3, CCR5), RhoC, Ras mutant, RGS5, ROBO4, Respiratory syncytial virus, RON, Sarcoma translocation breakpoints, SART3, Sclerostin, SLAMF7 (SLAM family member 7), Selectin P, SDC1 (Syndecan 1), sLe(a), Somatomedin C, SIP (Sphingosine-1-phosphate), Somatostatin, Sperm protein 17, SSX2, STEAP1 (six-transmembrane epithelial antigen of the prostate 1), STEAP2, STn, TAG-72 (tumor associated glycoprotein 72), Survivin, T-cell receptor, T cell transmembrane protein, TEM1 (Tumor endothelial marker 1), TENB2, Tenascin C (TN-C), TGF-α, TGF-β (Transforming growth factor beta), TGF-β1, TGF-β2 (Transforming growth factor-beta 2), Tie (CD202b), Tie2, TIM-1 (CDX-014), Tn, TNF, TNF-α, TNFRSF8, TNFRSF10B (tumor necrosis factor receptor superfamily member 10B), TNFRSF13B (tumor necrosis factor receptor superfamily member 13B), TPBG (trophoblast glycoprotein), TRAIL-R1 (Tumor necrosis apoprosis Inducing ligand Receptor 1), TRAILR2 (Death receptor 5 (DR5)), tumor-associated calcium signal transducer 2, tumor specific glycosylation of MUC1, TWEAK receptor, TYRP1 (glycoprotein 75), TROP-2, TRP-2, Tyrosinase, VCAM-1 (CD106), VEGF, VEGF-A, VEGF-2 (CD309), VEGFR-1, VEGFR2, or vimentin, WT1, XAGE 1, an insulin growth factor receptor, or an epidermal growth factor receptor, or any combination thereof.

In some embodiments, the number of unique first molecular label sequences associated with the unique identifier sequence for the first cellular component-binding reagent capable of specifically binding to the at least one cellular component target in the sequencing data indicates the number of copies of the at least one cellular component target in the one or more of the first and/or second plurality of cells. The cellular component-binding reagent specific oligonucleotide can comprise one or more of: (a) a second molecular label sequence (e.g., 2-20 nucleotides in length); (b) an alignment sequence adjacent to a poly(dA) region, optionally the alignment sequence is one or more nucleotides, or two or more nucleotides, in length; and (c) a linker, wherein the cellular component-binding reagent specific oligonucleotide is associated with the first cellular component-binding reagent through the linker. In some embodiments, the second molecular label sequences of at least two cellular component-binding reagent specific oligonucleotides are different, and wherein the unique identifier sequences of the at least two cellular component-binding reagent specific oligonucleotides are identical. In some embodiments, the second molecular label sequences of at least two cellular componentbinding reagent specific oligonucleotides are different, and wherein the unique identifier sequences of the at least two cellular component-binding reagent specific oligonucleotides are different. In some embodiments, the number of unique second molecular label sequences associated with the unique identifier sequence for the first cellular component-binding reagent capable of specifically binding to the at least one cellular component target in the sequencing data indicates the number of copies of the at least one cellular component target in the one or more of the first and/or second plurality of cells. In some embodiments, (a) the alignment sequence comprises a guanine, a cytosine, a thymine, a uracil, or a combination thereof, (b) the alignment sequence comprises a poly(dT) sequence, a poly(dG) sequence, a poly(dC) sequence, a poly(dU) sequence, or a combination thereof; and/or (c) the alignment sequence is 5' to the poly(dA) region. In some embodiments, the linker comprises a carbon chain. In some embodiments, the carbon chain comprises 2-30 carbons (e.g., 12 carbons). In some embodiments, the linker comprises 5' amino modifier C12 (5AmMC12), or a derivative thereof.

In some embodiments, the plurality of cellular component targets comprises a plurality of protein targets, and wherein the first cellular component-binding reagent is capable of specifically binding to at least one of the plurality of protein targets. In some embodiments, the plurality of cellular component targets comprises a cell-surface protein, an intracellular protein, a cell marker, a B-cell receptor, a T-cell receptor, an antibody, a major histocompatibility complex, a tumor antigen, a receptor, or a combination thereof.

The cellular component-binding reagent specific oligonucleotide can be associated with the first cellular component-binding reagent. In some embodiments, the cellular component-binding reagent specific oligonucleotide is covalently attached to the first cellular component-binding reagent and/or non-covalently attached to the first cellular component-binding reagent. The cellular component-binding reagent specific oligonucleotide can be conjugated to the first cellular component-binding reagent. In some embodiments, the cellular component-binding reagent specific oligonucleotide is conjugated to the first cellular component-binding reagent through a chemical group selected from the group consisting of a UV photocleavable group, a streptavidin, a biotin, an amine, and a combination thereof.

In some embodiments, the cellular component-binding reagent specific oligonucleotide is configured to be detachable from the first cellular component-binding reagent. The method can comprise: dissociating the cellular component-binding reagent specific oligonucleotide from the first cellular component-binding reagent. In some embodiments, dissociating the cellular component-binding reagent specific oligonucleotide comprises detaching the cellular component-binding reagent specific oligonucleotide from the first cellular component-binding reagent by UV photocleaving, chemical treatment, heating, enzyme treatment, or any combination thereof. In some embodiments, the dissociating occurs after barcoding the cellular component-binding reagent specific oligonucleotide and/or wherein the dissociating occurs before barcoding the cellular component-binding reagent specific oligonucleotide. In some embodiments, the cellular component-binding reagent specific oligonucleotide is configured to be non-detachable from the first cellular component-binding reagent.

The method can comprise: after contacting the plurality of first cellular component-binding reagents with the first and/or second plurality of cells, removing one or more first cellular component-binding reagents of the plurality of first cellular component-binding reagents that are not contacted with the first and/or second plurality of cells. In some embodiments, removing the one or more first cellular component-binding reagents not contacted with the first and/or second plurality of cells comprises removing the one or more first cellular component-binding reagents not contacted with the respective at least one of the plurality of cellular component targets. In some embodiments, the first and/or second plurality of cells comprises T cells, B cells, tumor cells, myeloid cells, blood cells, normal cells, fetal cells, maternal cells, or a mixture thereof.

In some embodiments, one or more single cells of the first plurality of cells and/or second plurality of cells comprises copies of a nucleic acid target. The method can comprise: contacting a plurality of oligonucleotide barcodes with the copies of the nucleic acid target for hybridization, wherein each oligonucleotide barcode of the plurality of oligonucleotide barcodes comprises a first universal sequence, a target-binding region capable of hybridizing to the copies of the nucleic acid target, and a first molecular label; extending the plurality of oligonucleotide barcodes hybridized to the copies of a nucleic acid target to generate a plurality of barcoded nucleic acid molecules each comprising a sequence complementary to at least a portion of the nucleic acid target; and obtaining sequence information of the plurality of barcoded nucleic acid molecules, or products thereof, to determine the copy number of the nucleic acid target in each of the one or more single cells. In some embodiments, determining the copy number of the nucleic acid target in each of the one or more single cells comprises determining the copy number of the nucleic acid target in each of the one or more single cells based on the number of first molecular labels with distinct sequences, complements thereof, or a combination thereof, associated with the plurality of barcoded nucleic acid molecules, or products thereof. In some embodiments, obtaining sequencing data comprises attaching sequencing adaptors to the plurality of barcoded nucleic acid molecules, or products thereof.

In some embodiments, the plurality of barcoded nucleic acid molecules comprise barcoded DNA molecules and/or barcoded RNA molecules. In some embodiments, the nucleic acid target comprises a nucleic acid molecule (e.g., RNA, messenger RNA (mRNA), microRNA, small interfering RNA (siRNA), RNA degradation product, RNA comprising a poly(A) tail, or any combination thereof). In some embodiments, the mRNA encodes an immune receptor).

In some embodiments, at least 10 of the plurality of oligonucleotide barcodes comprise different first molecular label sequences. In some embodiments, each first molecular label of the plurality of oligonucleotide barcodes comprises at least 6 nucleotides. In some embodiments, the plurality of oligonucleotide barcodes are associated with a solid support. In some embodiments, the plurality of oligonucleotide barcodes associated with the same solid support each comprise an identical sample label. In some embodiments, each sample label of the plurality of oligonucleotide barcodes comprises at least 6 nucleotides. In some embodiments, the plurality of oligonucleotide barcodes each comprise a cell label. In some embodiments, each cell label of the plurality of oligonucleotide barcodes comprises at least 6 nucleotides. In some embodiments, oligonucleotide barcodes associated with the same solid support comprise the same cell label. In some embodiments, oligonucleotide barcodes associated with different solid supports comprise different cell labels.

The method can comprise: associating a synthetic particle comprising the plurality of the oligonucleotide barcodes with a single cell in the first and/or second plurality of single cells. The method can comprise: lysing the single cell after associating the synthetic particle with the single cell. In some embodiments, lysing the single cell comprises heating the sample, contacting the sample with a detergent, changing the pH of the sample, or any combination thereof. In some embodiments, the synthetic particle and the single cell are in the same well. In some embodiments, the synthetic particle and the single cell are in the same droplet. In some embodiments, at least one of the plurality of oligonucleotide barcodes is immobilized or partially immobilized on the synthetic particle, or the at least one of the plurality of oligonucleotide barcodes is enclosed or partially enclosed in the synthetic particle. In some embodiments, the synthetic particle is disruptable. In some embodiments, the synthetic particle comprises a bead. In some embodiments, the bead comprises a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, an anti-biotin microbead, an anti-fluorochrome microbead, or any combination thereof. In some embodiments, the bead comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, and any combination thereof. In some embodiments, the bead comprises a disruptable hydrogel particle. In some embodiments, each of the plurality of oligonucleotide barcodes comprises a linker functional group, the synthetic particle comprises a third solid support functional group, and the support functional group and the linker functional group are associated with each other. In some embodiments, the linker functional group and the support functional group are individually selected from the group consisting of C6, biotin, streptavidin, primary amine(s), aldehyde(s), ketone(s), and any combination thereof. In some embodiments, the first plurality of cells and second plurality of cells are derived from the same sample.

### Generating and Detecting Amplicons

In some embodiments, generating amplicons comprises: amplifying the plurality of cellular component-binding reagent specific oligonucleotides using a primer capable of hybridizing to the second universal sequence, or a complement thereof, and a primer capable of hybridizing to the capture sequence, or a complement thereof, thereby generating amplicons of the unique identifier sequence of one or more of the first cellular component-binding reagents. In some embodiments, generating amplicons comprises: amplifying the plurality of cellular component-binding reagent specific oligonucleotides using a primer capable of hybridizing to the second universal sequence, or a complement thereof, and a primer capable of hybridizing to the capture sequence, or a complement thereof, thereby generating PCR1 products; and amplifying the PCR1 products using a primer capable of hybridizing to the second universal sequence, or a complement thereof, and a primer capable of hybridizing to the capture sequence, or a complement thereof, thereby generating amplicons of the unique identifier sequence of one or more of the first cellular component-binding reagents.

In some embodiments, generating amplicons comprises: amplifying the plurality of barcoded cellular component-binding reagent specific oligonucleotides using a primer capable of hybridizing to the second universal sequence, or a complement thereof, and a primer capable of hybridizing to the capture sequence, or a complement thereof, thereby generating amplicons of the unique identifier sequence of one or more of the first cellular component-binding reagents. In some embodiments, generating amplicons comprises: amplifying the plurality of barcoded cellular component-binding reagent specific oligonucleotides using a primer capable of hybridizing to the second universal sequence, or a complement thereof, and a primer capable of hybridizing to the capture sequence, or a complement thereof, thereby generating PCR1 products; and amplifying the PCR1 products using a primer capable of hybridizing to the second universal sequence, or a complement thereof, and a primer capable of hybridizing to the capture sequence, or a complement thereof, thereby generating amplicons of the unique identifier sequence of one or more of the first cellular component-binding reagents.

In some embodiments, generating amplicons comprises: amplifying the plurality of barcoded cellular component-binding reagent specific oligonucleotides using a primer capable of hybridizing to the first universal sequence, or a complement thereof, and a primer capable of hybridizing to the second universal sequence, or a complement thereof, thereby generating amplicons of the unique identifier sequence of one or more of the first cellular component-binding reagents. In some embodiments, generating amplicons comprises: amplifying the plurality of barcoded cellular component-binding reagent specific oligonucleotides using a primer capable of hybridizing to the first universal sequence, or a complement thereof, and a primer capable of hybridizing to the second universal sequence, or a complement thereof, thereby generating PCR1 products; and amplifying the PCR1 products using a primer capable of hybridizing to the second universal sequence, or a complement thereof, and a primer capable of hybridizing to the first universal sequence, or a complement thereof, thereby generating amplicons of the unique identifier sequence of one or more of the first cellular component-binding reagents.

In some embodiments, said amplification is carried out using a method selected from the group consisting of polymerase chain reaction (PCR), ligase chain reaction (LCR), loop-mediated isothermal amplification (LAMP), strand displacement amplification (SDA), replicase-mediated amplification, Immuno-amplification, nucleic acid sequence based amplification (NASBA), self-sustained sequence replication (3SR), rolling circle amplification, and transcription-mediated amplification (TMA). In some embodiments, said PCR is real-time PCR. In some embodiments, said PCR is quantitative real-time PCR (QRT-PCR). In some embodiments, said PCR is digital PCR (dPCR), such as, for example, droplet digital PCR (ddPCR). In some embodiments, determining the presence or amount of one or more amplicons comprises contacting the amplicons with a plurality of oligonucleotide probes, wherein each of the plurality of oligonucleotide probes comprises a sequence configured to bind a unique identifier sequence, or a complement thereof. In some embodiments, each probe is flanked by complementary sequences at the 5' end and 3' end. In some embodiments, one of the complementary sequences comprises a fluorescence emitter moiety and the other complementary sequence comprises a fluorescence quencher moiety. In some embodiments, at least one of the plurality of oligonucleotide probes comprises a fluorescence emitter moiety and a fluorescence quencher moiety. In some embodiments, the oligonucleotide probe comprises a TaqMan detection probe oligonucleotide, a molecular beacon detection probe oligonucleotide, or a molecular torch detection probe oligonucleotide. In some embodiments, the generating amplicons step and determining step are multiplexed. In some embodiments, at least about 4 unique identifier sequences are analyzed simultaneously. In some embodiments, the generating amplicons step and determining step are performed on single cells or multiple cells.

As used herein, "template" can refer to all or part of a polynucleotide containing at least one target nucleotide sequence. In some embodiments, the template is a cellular component-binding reagent specific oligonucleotide comprising a unique identifier sequence for the first cellular component-binding reagent (or a product thereof). In some embodiments, the template is a barcoded cellular component-binding reagent specific oligonucleotide (or a product thereof). In some embodiments, the target nucleotide sequence is the unique identifier sequence of one or more of the cellular component-binding reagent specific oligonucleotides associated with a first cellular component binding reagent. These templates can be employed in the amplification methods provided herein (e.g., generating amplicons of the unique identifier sequence of one or more of the first cellular component-binding reagents). The amount of one or more amplicons can be determined as described herein, which can indicate the abundance of a first cellular component-binding reagent and/or a cellular component target (bound by said first cellular component-binding reagent). Determining the presence or amount of one or more amplicons can comprise contacting the amplicons with a plurality of oligonucleotide probes which are configured to bind a unique identifier sequence, or a complement thereof. As discussed herein, these probes can comprise one or more detectable labels.

As used herein, a "primer" can refer to a polynucleotide that can serve to initiate a nucleic acid chain extension reaction. The length of a primer can vary, for example, from about 5 to about 100 nucleotides, from about 10 to about 50 nucleotides, from about 15 to about 40 nucleotides, or from about 20 to about 30 nucleotides. The length of a primer can be about 10 nucleotides, about 20 nucleotides, about 25 nucleotides, about 30 nucleotides, about 35 nucleotides, about 40 nucleotides, about 50 nucleotides, about 75 nucleotides, about 100 nucleotides, or a range between any two of these values. In some embodiments, the primer has a length of 10 to about 50 nucleotides, *i.e.,* 10, 11, 12, 13, 14, 15, 16,, 17, 18, 19, 20, 21, 22, 23, 24, 25 26, 27, 28, 29, 30, 31, 32, 33, 34, 35, 36, 37, 38, 39, 40, 41, 42, 43, 44, 45, 46, 47, 48, 49, 50, or more nucleotides. In some embodiments, the primer has a length of 18 to 32 nucleotides.

As used herein, a "probe" can refer to an polynucleotide that can hybridizes (e.g., specifically) to a target sequence (e.g., unique identifier sequence) in a nucleic acid (e.g., cellular component-binding reagent specific oligonucleotide), under conditions that allow hybridization, thereby allowing detection of the target sequence or amplified nucleic acid. A probe's "target" generally refers to a sequence within or a subset of an amplified nucleic acid sequence which hybridizes specifically to at least a portion of a probe oligomer by standard hydrogen bonding (i.e., base pairing). A probe may comprise target-specific sequences and other sequences that contribute to three-dimensional conformation of the probe. Sequences are "sufficiently complementary" if they allow stable hybridization in appropriate hybridization conditions of a probe oligomer to a target sequence that is not completely complementary to the probe's target-specific sequence. The length of a probe can vary, for example, from about 5 to about 100 nucleotides, from about 10 to about 50 nucleotides, from about 15 to about 40 nucleotides, or from about 20 to about 30 nucleotides. The length of a probe can be about 10 nucleotides, about 20 nucleotides, about 25 nucleotides, about 30 nucleotides, about 35 nucleotides, about 40 nucleotides, about 50 nucleotides, about 100 nucleotides, or a range between any two of these values. In some embodiments, the probe has a length of 10 to about 50 nucleotides. For example, the primers and or probes can be at least 10, 11, 12, 13, 14, 15, 16,, 17, 18, 19, 20, 21, 22, 23, 24, 25 26, 27, 28, 29, 30, 31, 32, 33, 34, 35, 36, 37, 38, 39, 40, 41, 42, 43, 44, 45, 46, 47, 48, 49, 50, or more nucleotides. In some embodiments, the probe can be non-sequence specific.

Preferably, the primers and/or probes can be between 8 and 45 nucleotides in length. For example, the primers and or probes can be at least 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, or more nucleotides in length. The primer and probe can be modified to contain additional nucleotides at the 5' or the 3' terminus, or both. One of skill in the art will appreciate that additional bases to the 3' terminus of amplification primers (not necessarily probes) are generally complementary to the template sequence. The primer and probe sequences can also be modified to remove nucleotides at the 5' or the 3' terminus. One of skill in the art will appreciate that in order to function for amplification, the primers or probes will be of a minimum length and annealing temperature as disclosed herein.

Primers and probes can bind to their targets at an annealing temperature, which is a temperature less than the melting temperature (Tₘ). As used herein, "Tₘ" and "melting temperature" are interchangeable terms which refer to the temperature at which 50% of a population of double-stranded polynucleotide molecules becomes dissociated into single strands. The formulae for calculating the Tₘ of polynucleotides are well known in the art. For example, the Tₘ may be calculated by the following equation: Tₘ = 69.3+0.41 × (G+C)%-6-50/L, wherein L is the length of the probe in nucleotides. The Tₘ of a hybrid polynucleotide may also be estimated using a formula adopted from hybridization assays in 1 M salt, and commonly used for calculating Tₘ for PCR primers: [(number of A+T) × 2°C + (number of G+C) x 4°C]. *See, e.g.,* C. R. Newton et al. PCR, 2nd ed., Springer-Verlag (New York: 1997), p.24. Other more sophisticated computations exist in the art, which take structural as well as sequence characteristics into account for the calculation of Tₘ. The melting temperature of an oligonucleotide can depend on complementarity between the oligonucleotide primer or probe and the binding sequence, and on salt conditions. In some embodiments, an oligonucleotide primer or probe provided herein has a Tₘ of less than about 90°C in 50mM KCl, 10 mM Tris-HCl buffer, for example about 89°C, 88, 87, 86, 85, 84, 83, 82, 81, 80 79, 78, 77, 76, 75, 74, 73, 72, 71, 70, 69, 68, 67, 66, 65, 64, 63, 62, 61, 60, 59, 58, 57, 56, 55, 54, 53, 52, 50, 49, 48, 47, 46, 45, 44, 43, 42, 41, 40, 39°C, or less, including ranges between any two of the listed values.

In some embodiments, the primers disclosed herein, e.g., amplification primers, can be provided as an amplification primer pair, e.g., comprising a forward primer and a reverse primer (first amplification primer and second amplification primer). Preferably, the forward and reverse primers have Tₘ's that do not differ by more than 10°C, *e.g.,* that differ by less than 10°C, less than 9°C, less than 8°C, less than 7°C, less than 6°C, less than 5°C, less than 4°C, less than 3°C, less than 2°C, or less than 1°C.

The primer and probe sequences may be modified by having nucleotide substitutions (relative to the target sequence) within the oligonucleotide sequence, provided that the oligonucleotide contains enough complementarity to hybridize specifically to the target nucleic acid sequence. In this manner, at least 1, 2, 3, 4, or up to about 5 nucleotides can be substituted. As used herein, the term "complementary" can refer to sequence complementarity between regions of two polynucleotide strands or between two regions of the same polynucleotide strand. A first region of a polynucleotide is complementary to a second region of the same or a different polynucleotide if, when the two regions are arranged in an antiparallel fashion, at least one nucleotide of the first region is capable of base pairing with a base of the second region. Therefore, it is not required for two complementary polynucleotides to base pair at every nucleotide position. "Fully complementary" can refer to a first polynucleotide that is 100% or "fully" complementary to a second polynucleotide and thus forms a base pair at every nucleotide position. "Partially complementary" also can refer to a first polynucleotide that is not 100% complementary (e.g., 90%, or 80% or 70% complementary) and contains mismatched nucleotides at one or more nucleotide positions. In some embodiments, an oligonucleotide includes a universal base.

As used herein, an "exogenous nucleotide sequence" can refer to a sequence introduced by primers or probes used for amplification, such that amplification products will contain exogenous nucleotide sequence and target nucleotide sequence in an arrangement not found in the original template from which the target nucleotide sequence was copied.

As used herein, "sequence identity" or "percent identical" as applied to nucleic acid molecules can refer to the percentage of nucleic acid residues in a candidate nucleic acid molecule sequence that are identical with a subject nucleic acid molecule sequence, after aligning the sequences to achieve the maximum percent identity, and not considering any nucleic acid residue substitutions as part of the sequence identity. Nucleic acid sequence identity can be determined using any method known in the art, for example CLUSTALW, T-COFFEE, BLASTN.

As used herein, the term "sufficiently complementary" can refer to a contiguous nucleic acid base sequence that is capable of hybridizing to another base sequence by hydrogen bonding between a series of complementary bases. Complementary base sequences can be complementary at each position in the oligomer sequence by using standard base pairing (e.g., G:C, A:T or A:U) or can contain one or more residues that are not complementary (including abasic positions), but in which the entire complementary base sequence is capable of specifically hybridizing with another base sequence in appropriate hybridization conditions. Contiguous bases can be at least about 80%, at least about 85%, at least about 90%, at least about 95%, at least about 99%, or 100% complementary to a sequence to which an oligomer is intended to hybridize. Substantially complementary sequences can refer to sequences ranging in percent identity from 100, 99, 98, 97, 96, 95, 94, 93, 92, 91, 90, 89, 88, 87, 86, 85, 84, 83, 82, 81, 80, 75, 70 or less, or any number in between, compared to the reference sequence. A skilled artisan can readily choose appropriate hybridization conditions which can be predicted based on base sequence composition, or be determined by using routine testing (*see e.g.,* Green and Sambrook, Molecular Cloning, A Laboratory Manual, 4th ed. (Cold Spring Harbor Laboratory Press, Cold Spring Harbor, N.Y., 2012)).

As used herein, the term "multiplex PCR" refers to a type of PCR where more than one set of primers is included in a reaction allowing one single target, or two or more different targets, to be amplified in a single reaction vessel (e.g., tube). The multiplex PCR can be, for example, a real-time PCR. A plurality of oligonucleotide probes, each configured to bind a unique identifier sequence, or a complement thereof, can be employed in a single amplification reaction. For example, in some embodiments, the number of different oligonucleotide probes being employed in a single reaction (each configured to detect a different target sequence (e.g., different unique identifier sequence) and thereby, detect a different cellular component target and cellular component binding reagent) can be, or can be about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, 1000, or a number or a range between any two of these values.

Oligonucleotide probes can, in some embodiments, include a detectable moiety. For example, the oligonucleotide probes disclosed herein can comprise a radioactive label. Non-limiting examples of radioactive labels include ³H, ¹⁴C, ³²P, and ³⁵S. In some embodiments, oligonucleotide probes can include one or more non-radioactive detectable markers or moieties, including but not limited to ligands, fluorophores, chemiluminescent agents, enzymes, and antibodies. Other detectable markers for use with probes, which can enable an increase in sensitivity of the method of the invention, include biotin and radio-nucleotides. It will become evident to the person of ordinary skill that the choice of a particular label dictates the manner in which it is bound to the probe. For example, oligonucleotide probes labeled with one or more dyes, such that upon hybridization to a template nucleic acid, a detectable change in fluorescence is generated. While non-specific dyes may be desirable for some applications, sequence-specific probes can provide more accurate measurements of amplification. One configuration of sequence-specific probe can include one end of the probe tethered to a fluorophore, and the other end of the probe tethered to a quencher. When the probe is unhybridized, it can maintain a stem-loop configuration, in which the fluorophore is quenched by the quencher, thus preventing the fluorophore from fluorescing. When the probe is hybridized to a template nucleic sequence, it is linearized, distancing the fluorophore from the quencher, and thus permitting the fluorophore to fluoresce. Another configuration of sequence-specific probe can include a first probe tethered to a first fluorophore of a FRET pair, and a second probe tethered to a second fluorophore of a FRET pair. The first probe and second probe can be configured to hybridize to sequences of an amplicon that are within sufficient proximity to permit energy transfer by FRET when the first probe and second probe are hybridized to the same amplicon.

In some embodiments the probe is a TaqMan probe. TaqMan probes can comprise a fluorophore and a quencher. The quencher molecule can quench the fluorescence emitted by the fluorophore when excited by the cycler's light source via Förster resonance energy transfer (FRET). As long as the fluorophore and the quencher are in proximity, quenching can inhibit any detectable (e.g., fluorescence) signals. TaqMan probes provided herein can designed such that they anneal within a DNA region amplified by primers provided herein. Without being bound by any particular theory, in some embodiments, as a PCR polymerase (e.g., Taq) extends the primer and synthesizes a nascent strand on a single-strand template, the 5' to 3' exonuclease activity of the PCR polymerase degrades the probe that has annealed to the template. Degradation of the probe can release the fluorophore from it and break the proximity to the quencher, thereby relieving the quenching effect and allowing fluorescence of the fluorophore. Hence, fluorescence detected in the quantitative PCR thermal cycler can, in some embodiments, be directly proportional to the fluorophore released and the amount of DNA template present in the PCR.

In some embodiments, the sequence specific probe comprises an oligonucleotide as disclosed herein (e.g., an oligonucleotide probe comprising a sequence configured to bind a unique identifier sequence, or a complement thereof) conjugated to a fluorophore. In some embodiments, the probe is conjugated to two or more fluorophores. Examples of fluorophores include: xanthene dyes, e.g., fluorescein and rhodamine dyes, such as fluorescein isothiocyanate (FITC), 2-[ethylamino)-3-(ethylimino)-2-7-dimethyl-3H-xanthen-9-yl]benzoic acid ethyl ester monohydrochloride (R6G)(emits a response radiation in the wavelength that ranges from about 500 to 560 nm), 1,1,3,3,3',3'-Hexamethylindodicarbocyanine iodide (HIDC) (emits a response radiation in the wavelength that ranged from about 600 to 660 nm), 6-carboxyfluorescein (commonly known by the abbreviations FAM and F), 6-carboxy-2',4',7',4,7-hexachlorofluorescein (HEX), 6-carboxy-4',5'-dichloro-2',7'-dimethoxyfluorescein (JOE or J), N,N,N',N'-tetramethyl-6-carboxyrhodamine (TAMRA or T), 6-carboxy-X-rhodamine (ROX or R), 5-carboxyrhodamine-6G (R6G5 or G5), 6-carboxyrhodamine-6G (R6G6 or G6), and rhodamine 110; cyanine dyes, e.g. Cy3, Cy5 and Cy7 dyes; coumarins, e.g., umbelliferone; benzimide dyes, e.g. Hoechst 33258; phenanthridine dyes, e.g. Texas Red; ethidium dyes; acridine dyes; carbazole dyes; phenoxazine dyes; porphyrin dyes; polymethine dyes, e.g. cyanine dyes such as Cy3 (emits a response radiation in the wavelength that ranges from about 540 to 580 nm), Cy5 (emits a response radiation in the wavelength that ranges from about 640 to 680 nm), etc; BODIPY dyes and quinoline dyes. Specific fluorophores of interest include: Pyrene, Coumarin, Diethylaminocoumarin, FAM, Fluorescein Chlorotriazinyl, Fluorescein, R110, Eosin, JOE, R6G, HIDC, Tetramethylrhodamine, TAMRA, Lissamine, ROX, Napthofluorescein, Texas Red, Napthofluorescein, Cy3, and Cy5, CAL fluor orange, and the like. Other examples of fluorescein dyes include 6-carboxyfluorescein (6-FAM), 2',4',1,4,-tetrachlorofluorescein (TET), 2',4',5',7',1,4-hexachlorofluorescein (HEX), 2',7'-dimethoxy-4',5'-dichloro-6-carboxyrhodamine (JOE), 2'-chloro-5'-fluoro-7',8'-fused phenyl-1,4-dichloro-6-carboxyfluorescein (NED), and 2'-chloro-7'-phenyl-1,4-dichloro-6-carboxyfluorescein (VIC). Probes can comprise SpC6, or functional equivalents and derivatives thereof. Probes can comprise a spacer moiety. A spacer moiety can comprise an alkyl group of at least 2 carbons to about 12 carbons. A probe can comprise a spacer comprising an abasic unit. A probe can comprise a spacer selected from the group comprising of idSp, iSp9, iS18, iSpC3, iSpC6, iSpC12, or any combination thereof.

In some embodiments, the probe is conjugated to a quencher. A quencher can absorb electromagnetic radiation and dissipate it as heat, thus remaining dark. Example quenchers include Dabcyl, NFQ's, such as BHQ-1 or BHQ-2 (Biosearch), IOWA BLACK FQ (IDT), and IOWA BLACK RQ (IDT). In some embodiments, the quencher is selected to pair with a fluorphore so as to absorb electromagnetic radiation emitted by the fluorophore. Flourophore/quencher pairs useful in the compositions and methods disclosed herein are well-known in the art, and can be found, e.g., described in Marras, "Selection of Fluorophore and Quencher Pairs for Fluorescent Nucleic Acid Hybridization Probes" available at www.molecular-beacons.org/download/marras,mmb06%28335%293.pdf. Examples of quencher moieties include, but are not limited to: a dark quencher, a Black Hole Quencher^{®} (BHQ^{®}) (e.g., BHQ-0, BHQ-1, BHQ-2, BHQ-3), a Qxl quencher, an ATTO quencher (e.g., ATTO 540Q, ATTO 580Q, and ATTO 612Q), dimethylaminoazobenzenesulfonic acid (Dabsyl), Iowa Black RQ, Iowa Black FQ, IRDye QC-1, a QSY dye (e.g., QSY 7, QSY 9, QSY 21), AbsoluteQuencher, Eclipse, and metal clusters such as gold nanoparticles, and the like. Examples of an ATTO quencher include, but are not limited to: ATTO 540Q, ATTO 580Q, and ATTO 612Q. Examples of a Black Hole Quencher^{®} (BHQ^{®}) include, but are not limited to: BHQ-0 (493 nm), BHQ-1 (534 nm), BHQ-2 (579 nm) and BHQ-3 (672 nm).

In some embodiments, a detectable label is a fluorescent label selected from: an Alexa Fluor^{®} dye (e.g., Alexa Fluor^{®} 350, Alexa Fluor^{®} 405, Alexa Fluor^{®} 430, Alexa Fluor^{®} 488, Alexa Fluor^{®} 500, Alexa Fluor^{®} 514, Alexa Fluor^{®} 532, Alexa Fluor^{®} 546, Alexa Fluor^{®} 555, Alexa Fluor^{®} 568, Alexa Fluor^{®} 594, Alexa Fluor^{®} 610, Alexa Fluor^{®} 633, Alexa Fluor^{®} 635, Alexa Fluor^{®} 647, Alexa Fluor^{®} 660, Alexa Fluor^{®} 680, Alexa Fluor^{®} 700, Alexa Fluor^{®} 750, Alexa Fluor^{®} 790), an ATTO dye (e.g., ATTO 390, ATTO 425, ATTO 465, ATTO 488, ATTO 495, ATTO 514, ATTO 520, ATTO 532, ATTO Rho6G, ATTO 542, ATTO 550, ATTO 565, ATTO Rho3B, ATTO Rhol 1, ATTO Rhol2, ATTO Thiol 2, ATTO RholOl, ATTO 590, ATTO 594, ATTO Rhol3, ATTO 610, ATTO 620, ATTO Rhol4, ATTO 633, ATTO 647, ATTO 647N, ATTO 655, ATTO Oxal2, ATTO 665, ATTO 680, ATTO 700, ATTO 725, ATTO 740), a DyFight dye, a cyanine dye (e.g., Cy2, Cy3, Cy3.5, Cy3b, Cy5, Cy5.5, Cy7, Cy7.5), a FluoProbes dye, a Sulfo Cy dye, a Seta dye, an IRIS Dye, a SeTau dye, an SRfluor dye, a Square dye, fluorescein (FITC), tetramethylrhodamine (TRITC), Texas Red, Oregon Green, Pacific Blue, Pacific Green, Pacific Orange, a quantum dot, and a tethered fluorescent protein.

In some embodiments, a fluorophore is attached to a first end of the probe, and a quencher is attached to a second end of the probe. In some embodiments, a probe can comprise two or more fluorophores. In some embodiments, a probe can comprise two or more quencher moieties. In some embodiments, a probe can comprise one or more quencher moieties and/or one or more fluorophores. A quencher moiety or a fluorophore can be attached to any portion of a probe (e.g., on the 5' end, on the 3' end, and/or in the middle of the probe). Any probe nucleotide can comprise a fluorophore or a quencher moiety, such as, for example, BHQ1dT. Attachment can include covalent bonding, and can optionally include at least one linker molecule positioned between the probe and the fluorophore or quencher. In some embodiments, a fluorophore is attached to a 5' end of a probe, and a quencher is attached to a 3' end of a probe. In some embodiments, a fluorophore is attached to a 3' end of a probe, and a quencher is attached to a 5' end of a probe. Examples of probes that can be used in quantitative nucleic acid amplification include molecular beacons, SCORPION^{™} probes (Sigma), TAQMAN^{™} probes (Life Technologies) and the like. Other nucleic acid detection technologies that are useful in the embodiments disclosed herein include, but are not limited to nanoparticle probe technology (*See,* Elghanian, et al. (1997) Science 277:1078-1081.) and Amplifluor probe technology (*See,* U.S. Pat. Nos: 5,866,366; 6,090,592; 6,117,635; and 6,117,986).

The composition can comprise: a plurality of oligonucleotide probes, wherein each of the plurality of oligonucleotide probes comprises a sequence configured to bind a unique identifier sequence, or a complement thereof. At least one of the plurality of probes can comprise a fluorescence emitter moiety and a fluorescence quencher moiety.

Any probes described herein can comprise a fluorescence emitter moiety, a fluorescence quencher moiety, or both.

As disclosed herein, a reaction mixture can comprise one or more of the primers disclosed herein, one or more of the probes disclosed herein (e.g., the fluorophore-containing probes), or any combination thereof. In some embodiments, the reaction mixture comprises one or more of the primer and/or probe-containing composition disclosed herein. The reaction mixture can also comprise various additional components. Examples of the additional components in the reaction mixture include, but are not limited to, template DNA, DNA polymerase (e.g., Taq DNA polymerase), deoxynucleotides (dNTPs), buffer solution, biovalent cations, monovalent cation potassium ions, and any combination thereof. In some embodiments, the reaction mixture is a master mix for real-time PCR.

As used herein, nucleic acid amplification can refer to any known procedure for obtaining multiple copies of a target nucleic acid sequence or its complement or fragments thereof, using sequence-specific methods. Examples of known amplification methods include, but are not limited to, polymerase chain reaction (PCR), ligase chain reaction (LCR), loop-mediated isothermal amplification (LAMP), strand displacement amplification (SDA) (e.g., multiple displacement amplification (MDA)), replicase-mediated amplification, immuno-amplification, nucleic acid sequence based amplification (NASBA), self-sustained sequence replication (3SR), rolling circle amplification, and transcription-mediated amplification (TMA).

For example, LCR amplification uses at least four separate oligonucleotides to amplify a target and its complementary strand by using multiple cycles of hybridization, ligation, and denaturation. SDA amplifies by using a primer that contains a recognition site for a restriction endonuclease which nicks one strand of a hemimodified DNA duplex that includes the target sequence, followed by amplification in a series of primer extension and strand displacement steps.

PCR is a method well-known in the art for amplification of nucleic acids. PCR involves amplification of a target sequence using two or more extendable sequence-specific oligonucleotide primers that flank the target sequence. The nucleic acid containing the target sequence of interest is subjected to a program of multiple rounds of thermal cycling (denaturation, annealing and extension) in the presence of the primers, a thermostable DNA polymerase (e.g., Taq polymerase) and various dNTPs, resulting in amplification of the target sequence. PCR uses multiple rounds of primer extension reactions in which complementary strands of a defined region of a DNA molecule are simultaneously synthesized by a thermostable DNA polymerase. At the end of each cycle, each newly synthesized DNA molecule acts as a template for the next cycle. During repeated rounds of these reactions, the number of newly synthesized DNA strands increases exponentially such that after 20 to 30 reaction cycles, the initial template DNA will have been replicated several thousand-fold or million-fold.

PCR can generate double-stranded amplification products suitable for post-amplification processing. Amplification products can be detected by visualization with agarose gel electrophoresis, by an enzyme immunoassay format using probe-based colorimetric detection, by fluorescence emission technology, or by other detection means known in the art.

Examples of PCR method include, but not limited to, Real-Time PCR, EndPoint PCR, Amplified fragment length polymorphism PCR (AFLP-PCR), Alu-PCR, Asymmetric PCR, Colony PCR, DD-PCR, Degenerate PCR, Hot-start PCR, In situ PCR, Inverse PCR Long-PCR, Multiplex PCR, Nested PCR, PCR-ELISA, PCR-RFLP, PCR-single strand conformation polymorphism (PCR-SSCP), quantitative competitive PCR (QC-PCR), rapid amplification of cDNA ends-PCR (RACE-PCR), Random Amplification of Polymorphic DNA-PCR (RAPD-PCR), Real-Time PCR, Repetitive extragenic palindromic-PCR (Rep-PCR), reverse transcriptase PCR (RT-PCR), TAIL-PCR, Touchdown PCR and Vectorette PCR.

As described herein, exemplary methods for polymerizing and/or amplifying nucleic acids include, for example, polymerase-mediated extension reactions. For instance, the polymerase- mediated extension reaction can be the polymerase chain reaction (PCR). In some embodiments, the nucleic acid amplification reaction is a multiplex reaction. For instance, exemplary methods for polymerizing and/or amplifying and detecting nucleic acids suitable for use as described herein are commercially available as TaqMan^{®} assays. TaqMan^{®} assays are typically carried out by performing nucleic acid amplification on a target polynucleotide using a nucleic acid polymerase having 5'-to-3' nuclease activity, a primer capable of hybridizing to the target polynucleotide, and an oligonucleotide probe capable of hybridizing to the target polynucleotide 3' relative to the primer. The oligonucleotide probe typically includes a detectable label (e.g., a fluorescent reporter molecule) and a quencher molecule capable of quenching the fluorescence of the reporter molecule. Typically, the detectable label and quencher molecule are part of a single probe. In some embodiments, the detectably labeled probe of the amplification reaction contains a fluorescent label at its 5' end and a quencher at its 3' end. As amplification proceeds, the polymerase digests the probe to separate the detectable label from the quencher molecule. Thus, in some preferred embodiments, the detectably labeled probe of the amplification reaction is configured to undergo cleavage by a polymerase in a 5' nuclease assay (such as in a TaqMan^{®} assay). The detectable label (e.g., fluorescence) is monitored during the reaction, where detection of the label corresponds to the occurrence of nucleic acid amplification (e.g., the higher the signal the greater the amount of amplification). Variations of TaqMan^{®} assays (e.g., LNA^{™} spiked TaqMan^{®} assay) are known in the art and would be suitable for use in the methods described herein.

Real-time PCR, also called quantitative real time polymerase chain reaction (QRT-PCR), can be used to simultaneously quantify and amplify a specific part of a given nucleic acid molecule. It can be used to determine whether a specific sequence is present in the sample; and if it is present, the number of copies of the sequence that are present. The term "real-time" can refer to periodic monitoring during PCR. Certain systems such as the ABI 7700 and 7900HT Sequence Detection Systems (Applied Biosystems, Foster City, CA.) conduct monitoring during each thermal cycle at a pre-determined or user-defined point. Real-time analysis of PCR with fluorescence resonance energy transfer (FRET) probes measures fluorescent dye signal changes from cycle-to-cycle, preferably minus any internal control signals. The real-time procedure follows the general pattern of PCR, but the nucleic acid is quantified after each round of amplification. Two examples of method of quantification are the use of fluorescent dyes (e.g., SYBRGreen) that intercalate into double-stranded DNA, and modified DNA oligonucleotide probes that fluoresce when hybridized with a complementary DNA. Intercalating agents have a relatively low fluorescence when unbound, and a relatively high fluorescence upon binding to double-stranded nucleic acids. As such, intercalating agents can be used to monitor the accumulation of double strained nucleic acids during a nucleic acid amplification reaction. Examples of such non-specific dyes useful in the embodiments disclosed herein include intercalating agents such as SYBR Green I (Molecular Probes), propidium iodide, ethidium bromide, and the like.

The oligonucleotide probe can be, for example, between about 10 and about 45 nucleotides in length, and comprises a detectable moiety (e.g., a signal moiety, a detectable label). In some embodiments, the contacting is performed under conditions allowing for the specific hybridization of the primers to the corresponding targeted gene region if the target organism is present in the sample. The presence and/or amount of probe that is specifically bound to the corresponding targeted gene region (if present in the sample being tested) can be determined, wherein bound probe is indicative of the presence of the corresponding target organism in the sample. In some embodiments, the amount of bound probe is used to determine the amount of the corresponding target organism in the sample.

The determining step can be achieved using any methods known to those skilled in the art, including but not limited to, *in situ* hybridization, following the contacting step. The detection of hybrid duplexes (*i.e.,* of a probe specifically bound to the targeted gene region) can be carried out by a number of methods. Typically, hybridization duplexes are separated from unhybridized nucleic acids and the labels bound to the duplexes are then detected. Such labels refer to radioactive, fluorescent, biological or enzymatic tags or labels of standard use in the art. A label can be conjugated to either the oligonucleotide probes or the nucleic acids derived from the biological sample. Those of skill in the art will appreciate that wash steps may be employed to wash away excess sample/target nucleic acids or oligonucleotide probe (as well as unbound conjugate, where applicable). Further, standard heterogeneous assay formats are suitable for detecting the hybrids using the labels present on the oligonucleotide primers and probes. Determining the presence or amount of one or more amplicons can comprise contacting said amplicons with a plurality of oligonucleotide probes. At least one of the plurality of oligonucleotide probes comprises a fluorescence emitter moiety and a fluorescence quencher moiety. In some embodiments, determining the presence or amount of one or more amplicons comprises measuring a detectable signal, such as, for example, a detectable signal from a probe.

In some embodiments, determining the presence or amount of one or more amplicons comprises measuring a detectable signal, such as, for example, a detectable signal from a probe (e.g., after cleavage of the probe by the 5'-3' exonuclease activity of a PCR polymerase (e.g., Taq)). Determining the presence or amount of one or more amplicons can comprise measuring a detectable signal, such as, for example, a detectable signal from a probe. The measuring can in some embodiments be quantitative, e.g., in the sense that the amount of signal detected can be used to determine the amount of target nucleic acid (e.g., a unique identifier sequence, or a complement thereof, of one or more first cellular component-binding reagents of interest) present in the sample. The measuring can in some embodiments be qualitative, e.g., in the sense that the presence or absence of detectable signal can indicate the presence or absence of targeted DNA (e.g., virus, SNP, etc.). In some embodiments, a detectable signal will not be present (e.g., above a given threshold level) unless the targeted DNA(s) (e.g., virus, SNP, etc.) is present above a particular threshold concentration. In some embodiments, a disclosed method can be used to determine the amount of a target nucleic acid (e.g., a unique identifier sequence, or a complement thereof, of one or more first cellular component-binding reagents of interest) in a sample (e.g., a sample comprising the target nucleic acid and a plurality of non-target nucleic acids). Determining the amount of a target nucleic acid in a sample can comprise comparing the amount of detectable signal generated from a test sample to the amount of detectable signal generated from a reference sample. Determining the amount of a target nucleic acid in a sample can comprise: measuring the detectable signal to generate a test measurement; measuring a detectable signal produced by a reference sample to generate a reference measurement; and comparing the test measurement to the reference measurement to determine an amount of target nucleic acid present in the sample. Determining the amount of a target nucleic acid in a sample can be used to derive the presence and/or amount of an organism comprising said target nucleic acid in a sample.

In some embodiments, a detectable signal is measured is produced by the fluorescence-emitting dye pair of a probe. For example, in some embodiments, a disclosed method includes contacting amplicons with a probe comprising a FRET pair or a quencher/fluor pair, or both. In some embodiments, a disclosed method includes contacting amplicons with a probe comprising a FRET pair. In some embodiments, a disclosed method includes contacting amplicons with a probe comprising a fluor/quencher pair.

Fluorescence-emitting dye pairs comprise a FRET pair or a quencher/fluor pair. In both embodiments of a FRET pair and a quencher/fluor pair, the emission spectrum of one of the dyes overlaps a region of the absorption spectrum of the other dye in the pair. As used herein, the term "fluorescence-emitting dye pair" is a generic term used to encompass both a "FRET pair" and a "quencher/fluor pair." The term "fluorescence-emitting dye pair" is used interchangeably with the phrase "a FRET pair and/or a quencher/fluor pair."

In some embodiments (e.g., when the probe includes a FRET pair) the probe produces an amount of detectable signal prior to being cleaved, and the amount of detectable signal that is measured is reduced when the probe is cleaved. In some embodiments, the probe produces a first detectable signal prior to being cleaved (e.g., from a FRET pair) and a second detectable signal when the probe is cleaved (e.g., from a quencher/fluor pair). As such, in some embodiments, the probe comprises a FRET pair and a quencher/fluor pair.

The probe can comprise a FRET pair. FRET is a process by which radiationless transfer of energy occurs from an excited state fluorophore to a second chromophore in close proximity. The range over which the energy transfer can take place is limited to approximately 10 nanometers (100 angstroms), and the efficiency of transfer is extremely sensitive to the separation distance between fluorophores. The term "FRET" (also known as "Forster resonance energy transfer") can refer to a physical phenomenon involving a donor fluorophore and a matching acceptor fluorophore selected so that the emission spectrum of the donor overlaps the excitation spectrum of the acceptor, and further selected so that when donor and acceptor are in close proximity (usually 10 nm or less) to one another, excitation of the donor will cause excitation of and emission from the acceptor, as some of the energy passes from donor to acceptor via a quantum coupling effect. Thus, a FRET signal serves as a proximity gauge of the donor and acceptor; only when they are in close proximity to one another is a signal generated. The FRET donor moiety (e.g., donor fluorophore) and FRET acceptor moiety (e.g., acceptor fluorophore) are collectively referred to herein as a "FRET pair".

The donor-acceptor pair (a FRET donor moiety and a FRET acceptor moiety) is referred to herein as a "FRET pair" or a "signal FRET pair." Thus, in some embodiments, a probe includes two signal partners (a signal pair), when one signal partner is a FRET donor moiety and the other signal partner is a FRET acceptor moiety. A probe that includes such a FRET pair (a FRET donor moiety and a FRET acceptor moiety) will thus exhibit a detectable signal (a FRET signal) when the signal partners are in close proximity (e.g., while on the same RNA molecule), but the signal will be reduced (or absent) when the partners are separated (e.g., after cleavage of the probe by the 5'-3' exonuclease activity of a PCR polymerase (e.g., Taq)). FRET donor and acceptor moieties (FRET pairs) will be known to one of skill in the art and any convenient FRET pair (e.g., any convenient donor and acceptor moiety pair) can be used.

In some embodiments, one signal partner of a signal quenching pair produces a detectable signal and the other signal partner is a quencher moiety that quenches the detectable signal of the first signal partner (e.g., the quencher moiety quenches the signal of the signal moiety such that the signal from the signal moiety is reduced (quenched) when the signal partners are in proximity to one another, e.g., when the signal partners of the signal pair are in close proximity).

For example, in some embodiments, an amount of detectable signal increases when the probe is cleaved. For example, in some embodiments, the signal exhibited by one signal partner (a signal moiety, a fluorescence emitter moiety) is quenched by the other signal partner (a quencher signal moiety, a fluorescence quencher moiety), e.g., when both are present on the same ssDNA molecule prior to cleavage by the 5'-3' exonuclease activity of a PCR polymerase (e.g., Taq). Such a signal pair is referred to herein as a "quencher/fluor pair", "quenching pair", or "signal quenching pair." For example, in some embodiments, one signal partner (e.g., the first signal partner) is a signal moiety that produces a detectable signal that is quenched by the second signal partner (e.g., a quencher moiety). The signal partners of such a quencher/fluor pair will thus produce a detectable signal when the partners are separated (e.g., after cleavage of the probe by the 5'-3' exonuclease activity of a PCR polymerase (e.g., Taq)), but the signal will be quenched when the partners are in close proximity (e.g., prior to cleavage of the probe by the 5'-3' exonuclease activity of a PCR polymerase (e.g., Taq)).

A quencher moiety can quench a signal from the signal moiety (e.g., prior to cleavage of the probe by the 5'-3' exonuclease activity of a PCR polymerase (e.g., Taq)) to various degrees. In some embodiments, a quencher moiety quenches the signal from the signal moiety where the signal detected in the presence of the quencher moiety (when the signal partners are in proximity to one another) is 95% or less of the signal detected in the absence of the quencher moiety (when the signal partners are separated). For example, the signal detected in the presence of the quencher moiety can be 90% or less, 80% or less, 70% or less, 60% or less, 50% or less, 40% or less, 30% or less, 20% or less, 15% or less, 10% or less, or 5% or less of the signal detected in the absence of the quencher moiety. In some embodiments, no signal (e.g., above background) is detected in the presence of the quencher moiety.

In some embodiments, the signal detected in the absence of the quencher moiety (when the signal partners are separated) is at least 1.2 fold greater (e.g., at least 1.3fold, at least 1.5 fold, at least 1.7 fold, at least 2 fold, at least 2.5 fold, at least 3 fold, at least 3.5 fold, at least 4 fold, at least 5 fold, at least 7 fold, at least 10 fold, at least 20 fold, or at least 50 fold greater, or a number or a range between any two of these values) than the signal detected in the presence of the quencher moiety (when the signal partners are in proximity to one another).

In some embodiments, the signal moiety is a fluorescent label. In some such embodiments, the quencher moiety quenches the signal (e.g., the light signal) from the fluorescent label (e.g., by absorbing energy in the emission spectra of the label). Thus, when the quencher moiety is not in proximity with the signal moiety, the emission (the signal) from the fluorescent label can be detectable because the signal is not absorbed by the quencher moiety. Any convenient donor acceptor pair (signal moiety /quencher moiety pair) can be used and many suitable pairs are known in the art.

In some embodiments, the quencher moiety absorbs energy from the signal moiety (also referred to herein as a "detectable label" or a "detectable moiety") and then emits a signal (e.g., light at a different wavelength). Thus, in some embodiments, the quencher moiety is itself a signal moiety (e.g., a signal moiety can be 6- carboxyfluorescein while the quencher moiety can be 6-carboxy-tetramethylrhodamine), and in some such embodiments, the pair could also be a FRET pair. In some embodiments, a quencher moiety is a dark quencher. A dark quencher can absorb excitation energy and dissipate the energy in a different way (e.g., as heat). Thus, a dark quencher has minimal to no fluorescence of its own (does not emit fluorescence).

Cleavage of a probe can be detected by measuring a colorimetric read-out. For example, the liberation of a fluorophore (e.g., liberation from a FRET pair, liberation from a quencher/fluor pair, and the like) can result in a wavelength shift (and thus color shift) of a detectable signal. Thus, in some embodiments, cleavage of a probe can be detected by a color-shift. Such a shift can be expressed as a loss of an amount of signal of one color (wavelength), a gain in the amount of another color, a change in the ration of one color to another, and the like.

Amplification can be carried out using one or more of polymerase chain reaction (PCR), ligase chain reaction (LCR), loop-mediated isothermal amplification (LAMP), strand displacement amplification (SDA), replicase-mediated amplification, Immuno-amplification, nucleic acid sequence based amplification (NASBA), self-sustained sequence replication (3SR), rolling circle amplification, and transcription-mediated amplification (TMA). The PCR can be real-time PCR or quantitative real-time PCR (QRT-PCR). Each primer can comprise exogenous nucleotide sequence.

The primers suitable for use in the methods and compositions described herein can comprise exogenous nucleotide sequence which allows post-amplification manipulation of amplification products without a significant effect on amplification itself. In some embodiments, the primer and/or probe can be flanked by complementary sequences comprising a fluorophore at the 5' end, and a fluorescence quencher at the 3' end.

Any of the oligonucleotide probes disclosed herein can comprise a fluorescence emitter moiety, a fluorescence quencher moiety, or both.

In some embodiments, amplification comprises a two-step reaction including without limitation, a pre-amplification step wherein a limited number of cycles of amplification occur (for example, but not limited to, 2, 3, 4, or 5 cycles of amplification), then the resulting amplicon is generally diluted and portions of the diluted amplicon are subjected to additional cycles of amplification in a subsequent amplification step.

An amplification reaction can comprises a plurality or multiplicity of single-plex reactions performed in parallel under the same assay conditions and/or at substantially the same time. In some embodiments, performing the plurality of amplification reactions in parallel forms a plurality of different amplification products. In some embodiments, performing the plurality of amplification reactions in parallel can form between 10 and 10,000 different amplification products, between 10 and 1000 different amplification products, between 10 and 100 different amplification products, or between 10 and 50 different amplification products. In some embodiments, an amplification reaction comprises multiplex amplification, in which a multiplicity of different target nucleic acids and/or a multiplicity of different amplification product species are simultaneously amplified using a multiplicity of different primer sets. In some embodiments, a multiplex amplification reaction and a single-plex amplification reaction, including a multiplicity of single-plex or lower -plexy reactions (for example, but not limited to a two-plex, a three-plex, a four-plex, a five-plex or a six-plex reaction) are performed in parallel.

In addition to 5 '-nuclease probes, such as the probes used in TaqMan^{®} assays, various probes are known in the art and suitable for use in detecting amplified nucleic acids in the provided methods. Exemplary probes include, but are not limited to, stem-loop molecular beacons, stemless or linear beacons, PNA Molecular Beacons^{™}, linear PNA beacons, non-FRET probes, Sunrise^{®}/Amplifluor^{®} probes, stem-loop and duplex Scorpions^{™} probes, bulge loop probes, pseudo knot probes, cyclicons, MGB Eclipse^{™} probe (Epoch Biosciences), hairpin probes, peptide nucleic acid (PNA) light-up probes, self- assembled nanoparticle probes, ferrocene-modified probes; QuantiProbes^{®} (Qiagen), HyBeacons^{®}, HybProbes, MGB Alert (www.nanogen.com), Q-PNA, Plexor^{™} (Promega), LUX^{™} primers, and DzyNA primers. Detectably-labeled probes can comprise non-detectable quencher moieties that quench the fluorescence of the detectable label, including, for example, black hole quenchers (Biosearch), Iowa Black^{™} quenchers (IDT), QSY quencher (Molecular Probes^{™}; Thermo Fisher Scientific), and Dabsyl and Dabcyl sulfonate/carboxylate Quenchers (Epoch). Detectably-labeled probes may also comprise two probes, wherein for example a fluorophore is on one probe, and a quencher is on the other, wherein hybridization of the two probes together on a target quenches the signal, or wherein hybridization on a target alters the signal signature via a change in fluorescence. Exemplary systems may also include FRET, salicylate/DTPA ligand systems. Detectable labels can also comprise sulfonate derivatives of fluorescein dyes with S03 instead of the carboxylate group, phosphoramidite forms of fluorescein, phosphoramidite forms of Cy5 (available for example from Amersham).

As described herein, one or more detectable labels and/or quenching agents may be attached to one or more primers and/or probes (e.g., detectable label). The detectable label may emit a signal when free or when bound to one of the target nucleic acids. The detectable label may also emit a signal when in proximity to another detectable label. Detectable labels may also be used with quencher molecules such that the signal is only detectable when not in sufficiently close proximity to the quencher molecule. For instance, in some embodiments, the assay system may cause the detectable label to be liberated from the quenching molecule. Any of several detectable labels may be used to label the primers and probes used in the methods described herein. As described herein, in some embodiments the detectable label may be attached to a probe, which may be incorporated into a primer, or may otherwise bind to amplified target nucleic acid (e.g., a detectable nucleic acid binding agent such as an intercalating or non-intercalating dye). When using more than one detectable label, each should differ in their spectral properties such that the labels may be distinguished from each other, or such that together the detectable labels emit a signal that is not emitted by either detectable label alone. Exemplary detectable labels include, for instance, a fluorescent dye or fluorophore (e.g., a chemical group that can be excited by light to emit fluorescence or phosphorescence), "acceptor dyes" capable of quenching a fluorescent signal from a fluorescent donor dye, and the like. Suitable detectable labels may include, for example, fluoresceins (e.g., 5-carboxy-2,7-dichlorofluorescein; 5- Carboxyfluorescein (5-FAM); 5-Hydroxy Tryptamine (5-HAT); 6-JOE; 6-carboxyfluorescein (6- FAM); FITC; 6-carboxy-1,4-dichloro-2',7'-dichlorofluorescein (TET); 6-carboxy-l,4-dichloro- 2' ,4 ' ,5 ' ,7 ' -tetrachlorofluorescein (HEX) ; 6-carboxy-4 ' ,5' -dichloro-2' ,7 ' -dimethoxyfluorescein (JOE) ; Alexa fluor^{®} fluorophores (e.g., 350, 405, 430, 488, 500, 514, 532, 546, 555, 568, 594, 610, 633, 635, 647, 660, 680, 700, 750); BODIPY^{™} fluorophores (e.g., 492/515, 493/503, 500/510, 505/515, 530/550, 542/563, 558/568, 564/570, 576/589, 581/591, 630/650-X, 650/665-X, 665/676, FL, FL ATP, Fl-Ceramide, R6G SE, TMR, TMR-X conjugate, TMR-X, SE, TR, TR ATP, TR-X SE), coumarins (e.g., 7-amino-4-methylcoumarin, AMC, AMCA, AMCA-S, AMCA-X, ABQ, CPM methylcoumarin, coumarin phalloidin, hydroxycoumarin, CMFDA, methoxycoumarin), calcein, calcein AM, calcein blue, calcium dyes (e.g., calcium crimson, calcium green, calcium orange, calcofluor white), Cascade Blue, Cascade Yellow; CyTM dyes (e.g., 3, 3.18, 3.5, 5, 5.18, 5.5, 7), cyan GFP, cyclic AMP Fluorosensor (FiCRhR), fluorescent proteins (e.g., green fluorescent protein (e.g., GFP. EGFP), blue fluorescent protein (e.g., BFP, EBFP, EBFP2, Azurite, mKalamal), cyan fluorescent protein (e.g., ECFP, Cerulean, CyPet), yellow fluorescent protein (e.g., YFP, Citrine, Venus, YPet), FRET donor/acceptor pairs (e.g., fluorescein/tetramethylrhodamine, IAEDANS/fluorescein, EDANS/dabcyl, fluorescein/fluorescein, BODIPY^{®} FL/BODIPY^{®} FL, Fluorescein/QSY7 and QSY9), LysoTracker^{®} and LysoSensor^{™} (e.g., LysoTracker^{®} Blue DND-22, LysoTracker^{®} Blue-White DPX, LysoTracker^{®} Yellow HCK-123, LysoTracker^{®} Green DND-26, LysoTracker^{®} Red DND-99, LysoSensor^{™} Blue DND-167, LysoSensor^{™} Green DND-189, LysoSensor^{™} Green DND-153, LysoSensor^{™} Yellow/Blue DND-160, LysoSensor^{™} Yellow/Blue 10,000 MW dextran), Oregon Green (e.g., 488, 488-X, 500, 514); rhodamines (e.g., 110, 123, B, B 200, BB, BG, B extra, 5-carboxytetramethylrhodamine (5-TAMRA), 5 GLD, 6-Carboxyrhodamine 6G, Lissamine, Lissamine Rhodamine B, Phallicidine, Phalloidine, Red, Rhod-2, ROX (6-carboxy-X- rhodamine), 5-ROX (carboxy-X -rhodamine), Sulphorhodamine B can C, Sulphorhodamine G Extra, TAMRA (6-carboxytetramethylrhodamine), Tetramethylrhodamine (TRITC), WT), Texas Red, Texas Red-X, VIC and other labels known to those of skill in the art. Other detectable labels may also be used. Any of these systems and detectable labels, as well as many others, may be used to detect amplified target nucleic acids.

As used herein, the term "detectable label" refers to any of a variety of signaling molecules indicative of amplification. In some embodiments, the reaction mixture may include a detectable label such as SYBR^{®} Green and/or other DNA-binding dyes. Such detectable labels may comprise or may be, for example, nucleic acid intercalating agents or non-intercalating agents. As used herein, an intercalating agent is an agent or moiety capable of non-covalent insertion between stacked base pairs of a double-stranded nucleic acid molecule. A non-intercalating agent is one that does not insert into the double-stranded nucleic acid molecule. The nucleic acid binding agent may produce a detectable signal directly or indirectly. The signal may be detectable directly using, for example, fluorescence and/or absorbance, or indirectly using, for example, any moiety or ligand that is detectably affected by proximity to double-stranded nucleic As used herein, an intercalating agent is an agent or moiety capable of non-covalent insertion between stacked base pairs of a double-stranded nucleic acid molecule. A non-intercalating agent acid is suitable such as a substituted label moiety or binding ligand attached to the nucleic acid binding agent. It is typically necessary for the nucleic acid binding agent to produce a detectable signal when bound to a double-stranded nucleic acid that is distinguishable from the signal produced when that same agent is in solution or bound to a single- stranded nucleic acid. For example, intercalating agents such as ethidium bromide fluoresce more intensely when intercalated into double-stranded DNA than when bound to single- stranded DNA, RNA, or in solution. Similarly, actinomycin D fluoresces in the red portion of the UV/VIS spectrum when bound to single-stranded nucleic acids, and fluoresces in the green portion of the UV/VIS spectrum when bound to double- stranded nucleic acids. And in another example, the photoreactive psoralen 4-aminomethyl-4-5',8- trimethylpsoralen (AMT) has been reported to exhibit decreased absorption at long wavelengths and fluorescence upon intercalation into double- stranded DNA. For example, U.S. Pat. No. 4,257,774 describes the direct binding of fluorescent intercalators to DNA (e.g., ethidium salts, daunomycin, mepacrine and acridine orange, 4',6- diamidino-a-phenylindole). Non-intercalating agents (e.g., minor groove binder moieties (MGBs) as described herein such as Hoechst 33258, distamycin, netropsin) may also be suitable for use.

Non-limiting examples of DNA binding dyes include acridines (e.g., acridine orange, acriflavine), actinomycin D, anthramycin, BOBO^{™}-1, BOBO^{™}-3, BO-PRO^{™}-1, cbromomycin, DAPI, daunomycin, distamycin (e.g., distamycin D), dyes described in U.S. Pat. No. 7,387,887, ellipticine, ethidium salts (e.g., ethidium bromide), fluorcoumanin, fluorescent intercalators, GelStar^{®} (Lonza), Hoechst 33258, Hoechst 33342, homidium, JO-PRO^{™}- 1, LIZ dyes, LO-PRO^{™}-1, mepacrine, mithramycin, NED dyes, netropsin, 4',6-diamidino-a-phenylindole, proflavine, POPO^{™}-1, POPO^{™}-3, PO-PRO^{™}-1, propidium iodide, ruthenium polypyridyls, S5, SYBR^{®} Gold, SYBR^{®} Green I, SYBR^{®} Green II, SYTOX^{®} blue, SYTOX^{®} green, SYTO^{®} 43, SYTO^{®} 44, SYTO^{®} 45, SYTOX^{®} Blue, TO-PRO^{®}-1, SYTO^{®} 11, SYTO^{®} 13, SYTO^{®} 15, SYTO^{®} 16, SYTO^{®} 20, SYTO^{®} 23, thiazole orange (Sigma- Aldrich Chemical Co.), TOTO^{™}-3, YO-PRO^{®}-1, and YOYO^{®}-3 (Molecular Probes; Thermo Fisher Scientific), among others. SYBR^{®} Green I, for example, has been used to monitor a PCR reactions. Other suitable DNA binding dyes may also be used.

The nucleic acid polymerases that may be employed in the disclosed nucleic acid amplification reactions may be any that function to carry out the desired reaction including, for example, a prokaryotic, fungal, viral, bacteriophage, plant, and/or eukaryotic nucleic acid polymerase. As used herein, the term "DNA polymerase" refers to an enzyme that synthesizes a DNA strand de novo using a nucleic acid strand as a template. DNA polymerase uses an existing DNA or RNA as the template for DNA synthesis and catalyzes the polymerization of deoxyribonucleotides alongside the template strand, which it reads. The newly synthesized DNA strand is complementary to the template strand. DNA polymerase can add free nucleotides only to the 3'-hydroxyl end of the newly forming strand. It synthesizes oligonucleotides via transfer of a nucleoside monophosphate from a deoxyribonucleoside triphosphate (dNTP) to the 3'-hydroxyl group of a growing oligonucleotide chain. This results in elongation of the new strand in a 5'-to-3' direction. Since DNA polymerase can only add a nucleotide onto a pre-existing 3'-OH group, to begin a DNA synthesis reaction, the DNA polymerase needs a primer to which it can add the first nucleotide. Suitable primers may comprise oligonucleotides of RNA or DNA, or chimeras thereof (e.g., RNA/DNA chimerical primers). The DNA polymerases may be a naturally occurring DNA polymerases or a variant of natural enzyme having the above-mentioned activity. For example, it may include a DNA polymerase having a strand displacement activity, a DNA polymerase lacking 5'-to-3' exonuclease activity, a DNA polymerase having a reverse transcriptase activity, or a DNA polymerase having an endonuclease activity.

Polymerases used in accordance with the present teachings may be any enzyme that can synthesize a nucleic acid molecule from a nucleic acid template, typically in the 5' to 3' direction. Suitable nucleic acid polymerases may also comprise holoenzymes, functional portions of the holoenzymes, chimeric polymerase, or any modified polymerase that can effectuate the synthesis of a nucleic acid molecule. Within this disclosure, a DNA polymerase may also include a polymerase, terminal transferase, reverse transcriptase, telomerase, and/or polynucleotide phosphorylase.

The nucleic acid polymerases used in the methods disclosed herein may be mesophilic or thermophilic. Exemplary mesophilic DNA polymerases include T7 DNA polymerase, T5 DNA polymerase, Klenow fragment DNA polymerase, DNA polymerase III and the like. Non-limiting examples of polymerases may include, for example, T7 DNA polymerase, eukaryotic mitochondrial DNA Polymerase γ, prokaryotic DNA polymerase I, II, III, IV, and/or V; eukaryotic polymerase α, B, γ, δ, ε, η, ζ, i,and/or κ; E. coli DNA polymerase I; E. coli DNA polymerase III alpha and/or epsilon subunits; E. coli polymerase IV, E. coli polymerase V; T. aquaticus DNA polymerase I; B. stearothermophilus DNA polymerase I; Euryarchaeota polymerases; terminal deoxynucleotidyl transferase (TdT); S. cerevisiae polymerase 4; translesion synthesis polymerases; reverse transcriptase; and/or telomerase. Non-limiting examples of suitable thermostable DNA polymerases that may be used include, but are not limited to, Thermus thermophilus (Tth) DNA polymerase, Thermus aquaticus (Taq) DNA polymerase, Thermotoga neopolitana (Tne) DNA polymerase, Thermotoga maritima (Tma) DNA polymerase, Thermococcus litoralis (Tli or VENT^{™}) DNA polymerase, Pyrococcus furiosus (Pfu) DNA polymerase, DEEPVENT^{™} DNA polymerase, Pyrococcus woosii (Pwo) DNA polymerase, Bacillus sterothermophilus (Bst) DNA polymerase, Bacillus caldophilus (Bca) DNA polymerase, Sulfobus acidocaldarius (Sac) DNA polymerase, Thermoplasma acidophilum (Tac) DNA polymerase, Thermus flavus (Tfl/Tub) DNA polymerase, Thermus ruber (Tru) DNA polymerase, Thermus brockianus (DYNAZYME^{™}) DNA polymerase, Methanobacterium thermoautotrophicum (Mth) DNA polymerase, mycobacterium DNA polymerase (Mtb, Mlep), and mutants, and variants and derivatives thereof. RNA polymerases such as T3, T5 and SP6 and mutants, variants and derivatives thereof may also be used in accordance with the present teachings. Generally, any type I DNA polymerase may be used in accordance with the invention although other DNA polymerases may be used including, but not limited to, type III or family A, B, C etc. DNA polymerases. In addition, any genetically engineered DNA polymerases, any having reduced or insignificant 3'-to-5' exonuclease activity (e.g., Superscript^{™} DNA polymerase), and/or genetically engineered DNA polymerases (e.g., those having the active site mutation F667Y or the equivalent of F667Y (e.g., in Tth), AmpliTaq^{™}, ThermoSequenase^{™}), AmpliTaq^{™} Gold, Platinum^{™} Taq DNA Polymerase, Therminator I, Therminator II, Therminator III, Therminator Gamma (New England Biolabs, Beverly, MA), and/or any derivatives and fragments thereof, may be used in accordance with the present teachings. Examples of DNA polymerases substantially lacking in 3' exonuclease activity include, but are not limited to, Taq, Tne(exo-), Tma(exo-), Pfu (exo-), Pwo(exo-) and Tth DNA polymerases, and mutants, variants and derivatives thereof. Other nucleic acid polymerases may also be suitable as would be understood by one of skill in the art. DNA polymerases for use in the methods disclosed herein may be obtained commercially, for example, from Invitrogen^{™} (Thermo Fisher Scientific), Pharmacia (Piscataway, NJ), Sigma (St. Louis, MO), Boehringer Mannheim, and New England Biolabs (Beverly, MA).

The detection of the signal may be using any reagents or instruments that detect a change in fluorescence from a fluorophore. For example, detection may be performed using any spectrophotometric thermal cycler. Examples of spectrophotometric thermal cyclers include, but are not limited to, Applied Biosystems (AB) PRISM^{®} 7000, AB 7300 real-time PCR system, AB 7500 real-time PCR system, AB PRISM^{™} 7900HT, Bio-Rad ICycler IQ^{™}, Cepheid SmartCycler^{®} II, Corbett Research Rotor-Gene 3000, Idaho Technologies R.A.P.I.D.^{™}, MJ Research Chromo 4^{™}, Roche Applied Science LightCycler^{®}, Roche Applied Science LightCycler^{®}2.0, Stratagene Mx3000P^{™}, and Stratagene Mx4000^{™}. It should be noted that new instruments are being developed at a rapid rate and any like instruments may be used for the methods.

### Kits (are not part of the invention)

Kits for performing the methods described herein are also provided. As used herein, the term "kit" refers to a packaged set of related components, typically one or more compounds or compositions. The kit may comprise a pair of oligonucleotides for polymerizing and/or amplifying at least one target nucleic acid from a sample, one or more detergents, a nucleic acid polymerase, and/or corresponding one or more probes labeled with a detectable label. The kit may also include samples containing pre-defined target nucleic acids to be used in control reactions. The kit may also optionally include stock solutions, buffers, enzymes, detectable labels or reagents required for detection, tubes, membranes, and the like that may be used to complete the amplification reaction. In some embodiments, multiple primer sets are included. For example, the kit can include one or more of a buffer (e.g., Tris), one or more salts (e.g., KG), glycerol, dNTPs (dATP, dTTP, dGTP, dCTP, dUTP), ddNTPs (ddATP, ddTTP, ddGTP, ddCTP), recombinant BSA (bovine serum albumin), a dye (e.g., ROX passive reference dye), one or more detergents, polyethylene glycol (PEG), polyvinyl pyrrolidone (PVP), gelatin (e.g., fish or bovine source) and/or antifoam agent.

Disclosed herein include kits. In some embodiments, the kit comprises: a plurality of oligonucleotide probes, wherein each of the plurality of oligonucleotide probes comprises a sequence configured to bind a unique identifier sequence, or a complement thereof. The kit can comprise: a plurality of first cellular component-binding reagents, wherein each of the plurality of first cellular component-binding reagents comprises a cellular component-binding reagent specific oligonucleotide comprising a unique identifier sequence for the first cellular component-binding reagent, and wherein the first cellular component-binding reagent is capable of specifically binding to at least one of a plurality of cellular component targets. The kit can comprise: a plurality of second cellular component-binding reagents, wherein each of the plurality of second cellular component-binding reagents comprises a detectable moiety, or a precursor thereof, wherein a second cellular component-binding reagent is capable of specifically binding to at least one of the plurality of cellular component targets, wherein second cellular component-binding reagents capable of binding the same cellular component target comprise the same detectable moiety, or a precursor thereof, and wherein second cellular component-binding reagents capable of binding different cellular component targets comprise different detectable moieties, or precursors thereof. The kit can comprise: a plurality of third cellular component-binding reagents, wherein a third cellular component-binding reagent is capable of specifically binding to at least one of the plurality of cellular component targets, wherein the third cellular component-binding reagents do not comprise a cellular component-binding reagent specific oligonucleotide, wherein one or more of the first cellular component-binding reagents and one or more of the third cellular component-binding reagents are capable of binding the same cellular component target. In some embodiments, first cellular component-binding reagents and third cellular component-binding reagents capable of binding the same cellular component target have the same clonotype.

The one or more of the first cellular component-binding reagents and one or more of the second cellular component-binding reagents can be capable of binding the same cellular component target. In some embodiments, first cellular component-binding reagents and second cellular component-binding reagents capable of binding the same cellular component target have the same clonotype In some embodiments, each probe is flanked by complementary sequences at the 5' end and 3' end. In some embodiments, one of the complementary sequences comprises a fluorescence emitter moiety and the other complementary sequence comprises a fluorescence quencher moiety. In some embodiments, at least one of the plurality of oligonucleotide probes comprises a fluorescence emitter moiety and a fluorescence quencher moiety. In some embodiments, the oligonucleotide probe comprises a TaqMan detection probe oligonucleotide, a molecular beacon. The kit can comprise: a primer capable of hybridizing to a first universal sequence, or a complement thereof. The kit can comprise: a primer capable of hybridizing to a second universal sequence, or a complement thereof. The kit can comprise: a primer capable of hybridizing to a capture sequence, or a complement thereof.

### Detectable Moieties

In some embodiments, the detectable moiety (e.g., detectable label) comprises an optical moiety, a luminescent moiety, an electrochemically active moiety, a nanoparticle, or a combination thereof. In some embodiments, the luminescent moiety comprises a chemiluminescent moiety, an electroluminescent moiety, a photoluminescent moiety, or a combination thereof. In some embodiments, the photoluminescent moiety comprises a fluorescent moiety, a phosphorescent moiety, or a combination thereof. In some embodiments, the fluorescent moiety comprises a fluorescent dye. In some embodiments, the nanoparticle comprises a quantum dot. In some embodiments, the methods comprise performing a reaction to convert the detectable moiety precursor into the detectable moiety. In some embodiments, performing a reaction to convert the detectable moiety precursor into the detectable moiety comprises contacting the detectable moiety precursor with a substrate. In some such embodiments, contacting the detectable moiety precursor with a substrate yields a detectable byproduct of a reaction between the two molecules.

### Detectable Moiety Properties and Structures

In some embodiments, detectable labels, moieties, or markers can be detectible based on, for example, fluorescence emission, absorbance, fluorescence polarization, fluorescence lifetime, fluorescence wavelength, absorbance wavelength, Stokes shift, light scatter, mass, molecular mass, redox, acoustic, Raman, magnetism, radio frequency, enzymatic reactions (including chemiluminescence and electro- chemiluminescence) or combinations thereof. For example, the label may be a fluorophore, a chromophore, an enzyme, an enzyme substrate, a catalyst, a redox label, a radio label, an acoustic label, a Raman (SERS) tag, a mass tag, an isotope tag (e.g., isotopically pure rare earth element), a magnetic particle, a microparticle, a nanoparticle, an oligonucleotide, or any combination thereof. In some embodiments, the label is a fluorophore (i.e., a fluorescent label, fluorescent dye, etc.). Fluorophores of interest may include but are not limited to dyes suitable for use in analytical applications (e.g., flow cytometry, imaging, etc.) , such as an acridine dye, anthraquinone dyes, arylmethane dyes, diarylmethane dyes (e.g., diphenyl methane dyes), chlorophyll containing dyes, triarylmethane dyes (e.g., triphenylmethane dyes), azo dyes, diazonium dyes, nitro dyes, nitroso dyes, phthalocyanine dyes, cyanine dyes, asymmetric cyanine dyes, quinon-imine dyes, azine dyes, eurhodin dyes, safranin dyes, indamins, indophenol dyes, fluorine dyes, oxazine dye, oxazone dyes, thiazine dyes, thiazole dyes, xanthene dyes, fluorene dyes, pyronin dyes, fluorine dyes, rhodamine dyes, phenanthridine dyes, as well as dyes combining two or more of the aforementioned dyes (e.g., in tandem), polymeric dyes having one or more monomeric dye units and mixtures of two or more of the aforementioned dyes thereof. A large number of dyes are commercially available from a variety of sources, such as, for example, Molecular Probes (Eugene, OR), Dyomics GmbH (Jena, Germany), Sigma-Aldrich (St. Louis, MO), Sirigen, Inc. (Santa Barbara, CA) and Exciton (Dayton, OH). For example, the fluorophore may include 4-acetamido-4'-isothiocyanatostilbene-2,2'disulfonic acid; acridine and derivatives such as acridine, acridine orange, acridine yellow, acridine red, and acridine isothiocyanate; allophycocyanin, phycoerythrin, peridinin-chlorophyll protein, 5-(2'-aminoethyl)aminonaphthalene-1-sulfonic acid (EDANS); 4-amino-N-[3-vinylsulfonyl)phenyl]naphthalimide-3,5 disulfonate (Lucifer Yellow VS); N-(4-anilino-1-naphthyl)maleimide; anthranilamide; Brilliant Yellow; coumarin and derivatives such as coumarin, 7-amino-4-methylcoumarin (AMC, Coumarin 120), 7-amino-4-trifluoromethylcouluarin (Coumaran 151); cyanine and derivatives such as cyanosine, Cy3, Cy3.5, Cy5, Cy5.5, and Cy7; 4',6-diaminidino-2-phenylindole (DAPI); 5', 5"-dibromopyrogallol-sulfonephthalein (Bromopyrogallol Red); 7-diethylamino-3-(4'-isothiocyanatophenyl)-4-methylcoumarin; diethylaminocoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5- carboxyfluorescein (FAM), 5-(4,6-dichlorotriazin-2-yl)aminofluorescein (DTAF), 2'7' -dimethoxy-4'5' -dichloro-6-carboxyfluorescein (JOE), fluorescein isothiocyanate (FITC), fluorescein chlorotriazinyl, naphthofluorescein, and QFITC (XRITC); fluorescamine; IR144; IR1446; Green Fluorescent Protein (GFP); Reef Coral Fluorescent Protein (RCFP); Lissamine^{™}; Lissamine rhodamine, Lucifer yellow; Malachite Green isothiocyanate; 4-methylumbelliferone; ortho cresolphthalein; nitrotyrosine; pararosaniline; Nile Red; Oregon Green; Phenol Red; B-phycoerythrin; o-phthaldialdehyde; pyrene and derivatives such as pyrene, pyrene butyrate and succinimidyl 1-pyrene butyrate; Reactive Red 4 (Cibacron^{™} Brilliant Red 3B-A); rhodamine and derivatives such as 6-carboxy-X-rhodamine (ROX), 6-carboxyrhodamine (R6G), 4,7-dichlororhodamine lissamine, rhodamine B sulfonyl chloride, rhodamine (Rhod), rhodamine B, rhodamine 123, rhodamine X isothiocyanate, sulforhodamine B, sulforhodamine 101, sulfonyl chloride derivative of sulforhodamine 101 (Texas Red), N,N,N',N'-tetramethyl-6-carboxyrhodamine (TAMRA), tetramethyl rhodamine, and tetramethyl rhodamine isothiocyanate (TRITC); riboflavin; rosolic acid and terbium chelate derivatives; xanthene; dye-conjugated polymers (i.e., polymer-attached dyes) such as fluorescein isothiocyanate-dextran as well as dyes combining two or more dyes (e.g., in tandem), polymeric dyes having one or more monomeric dye units and mixtures of two or more of the aforementioned dyes or combinations thereof.

The detectable moiety can be selected from a group of spectrally-distinct detectable moieties. Spectrally-distinct detectable moieties include detectable moieties with distinguishable emission spectra even if their emission spectral may overlap. Non-limiting examples of detectable moieties include Xanthene derivatives: fluorescein, rhodamine, Oregon green, eosin, and Texas red; Cyanine derivatives: cyanine, indocarbocyanine, oxacarbocyanine, thiacarbocyanine, and merocyanine; Squaraine derivatives and ring-substituted squaraines, including Seta, SeTau, and Square dyes; Naphthalene derivatives (dansyl and prodan derivatives); Coumarin derivatives; oxadiazole derivatives: pyridyloxazole, nitrobenzoxadiazole and benzoxadiazole; Anthracene derivatives: anthraquinones, including DRAQ5, DRAQ7 and CyTRAK Orange; Pyrene derivatives: cascade blue; Oxazine derivatives: Nile red, Nile blue, cresyl violet, oxazine 170; Acridine derivatives: proflavin, acridine orange, acridine yellow; Arylmethine derivatives: auramine, crystal violet, malachite green; and Tetrapyrrole derivatives: porphin, phthalocyanine, bilirubin. Other non-limiting examples of detectable moieties include Hydroxycoumarin, Aminocoumarin, Methoxycoumarin, Cascade Blue, Pacific Blue, Pacific Orange, Lucifer yellow, NBD, R-Phycoerythrin (PE), PE-Cy5 conjugates, PE-Cy7 conjugates, Red 613, PerCP, TruRed, FluorX, Fluorescein, BODIPY-FL, Cy2, Cy3, Cy3B, Cy3.5, Cy5, Cy5.5, Cy7, TRITC, X-Rhodamine, Lissamine Rhodamine B, Texas Red, Allophycocyanin (APC), APC-Cy7 conjugates, Hoechst 33342, DAPI, Hoechst 33258, SYTOX Blue, Chromomycin A3, Mithramycin, YOYO-1, Ethidium Bromide, Acridine Orange, SYTOX Green, TOTO-1, TO-PRO-1, TO-PRO: Cyanine Monomer, Thiazole Orange, CyTRAK Orange, Propidium Iodide (PI), LDS 751, 7-AAD, SYTOX Orange, TOTO-3, TO-PRO-3, DRAQ5, DRAQ7, Indo-1, Fluo-3, Fluo-4, DCFH, DHR, and SNARF.

Fluorophores of interest can include, but are not limited to, dyes suitable for use in analytical applications (e.g., flow cytometry, imaging, etc.), such as an acridine dye, anthraquinone dyes, arylmethane dyes, diarylmethane dyes (e.g., diphenyl methane dyes), chlorophyll containing dyes, triarylmethane dyes (e.g., triphenylmethane dyes), azo dyes, diazonium dyes, nitro dyes, nitroso dyes, phthalocyanine dyes, cyanine dyes, asymmetric cyanine dyes, quinon-imine dyes, azine dyes, eurhodin dyes, safranin dyes, indamins, indophenol dyes, fluorine dyes, oxazine dye, oxazone dyes, thiazine dyes, thiazole dyes, xanthene dyes, fluorene dyes, pyronin dyes, fluorine dyes, rhodamine dyes, phenanthridine dyes, as well as dyes combining two or more dyes (e.g., in tandem) as well as polymeric dyes having one or more monomeric dye units, as well as mixtures of two or more dyes thereof. For example, the fluorophore may be 4- acetamido-4'-isothiocyanatostilbene-2,2'disulfonic acid; acridine and derivatives such as acridine, acridine orange, acrindine yellow, acridine red, and acridine isothiocyanate; allophycocyanin, phycoerythrin, peridinin-chlorophyll protein, 5-(2'-aminoethyl)aminonaphthalene-1-sulfonic acid (EDANS); 4-amino-N-[3-vinylsulfonyl)phenyl]naphthalimide-3,5 disulfonate (Lucifer Yellow VS); N-(4-anilino-1-naphthyl)maleimide; anthranilamide; Brilliant Yellow; coumarin and derivatives such as coumarin, 7-amino-4-methylcoumarin (AMC, Coumarin 120), 7-amino-4-trifluoromethylcouluarin (Coumaran 151); cyanine and derivatives such as cyanosine, Cy3, Cy5, Cy5.5, and Cy7; 4',6-diaminidino-2-phenylindole (DAPI); 5', 5"-dibromopyrogallol-sulfonephthalein (Bromopyrogallol Red); 7-diethylamino-3-(4'-isothiocyanatophenyl)-4-methylcoumarin; diethylaminocoumarin; diethylenetriamine pentaacetate; 4,4'-diisothiocyanatodihydro-stilbene-2,2'-disulfonic acid; 4,4'- diisothiocyanatostilbene-2,2'-disulfonic acid; 5-[dimethylamino]naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5- carboxyfluorescein (FAM), 5-(4,6-dichlorotriazin-2-yl)aminofluorescein (DTAF), 2'7'-dimethoxy-4'5'-dichloro-6-carboxyfluorescein (JOE), fluorescein isothiocyanate (FITC), fluorescein chlorotriazinyl, naphthofluorescein, and QFITC (XRITC); fluorescamine; IR144; IR1446; Green Fluorescent Protein (GFP); Reef Coral Fluorescent Protein (RCFP); Lissamine^{™}; Lissamine rhodamine, Lucifer yellow; Malachite Green isothiocyanate; 4-methylumbelliferone; ortho cresolphthalein; nitrotyrosine; pararosaniline; Nile Red; Oregon Green; Phenol Red; B-phycoerythrin; o- phthaldialdehyde; pyrene and derivatives such as pyrene, pyrene butyrate and succinimidyl 1-pyrene butyrate; Reactive Red 4 (Cibacron^{™} Brilliant Red 3B-A); rhodamine and derivatives such as 6-carboxy-X-rhodamine (ROX), 6-carboxyrhodamine (R6G), 4,7-dichlororhodamine lissamine, rhodamine B sulfonyl chloride, rhodamine (Rhod), rhodamine B, rhodamine 123, rhodamine X isothiocyanate, sulforhodamine B, sulforhodamine 101, sulfonyl chloride derivative of sulforhodamine 101 (Texas Red), N,N,N',N'-tetramethyl-6-carboxyrhodamine (TAMRA), tetramethyl rhodamine, and tetramethyl rhodamine isothiocyanate (TRITC); riboflavin; rosolic acid and terbium chelate derivatives; xanthene; dye-conjugated polymers (i.e., polymer-attached dyes) such as fluorescein isothiocyanate-dextran as well as dyes combining two or more of the aforementioned dyes (e.g., in tandem), polymeric dyes having one or more monomeric dye units and mixtures of two or more of the aforementioned dyes thereof.

The group of spectrally distinct detectable moieties can, for example, include five different fluorophores, five different chromophores, a combination of five fluorophores and chromophores, a combination of four different fluorophores and a non-fluorophore, a combination of four chromophores and a non-chromophore, or a combination of four fluorophores and chromophores and a non-fluorophore non-chromophore. In some embodiments, the detectable moieties can be one of 1, 2, 3, 4, 5, 6, 7, 8, 9, 10, 20, 30, 40, 50, 60, 70, 80, 90, 100, 1000, 2000, 3000, 4000, 5000, 6000, 7000, 8000, 9000, 10000, or a number or a range between any two of these values, of spectrally-distinct moieties.

The excitation wavelength of the detectable moieties can vary, for example be, or be about, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 nanometers, or a number or a range between any two of these values. The emission wavelength of the detectable moieties can also vary, for example be, or be about, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 nanometers, or a number or a range between any two of these values.

The molecular weights of the detectable moieties can vary, for example be, or be about, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 Daltons (Da), or a number or a range between any two of these values. The molecular weights of the detectable moieties can also vary, for example be, or be about, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 kilo Daltons (kDa), or a number or a range between any two of these values.

### Polymeric Dyes

In some instances, the fluorophore (i.e., dye) is a fluorescent polymeric dye. Fluorescent polymeric dyes that find use in the subject methods and systems can vary. In some instances of the method, the polymeric dye includes a conjugated polymer.

Conjugated polymers (CPs) are characterized by a delocalized electronic structure which includes a backbone of alternating unsaturated bonds (e.g., double and/or triple bonds) and saturated (e.g., single bonds) bonds, where π-electrons can move from one bond to the other. As such, the conjugated backbone may impart an extended linear structure on the polymeric dye, with limited bond angles between repeat units of the polymer. For example, proteins and nucleic acids, although also polymeric, in some cases do not form extended-rod structures but rather fold into higher-order three- dimensional shapes. In addition, CPs may form "rigid-rod" polymer backbones and experience a limited twist (e.g., torsion) angle between monomer repeat units along the polymer backbone chain. In some instances, the polymeric dye includes a CP that has a rigid rod structure. As summarized above, the structural characteristics of the polymeric dyes can have an effect on the fluorescence properties of the molecules.

Any convenient polymeric dye may be utilized in the subject methods and systems. In some instances, a polymeric dye is a multichromophore that has a structure capable of harvesting light to amplify the fluorescent output of a fluorophore. In some instances, the polymeric dye is capable of harvesting light and efficiently converting it to emitted light at a longer wavelength. In some embodiments, the polymeric dye has a light-harvesting multichromophore system that can efficiently transfer energy to nearby luminescent species (e.g., a "signaling chromophore"). Mechanisms for energy transfer include, for example, resonant energy transfer (e.g., Forster (or fluorescence) resonance energy transfer, FRET), quantum charge exchange (Dexter energy transfer) and the like. In some instances, these energy transfer mechanisms are relatively short range; that is, close proximity of the light harvesting multichromophore system to the signaling chromophore provides for efficient energy transfer. Under conditions for efficient energy transfer, amplification of the emission from the signaling chromophore occurs when the number of individual chromophores in the light harvesting multichromophore system is large; that is, the emission from the signaling chromophore is more intense when the incident light (the "excitation light") is at a wavelength which is absorbed by the light harvesting multichromophore system than when the signaling chromophore is directly excited by the pump light.

The multichromophore can be a conjugated polymer. Conjugated polymers (CPs) are characterized by a delocalized electronic structure and can be used as highly responsive optical reporters for chemical and biological targets. Because the effective conjugation length is substantially shorter than the length of the polymer chain, the backbone contains a large number of conjugated segments in close proximity. Thus, conjugated polymers are efficient for light harvesting and enable optical amplification via energy transfer.

The polymer can be used as a direct fluorescent reporter, for example fluorescent polymers having high extinction coefficients, high brightness, etc. In some instances, the polymer may be used as a strong chromophore where the color or optical density is used as an indicator.

Polymeric dyes of interest include, but are not limited to, those dyes described by Gaylord et al. in US Publication Nos. 20040142344, 20080293164, 20080064042, 20100136702, 20110256549, 20120028828, 20120252986, 20130190193 and 20160025735.

The polymeric dye can include a conjugated polymer including a plurality of first optically active units forming a conjugated system, having a first absorption wavelength (e.g., as described herein) at which the first optically active units absorb light to form an excited state. The CP may be polycationic, polyanionic and/or a charge-neutral conjugated polymer.

The CPs can be water soluble for use in biological samples. Any convenient substituent groups may be included in the polymeric dyes to provide for increased water-solubility, such as a hydrophilic substituent group, e.g., a hydrophilic polymer, or a charged substituent group, e.g., groups that are positively or negatively charged in an aqueous solution, e.g., under physiological conditions. Any convenient water-soluble groups (WSGs) may be utilized in the subject light harvesting multichromophores. The term "water-soluble group" refers to a functional group that is well solvated in aqueous environments and that imparts improved water solubility to the molecules to which it is attached. In some embodiments, a WSG increases the solubility of the multichromophore in a predominantly aqueous solution (e.g., as described herein), as compared to a multichromophore which lacks the WSG. The water-soluble groups may be any convenient hydrophilic group that is well solvated in aqueous environments. In some embodiments, the hydrophilic water-soluble group is charged, e.g., positively or negatively charged or zwitterionic. In some embodiments, the hydrophilic water-soluble group is a neutral hydrophilic group. In some embodiments, the WSG is a hydrophilic polymer, e.g., a polyethylene glycol, a cellulose, a chitosan, or a derivative thereof.

As used herein, the terms "polyethylene glycol" and "PEG" refer to a polymer including a chain described by the formula -(CH₂-CH₂-O-)ₙ- or a derivative thereof. In some embodiments, "n" is 5000 or less, such as 1000 or less, 500 or less, 200 or less, 100 or less, 50 or less, 40 or less, 30 or less, 20 or less, 15 or less, such as 5 to 15, or 10 to 15. The PEG polymer can be of any convenient length and can include a variety of terminal groups, including but not limited to, alkyl, aryl, hydroxyl, amino, acyl, acyloxy, and amido terminal groups.

The length of polymeric dye can vary. In some embodiments, the particular number of monomeric repeat units or segments of the polymeric dye may fall within the range of 2 to 500,000, such as 2 to 100,000, 2 to 30,000, 2 to 10,000, 2 to 3,000 or 2 to 1,000 units or segments, or such as 100 to 100,000, 200 to 100,000, or 500 to 50,000 units or segments. In some embodiments, the number of monomeric repeat units or segments of the polymeric dye is within the range of 2 to 1000 units or segments, such as from 2 to 750 units or segments, such as from 2 to 500 units or segments, such as from 2 to 250 units or segment, such as from 2 to 150 units or segment, such as from 2 to 100 units or segments, such as from 2 to 75 units or segments, such as from 2 to 50 units or segments and including from 2 to 25 units or segments.

The MW of polymeric dyes can vary. In some embodiments, the MW of the polymeric dye may be expressed as an average molecular weight. In some instances, the polymeric dye has an average molecular weight of from 500 to 500,000, such as from 1,000 to 100,000, from 2,000 to 100,000, from 10,000 to 100,000 or even an average molecular weight of from 50,000 to 100,000. In some embodiments, the polymeric dye has an average molecular weight of 70,000.

The polymeric dye can have one or more desirable spectroscopic properties, such as a particular absorption maximum wavelength, a particular emission maximum wavelength, extinction coefficient, quantum yield, and the like.

The polymeric dye can have an absorption curve between 280 and 850 nm. In some embodiments, the polymeric dye has an absorption maximum in the range 280 and 850 nm. In some embodiments, the polymeric dye absorbs incident light having a wavelength in the range between 280 and 850 nm, where specific examples of absorption maxima of interest include, but are not limited to: 348nm, 355nm, 405nm, 407nm, 445nm, 488nm, 640nm and 652nm. In some embodiments, the polymeric dye has an absorption maximum wavelength in a range selected from the group consisting of 280-310nm, 305-325nm, 320-350nm, 340-375nm, 370-425nm, 400- 450nm, 440-500nm, 475-550nm, 525-625nm, 625-675nm and 650-750nm. In some embodiments, the polymeric dye has an absorption maximum wavelength of 348nm, 355nm, 405nm, 407nm, 445nm, 488nm, 640nm, 652nm, or a range between any two of these values.

In some embodiments, the polymeric dye has an emission maximum wavelength ranging from 400 to 850 nm, such as 415 to 800 nm, where specific examples of emission maxima of interest include, but are not limited to: 395 nm, 421nm, 445nm, 448nm, 452nm, 478nm, 480nm, 485nm, 491nm, 496nm, 500nm, 510nm, 515nm, 519nm, 520nm, 563nm, 570nm, 578nm, 602nm, 612nm, 650nm, 661nm, 667nm, 668nm, 678nm, 695nm, 702nm, 711nm, 719nm, 737nm, 785nm, 786nm, 805nm. In some embodiments, the polymeric dye has an emission maximum wavelength in a range selected from the group consisting of 380-400nm, 410-430nm, 470-490nm, 490-510nm, 500-520nm, 560-580nm, 570-595nm, 590-610nm, 610-650nm, 640-660nm, 650-700nm, 700-720nm, 710-750nm, 740-780nm and 775-795nm. In some embodiments, the polymeric dye has an emission maximum of 395nm, 421nm, 478nm, 480nm, 485nm, 496nm, 510nm, 570nm, 602nm, 650nm, 711nm, 737nm, 750nm, 786nm, or a range of any two of these values. In some embodiments, the polymeric dye has an emission maximum wavelength of 421nm ± 5nm, 510nm ± 5nm, 570nm ± 5nm, 602nm ± 5nm, 650nm ± 5nm, 711nm ± 5nm, 786nm ± 5nm, or a range of any two of these values. In some embodiments, the polymeric dye has an emission maximum selected from the group consisting of 421nm, 510nm, 570nm, 602nm, 650nm, 711nm and 786nm.

In some embodiments, the polymeric dye has an extinction coefficient of 1 x 106 cm- 1M⁻¹ or more, such as 2 x 10⁶ cm⁻¹M⁻¹ or more, 2.5 x 10⁶ cm⁻¹M⁻¹ or more, 3 x 10⁶ cm⁻¹M⁻¹ or more, 4 x 10⁶ cm⁻¹M⁻¹ or more, 5 x 10⁶ cm⁻¹M⁻¹ or more, 6 x 10⁶ cm⁻¹M⁻¹ or more, 7 x 10⁶ cm⁻¹M⁻¹ or more, or 8 x 10⁶ cm⁻¹M⁻¹ or more. In some embodiments, the polymeric dye has a quantum yield of 0.05 or more, such as 0.1 or more, 0.15 or more, 0.2 or more, 0.25 or more, 0.3 or more, 0.35 or more, 0.4 or more, 0.45 or more, 0.5 or more, 0.6 or more, 0.7 or more, 0.8 or more, 0.9 or more, 0.95 or more, 0.99 or more and including 0.999 or more. For example, the quantum yield of polymeric dyes of interest may range from 0.05 to 1, such as from 0.1 to 0.95, such as from 0.15 to 0.9, such as from 0.2 to 0.85, such as from 0.25 to 0.75, such as from 0.3 to 0.7 and including a quantum yield of from 0.4 to 0.6. In some embodiments, the polymeric dye has a quantum yield of 0.1 or more, 0.3 or more, 0.5 or more, 0.6 or more. In some embodiments, the polymeric dye has a quantum yield of 0.7 or more, 0.8 or more, 0.9 or more, or 0.95 or more. In some embodiments, the polymeric dye has an extinction coefficient of 1 x 10⁶ or more and a quantum yield of 0.3 or more. In some embodiments, the polymeric dye has an extinction coefficient of 2 x 10⁶ or more and a quantum yield of 0.5 or more.

### EXAMPLES

Some aspects of the embodiments discussed above are disclosed in further detail in the following examples, which are not in any way intended to limit the scope of the present disclosure.

### Example 1. Oligonucleotides for Associating with Protein Binding Reagents

This example demonstrates designing of oligonucleotides that can be conjugated with protein binding reagents. The oligonucleotides can be used to determine protein expression and gene expression simultaneously. The oligonucleotides can also be used for sample indexing to determine cells of the same or different samples.

### 95mer Oligonucleotide Design

The following method was used to generate candidate oligonucleotide sequences and corresponding primer sequences for simultaneous determination of protein expression and gene expression or sample indexing.

### 1. Sequence generation and elimination

The following process was used to generate candidate oligonucleotide sequences for simultaneous determination of protein expression and gene expression or sample indexing.

Step 1a. Randomly generate a number of candidate sequences (50000 sequences) with the desired length (45 bps).

Step 1b. Append the transcriptional regulator LSRR sequence to the 5' end of the sequences generated and a poly(A) sequence (25 bps) to the 3' end of the sequences generated.

Step 1c. Remove sequences generated and appended that do not have GC contents in the range of 40% to 50%.

Step 1d. Remove remaining sequences with one or more hairpin structures each.

The number of remaining candidate oligonucleotide sequences was 423.

### 2. Primer design

The following method was used to design primers for the remaining 423 candidate oligonucleotide sequences.

2.1 N1 Primer: Use the universal N1 sequence: 5'-GTTGTCAAGATGCTACCGTTCAGAG-3' (LSRR sequence; SEQ ID NO. 3) as the N1 primer.

2.2 N2 Primer (for amplifying specific sample index oligonucleotides; e.g., N2 primer in FIGS. 9B-9D):
2.2a. Remove candidate N2 primers that do not start downstream of the N1 sequence.
2.2b. Remove candidate N2 primers that overlap in the last 35 bps of the candidate oligonucleotide sequence.
2.2c. Remove the primer candidates that are aligned to the transcriptome of the species of cells being studied using the oligonucleotides (e.g., the human transcriptome or the mouse transcriptome).

2.2d. Use the ILR2 sequence as the default control (ACACGACGCTCTTCCGATCT; SEQ ID NO. 4) to minimize or avoid primer-primer interactions.

Of the 423 candidate oligonucleotide sequences, N2 primers for 390 candidates were designed.

### 3. Filtering

The following process was used to filter the remaining 390 candidate primer sequences.

3a. Eliminate any candidate oligonucleotide sequence with a random sequence ending in As (i.e., the effective length of the poly(A) sequence is greater than 25 bps) to keep poly(A) tail the same length for all barcodes.

3b. Eliminate any candidate oligonucleotide sequences with 4 or more consecutive Gs (> 3Gs) because of extra cost and potentially lower yield in oligo synthesis of runs of Gs.

FIG. 9A shows a non-limiting exemplary candidate oligonucleotide sequence generated using the method above.

### 200mer Oligonucleotide Design

The following method was used to generate candidate oligonucleotide sequences and corresponding primer sequences for simultaneous determination of protein expression and gene expression and sample indexing.

### 1. Sequence generation and elimination

The following was used to generate candidate oligonucleotide sequences for simultaneous determination of protein expression and gene expression and sample indexing.

1a. Randomly generate a number of candidate sequences (100000 sequences) with the desired length (128 bps).

1b. Append the transcriptional regulator LSRR sequence and an additional anchor sequence that is non-human, non-mouse to the 5' end of the sequences generated and a poly(A) sequence (25 bps) to the 3' end of the sequences generated.

1c. Remove sequences generated and appended that do not have GC contents in the range of 40% to 50%.

1d. Sort remaining candidate oligonucleotide sequences based on hairpin structure scores.

1e. Select 1000 remaining candidate oligonucleotide sequences with the lowest hairpin scores.

### 2. Primer design

The following method was used to design primers for 400 candidate oligonucleotide sequences with the lowest hairpin scores.

2.1 N1 Primer: Use the universal N1 sequence: 5'-GTTGTCAAGATGCTACCGTTCAGAG-3' (LSRR sequence; SEQ ID NO. 3) as the N1 primer.

2.2 N2 Primer (for amplifying specific sample index oligonucleotides; e.g., N2 primer in FIGS. 9B and 9C):
2.2a. Remove candidate N2 primers that do not start 23 nts downstream of the N1 sequence (The anchor sequence was universal across all candidate oligonucleotide sequences).
2.2b. Remove candidate N2 primers that overlap in the last 100 bps of the target sequence. The resulting primer candidates can be between the 48th nucleotide and 100th nucleotide of the target sequence.
2.2c. Remove the primer candidates that are aligned to the transcriptome of the species of cells being studied using the oligonucleotides (e.g., the human transcriptome or the mouse transcriptome).
2.2d. Use the ILR2 sequence, 5'-ACACGACGCTCTTCCGATCT-3' (SEQ ID NO. 4) as the default control to minimize or avoid primer-primer interactions.
2.2e. Remove N2 primer candidates that overlap in the last 100 bps of the target sequence.

Of the 400 candidate oligonucleotide sequences, N2 primers for 392 candidates were designed.

### 3. Filtering

The following was used to filter the remaining 392 candidate primer sequences.

3a. Eliminate any candidate oligonucleotide sequence with a random sequence ending in As (i.e., the effective length of the poly(A) sequence is greater than 25 bps) to keep poly(A) tail the same length for all barcodes.

3b. Eliminate any candidate oligonucleotide sequences with 4 or more consecutive Gs (> 3Gs) because of extra cost and potentially lower yield in oligo synthesis of runs of Gs.

FIG. 9B shows a non-limiting exemplary candidate oligonucleotide sequence generated using the method above. The nested N2 primer shown in FIG. 9B can bind to the antibody or sample specific sequence for targeted amplification. FIG. 9C shows the same non-limiting exemplary candidate oligonucleotide sequence with a nested universal N2 primer that corresponds to the anchor sequence for targeted amplification. FIG. 9D shows the same non-limiting exemplary candidate oligonucleotide sequence with a N2 primer for one step targeted amplification.

Altogether, these data indicate that oligonucleotide sequences of different lengths can be designed for simultaneous determination of protein expression and gene expression or sample indexing. The oligonucleotide sequences can include a universal primer sequence, an antibody specific oligonucleotide sequence or a sample indexing sequence, and a poly(A) sequence.

### Example 2. Oligonucleotide-Associated Antibody Workflow

This example demonstrates a workflow of using an oligonucleotide-conjugated antibody for determining the expression profile of a protein target.

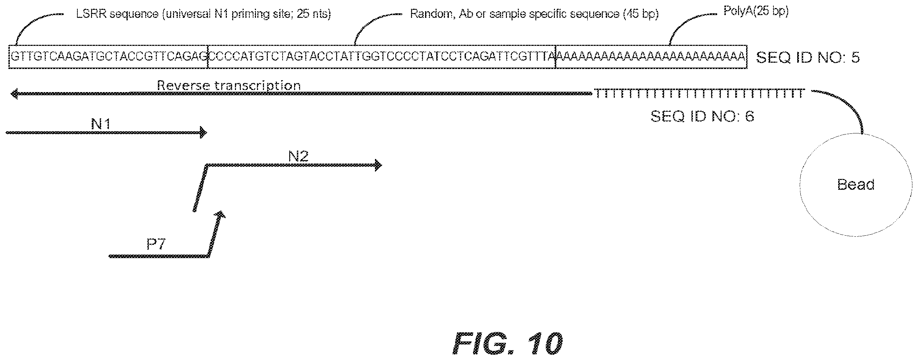

Frozen cells (e.g., frozen peripheral blood mononuclear cells (PBMCs)) of a subject are thawed. The thawed cells are stained with an oligonucleotide-conjugated antibody (e.g., an anti-CD4 antibody at 0.06 µg/100 µl (1:333 dilution of an oligonucleotide-conjugated antibody stock)) at a temperature for a duration (e.g., room temperature for 20 minutes). The oligonucleotide-conjugated antibody is conjugated with 1, 2, or 3 oligonucleotides ("antibody oligonucleotides"). The sequence of the antibody oligonucleotide is shown in FIG. 10. The cells are washed to remove unbound oligonucleotide-conjugated antibody. The cells are optionally stained with Calcein AM (BD (Franklin Lake, New Jersey)) and Draq7^{™} (Abcam (Cambridge, United Kingdom)) for sorting with flow cytometry to obtain cells of interest (e.g., live cells). The cells are optionally washed to remove excess Calcein AM and Draq7^{™}. Single cells stained with Calcein AM (live cells) and not Draq7^{™} (cells that are not dead or permeabilized) are sorted, using flow cytometry, into a BD Rhapsody^{™} cartridge.

Of the wells containing a single cell and a bead, the single cells in the wells (e.g., 3500 live cells) are lysed in a lysis buffer (e.g., a lysis buffer with 5 mM DTT). The mRNA expression profile of a target (e.g., CD4) is determined using BD Rhapsody^{™} beads. The protein expression profile of a target (e.g., CD4) is determined using BD Rhapsody^{™} beads and the antibody oligonucleotides. Briefly, the mRNA molecules are released after cell lysis. The Rhapsody^{™} beads are associated with barcodes (e.g., stochastic barcodes) each containing a molecular label, a cell label, and an oligo(dT) region. The poly(A) regions of the mRNA molecules released from the lysed cells hybridize to the poly(T) regions of the stochastic barcodes. The poly(dA) regions of the antibody oligonucleotides hybridize to the oligo(dT) regions of the barcodes. The mRNA molecules were reverse transcribed using the barcodes. The antibody oligonucleotides are replicated using the barcodes. The reverse transcription and replication optionally occur in one sample aliquot at the same time.

The reverse transcribed products and replicated products are PCR amplified using primers for determining mRNA expression profiles of genes of interest, using N1 primers, and the protein expression profile of a target, using the antibody oligonucleotide N1 primer. For example, the reverse transcribed products and replicated products can be PCR amplified for 15 cycles at 60 degrees annealing temperature using primers for determining the mRNA expression profiles of 488 blood panel genes, using blood panel N1 primers, and the expression profile of CD4 protein, using the antibody oligonucleotide N1 primer ("PCR 1"). Excess barcodes are optionally removed with Ampure cleanup. The products from PCR 1 are optionally divided into two aliquots, one aliquot for determining the mRNA expression profiles of the genes of interest, using the N2 primers for the genes of interest, and one aliquot for determining the protein expression profile of the target of interest, using the antibody oligonucleotide N2 primer ("PCR 2"). Both aliquots are PCR amplified (e.g., for 15 cycles at 60 degrees annealing temperature). The protein expression of the target in the cells are determined based on the antibody oligonucleotides as illustrated in FIG. 10 ("PCR 2"). Sequencing data is obtained and analyzed after sequencing adaptor addition ("PCR 3"), such as sequencing adaptor ligation. Cell types are determined based on the mRNA expression profiles of the genes of interest.

Altogether, this example describes using an oligonucleotide-Conjugated antibody for determining the protein expression profile of a target of interest. This example further describes that the protein expression profile of the target of interest and the mRNA expression profiles of genes of interest can be determine simultaneously.

### Example 3. Cellular Component-Binding Reagent Oligonucleotides

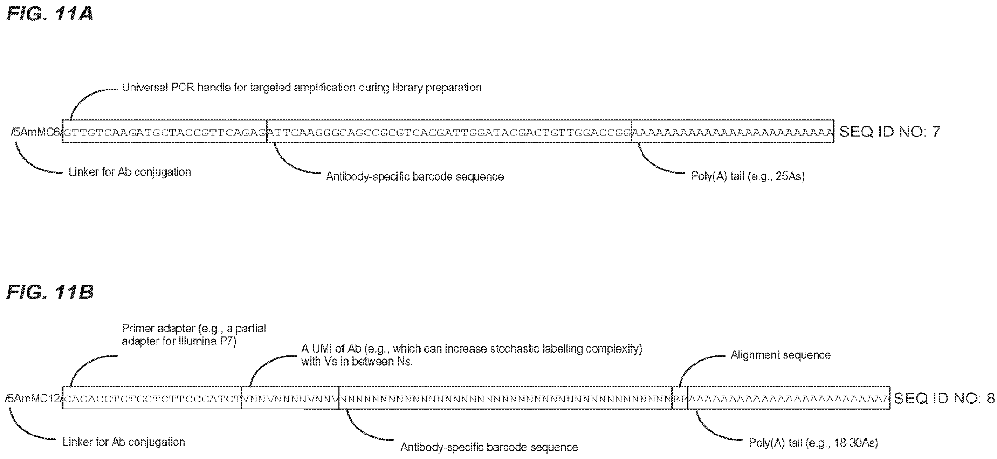

FIGS. 11A-11B show non-limiting exemplary designs of oligonucleotides for determining protein expression and gene expression simultaneously and for sample indexing. FIG. 11A shows a non-limiting exemplary cellular component-binding reagent oligonucleotide (SEQ ID NO: 7) comprising a 5' amino modifier C6 (5AmMC6) linker for antibody conjugation (e.g., can be modified prior to antibody conjugation), a universal PCR handle, an antibody-specific barcode sequence, and a poly(A) tail. While this embodiment depicts a poly(A) tail that is 25 nucleotides long, the length of the poly(A) tail can vary. In some embodiments, the antibody-specific barcode sequence is antibody clone-specific barcode for use in methods of protein expression profiling. In some embodiments, the antibody-specific barcode sequence is a sample tag sequence for use in methods of sample indexing. Exemplary design characteristics of the antibody-specific barcode sequence are, in some embodiments, a Hamming distance greater than 3, a GC content in the range of 40% to 60%, and an absence of predicted secondary structures (e.g., hairpin). In some embodiments, the universal PCR handle is employed for targeted PCR amplification during library preparation that attaches Illumina sequencing adapters to the amplicons. In some embodiments, high quality sequencing reads can be achieved by reducing sequencing diversity.

FIG. 11B shows a non-limiting exemplary cellular component-binding reagent oligonucleotide (SEQ ID NO: 8) comprising a 5' amino modifier C12 (5AmMC12) linker for antibody conjugation, a primer adapter (e.g., a partial adapter for Illumina P7), an antibody unique molecular identifier (UMI), an antibody-specific barcode sequence, an alignment sequence, and a poly(A) tail. While this embodiment depicts a poly(A) tail that is 25 nucleotides long, the length of the poly(A) tail can range, in some embodiments, from 18-30 nucleotides. Exemplary design characteristics of the antibody-specific barcode sequence (wherein "X" indicates any nucleotide), in addition to those described in FIG. 11A, include, in some embodiments, an absence of homopolymers and an absence of sequences predicted *in silico* to bind human transcripts, mouse transcripts, Rhapsody system primers, and/or SCMK system primers. In some embodiments, the alignment sequence comprises the sequence BB (in which B is C, G, or T). Alignment sequences 1 nucleotide in length and more than 2 nucleotides in length are provided in some embodiments. The 5AmMC12 linker, can, in some embodiments, achieve a higher efficiency (e.g., for antibody conjugation or the modification prior to antibody conjugation) as compared to a shorter linker (e.g., 5AmMC6). The antibody UMI sequence can comprise "VN" and/or "NV" doublets (in which each "V" is any of A, C, or G, and in which "N" is any of A, G, C, or T), which, in some embodiments, improve informatics analysis by serving as a geomarker and/or reduce the incidence of homopolymers. In some embodiments, the presence of a unique molecular labeling sequence on the binding reagent oligonucleotide increases stochastic labelling complexity. In some embodiments, the primer adapter comprises the sequence of a first universal primer, a complimentary sequence thereof, a partial sequence thereof, or a combination thereof. In some embodiments, the primer adapter eliminates the need for a PCR amplification step for attachment of Illumina sequencing adapters that would typically be required before sequencing. In some embodiments, the primer adapter sequence (or a subsequence thereof) is not part of the sequencing readout of a sequencing template comprising a primer adapter sequence and therefore does not affect read quality of a template comprising a primer adapter.

### Example 4. Design and Testing of AbSeq Probes

In this example, proof of concept for AbSeq probes provided herein is demonstrated. Four mini panels of probes were developed to detect 14 AbSeq being employed (Tables 1 and 2).

**TABLE 1: 14 plex AbSeq**

| | | | Cat# | AHS |
|---|---|---|---|---|
| Naive/Effector T cell Panel | 1 | CD4 | 940001 | 0032 |
| | 2 | CD8 | 940003 | 0027 |
| | 3 | CCR7 | 940014 | 0007 |
| | 4 | CD45RO | 940022 | 0036 |
| Treg Panel | | CD4 | 940001 | 0032 |
| | 5 | CD25 | 940009 | 0026 |
| | 6 | HLA-DR | 940010 | 0035 |
| | 7 | CD45RA | 940011 | 0009 |
| B cell Panel | 8 | CD19 | 940004 | 0030 |
| | 9 | CD3 | 940000 | 0033 |
| | 10 | CD27 | 940018 | 0025 |
| | 11 | IgD | 940026 | 0058 |
| Dendritic cell panel | | HLA-DR | 940010 | 0035 |
| | 12 | CD11c | 940024 | 0056 |
| | 13 | CD16 | 940006 | 0053 |

**TABLE 2: Oligo probes-mini panels**

| | |
|---|---|
| Naïve/effector T cell panel | CD4 Cy5 |
| | CD8 Texas-Red |
| | CCR7 HEX |
| | CD45RO FAM |
| Treg panel | CD4 Cy5 |
| | CD25 HEX |
| | HLA-DR Texas-Red |
| | CD45RA FAM |
| B cell panel | CD19 HEX |
| | CD3 Texas-Red |
| | CD27 FAM |
| | IgD Cy5 |
| Dendritic cell panel | HLA-DR Texas-Red |
| | CD11c Cy5 |
| | CD16 HEX |
| | CD123 FAM |

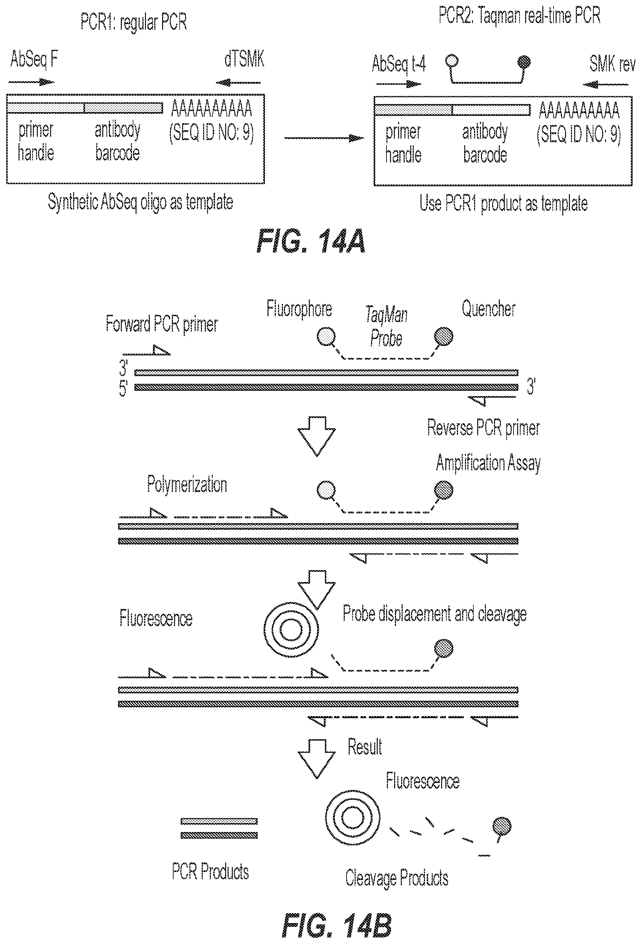

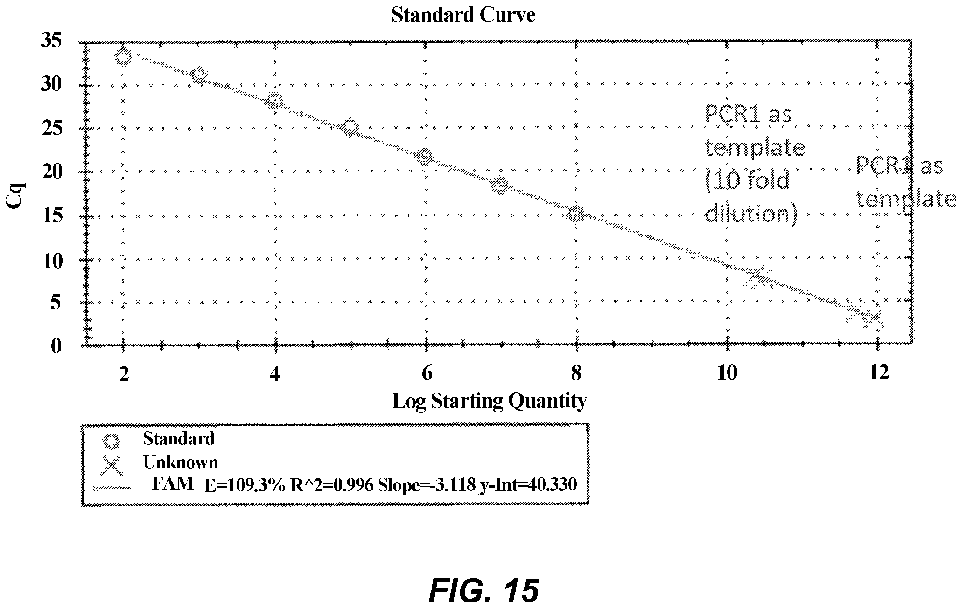

FIGS. 14A and 14B depict non-limiting exemplary qPCR workflows. It was found that AbSeq oligo can be detected using the oligo itself as the template (no cells used in this experiment). FIG. 15 depicts non-limiting exemplary data related to a standard curve generated using the disclosed methods and compositions. One template was tested, using the CD45RO AbSeq oligo and its specific Taqman probe. The experiment incorporated "non-template control" to ensure there is little contamination (~85 copies). It was found that nothing could be amplified if using CD123 probe against CD45RO template (N/A: not detectable). These data support the feasibility of the 2-step PCR approach. These data confirm the accuracy of primers and oligo probes provided herein (at least for CD45RO probe). Accordingly, profiling of cells was next explored in the following example.

### Example 5. Profiling Single Cells

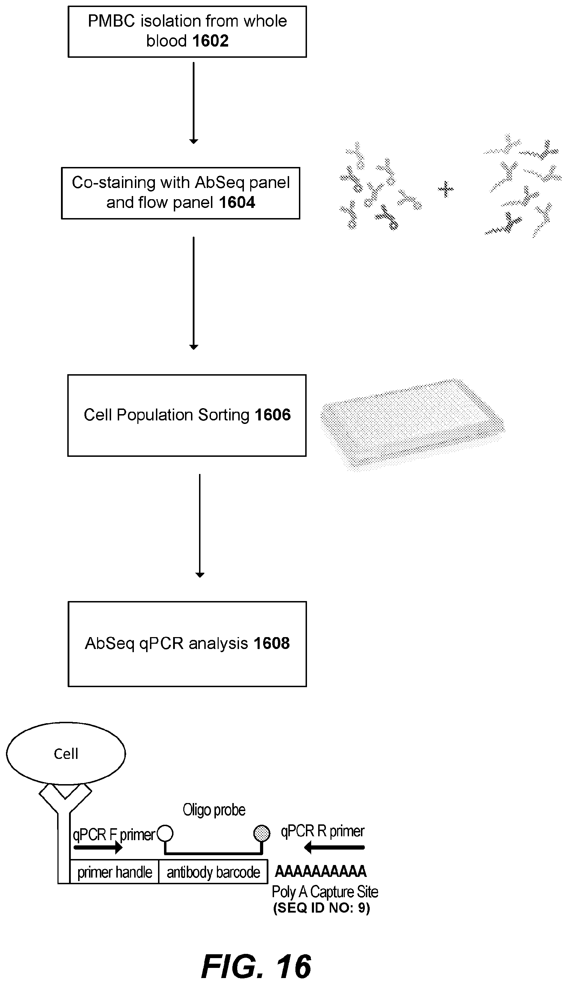

In this example, proof of concept of profiling single cells with the methods and compositions provided herein is demonstrated. FIG. 16 depicts a non-limiting exemplary experimental workflow. The workflow can comprise PBMC isolation from whole blood (step 1602). The workflow can comprise co-staining with a flow panel and AbSeq panel (step 1604). The workflow can comprise cell sorting, e.g., employing a StepSorter (384 well plate with 24 wells X 16 rows) (step 1606). Accordingly, the workflow can comprise sorting into a 384 well plate the cell populations provided in Table 3. The workflow can comprise AbSeq qPCR analysis (step 1608). The workflow shown in FIG. 16 was performed with the goal of confirming StepSort accuracy by amplifying the oligo conjugates on the specific AbSeq antibody using oligo probes.

**TABLE 3: Populations Sorted into a 384 Well Plate**

| | |
|---|---|
| Population 1 | Naïve CD4 T cells |
| Population 2 | Effector memory CD4 T cells |
| Population 3 | Naïve CD8 T cells |
| Population 4 | Effector memory CD8 T cells |
| Population 5 | Naïve CD4 Treg cells |
| Population 6 | Effector memory CD4 Treg cells |
| Population 7 | Naïve B cells |
| Population 8 | Memory B cells |
| Population 9 | CD16 DC |
| Population 10 | plasmacytoid DC |

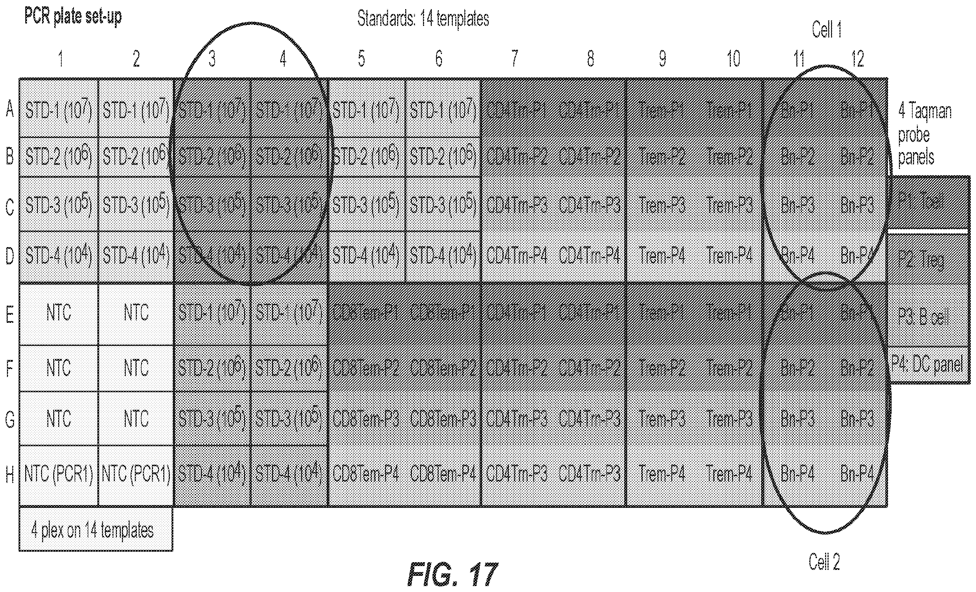

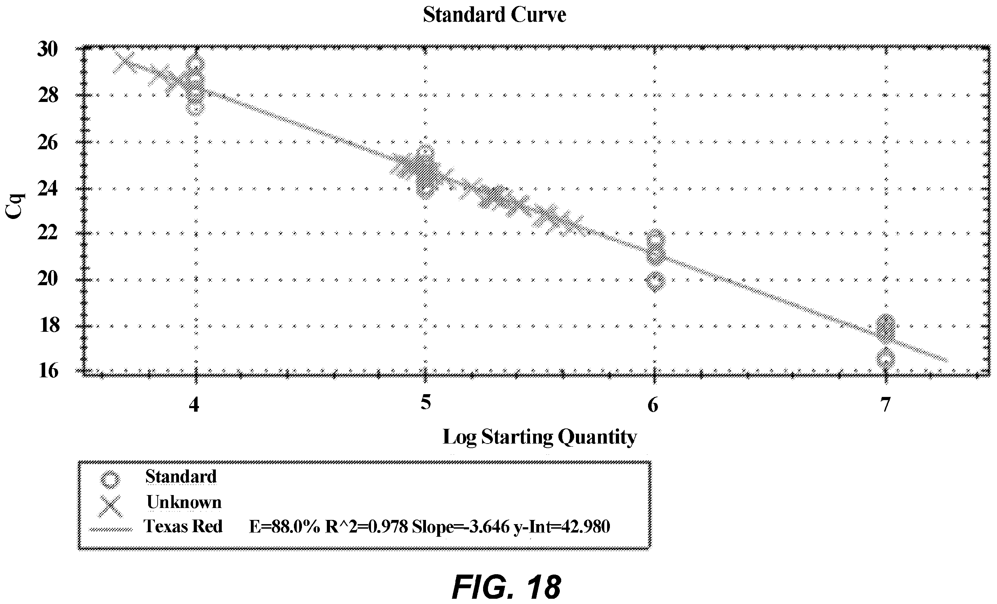

FIG. 17 depicts a non-limiting exemplary PCR plate set-up. Circles indicate a first cell, a second cell, and standards (14 templates). 4 Taqman probe panels were employed. Six plates were set up like FIG. 17 to cover 2 sorted cells per immune cell type, for a total 10 different immune populations (Table 3). FIG. 18 depicts non-limiting exemplary standard curve generated.

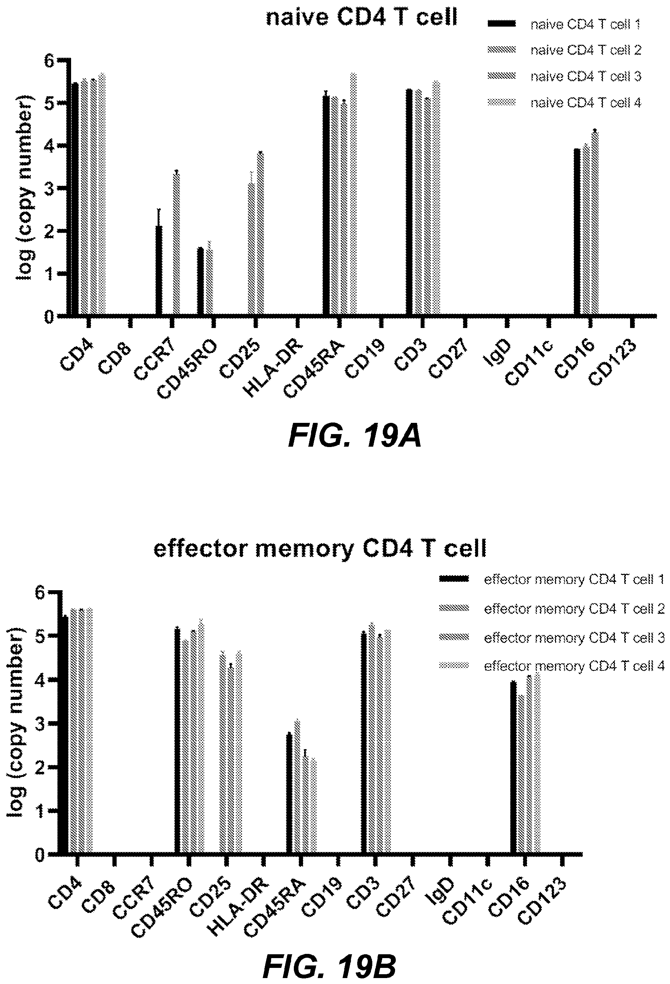

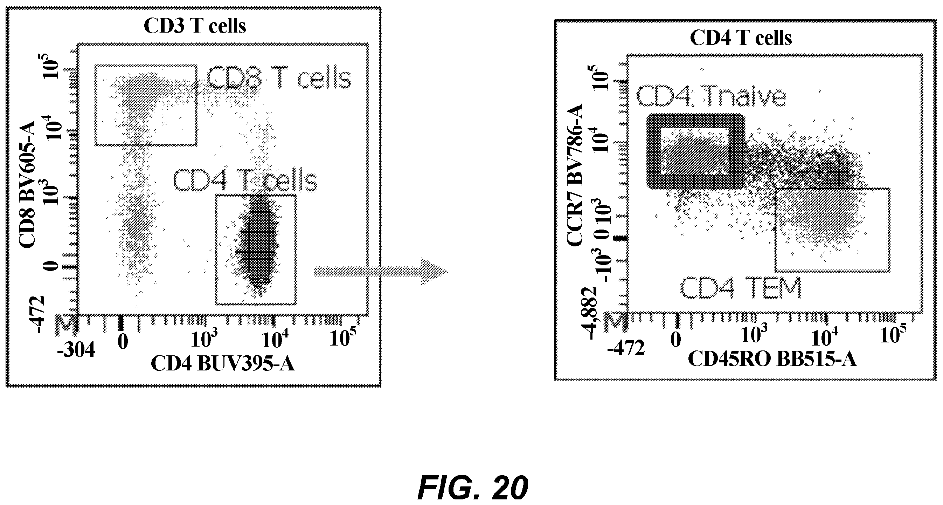

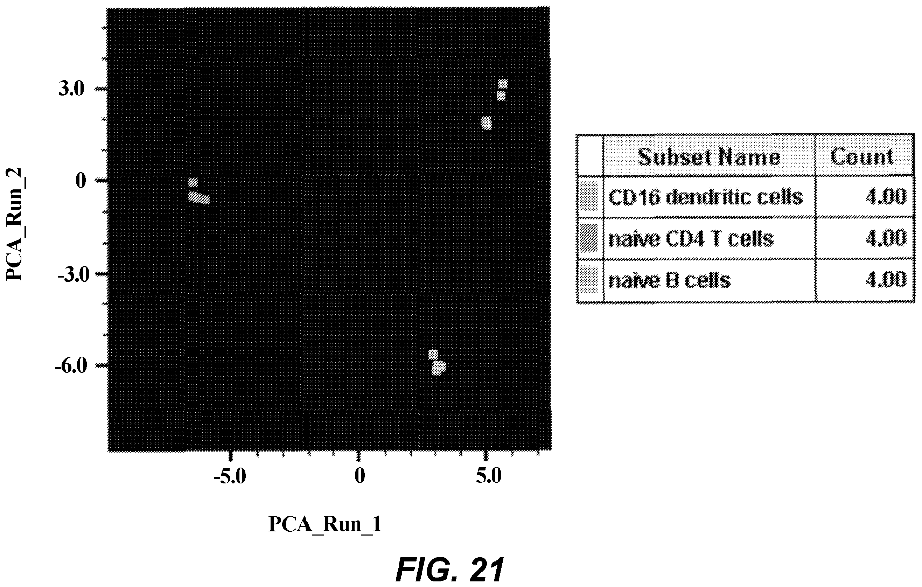

The experiment identified the copy number of each marker on a particular cell type. FIGS. 19A-19B depict non-limiting exemplary data generated using the methods and compositions described herein for naive CD4 T cell (FIG. 19A) and effector memory CD4 T cell (FIG. 19B). FIG. 20 depicts non-limiting exemplary data generated using the methods and compositions described herein. Results relate to cells that are CD3+, CD4+, CCR7+, and CD45RO-. Cellular heterogeneity was revealed. CD45RA was only present in AbSeq panel but not in flow cytometry panel (characterization of marker signature of individual cells at the qPCR level). Different levels of CD45RA and CD45RO were found to help define cell types. Heterogeneity of CCR7 detection may be due to CCR7 AbSeq clone. Multivariate analysis shows cell type specific clustering (FIG. 21).

### Terminology

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. All such modifications and changes are intended to fall within the scope of the subject matter, as defined by the appended claims.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. As used in this specification and the appended claims, the singular forms "a," "an," and "the" include plural references unless the context clearly dictates otherwise. Any reference to "or" herein is intended to encompass "and/or" unless otherwise stated.

It will be understood by those within the art that, in general, terms used herein, and especially in the appended claims (*e.g.,* bodies of the appended claims) are generally intended as "open" terms (*e.g.,* the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g.,* "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g.,* the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (*e.g.,* " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

## Claims

1. A method for measuring cellular component target expression in cells, comprising:
contacting a plurality of first cellular component-binding reagents with a first plurality of cells comprising a plurality of cellular component targets, wherein each of the plurality of first cellular component-binding reagents comprises a cellular component-binding reagent specific oligonucleotide comprising a unique identifier sequence for the first cellular component-binding reagent, and wherein the first cellular component-binding reagent is capable of specifically binding to at least one of the plurality of cellular component targets;
generating amplicons of the unique identifier sequence of one or more of the first cellular component-binding reagents;
determining the amount of one or more amplicons as an indication of the amount of the one or more of the first cellular component-binding reagents; and
if the amount of one or more amplicons indicates an abundance of a first cellular component-binding reagent above or below a predetermined abundance range, repeating the contacting step with a second plurality of cells in a manner configured to achieve an abundance of said first cellular component-binding reagent within said predetermined abundance range.

2. The method of claim 1, wherein:
(a) the predetermined abundance range comprises the dynamic range for which acceptable linearity and efficiency of detection of the first cellular component-binding reagent are observed;
(b) the method comprises contacting the first and/or second plurality of cells with a plurality of third cellular component-binding reagents, wherein a third cellular component-binding reagent is capable of specifically binding to at least one of the plurality of cellular component targets, wherein the third cellular component-binding reagents do not comprise a cellular component-binding reagent specific oligonucleotide, wherein one or more of the first cellular component-binding reagents and one or more of the third cellular component-binding reagents are capable of binding the same cellular component target, optionally first cellular component-binding reagents and third cellular component-binding reagents capable of binding the same cellular component target have the same clonotype; and/or
(c) repeating the contacting step with a second plurality of cells in a manner configured to achieve an abundance of said first cellular component-binding reagent within said predetermined abundance range comprises contacting the second plurality of cells with mixture of said first cellular component-binding reagent and a third cellular component-binding reagent capable of binding the same cellular component target as said first cellular component-binding reagent at a titration ratio configured to achieve an abundance of said first cellular component-binding reagent within said predetermined abundance range, optionally the titration ratio is configured such that sequencing reads comprising the unique identifier sequence of said first cellular component-binding reagent account for less than about 5% of total sequencing reads.

3. A method for measuring cellular component target expression in cells, comprising:
contacting a plurality of first cellular component-binding reagents with the first plurality of cells comprising a plurality of cellular component targets, wherein each of the plurality of first cellular component-binding reagents comprises a cellular component-binding reagent specific oligonucleotide comprising a unique identifier sequence for the first cellular component-binding reagent, and wherein the first cellular component-binding reagent is capable of specifically binding to at least one of the plurality of cellular component targets;
isolating one or more cell types of interest from the first plurality of cells, wherein a first cell type of interest is **characterized by** abundance of: (i) one or more positive cellular component targets above a positive abundance threshold and/or (ii) one or more negative cellular component targets below a negative abundance threshold;
generating amplicons of the unique identifier sequence of one or more of the first cellular component-binding reagents; and
determining the amount of one or more amplicons as an indication of the amount of the cellular component targets bound by said one or more of the first cellular component-binding reagents,
wherein one or more cells suspected of being a first cell type of interest is determined to not be a first cell type of interest if the amount of one or more amplicons indicates an abundance of: (i) one or more positive cellular component targets below a positive abundance threshold and/or (ii) one or more negative cellular component targets above a negative abundance threshold.

4. The method of claim 3, comprising, if one or more cells suspected of being a first cell type of interest is determined to not be a first cell type of interest, repeating the contacting step and/or isolating step with a second plurality of cells in a manner configured to achieve isolation of the one or more cell types of interest.

5. The method of any one of claims 1-4, comprising:
contacting a plurality of oligonucleotide barcodes with the cellular component-binding reagent specific oligonucleotides for hybridization, wherein the oligonucleotide barcodes each comprise a first molecular label and a first universal sequence; and
extending the plurality of oligonucleotide barcodes hybridized to the cellular component-binding reagent specific oligonucleotides to generate a plurality of barcoded cellular component-binding reagent specific oligonucleotides each comprising a sequence complementary to at least a portion of the unique identifier sequence and the first molecular label, optionally comprising obtaining sequence information of the plurality of barcoded cellular component-binding reagent specific oligonucleotides, or products thereof, to determine the number of copies of at least one cellular component target of the plurality of cellular component targets in one or more of the first plurality of cells and/or second plurality of cells, optionally wherein obtaining the sequence information comprises attaching sequencing adaptors to the plurality of barcoded cellular component-binding reagent specific oligonucleotides, or products thereof.

6. The method of any one of claims 1-5, wherein:
(a) the cellular component-binding reagent specific oligonucleotide comprises a sequence complementary to a capture sequence of an oligonucleotide barcode configured to capture the sequence of the cellular component-binding reagent specific oligonucleotide, optionally the sequence of the cellular component-binding reagent specific oligonucleotide complementary to the capture sequence comprises a poly(dA) region; and/or
(b) the cellular component-binding reagent specific oligonucleotide comprises a second universal sequence.

7. The method of any one of claims 1-6, wherein generating amplicons comprises:
(a) amplifying the plurality of cellular component-binding reagent specific oligonucleotides using a primer capable of hybridizing to the second universal sequence, or a complement thereof, and a primer capable of hybridizing to the capture sequence, or a complement thereof, thereby generating amplicons of the unique identifier sequence of one or more of the first cellular component-binding reagents;
(b) amplifying the plurality of cellular component-binding reagent specific oligonucleotides using a primer capable of hybridizing to the second universal sequence, or a complement thereof, and a primer capable of hybridizing to the capture sequence, or a complement thereof, thereby generating PCR1 products; and amplifying the PCR1 products using a primer capable of hybridizing to the second universal sequence, or a complement thereof, and a primer capable of hybridizing to the capture sequence, or a complement thereof, thereby generating amplicons of the unique identifier sequence of one or more of the first cellular component-binding reagents;
(c) amplifying the plurality of barcoded cellular component-binding reagent specific oligonucleotides using a primer capable of hybridizing to the second universal sequence, or a complement thereof, and a primer capable of hybridizing to the capture sequence, or a complement thereof, thereby generating amplicons of the unique identifier sequence of one or more of the first cellular component-binding reagents;
(d) amplifying the plurality of barcoded cellular component-binding reagent specific oligonucleotides using a primer capable of hybridizing to the second universal sequence, or a complement thereof, and a primer capable of hybridizing to the capture sequence, or a complement thereof, thereby generating PCR1 products; and amplifying the PCR1 products using a primer capable of hybridizing to the second universal sequence, or a complement thereof, and a primer capable of hybridizing to the capture sequence, or a complement thereof, thereby generating amplicons of the unique identifier sequence of one or more of the first cellular component-binding reagents;
(e) amplifying the plurality of barcoded cellular component-binding reagent specific oligonucleotides using a primer capable of hybridizing to the first universal sequence, or a complement thereof, and a primer capable of hybridizing to the second universal sequence, or a complement thereof, thereby generating amplicons of the unique identifier sequence of one or more of the first cellular component-binding reagents; and/or
(f) amplifying the plurality of barcoded cellular component-binding reagent specific oligonucleotides using a primer capable of hybridizing to the first universal sequence, or a complement thereof, and a primer capable of hybridizing to the second universal sequence, or a complement thereof, thereby generating PCR1 products; and amplifying the PCR1 products using a primer capable of hybridizing to the second universal sequence, or a complement thereof, and a primer capable of hybridizing to the first universal sequence, or a complement thereof, thereby generating amplicons of the unique identifier sequence of one or more of the first cellular component-binding reagents.

8. The method of any one of claims 1-7, wherein said amplification is carried out using a method selected from the group consisting of polymerase chain reaction (PCR), ligase chain reaction (LCR), loop-mediated isothermal amplification (LAMP), strand displacement amplification (SDA), replicase-mediated amplification, Immuno-amplification, nucleic acid sequence based amplification (NASBA), self-sustained sequence replication (3SR), rolling circle amplification, and transcription-mediated amplification (TMA), optionally wherein said PCR is:
(a) real-time PCR;
(b) quantitative real-time PCR (QRT-PCR); or
(c) digital PCR (dPCR), optionally droplet digital PCR (ddPCR).

9. The method of any one of claims 1-8, wherein:
(a) determining the presence or amount of one or more amplicons comprises contacting the amplicons with a plurality of oligonucleotide probes, wherein each of the plurality of oligonucleotide probes comprises a sequence configured to bind a unique identifier sequence, or a complement thereof, optionally wherein each probe is flanked by complementary sequences at the 5' end and 3' end, optionally wherein one of the complementary sequences comprises a fluorescence emitter moiety and the other complementary sequence comprises a fluorescence quencher moiety;
(b) at least one of the plurality of oligonucleotide probes comprises a fluorescence emitter moiety and a fluorescence quencher moiety;
(c) the oligonucleotide probe comprises a TaqMan detection probe oligonucleotide, a molecular beacon detection probe oligonucleotide, or a molecular torch detection probe oligonucleotide;
(d) the generating amplicons step and determining step are multiplexed, optionally at least about 4 unique identifier sequences are analyzed simultaneously;
(e) the generating amplicons step and determining step are performed on single cells or multiple cells; and/or
(f) the isolating step comprises single cell sorting or bulk sorting.

10. The method of any one of claims 1-9, wherein:
(a) the one or more cell types of interest comprise hemogenic endothelium cells, hematopoietic stem and progenitor cells (HSC), hematopoietic multipotent progenitor cell (MPP), pre-T cell progenitor cells, pre- K cell progenitor cells, T cell progenitor cells, NK cell progenitor cells, T cells, NK cells, NKT cells, B cells, macrophage, neutrophils, CD4 cells, CD8 cells, naive T-cells, memory stem T-cells, central memory T- cells, double negative T-cells, effector memory T-cells, effector T-cells, ThO cells, TcO cells, Thl cells, Tel cells, Th2 cells, Tc2 cells, Thl7 cells, Th22 cells, gamma/delta T-cells, natural killer (NK) cells, natural killer T (NKT) cells, hematopoietic stem cells pluripotent stem cells, or any combination thereof;
(b) the one or more cell types of interest comprise or more immune cell types, optionally naive CD4 T cells, effector memory CD4 T cells, naive CD8 T cells, effector memory CD8 T cells, naive CD4 Treg cells, effector memory CD4 Treg cells, naive B cells, memory B cells, CD16 DC, plasmacytoid DC, or any combination thereof;
(c) the method comprises contacting a plurality of second cellular component-binding reagents with the first and/or second plurality of cells, wherein each of the plurality of second cellular component-binding reagents comprises a detectable moiety, or a precursor thereof, wherein a second cellular component-binding reagent is capable of specifically binding to at least one of the plurality of cellular component targets, wherein second cellular component-binding reagents capable of binding the same cellular component target comprise the same detectable moiety, or a precursor thereof, and wherein second cellular component-binding reagents capable of binding different cellular component targets comprise different detectable moieties, or precursors thereof;
(d) the first plurality of cells and/or second plurality of cells are contacted with the first cellular component-binding reagents and second cellular component-binding reagents simultaneously;
(e) one or more of the first cellular component-binding reagents and one or more of the second cellular component-binding reagents are capable of binding the same cellular component target, optionally first cellular component-binding reagents and second cellular component-binding reagents capable of binding the same cellular component target have the same clonotype; And/or
(f) the isolating step comprises:
(i) fluorescence-activated cell sorting, optionally fluorescence-activated cell sorting employing the second cellular component-binding reagents;
(ii) depositing the one or more cells of interest of the first and/or second plurality of cells into one or more microtiter plates; and/or
(iii) detecting emissions of the detectable moiety of second cellular component-binding reagents with a flow cytometer, optionally the flow cytometer comprises a conventional flow cytometer, a spectral flow cytometer, a hyperspectral flow cytometer, an imaging flow cytometer, or any combination thereof; and/or
(g) the detectable moiety comprises an optical moiety, a luminescent moiety, an electrochemically active moiety, a nanoparticle, or a combination thereof, optionally the nanoparticle comprises a quantum dot;
(h) the luminescent moiety comprises a chemiluminescent moiety, an electroluminescent moiety, a photoluminescent moiety, or a combination thereof;
(i) the photoluminescent moiety comprises a fluorescent moiety, a phosphorescent moiety, or a combination thereof, optionally the fluorescent moiety comprises a fluorescent dye;
(j) the method comprises performing a reaction to convert the detectable moiety precursor into the detectable moiety;
(k) the one or more positive cellular component targets and/or one or more negative cellular component targets comprises CD4, CD8, CCR7, CD45RO, CD25, HLA-DR, CD45RA, CD19, CD3, CD27, IgD, CD11c, CD16, or any combination thereof; and/or
(l) the number of unique first molecular label sequences associated with the unique identifier sequence for the first cellular component-binding reagent capable of specifically binding to the at least one cellular component target in the sequencing data indicates the number of copies of the at least one cellular component target in the one or more of the first and/or second plurality of cells.

11. The method of any one of claims 1-10, wherein:
(a) the cellular component-binding reagent specific oligonucleotide comprises one or more of:
(i) a second molecular label sequence, optionally wherein:
(A) the second molecular label sequence is 2-20 nucleotides in length;
(B) the second molecular label sequences of at least two cellular component-binding reagent specific oligonucleotides are different, and wherein the unique identifier sequences of the at least two cellular component-binding reagent specific oligonucleotides are identical; and/or
(C) the second molecular label sequences of at least two cellular component-binding reagent specific oligonucleotides are different, and wherein the unique identifier sequences of the at least two cellular component-binding reagent specific oligonucleotides are different; and/or
(ii) an alignment sequence adjacent to a poly(dA) region, optionally the alignment sequence is one or more nucleotides, or two or more nucleotides, in length; and
(iii) a linker, wherein the cellular component-binding reagent specific oligonucleotide is associated with the first cellular component-binding reagent through the linker; and/or
(b) the number of unique second molecular label sequences associated with the unique identifier sequence for the first cellular component-binding reagent capable of specifically binding to the at least one cellular component target in the sequencing data indicates the number of copies of the at least one cellular component target in the one or more of the first and/or second plurality of cells; and/or
(c) the alignment sequence:
(i) comprises a guanine, a cytosine, a thymine, a uracil, or a combination thereof;
(ii) comprises a poly(dT) sequence, a poly(dG) sequence, a poly(dC) sequence, a poly(dU) sequence, or a combination thereof; and/or
(iii) is 5' to the poly(dA) region; and/or
(d) the linker comprises:
(i) a carbon chain, optionally the carbon chain comprises 2-30 carbons, and further optionally the carbon chain comprises 12 carbons; and/or
(ii) 5' amino modifier C12 (5AmMC12), or a derivative thereof; and/or
(e) the plurality of cellular component targets comprises a plurality of protein targets, and wherein the first cellular component-binding reagent is capable of specifically binding to at least one of the plurality of protein targets, optionally the plurality of cellular component targets comprises a cell-surface protein, an intracellular protein, a cell marker, a B-cell receptor, a T-cell receptor, an antibody, a major histocompatibility complex, a tumor antigen, a receptor, or a combination thereof; and/or
(f) the cellular component-binding reagent specific oligonucleotide is:
(i) associated with the first cellular component-binding reagent, optionally the cellular component-binding reagent specific oligonucleotide is covalently attached to the first cellular component-binding reagent and/or non-covalently attached to the first cellular component-binding reagent;
(ii) conjugated to the first cellular component-binding reagent, and optionally the cellular component-binding reagent specific oligonucleotide is conjugated to the first cellular component-binding reagent through a chemical group selected from the group consisting of a UV photocleavable group, a streptavidin, a biotin, an amine, and a combination thereof; and/or
(iii) configured to be detachable from the first cellular component-binding reagent ; and/or
(g) the method comprises dissociating the cellular component-binding reagent specific oligonucleotide from the first cellular component-binding reagent, optionally wherein:
(i) dissociating the cellular component-binding reagent specific oligonucleotide comprises detaching the cellular component-binding reagent specific oligonucleotide from the first cellular component-binding reagent by UV photocleaving, chemical treatment, heating, enzyme treatment, or any combination thereof; and/or
(ii) the dissociating occurs after barcoding the cellular component-binding reagent specific oligonucleotide and/or wherein the dissociating occurs before barcoding the cellular component-binding reagent specific oligonucleotide; and/or
(h) the cellular component-binding reagent specific oligonucleotide is configured to be non-detachable from the first cellular component-binding reagent; and/or
(i) the method comprises after contacting the plurality of first cellular component-binding reagents with the first and/or second plurality of cells, removing one or more first cellular component-binding reagents of the plurality of first cellular component-binding reagents that are not contacted with the first and/or second plurality of cells, optionally removing the one or more first cellular component-binding reagents not contacted with the first and/or second plurality of cells comprises removing the one or more first cellular component-binding reagents not contacted with the respective at least one of the plurality of cellular component targets.

12. The method of any one of claims 1-11, wherein:
(a) the first and/or second plurality of cells comprises T cells, B cells, tumor cells, myeloid cells, blood cells, normal cells, fetal cells, maternal cells, or a mixture thereof; and/or
(b) one or more single cells of the first plurality of cells and/or second plurality of cells comprises copies of a nucleic acid target, further comprising:
contacting a plurality of oligonucleotide barcodes with the copies of the nucleic acid target for hybridization, wherein each oligonucleotide barcode of the plurality of oligonucleotide barcodes comprises a first universal sequence, a target-binding region capable of hybridizing to the copies of the nucleic acid target, and a first molecular label;
extending the plurality of oligonucleotide barcodes hybridized to the copies of a nucleic acid target to generate a plurality of barcoded nucleic acid molecules each comprising a sequence complementary to at least a portion of the nucleic acid target; and
obtaining sequence information of the plurality of barcoded nucleic acid molecules, or products thereof, to determine the copy number of the nucleic acid target in each of the one or more single cells.

13. The method of any one of claims 1-12, wherein:
(a) determining the copy number of the nucleic acid target in each of the one or more single cells comprises determining the copy number of the nucleic acid target in each of the one or more single cells based on the number of first molecular labels with distinct sequences, complements thereof, or a combination thereof, associated with the plurality of barcoded nucleic acid molecules, or products thereof;
(b) obtaining sequencing data comprises attaching sequencing adaptors to the plurality of barcoded nucleic acid molecules, or products thereof;
(c) the plurality of barcoded nucleic acid molecules comprise barcoded deoxyribonucleic acid (DNA) molecules and/or barcoded ribonucleic acid (RNA) molecules; and/or
(d) the nucleic acid target comprises a nucleic acid molecule, optionally ribonucleic acid (RNA), messenger RNA (mRNA), microRNA, small interfering RNA (siRNA), RNA degradation product, RNA comprising a poly(A) tail, or any combination thereof, and further optionally the mRNA encodes an immune receptor.

14. The method of any one of claims 1-13, wherein:
(a) at least 10 of the plurality of oligonucleotide barcodes comprise different first molecular label sequences;
(b) each first molecular label of the plurality of oligonucleotide barcodes comprises at least 6 nucleotides;
(c) the plurality of oligonucleotide barcodes are associated with a solid support, and optionally the plurality of oligonucleotide barcodes associated with the same solid support each comprise an identical sample label, and further optionally each sample label of the plurality of oligonucleotide barcodes comprises at least 6 nucleotides; and/or
(d) the plurality of oligonucleotide barcodes each comprise a cell label, and optionally each cell label of the plurality of oligonucleotide barcodes comprises at least 6 nucleotides, optionally wherein oligonucleotide barcodes associated with the same solid support comprise:
(i) the same cell label; or
(ii) different cell labels; and/or
(e) the first plurality of cells and second plurality of cells are derived from the same sample.

15. The method of any one of claims 1-14, the method comprising associating a synthetic particle comprising the plurality of the oligonucleotide barcodes with a single cell in the first and/or second plurality of single cells, optionally wherein:
(a) the method comprises lysing the single cell after associating the synthetic particle with the single cell, and optionally lysing the single cell comprises heating the sample, contacting the sample with a detergent, changing the pH of the sample, or any combination thereof;
(b) the synthetic particle and the single cell are in the same well;
(c) the synthetic particle and the single cell are in the same droplet;
(d) at least one of the plurality of oligonucleotide barcodes is immobilized or partially immobilized on the synthetic particle, or the at least one of the plurality of oligonucleotide barcodes is enclosed or partially enclosed in the synthetic particle;
(e) the synthetic particle is disruptable; and/or
(f) the synthetic particle comprises a bead, and optionally the bead comprises:
(i) a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, an anti-biotin microbead, an anti-fluorochrome microbead, or any combination thereof;
(ii) a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, and any combination thereof; or
(iii) a disruptable hydrogel particle; and/or
(g) each of the plurality of oligonucleotide barcodes comprises a linker functional group, wherein the synthetic particle comprises a third solid support functional group, and wherein the support functional group and the linker functional group are associated with each other, and optionally the linker functional group and the support functional group are individually selected from the group consisting of C6, biotin, streptavidin, primary amine(s), aldehyde(s), ketone(s), and any combination thereof.

## Patentansprüche

1. Verfahren zum Messen der Expression von Zellkomponentenzielen in Zellen, umfassend:
In-Kontakt-Bringen einer Vielzahl von ersten zellkomponentenbindenden Reagentien mit einer ersten Vielzahl von Zellen, die eine Vielzahl von Zellkomponentenzielen umfasst, wobei jedes aus der Vielzahl von ersten zellkomponentenbindenden Reagentien ein zellkomponentenbindendes-Reagens-spezifisches Oligonukleotid, umfassend eine einmalige Identifikatorsequenz für das erste zellkomponentenbindende Reagens, umfasst, und wobei das erste zellkomponentenbindende Reagens zum spezifischen Binden an wenigstens eines aus der Vielzahl von Zellkomponentenzielen in der Lage ist;
Erzeugen von Amplikons der einmaligen Identifikatorsequenz von einem oder mehreren der ersten zellkomponentenbindenden Reagentien;
Bestimmen der Menge von einem oder mehreren Amplikons als Anzeige der Menge des einen oder der mehreren der ersten zellkomponentenbindenden Reagentien; und
wenn die Menge von einem oder mehreren Amplikons eine Häufigkeit eines ersten zellkomponentenbindenden Reagens über oder unter einem vorbestimmten Häufigkeitsbereich anzeigt, Wiederholen des In-Kontakt-Bringen-Schrittes mit einer zweiten Vielzahl von Zellen auf eine Weise, konfiguriert zum Erzielen einer Häufigkeit des ersten zellkomponentenbindenden Reagens innerhalb des vorbestimmten Häufigkeitsbereichs.

2. Verfahren nach Anspruch 1, wobei:
(a) der vorbestimmte Häufigkeitsbereich den dynamischen Bereich umfasst, für den akzeptable Linearität und Effizienz der Detektion des ersten zellkomponentenbindenden Reagens beobachtet werden;
(b) das Verfahren das In-Kontakt-Bringen der ersten und/oder zweiten Vielzahl von Zellen mit einer Vielzahl von dritten zellkomponentenbindenden Reagentien umfasst, wobei ein drittes zellkomponentenbindendes Reagens in der Lage ist, an wenigstens eines aus der Vielzahl von Zellkomponentenzielen spezifisch zu binden, wobei die dritten zellkomponentenbindenden Reagentien kein zellkomponentenbindendes-Reagens-spezifisches Oligonukleotid umfassen, wobei ein oder mehrere von den ersten zellkomponentenbindenden Reagentien und ein oder mehrere von den dritten zellkomponentenbindenden Reagentien zum Binden desselben Zellkomponentenziels in der Lage sind, wobei gegebenenfalls erste zellkomponentenbindende Reagentien und dritte zellkomponentenbindende Reagentien, die zum Binden desselben Zellkomponentenziels in der Lage sind, den gleichen Klonotyp aufweisen; und/oder
(c) das Wiederholen des In-Kontakt-Bringen-Schrittes mit einer zweiten Vielzahl von Zellen auf eine Weise, konfiguriert zum Erzielen einer Häufigkeit des ersten zellkomponentenbindenden Reagens innerhalb des vorbestimmten Häufigkeitsbereichs, das In-Kontakt-Bringen der zweiten Vielzahl von Zellen mit einem Gemisch aus dem ersten zellkomponentenbindenden Reagens und einem dritten zellkomponentenbindenden Reagens, das zum Binden desselben Zellkomponentenziels wie das erste zellkomponentenbindende Reagens in der Lage ist, bei einem Titrationsverhältnis, konfiguriert zum Erzielen einer Häufigkeit des ersten zellkomponentenbindenden Reagens innerhalb des vorbestimmten Häufigkeitsbereichs, umfasst, wobei gegebenenfalls das Titrationsverhältnis so konfiguriert wird, dass Sequenzierablesungen, umfassend die einmalige Identifikatorsequenz des ersten zellkomponentenbindenden Reagens, weniger als etwa 5 % der gesamten Sequenzierablesungen ausmachen.

3. Verfahren zum Messen der Expression von Zellkomponentenzielen in Zellen, umfassend:
In-Kontakt-Bringen einer Vielzahl von ersten zellkomponentenbindenden Reagentien mit der ersten Vielzahl von Zellen, die eine Vielzahl von Zellkomponentenzielen umfasst, wobei jedes der Vielzahl von ersten zellkomponentenbindenden Reagentien ein zellkomponentenbindendes-Reagens-spezifisches Oligonukleotid, umfassend eine einmalige Identifikatorsequenz für das erste zellkomponentenbindende Reagens, umfasst, und wobei das erste zellkomponentenbindende Reagens zum spezifischen Binden an wenigstens eines aus der Vielzahl von Zellkomponentenzielen in der Lage ist;
Isolieren eines oder mehrerer Zelltypen von Interesse aus der ersten Vielzahl von Zellen, wobei ein erster Zelltyp von Interesse **gekennzeichnet ist durch** die Häufigkeit von: (i) einem oder mehreren positiven Zellkomponentenzielen über einem positiven Häufigkeitsschwellenwert und/oder (ii) einem oder mehreren negativen Zellkomponentenzielen unter einem negativen Häufigkeitsschwellenwert;
Erzeugen von Amplikons der einmaligen Identifikatorsequenz von einem oder mehreren der ersten zellkomponentenbindenden Reagentien; und
Bestimmen der Menge von einem oder mehreren Amplikons als Anzeige der Menge der von dem einen oder den mehreren der ersten zellkomponentenbindenden Reagentien gebundenen Zellkomponentenziele,
wobei von einer oder mehreren Zellen, die vermutlich von einem ersten Zelltyp von Interesse ist/sind, festgestellt wird, nicht von einem ersten Zelltyp von Interesse zu sein, wenn die Menge von einem oder mehreren Amplikons eine Häufigkeit anzeigt von: (i) einem oder mehreren positiven Zellkomponentenzielen unter einem positiven Häufigkeitsschwellenwert und/oder (ii) einem oder mehreren negativen Zellkomponentenzielen über einem negativen Häufigkeitsschwellenwert.

4. Verfahren nach Anspruch 3, umfassend, wenn von einer oder mehreren Zellen, die vermutlich von einem ersten Zelltyp von Interesse ist/sind, festgestellt wird, nicht von einem ersten Zelltyp von Interesse zu sein, das Wiederholen des In-Kontakt-Bringen-Schrittes und/oder Isolierungsschrittes mit einer zweiten Vielzahl von Zellen auf eine Weise, konfiguriert zum Erzielen der Isolierung des einen oder der mehreren Zelltypen von Interesse.

5. Verfahren nach einem der Ansprüche 1-4, umfassend:
In-Kontakt-Bringen einer Vielzahl von Oligonukleotid-Barcodes mit den zellkomponentenbindendes-Reagens-spezifischen Oligonukleotiden zur Hybridisierung, wobei die Oligonukleotid-Barcodes jeweils eine erste Molekülmarkierung und eine erste universelle Sequenz umfassen; und
Verlängern der Vielzahl von Oligonukleotid-Barcodes, hybridisiert an die zellkomponentenbindendes-Reagens-spezifischen Oligonukleotide, zum Erzeugen einer Vielzahl von barcodierten zellkomponentenbindendes-Reagens-spezifischen Oligonukleotiden, jeweils umfassend eine Sequenz, komplementär zu wenigstens einem Teil der einmaligen Identifikatorsequenz und der ersten Molekülmarkierung, gegebenenfalls umfassend das Gewinnen von Sequenzinformationen der Vielzahl von barcodierten zellkomponentenbindendes-Reagens-spezifischen Oligonukleotiden, oder Produkten davon, zum Bestimmen der Kopienzahl von wenigstens einem Zellkomponentenziel aus der Vielzahl von Zellkomponentenzielen in einer oder mehreren der ersten Vielzahl von Zellen und/oder zweiten Vielzahl von Zellen, gegebenenfalls wobei das Gewinnen der Sequenzinformationen das Anbringen von Sequenzieradaptoren an die Vielzahl von barcodierten zellkomponentenbindendes-Reagens-spezifischen Oligonukleotiden, oder Produkten davon, umfasst.

6. Verfahren nach einem der Ansprüche 1-5, wobei:
(a) das zellkomponentenbindendes-Reagens-spezifische Oligonukleotid eine Sequenz, komplementär zu einer Einfangsequenz eines Oligonukleotid-Barcodes, konfiguriert zum Einfangen der Sequenz des zellkomponentenbindendes-Reagens-spezifischen Oligonukleotids, umfasst, wobei gegebenenfalls die Sequenz des zellkomponentenbindendes-Reagens-spezifischen Oligonukleotids, komplementär zu der Einfangsequenz, eine Poly(dA)-Region umfasst; und/oder
(b) das zellkomponentenbindendes-Reagens-spezifische Oligonukleotid eine zweite universelle Sequenz umfasst.

7. Verfahren nach einem der Ansprüche 1-6, wobei das Erzeugen von Amplikons umfasst:
(a) Amplifizieren der Vielzahl von zellkomponentenbindendes-Reagens-spezifischen Oligonukleotiden unter Verwendung eines Primers, fähig zum Hybridisieren an die zweite universelle Sequenz, oder ein Komplement davon, und eines Primers, fähig zum Hybridisieren an die Einfangsequenz, oder ein Komplement davon, wodurch Amplikons der einmaligen Identifikatorsequenz von einem oder mehreren der ersten zellkomponentenbindenden Reagentien erzeugt werden;
(b) Amplifizieren der Vielzahl von zellkomponentenbindendes-Reagens-spezifischen Oligonukleotiden unter Verwendung eines Primers, fähig zum Hybridisieren an die zweite universelle Sequenz, oder ein Komplement davon, und eines Primers, fähig zum Hybridisieren an die Einfangsequenz, oder ein Komplement davon, wodurch PCR1-Produkte erzeugt werden; und Amplifizieren der PCR1-Produkte unter Verwendung eines Primers, fähig zum Hybridisieren an die zweite universelle Sequenz, oder ein Komplement davon, und eines Primers, fähig zum Hybridisieren an die Einfangsequenz, oder ein Komplement davon, wodurch Amplikons der einmaligen Identifikatorsequenz von einem oder mehreren der ersten zellkomponentenbindenden Reagentien erzeugt werden;
(c) Amplifizieren der Vielzahl von barcodierten zellkomponentenbindendes-Reagens-spezifischen Oligonukleotiden unter Verwendung eines Primers, fähig zum Hybridisieren an die zweite universelle Sequenz, oder ein Komplement davon, und eines Primers, fähig zum Hybridisieren an die Einfangsequenz, oder ein Komplement davon, wodurch Amplikons der einmaligen Identifikatorsequenz von einem oder mehreren der ersten zellkomponentenbindenden Reagentien erzeugt werden;
(d) Amplifizieren der Vielzahl von barcodierten zellkomponentenbindendes-Reagens-spezifischen Oligonukleotiden unter Verwendung eines Primers, fähig zum Hybridisieren an die zweite universelle Sequenz, oder ein Komplement davon, und eines Primers, fähig zum Hybridisieren an die Einfangsequenz, oder ein Komplement davon, wodurch PCR1-Produkte erzeugt werden; und Amplifizieren der PCR1-Produkte unter Verwendung eines Primers, fähig zum Hybridisieren an die zweite universelle Sequenz, oder ein Komplement davon, und eines Primers, fähig zum Hybridisieren an die Einfangsequenz, oder ein Komplement davon, wodurch Amplikons der einmaligen Identifikatorsequenz von einem oder mehreren der ersten zellkomponentenbindenden Reagentien erzeugt werden;
(e) Amplifizieren der Vielzahl von barcodierten zellkomponentenbindendes-Reagens-spezifischen Oligonukleotiden unter Verwendung eines Primers, fähig zum Hybridisieren an die erste universelle Sequenz, oder ein Komplement davon, und eines Primers, fähig zum Hybridisieren an die zweite universelle Sequenz, oder ein Komplement davon, wodurch Amplikons der einmaligen Identifikatorsequenz von einem oder mehreren der ersten zellkomponentenbindenden Reagentien erzeugt werden; und/oder
(f) Amplifizieren der Vielzahl von barcodierten zellkomponentenbindendes-Reagens-spezifischen Oligonukleotiden unter Verwendung eines Primers, fähig zum Hybridisieren an die erste universelle Sequenz, oder ein Komplement davon, und eines Primers, fähig zum Hybridisieren an die zweite universelle Sequenz, oder ein Komplement davon, wodurch PCR1-Produkte erzeugt werden; und
Amplifizieren der PCR1-Produkte unter Verwendung eines Primers, fähig zum Hybridisieren an die zweite universelle Sequenz, oder ein Komplement davon, und eines Primers, fähig zum Hybridisieren an die erste universelle Sequenz, oder ein Komplement davon, wodurch Amplikons der einmaligen Identifikatorsequenz von einem oder mehreren der ersten zellkomponentenbindenden Reagentien erzeugt werden.

8. Verfahren nach einem der Ansprüche 1-7, wobei die Amplifikation durchgeführt wird unter Verwendung eines Verfahrens, ausgewählt aus der Gruppe bestehend aus Polymerasekettenreaktion (PCR), Ligasekettenreaktion (LCR), Loop-vermittelter isothermaler Amplifikation (LAMP), Strangverdrängungs-Amplifikation (SDA), Replikase-vermittelter Amplifikation, Immun-Amplifikation, Nukleinsäuresequenz-basierter Amplifikation (NASBA), Self-Sustained-Sequence-Replikation (3SR), Rolling-Circle-Amplifikation und Transkriptions-vermittelter Amplifikation (TMA), gegebenenfalls wobei die PCR eine:
(a) Echtzeit-PCR;
(b) quantitative Echtzeit-PCR (QRT-PCR); oder
(c) digitale PCR (dPCR), gegebenenfalls digitale Tröpfchen-PCR (ddPCR), ist.

9. Verfahren nach einem der Ansprüche 1-8, wobei:
(a) das Bestimmen des Vorhandenseins oder der Menge von einem oder mehreren Amplikons das In-Kontakt-Bringen der Amplikons mit einer Vielzahl von Oligonukleotidsonden umfasst, wobei jede aus der Vielzahl von Oligonukleotidsonden eine Sequenz, konfiguriert zum Binden einer einmaligen Identifikatorsequenz, oder eines Komplements davon, umfasst, gegebenenfalls wobei jede Sonde von komplementären Sequenzen am 5'-Ende und 3'-Ende flankiert ist, gegebenenfalls wobei eine der komplementären Sequenzen eine Fluoreszenzemitter-Einheit umfasst und die andere komplementäre Sequenz eine Fluoreszenzquencher-Einheit umfasst;
(b) wenigstens eine aus der Vielzahl von Oligonukleotidsonden eine Fluoreszenzemitter-Einheit und eine Fluoreszenzquencher-Einheit umfasst;
(c) die Oligonukleotidsonde ein TaqMan-Nachweissonden-Oligonukleotid, ein Molecular-Beacon-Nachweissonden-Oligonukleotid oder ein Molecular-Torch-Nachweissonden-Oligonukleotid umfasst;
(d) der Schritt zum Erzeugen von Amplikons und der Bestimmungsschritt gemultiplext werden, wobei gegebenenfalls wenigstens etwa 4 einmalige Identifikatorsequenzen gleichzeitig analysiert werden;
(e) der Schritt zum Erzeugen von Amplikons und der Bestimmungsschritt an Einzelzellen oder mehreren Zellen durchgeführt werden; und/oder
(f) der Isolierungsschritt Einzelzell-Sortierung oder Bulk-Sortierung umfasst.

10. Verfahren nach einem der Ansprüche 1-9, wobei:
(a) der eine oder die mehreren Zelltypen von Interesse hämogene Endothelzellen, hämatopoetische Stamm- und Progenitorzellen (HSC), hämatopoetische multipotente Progenitorzellen (MPP), prä-T-Zell-Progenitorzellen, prä-K-Zell-Progenitorzellen, T-Zell-Progenitorzellen, NK-Zell-Progenitorzellen, T-Zellen, NK-Zellen, NKT-Zellen, B-Zellen, Makrophagen, Neutrophile, CD4-Zellen, CD8-Zellen, naive T-Zellen, T-Gedächtnisstammzellen, zentrale T-Gedächtniszellen, doppelt-negative T-Zellen, Effektor-Gedächtnis-T-Zellen, Effektor-T-Zellen, ThO-Zellen, TcO-Zellen, Th1-Zellen, Te1-Zellen, Th2-Zellen, Tc2-Zellen, Th17-Zellen, Th22-Zellen, Gamma/Delta-T-Zellen, natürliche Killerzellen (NK-Zellen), natürliche Killer-T-Zellen (NKT-Zellen), hämatopoetische Stammzellen, pluripotente Stammzellen oder irgendeine Kombination davon umfassen;
(b) der eine oder die mehreren Zelltypen von Interesse umfassen eine oder mehrere Immunzelltypen, gegebenenfalls naive CD4-T-Zellen, Effektor-Gedächtnis-CD4-T-Zellen, naive CD8-T-Zellen, Effektor-Gedächtnis-CD8-T-Zellen, naive CD4-Treg-Zellen, Effektor-Gedächtnis-CD4-Treg-Zellen, naive B-Zellen, B-Gedächtniszellen, CD16-DC, plasmazytoide DC oder irgendeine Kombination davon;
(c) das Verfahren das In-Kontakt-Bringen einer Vielzahl von zweiten zellkomponentenbindenden Reagentien mit der ersten und/oder zweiten Vielzahl von Zellen umfasst, wobei jede aus der Vielzahl von zweiten zellkomponentenbindenden Reagentien eine nachweisbare Einheit, oder einen Vorläufer davon, umfasst, wobei ein zweites zellkomponentenbindenden Reagens in der Lage ist, an wenigstens eines der Vielzahl von Zellkomponentenzielen spezifisch zu binden, wobei zweite zellkomponentenbindende Reagentien, die zum Binden desselben Zellkomponentenziels in der Lage sind, die gleiche nachweisbare Einheit, oder einen Vorläufer davon, umfassen, und wobei zweite zellkomponentenbindende Reagentien, die zum Binden unterschiedlicher Zellkomponentenziele in der Lage sind, unterschiedliche nachweisbare Einheiten, oder Vorläufer davon, umfassen;
(d) die erste Vielzahl von Zellen und/oder zweite Vielzahl von Zellen gleichzeitig mit den ersten zellkomponentenbindenden Reagentien und zweiten zellkomponentenbindenden Reagentien in Kontakt gebracht werden;
(e) ein oder mehrere von den ersten zellkomponentenbindenden Reagentien und ein oder mehrere von den zweiten zellkomponentenbindenden Reagentien zum Binden an dasselbe Zellkomponentenziel in der Lage sind, wobei gegebenenfalls erste zellkomponentenbindende Reagentien und zweite zellkomponentenbindende Reagentien, die zum Binden an dasselbe Zellkomponentenziel in der Lage sind, den gleichen Klonotyp aufweisen; und/oder
(f) der Isolierungsschritt Folgendes umfasst:
(i) Fluoreszenz-aktivierte Zellsortierung, gegebenenfalls Fluoreszenz-aktivierte Zellsortierung unter Nutzung der zweiten zellkomponentenbindenden Reagentien;
(ii) Abscheiden der einen oder mehreren Zellen von Interesse der ersten und/oder zweiten Vielzahl von Zellen in eine oder mehrere Mikrotiterplatten; und/oder
(iii) Nachweisen von Emissionen der nachweisbaren Einheit von zweiten zellkomponentenbindenden Reagentien mithilfe eines Durchflusszytometers, gegebenenfalls wobei das Durchflusszytometer ein herkömmliches Durchflusszytometer, ein Spektral-Durchflusszytometer, ein Hyperspektral-Durchflusszytometer, ein Bildgebungs-Durchflusszytometer, oder irgendeine Kombination davon umfasst; und/oder
(g) die nachweisbare Einheit eine optische Einheit, eine lumineszente Einheit, eine elektrochemisch aktive Einheit, ein Nanopartikel oder eine Kombination davon umfasst, wobei das Nanopartikel gegebenenfalls einen Quantenpunkt umfasst;
(h) die lumineszente Einheit eine chemolumineszente Einheit, eine elektrolumineszente Einheit, eine photolumineszente Einheit oder eine Kombination davon umfasst;
(i) die photolumineszente Einheit eine fluoreszierende Einheit, eine phosphoreszierende Einheit oder eine Kombination davon umfasst, gegebenenfalls wobei die fluoreszierende Einheit einen Fluoreszenzfarbstoff umfasst;
(j) das Verfahren das Durchführen einer Reaktion zum Umwandeln des Vorläufers der nachweisbaren Einheit in die nachweisbare Einheit umfasst;
(k) das eine oder die mehreren positiven Zellkomponentenziele und/oder das eine oder die mehreren negativen Zellkomponentenziele CD4, CD8, CCR7, CD45RO, CD25, HLA-DR, CD45RA, CD19, CD3, CD27, IgD, CD11c, CD16 oder irgendeine Kombination davon umfassen; und/oder
(l) die Anzahl einmaliger erster Molekülmarkierungssequenzen, die mit der einmaligen Identifikatorsequenz für das erste zellkomponentenbindende Reagens, das in der Lage ist, an das wenigstens eine Zellkomponentenziel spezifisch zu binden, assoziiert sind, in den Sequenzierdaten die Kopienzahl des wenigstens einen Zellkomponentenziels in der einen oder den mehreren aus der ersten und/oder zweiten Vielzahl von Zellen anzeigt.

11. Verfahren nach einem der Ansprüche 1-10, wobei:
(a) das zellkomponentenbindendes-Reagens-spezifische Oligonukleotid eines oder mehrere der Folgenden umfasst:
(i) eine zweite Molekülmarkierungssequenz, gegebenenfalls wobei:
(A) die zweite Molekülmarkierungssequenz 2-20 Nukleotide lang ist;
(B) die zweiten Molekülmarkierungssequenzen von wenigstens zwei zellkomponentenbindendes-Reagens-spezifischen Oligonukleotiden unterschiedlich sind, und wobei die einmaligen Identifikatorsequenzen von den wenigstens zwei zellkomponentenbindendes-Reagens-spezifischen Oligonukleotiden identisch sind; und/oder
(C) die zweiten Molekülmarkierungssequenzen von wenigstens zwei zellkomponentenbindendes-Reagens-spezifischen Oligonukleotiden unterschiedlich sind, und wobei die einmaligen Identifikatorsequenzen von den wenigstens zwei zellkomponentenbindendes-Reagens-spezifischen Oligonukleotiden unterschiedlich sind; und/oder
(ii) eine Alignmentsequenz benachbart zu einer Poly(dA)-Region, wobei gegebenenfalls die Alignmentsequenz ein oder mehr Nukleotide oder zwei oder mehr Nukleotide lang ist; und
(iii) einen Linker, wobei das zellkomponentenbindendes-Reagens-spezifische Oligonukleotid über den Linker mit dem ersten zellkomponentenbindenden Reagens assoziiert ist; und/oder
(b) die Anzahl einmaliger zweiter Molekülmarkierungssequenzen, die mit der einmaligen Identifikatorsequenz für das erste zellkomponentenbindende Reagens, das in der Lage ist, an das wenigstens eine Zellkomponentenziel spezifisch zu binden, assoziiert sind, in den Sequenzierdaten die Kopienzahl des wenigstens einen Zellkomponentenziels in der einen oder den mehreren aus der ersten und/oder zweiten Vielzahl von Zellen anzeigt; und/oder
(c) die Alignmentsequenz:
(i) ein Guanin, ein Cytosin, ein Thymin, ein Uracil oder eine Kombination davon umfasst;
(ii) eine Poly(dT)-Sequenz, eine Poly(dG)-Sequenz, eine Poly(dC)-Sequenz, eine Poly(dU)-Sequenz oder eine Kombination davon umfasst; und/oder
(iii) sich 5' zur Poly(dA)-Region befindet; und/oder
(d) der Linker Folgendes umfasst:
(i) eine Kohlenstoffkette, wobei die Kohlenstoffkette gegebenenfalls 2-30 Kohlenstoffatome umfasst, und ferner die Kohlenstoffkette gegebenenfalls 12 Kohlenstoffatome umfasst; und/oder
(ii) 5'-Aminomodifikator C12 (5AmMC12) oder ein Derivat davon; und/oder
(e) die Vielzahl von Zellkomponentenzielen eine Vielzahl von Proteinzielen umfasst, und wobei das erste zellkomponentenbindende Reagens in der Lage ist, an wenigstens eines der Vielzahl von Proteinzielen spezifisch zu binden, gegebenenfalls wobei die Vielzahl von Zellkomponentenzielen ein Zelloberflächenprotein, ein intrazelluläres Protein, einen Zellmarker, einen B-Zell-Rezeptor, einen T-Zell-Rezeptor, einen Antikörper, einen Haupthistokompatibilitätskomplex, ein Tumorantigen, einen Rezeptor oder eine Kombination davon umfasst; und/oder
(f) das zellkomponentenbindendes-Reagens-spezifische Oligonukleotid:
(i) mit dem ersten zellkomponentenbindenden Reagens assoziiert ist, gegebenenfalls wobei das zellkomponentenbindendes-Reagens-spezifische Oligonukleotid kovalent an das erste zellkomponentenbindende Reagens gebunden ist und/oder nichtkovalent an das erste zellkomponentenbindende Reagens gebunden ist;
(ii) an das erste zellkomponentenbindende Reagens konjugiert ist, und gegebenenfalls das zellkomponentenbindendes-Reagens-spezifische Oligonukleotid über eine chemische Gruppe, ausgewählt aus der Gruppe bestehend aus einer UV-photospaltbaren Gruppe, einem Streptavidin, einem Biotin, einem Amin und einer Kombination davon, an das erste zellkomponentenbindende Reagens konjugiert ist; und/oder
(iii) so konfiguriert ist, dass es von dem ersten zellkomponentenbindenden Reagens abtrennbar ist; und/oder
(g) das Verfahren das Dissoziieren des zellkomponentenbindendes-Reagens-spezifischen Oligonukleotids von dem ersten zellkomponentenbindenden Reagens umfasst, gegebenenfalls wobei:
(i) das Dissoziieren des zellkomponentenbindendes-Reagens-spezifischen Oligonukleotids das Abtrennen des zellkomponentenbindendes-Reagens-spezifischen Oligonukleotids von dem ersten zellkomponentenbindenden Reagens mittels UV-Photospaltung, Chemikalienbehandlung, Erhitzen, Enzymbehandlung oder irgendeiner Kombination davon umfasst; und/oder
(ii) das Dissoziieren nach dem Barcodieren des zellkomponentenbindendes-Reagens-spezifischen Oligonukleotids erfolgt und/oder wobei das Dissoziieren vor dem Barcodieren des zellkomponentenbindendes-Reagens-spezifischen Oligonukleotids erfolgt; und/oder
(h) das zellkomponentenbindendes-Reagens-spezifische Oligonukleotid so konfiguriert ist, dass es von dem ersten zellkomponentenbindenden Reagens nicht abtrennbar ist; und/oder
(i) das Verfahren nach In-Kontakt-Bringen der Vielzahl von ersten zellkomponentenbindenden Reagentien mit der ersten und/oder zweiten Vielzahl von Zellen das Entfernen eines oder mehrerer erster zellkomponentenbindender Reagentien der Vielzahl von ersten zellkomponentenbindenden Reagentien, die nicht mit der ersten und/oder zweiten Vielzahl von Zellen in Kontakt gebracht werden, umfasst, gegebenenfalls wobei das Entfernen des einen bzw. der mehreren nicht mit der ersten und/oder zweiten Vielzahl von Zellen in Kontakt gebrachten ersten zellkomponentenbindenden Reagentien das Entfernen des einen bzw. der mehreren nicht mit dem entsprechenden wenigstens einen aus der Vielzahl von Zellkomponentenzielen in Kontakt gebrachten ersten zellkomponentenbindenden Reagentien umfasst.

12. Verfahren nach einem der Ansprüche 1-11, wobei:
(a) die erste und/oder zweite Vielzahl von Zellen T-Zellen, B-Zellen, Tumorzellen, myeloische Zellen, Blutkörperchen, normale Zellen, fötale Zellen, maternale Zellen oder eine Mischung davon umfasst; und/oder
(b) eine oder mehrere Einzelzellen der ersten Vielzahl von Zellen und/oder zweiten Vielzahl von Zellen Kopien eines Nukleinsäureziels umfasst, ferner umfassend:
In-Kontakt-Bringen einer Vielzahl von Oligonukleotid-Barcodes mit den Kopien des Nukleinsäureziels zur Hybridisierung, wobei jeder Oligonukleotid-Barcode der Vielzahl von Oligonukleotid-Barcodes eine erste universelle Sequenz, eine Zielbindungsregion, fähig zum Hybridisieren an die Kopien des Nukleinsäureziels, und eine erste Molekülmarkierung umfasst;
Verlängern der Vielzahl von an die Kopien eines Nukleinsäureziels hybridisierten Oligonukleotid-Barcodes, so dass eine Vielzahl von barcodierten Nukleinsäuremolekülen erzeugt wird, die jeweils eine Sequenz umfassen, die zu wenigstens einem Teil des Nukleinsäureziels komplementär ist; und
Gewinnen von Sequenzinformationen der Vielzahl von barcodierten Nukleinsäuremolekülen, oder Produkten davon, um die Kopienzahl des Nukleinsäureziels in jeder von den ein oder mehreren Einzelzellen zu bestimmen.

13. Verfahren nach einem der Ansprüche 1-12, wobei:
(a) das Bestimmen der Kopienzahl des Nukleinsäureziels in jeder von den ein oder mehreren Einzelzellen das Bestimmen der Kopienzahl des Nukleinsäureziels in jeder von den ein oder mehreren Einzelzellen anhand der Anzahl erster Molekülmarkierungen mit unterschiedlichen Sequenzen, Komplementen davon, oder einer Kombination davon, die mit der Vielzahl von barcodierten Nukleinsäuremolekülen oder Produkten davon assoziiert sind, umfasst;
(b) das Gewinnen von Sequenzierdaten das Anbringen von Sequenzieradaptoren an die Vielzahl von barcodierten Nukleinsäuremolekülen oder Produkte davon umfasst;
(c) die Vielzahl von barcodierten Nukleinsäuremolekülen barcodierte Desoxyribonukleinsäure(DNA)-Moleküle und/oder barcodierte Ribonukleinsäure(RNA)-Moleküle umfasst; und/oder
(d) das Nukleinsäureziel ein Nukleinsäuremolekül, gegebenenfalls Ribonukleinsäure (RNA), mRNA (messenger RNA), microRNA, siRNA (small interfering RNA), RNA-Abbauprodukt, RNA, die ein Poly(A)-Ende umfasst, oder irgendeine Kombination davon umfasst, und ferner gegebenenfalls die mRNA einen Immunrezeptor codiert.

14. Verfahren nach einem der Ansprüche 1-13, wobei:
(a) wenigstens 10 aus der Vielzahl von Oligonukleotid-Barcodes unterschiedliche erste Molekülmarkierungssequenzen umfassen;
(b) jede erste Molekülmarkierung der Vielzahl von Oligonukleotid-Barcodes wenigstens 6 Nukleotide umfasst;
(c) die Vielzahl von Oligonukleotid-Barcodes mit einem festen Träger assoziiert ist, und gegebenenfalls die Vielzahl von Oligonukleotid-Barcodes, die mit demselben festen Träger assoziiert sind, jeweils eine identische Probenmarkierung umfassen, und ferner gegebenenfalls jede Probenmarkierung der Vielzahl von Oligonukleotid-Barcodes wenigstens 6 Nukleotide umfasst; und/oder
(d) die Vielzahl von Oligonukleotid-Barcodes jeweils eine Zellmarkierung umfasst, und gegebenenfalls jede Zellmarkierung der Vielzahl von Oligonukleotid-Barcodes wenigstens 6 Nukleotide umfasst, gegebenenfalls wobei Oligonukleotid-Barcodes, die mit demselben festen Träger assoziiert sind, umfassen:
(i) die gleiche Zellmarkierung; oder
(ii) unterschiedliche Zellmarkierungen; und/oder (e) die erste Vielzahl von Zellen und die zweite Vielzahl von Zellen aus derselben Probe abgeleitet sind.

15. Verfahren nach einem der Ansprüche 1-14, wobei das Verfahren das Assoziieren eines synthetischen Partikels, umfassend die Vielzahl der Oligonukleotid-Barcodes, mit einer Einzelzelle in der ersten und/oder zweiten Vielzahl von Einzelzellen umfasst, gegebenenfalls wobei:
(a) das Verfahren das Lysieren der Einzelzelle nach dem Assoziieren des synthetischen Partikels mit der Einzelzelle umfasst, und gegebenenfalls das Lysieren der Einzelzelle Erhitzen der Probe, in-Kontakt-Bringen der Probe mit einem Detergens, Ändern des pH der Probe oder irgendeine Kombination davon umfasst;
(b) sich das synthetische Partikel und die Einzelzelle in derselben Mulde befinden;
(c) sich das synthetische Partikel und die Einzelzelle in demselben Tröpfchen befinden;
(d) wenigstens einer der Vielzahl von Oligonukleotid-Barcodes auf dem synthetischen Partikel immobilisiert oder teilimmobilisiert ist, oder der wenigstens eine der Vielzahl von Oligonukleotid-Barcodes in dem synthetischen Partikel eingeschlossen oder teilweise eingeschlossen ist;
(e) das synthetische Partikel aufschließbar ist; und/oder
(f) das synthetische Partikel ein Kügelchen umfasst, und gegebenenfalls das Kügelchen:
(i) ein Sepharose-Kügelchen, ein Streptavidin-Kügelchen, ein Agarose-Kügelchen, ein Magnetkügelchen, ein konjugiertes Kügelchen, ein mit Protein A konjugiertes Kügelchen, ein mit Protein G konjugiertes Kügelchen, ein mit Protein A/G konjugiertes Kügelchen, ein mit Protein L konjugiertes Kügelchen, ein mit Oligo(dT) konjugiertes Kügelchen, ein Siliciumdioxid-Kügelchen, ein siliciumdioxidähnliches Kügelchen, ein Anti-Biotin-Mikrokügelchen, ein Anti-Fluorochrom-Mikrokügelchen oder irgendeine Kombination davon umfasst;
(ii) ein Material umfasst, das aus der Gruppe bestehend aus Polydimethylsiloxan (PDMS), Polystyrol, Glas, Polypropylen, Agarose, Gelatine, Hydrogel, paramagnetischem Teilchen, Keramik, Kunststoff, Glas, Methylstyrol, Acrylpolymer, Titan, Latex, Sepharose, Cellulose, Nylon, Silicon und irgendeiner Kombination davon ausgewählt ist; oder
(iii) ein aufschließbares Hydrogelpartikel umfasst; und/oder
(g) jeder aus der Vielzahl von Oligonukleotid-Barcodes eine Linker-Funktionsgruppe umfasst, wobei das synthetische Partikel eine dritte Fester-Träger-Funktionsgruppe umfasst, und wobei die Träger-Funktionsgruppe und die Linker-Funktionsgruppe miteinander assoziiert sind, und gegebenenfalls wobei die Linker-Funktionsgruppe und die Träger-Funktionsgruppe individuell aus der Gruppe bestehend aus C6, Biotin, Streptavidin, primärem Amin (primären Aminen), Aldehyd(en), Keton(en) und irgendeiner Kombination davon ausgewählt sind.

## Revendications

1. Procédé de mesure de l'expression d'un composant cellulaire dans des cellules, comprenant :
la mise en contact d'une pluralité de premiers réactifs de liaison de composants cellulaires avec une première pluralité de cellules comprenant une pluralité de cibles de composants cellulaires, chacun de la pluralité de premiers réactifs de liaison de composants cellulaires comprenant un oligonucléotide spécifique à un réactif de liaison de composants cellulaires comprenant une séquence d'identifiant unique pour le premier réactif de liaison de composants cellulaires, et le premier réactif de liaison de composants cellulaires pouvant se lier spécifiquement à au moins l'une des cibles de composants cellulaires ;
la génération d'amplicons de la séquence d'identifiant unique d'un ou plusieurs premiers réactifs de liaison de composants cellulaires ;
la détermination de la quantité d'un ou plusieurs amplicons comme indication de la quantité du ou des premiers réactifs de liaison de composants cellulaires ; et
si la quantité d'un ou plusieurs amplicons indique une abondance d'un premier réactif de liaison de composants cellulaires supérieure ou inférieure à une plage d'abondance prédéterminée, la répétition de l'étape de mise en contact avec une deuxième pluralité de cellules d'une manière conçue pour obtenir une abondance dudit premier réactif de liaison de composants cellulaires dans ladite plage d'abondance prédéterminée.

2. Procédé selon la revendication 1, dans lequel :
(a) la plage d'abondance prédéterminée comprend la plage dynamique pour laquelle une linéarité et une efficacité de détection acceptables du premier réactif de liaison de composants cellulaires sont observées ;
(b) le procédé comprend la mise en contact de la première et/ou de la deuxième pluralité de cellules avec une pluralité de troisièmes réactifs de liaison de composants cellulaires, un troisième réactif de liaison de composants cellulaires pouvant se lier spécifiquement à au moins l'une des cibles de composants cellulaires, les troisièmes réactifs de liaison de composants cellulaires ne comprenant pas d'oligonucléotide spécifique à un réactif de liaison de composants cellulaires, un ou plusieurs premiers réactifs de liaison de composants cellulaires et un ou plusieurs troisièmes réactifs de liaison de composants cellulaires pouvant se lier à la même cible de composants cellulaires, facultativement, les premiers réactifs de liaison de composants cellulaires et les troisièmes réactifs de liaison de composants cellulaires pouvant se lier à la même cible de composants cellulaires présentant le même clonotype ; et/ou
(c) la répétition de l'étape de mise en contact avec une deuxième pluralité de cellules d'une manière conçue pour obtenir une abondance dudit premier réactif de liaison de composants cellulaires dans ladite plage d'abondance prédéterminée comprend la mise en contact de la deuxième pluralité de cellules avec un mélange dudit premier réactif de liaison de composants cellulaires et d'un troisième réactif de liaison de composants cellulaires pouvant se lier à la même cible de composants cellulaires que ledit premier réactif de liaison de composants cellulaires à un rapport de titrage conçu pour obtenir une abondance dudit premier réactif de liaison de composants cellulaires dans ladite plage d'abondance prédéterminée, facultativement le rapport de titrage étant conçu de telle sorte que les lectures de séquençage comprenant la séquence d'identifiant unique dudit premier réactif de liaison de composants cellulaires représentent moins d'environ 5 % de l'ensemble des lectures de séquençage.

3. Procédé de mesure de l'expression d'un composant cellulaire dans des cellules, comprenant :
la mise en contact d'une pluralité de premiers réactifs de liaison de composants cellulaires avec la première pluralité de cellules comprenant une pluralité de cibles de composants cellulaires, chacun des premiers réactifs de liaison de composants cellulaires comprenant un oligonucléotide spécifique à un réactif de liaison de composants cellulaires comprenant une séquence d'identifiant unique pour le premier réactif de liaison de composants cellulaires, et le premier réactif de liaison de composants cellulaires pouvant se lier spécifiquement à au moins l'une des cibles de composants cellulaires ;
l'isolement d'un ou plusieurs types de cellules d'intérêt à partir de la première pluralité de cellules, un premier type de cellule d'intérêt étant **caractérisé par** l'abondance de : (i) une ou plusieurs cibles de composants cellulaires positives au-dessus d'un seuil d'abondance positif et/ou (ii) une ou plusieurs cibles de composants cellulaires négatives au-dessous d'un seuil d'abondance négatif ;
la génération d'amplicons de la séquence d'identifiant unique d'un ou plusieurs premiers réactifs de liaison de composants cellulaires ; et
la détermination de la quantité d'un ou plusieurs amplicons comme indication de la quantité du ou des premiers réactifs de liaison de composants cellulaires,
dans lequel il est déterminé qu'une ou plusieurs cellules suspectées d'être d'un premier type de cellule d'intérêt ne sont pas d'un premier type de cellule d'intérêt si la quantité d'un ou plusieurs amplicons indique une abondance de : (i) une ou plusieurs cibles de composants cellulaires positives au-dessus d'un seuil d'abondance positif et/ou (ii) une ou plusieurs cibles de composants cellulaires négatives au-dessous d'un seuil d'abondance négatif.

4. Procédé selon la revendication 3, comprenant, si une ou plusieurs cellules suspectées d'être d'un premier type de cellule d'intérêt sont déterminées comme n'étant pas d'un premier type de cellule d'intérêt, la répétition de l'étape de mise en contact et/ou de l'étape d'isolement avec une deuxième pluralité de cellules d'une manière conçue pour réaliser l'isolement du ou des types de cellules d'intérêt.

5. Procédé selon l'une quelconque des revendications 1 à 4, comprenant :
la mise en contact d'une pluralité de codes-barres oligonucléotidiques avec les oligonucléotides spécifiques à des réactifs de liaison de composants cellulaires pour réaliser une hybridation, les codes-barres oligonucléotidiques comprenant chacun un premier marqueur moléculaire et une première séquence universelle ; et
l'allongement de la pluralité de codes-barres oligonucléotidiques hybridés aux oligonucléotides spécifiques à des réactifs de liaison de composants cellulaires pour générer une pluralité d'oligonucléotides spécifiques à des réactifs de liaison de composants cellulaires marqués par code-barres comprenant chacun une séquence complémentaire d'au moins une partie de la séquence d'identifiant unique et du premier marqueur moléculaire, comprenant facultativement l'obtention d'informations de séquence de la pluralité d'oligonucléotides spécifiques à des réactifs de liaison de composants cellulaires marqués par code-barres, ou de produits de ceux-ci, afin de déterminer le nombre de copies d'au moins une cible de composants cellulaires de la pluralité de cibles de composants cellulaires dans l'une ou plusieurs de la première pluralité de cellules et/ou deuxième pluralité de cellules, facultativement,
l'obtention des informations de séquence comprenant la liaison d'adaptateurs de séquençage à la pluralité d'oligonucléotides spécifiques à des réactifs de liaison de composants cellulaires marqués par code-barres, ou de produits de ceux-ci.

6. Procédé selon l'une quelconque des revendications 1 à 5, dans lequel :
(a) l'oligonucléotide spécifique à un réactif de liaison de composant cellulaire comprend une séquence complémentaire d'une séquence de capture d'un code-barres oligonucléotidique conçue pour capturer la séquence de l'oligonucléotide spécifique à un réactif de liaison de composants cellulaires, facultativement, la séquence de l'oligonucléotide spécifique à un réactif de liaison de composants cellulaires complémentaire de la séquence de capture comprend une région poly(dA) ; et/ou
(b) l'oligonucléotide spécifique à un réactif de liaison de composants cellulaires comprend une deuxième séquence universelle.

7. Procédé selon l'une quelconque des revendications 1 à 6, dans lequel la génération d'amplicons comprend :
(a) l'amplification de la pluralité d'oligonucléotides spécifiques à des réactifs de liaison de composants cellulaires au moyen d'une amorce pouvant s'hybrider à la deuxième séquence universelle, ou un complément de celle-ci, et d'une amorce pouvant s'hybrider à la séquence de capture, ou un complément de celle-ci, générant ainsi des amplicons de la séquence d'identifiant unique d'un ou plusieurs premiers réactifs de liaison de composants cellulaires ;
(b) l'amplification de la pluralité d'oligonucléotides spécifiques à des réactifs de liaison de composants cellulaires au moyen d'une amorce pouvant s'hybrider à la deuxième séquence universelle, ou un complément de celle-ci, et d'une amorce pouvant s'hybrider à la séquence de capture, ou un complément de celle-ci, générant ainsi des produits PCR1 ; et
l'amplification des produits PCR1 au moyen d'une amorce pouvant s'hybrider à la deuxième séquence universelle, ou un complément de celle-ci, et d'une amorce pouvant s'hybrider à la séquence de capture, ou un complément de celle-ci, générant ainsi des amplicons de la séquence d'identifiant unique d'un ou plusieurs premiers réactifs de liaison de composants cellulaires ;
(c) l'amplification de la pluralité d'oligonucléotides spécifiques à des réactifs de liaison de composants cellulaires marqués par code-barres au moyen d'une amorce pouvant s'hybrider à la deuxième séquence universelle, ou un complément de celle-ci, et d'une amorce pouvant s'hybrider à la séquence de capture, ou un complément de celle-ci, générant ainsi des amplicons de la séquence d'identifiant unique d'un ou plusieurs premiers réactifs de liaison de composants cellulaires ;
(d) l'amplification de la pluralité d'oligonucléotides spécifiques à des réactifs de liaison de composants cellulaires au moyen d'une amorce pouvant s'hybrider à la deuxième séquence universelle, ou un complément de celle-ci, et d'une amorce pouvant s'hybrider à la séquence de capture, ou un complément de celle-ci, générant ainsi des produits PCR1 ; et
l'amplification des produits PCR1 au moyen d'une amorce pouvant s'hybrider à la deuxième séquence universelle, ou un complément de celle-ci, et d'une amorce pouvant s'hybrider à la séquence de capture, ou un complément de celle-ci, générant ainsi des amplicons de la séquence d'identifiant unique d'un ou plusieurs premiers réactifs de liaison de composants cellulaires ;
(e) l'amplification de la pluralité d'oligonucléotides spécifiques à des réactifs de liaison de composants cellulaires marqués par code-barres au moyen d'une amorce pouvant s'hybrider à la première séquence universelle, ou un complément de celle-ci, et d'une amorce pouvant s'hybrider à la deuxième séquence universelle, ou un complément de celle-ci, générant ainsi des amplicons de la séquence d'identifiant unique d'un ou plusieurs premiers réactifs de liaison de composants cellulaires ; et/ou
(f) l'amplification de la pluralité d'oligonucléotides spécifiques à des réactifs de liaison de composants cellulaires au moyen d'une amorce pouvant s'hybrider à la première séquence universelle, ou un complément de celle-ci, et d'une amorce pouvant s'hybrider à la deuxième séquence universelle, ou un complément de celle-ci, générant ainsi des produits PCR1 ; et
l'amplification des produits PCR1 au moyen d'une amorce pouvant s'hybrider à la deuxième séquence universelle, ou un complément de celle-ci, et d'une amorce pouvant s'hybrider à la première séquence universelle, ou un complément de celle-ci, générant ainsi des amplicons de la séquence d'identifiant unique d'un ou plusieurs premiers réactifs de liaison de composants cellulaires.

8. Procédé selon l'une quelconque des revendications 1 à 7, dans lequel ladite amplification est réalisée par une méthode choisie dans le groupe constitué par la réaction en chaîne par polymérase (PCR), la réaction en chaîne par ligase (LCR), l'amplification isotherme médiée par les boucles (LAMP), l'amplification par déplacement de brin (SDA), l'amplification médiée par réplicase, l'immunoamplification, l'amplification basée sur la séquence d'acide nucléique (NASBA), la réplication de séquence auto-entretenue (3SR), l'amplification par cercle roulant et l'amplification médiée par la transcription (TMA), facultativement, dans lequel ladite PCR est :
(a) la PCR en temps réel ;
(b) la PCR quantitative en temps réel (QRT-PCR) ; ou
(c) la PCR numérique (dPCR), facultativement la PCR numérique en gouttelettes (ddPCR).

9. Procédé selon l'une quelconque des revendications 1 à 8, dans lequel :
(a) la détermination de la présence ou de la quantité d'un ou plusieurs amplicons comprend la mise en contact des amplicons avec une pluralité de sondes oligonucléotidiques, chacune de la pluralité de sondes oligonucléotidiques comprenant une séquence conçue pour se lier à une séquence d'identifiant unique, ou à un complément de celle-ci, facultativement chaque sonde étant flanquée de séquences complémentaires de l'extrémité 5' et de l'extrémité 3', facultativement, l'une des séquences complémentaires comprenant un fragment émetteur de fluorescence et l'autre séquence complémentaire comprenant un fragment extincteur de fluorescence ;
(b) au moins l'une des sondes oligonucléotidiques comprend un fragment émetteur de fluorescence et fragment extincteur de fluorescence ;
(c) la sonde oligonucléotidique comprend un oligonucléotide de sonde de détection TaqMan, un oligonucléotide de sonde de détection de balise moléculaire ou un oligonucléotide de sonde de détection de torche moléculaire ;
(d) l'étape de génération d'amplicons et l'étape de détermination sont multiplexées, facultativement, au moins environ 4 séquences d'identifiant unique sont analysées simultanément ;
(e) l'étape de génération d'amplicons et l'étape de détermination sont réalisées sur des cellules individuelles ou sur plusieurs cellules ; et/ou
(f) l'étape d'isolement comprend le tri de cellules individuelles ou le tri en vrac.

10. Procédé selon l'une quelconque des revendications 1 à 9, dans lequel :
(a) les un ou plusieurs types de cellule d'intérêt comprennent les cellules endothéliales hémogéniques, les cellules souches et progénitrices hématopoïétiques (HSC), les cellules progénitrices multipotentes hématopoïétiques (MPP), les progéniteurs de cellules pré-T, les progéniteurs de cellules pré-K, les progéniteurs de lymphocytes T, les progéniteurs de lymphocytes NK, les lymphocytes T, les lymphocytes NK, les lymphocytes NKT, les lymphocytes B, les macrophages, les neutrophiles, les cellules CD4, les cellules CD8, les lymphocytes T naïfs, les lymphocytes T souches mémoires, les lymphocytes T mémoires centraux, les lymphocytes T doublement négatifs, les lymphocytes T mémoires effecteurs, les lymphocytes T effecteurs, les lymphocytes ThO, les lymphocytes TcO, les lymphocytes Th1, les lymphocytes Te1, les lymphocytes Th2, les lymphocytes Tc2, les lymphocytes Th17, les lymphocytes Th22, les lymphocytes T gamma/delta, les lymphocytes tueurs naturels (NK), les cellules T tueuses naturelles (NKT), les cellules souches hématopoïétiques, les cellules souches pluripotentes, ou une combinaison quelconque de ceux-ci ;
(b) le ou les types de cellules d'intérêt comprennent un ou plusieurs types de cellules immunitaires, facultativement des lymphocytes T CD4 naïfs, des lymphocytes T CD4 mémoires effecteurs, des lymphocytes T CD8 naïfs, des lymphocytes T CD8 mémoires effecteurs, des lymphocytes Treg CD4 naïfs, des lymphocytes Treg CD4 mémoires effecteurs, des lymphocytes B naïfs, des lymphocytes B mémoires, des cellules dendritiques CD16, des cellules dendritiques plasmacytoïdes, ou une combinaison quelconque de ceux-ci ;
(c) le procédé comprend la mise en contact d'une pluralité de deuxièmes réactifs de liaison de composants cellulaires avec la première et/ou la deuxième pluralité de cellules, chacun de la pluralité de deuxièmes réactifs de liaison de composants cellulaires comprenant un fragment détectable, ou un précurseur de celui-ci, un deuxième réactif de liaison de composants cellulaires pouvant se lier spécifiquement à au moins l'une de la pluralité de cibles de composants cellulaires, les deuxièmes réactifs de liaison de composants cellulaires pouvant se lier à la même cible de composants cellulaires comprenant le même fragment détectable, ou un précurseur de celui-ci, et les deuxièmes réactifs de liaison de composants cellulaires pouvant se lier à différentes cibles de composants cellulaires comprenant différents fragments détectables, ou des précurseurs de ceux-ci ;
(d) la première pluralité de cellules et/ou la deuxième pluralité de cellules sont mises en contact avec les premiers réactifs de liaison de composants cellulaires et les deuxièmes réactifs de liaison de composants cellulaires simultanément ;
(e) un ou plusieurs premiers réactifs de liaison de composants cellulaires et un ou plusieurs deuxièmes réactifs de liaison de composants cellulaires peuvent se lier à la même cible de composants cellulaires, facultativement, les premiers réactifs de liaison de composants cellulaires et les deuxièmes réactifs de liaison de composants cellulaires pouvant se lier à la même cible de composants cellulaires présentent le même clonotype ; et/ou
(f) l'étape d'isolement comprend :
(i) un tri cellulaire activé par fluorescence, facultativement, le tri cellulaire activé par fluorescence utilisant les deuxièmes réactifs de liaison de composants cellulaires ;
(ii) le dépôt de la ou des cellules d'intérêt de la première et/ou la deuxième pluralité de cellules dans une ou plusieurs plaques de microtitration ; et/ou
(iii) la détection d'émissions du fragment détectable des deuxièmes réactifs de liaison de composants cellulaires à l'aide d'un cytomètre en flux, facultativement, le cytomètre en flux comprenant un cytomètre en flux conventionnel, un cytomètre en flux spectral, un cytomètre en flux hyperspectral, un cytomètre en flux d'imagerie ou une combinaison quelconque de ceux-ci ; et/ou
(g) le fragment détectable comprend un fragment optique, un fragment luminescent, un fragment électrochimiquement actif, une nanoparticule ou une combinaison de celles-ci, facultativement, la nanoparticule comprend un point quantique ;
(h) le fragment luminescent comprend un fragment chimioluminescent, un fragment électroluminescent, un fragment photoluminescent ou une combinaison de ceux-ci ;
(i) le fragment photoluminescent comprend un fragment fluorescent, un fragment phosphorescent ou une combinaison de ceux-ci, facultativement, le fragment fluorescent comprend un colorant fluorescent ;
(j) le procédé comprend la réalisation d'une réaction pour convertir le précurseur de fragment détectable en fragment détectable ;
(k) la ou les cibles de composants cellulaires positives et/ou la ou les cibles de composants cellulaires négatives comprennent CD4, CD8, CCR7, CD45RO, CD25, HLA-DR, CD45RA, CD19, CD3, CD27, IgD, CD11c, CD16, ou une combinaison quelconque de ceux-ci ; et/ou
(l) le nombre de premières séquences de marqueurs moléculaires uniques associées à la séquence d'identifiant unique pour le premier réactif de liaison de composants cellulaires pouvant se lier de manière spécifique à la ou aux cibles de composants cellulaires dans les données de séquençage indique le nombre de copies de la ou des cibles de composants cellulaires dans la ou les cellules de la première et/ou la deuxième pluralité de cellules.

11. Procédé selon l'une quelconque des revendications 1 à 10, dans lequel :
(a) l'oligonucléotide spécifique à un réactif de liaison de composants cellulaires comprend l'un ou plusieurs parmi :
(i) une deuxième séquence de marquage moléculaire, facultativement :
(A) la deuxième séquence d'étiquette moléculaire a une longueur de 2 à 20 nucléotides ;
(B) les deuxièmes séquences de marqueurs moléculaires d'au moins deux oligonucléotides spécifiques à des réactifs de liaison de composants cellulaires sont différentes, et les séquences d'identifiants uniques des au moins deux oligonucléotides spécifiques à des réactifs de liaison de composants cellulaires sont identiques ; et/ou
(C) les deuxièmes séquences de marqueurs moléculaires d'au moins deux oligonucléotides spécifiques à des réactifs de liaison de composants cellulaires sont différentes, et les séquences d'identifiants uniques des au moins deux oligonucléotides spécifiques à des réactifs de liaison de composants cellulaires sont différentes ; et/ou
(ii) une séquence d'alignement adjacente à une région poly(dA), facultativement, la séquence d'alignement a une longueur d'un ou plusieurs nucléotides, ou d'au moins deux nucléotides ; et
(iii) un lieur, l'oligonucléotide spécifique à un réactif de liaison de composants cellulaires étant associé au premier réactif de liaison de composants cellulaires par l'intermédiaire du lieur ; et/ou
(b) le nombre de deuxièmes séquences de marqueurs moléculaires uniques associées à la séquence d'identifiant unique pour le premier réactif de liaison de composants cellulaires pouvant se lier de manière spécifique à la ou aux cibles de composants cellulaires dans les données de séquençage indique le nombre de copies de la ou des cibles de composants cellulaires dans la ou les cellules de la première et/ou la deuxième pluralité de cellules ; et/ou
(c) la séquence d'alignement :
(i) comprend une guanine, une cytosine, une thymine, un uracile, ou une combinaison de ceux-ci ;
(ii) comprend une séquence poly(dT), une séquence poly(dG), une séquence poly(dC), une séquence poly(dU), ou une combinaison de ceux-ci ; et/ou
(iii) est en 5' par rapport à la région poly(dA) ; et/ou
(d) le lieur comprend :
(i) une chaîne carbonée, facultativement, la chaîne carbonée comprend 2 à 30 atomes de carbone, et en outre, facultativement, la chaîne carbonée comprenant 12 atomes de carbone ; et/ou
(ii) un modificateur amino en C12 en 5' (5AmMC12), ou un dérivé de celui-ci ; et/ou
(e) la pluralité de cibles de composants cellulaires comprend une pluralité de cibles protéiques, et le premier réactif de liaison de composants cellulaires peut se lier de manière spécifique à au moins l'une des cibles protéiques, facultativement, la pluralité de cibles de composants cellulaires comprend une protéine de surface cellulaire, une protéine intracellulaire, un marqueur cellulaire, un récepteur de lymphocytes B, un récepteur de lymphocytes T, un anticorps, un complexe majeur d'histocompatibilité, un antigène tumoral, un récepteur, ou une combinaison de ceux-ci ; et/ou
(f) l'oligonucléotide spécifique à un réactif de liaison de composants cellulaires est :
(i) associé au premier réactif de liaison de composants cellulaires, facultativement l'oligonucléotide spécifique à un réactif de liaison de composants cellulaires est lié de manière covalente au premier réactif de liaison de composants cellulaires et/ou lié de manière non covalente au premier réactif de liaison de composants cellulaires ;
(ii) conjugué au premier réactif de liaison de composants cellulaires, et facultativement, l'oligonucléotide spécifique à un réactif de liaison de composants cellulaires est conjugué au premier réactif de liaison de composants cellulaires par l'intermédiaire d'un groupe chimique choisi dans le groupe constitué par un groupe photoclivable par les UV, une streptavidine, une biotine, une amine et une combinaison de ceux-ci ; et/ou
(iii) conçu pour être séparable du premier réactif de liaison de composants cellulaires ; et/ou
(g) le procédé comprend la dissociation de l'oligonucléotide spécifique à un réactif de liaison de composants cellulaires du premier réactif de liaison de composants cellulaires, facultativement dans lequel :
(i) la dissociation de l'oligonucléotide spécifique à un réactif de liaison de composants cellulaires comprend la séparation de l'oligonucléotide spécifique à un premier réactif de liaison de composants cellulaires par photoclivage par les UV, traitement chimique, chauffage, traitement enzymatique ou une combinaison quelconque de ceux-ci ; et/ou
(ii) la dissociation survient après le marquage par code-barres de l'oligonucléotide spécifique à un réactif de liaison de composants cellulaires et/ou la dissociation survient avant le marquage par code-barres de l'oligonucléotide spécifique à un réactif de liaison de composants cellulaires ; et/ou
(h) l'oligonucléotide spécifique à un réactif de liaison de composants cellulaires est conçu pour être non séparable du premier réactif de liaison de composants cellulaires ; et/ou
(i) le procédé comprend, après la mise en contact de la pluralité de premiers réactifs de liaison de composants cellulaires avec la première et/ou deuxième pluralité de cellules, l'élimination d'un ou plusieurs premiers réactifs de liaison de composants cellulaires parmi la pluralité de premiers réactifs de liaison de composants cellulaires qui ne sont pas mis en contact avec la première et/ou deuxième pluralité de cellules, facultativement, l'élimination du ou des réactifs de liaison de composants cellulaires non mis en contact avec la première et/ou deuxième pluralité de cellules comprenant l'élimination du ou des premiers réactifs de liaison de composants cellulaires non mis en contact avec la ou les cibles respectives parmi la pluralité de cibles de composants cellulaires.

12. Procédé selon l'une quelconque des revendications 1 à 11, dans lequel :
(a) la première et/ou deuxième pluralité de cellules comprend des lymphocytes T, des lymphocytes B, des cellules tumorales, des cellules myéloïdes, des cellules sanguines, des cellules normales, des cellules fœtales, des cellules maternelles ou un mélange de ceux-ci ; et/ou
(b) une ou plusieurs cellules individuelles de la première pluralité de cellules et/ou deuxième pluralité de cellules comprennent des copies d'une cible d'acide nucléique, le procédé comprenant en outre :
la mise en contact d'une pluralité de codes-barres oligonucléotidiques avec les copies de la cible d'acide nucléique pour réaliser une hybridation, chaque code-barres oligonucléotidique de la pluralité de codes-barres oligonucléotidiques comprenant une première séquence universelle, une région de liaison à une cible pouvant s'hybrider aux copies de la cible d'acide nucléique, et un premier marqueur moléculaire ;
l'allongement de la pluralité de codes-barres oligonucléotidiques hybridés aux copies d'une cible d'acide nucléique pour générer une pluralité de molécules d'acide nucléique marquées par code-barres comprenant chacune une séquence complémentaire d'au moins une partie de la cible d'acide nucléique ; et
l'obtention d'informations de séquence de la pluralité de molécules d'acide nucléique marquées par code-barres, ou de produits de celles-ci, afin de déterminer le nombre de copies de la cible d'acide nucléique dans chacune des cellules individuelles.

13. Procédé selon l'une quelconque des revendications 1 à 12, dans lequel :
(a) la détermination du nombre de copies de la cible d'acide nucléique dans chacune des cellules individuelles comprend la détermination du nombre de copies de la cible d'acide nucléique dans chacune des cellules individuelles sur la base du nombre de premiers marqueurs moléculaires avec des séquences distinctes, des compléments de ceux-ci, ou une combinaison de ceux-ci, associés à la pluralité de molécules d'acide nucléique marquées par code-barres, ou de produits de ceux-ci ;
(b) l'obtention de données de séquençage comprend la liaison d'adaptateurs de séquençage à la pluralité de molécules d'acide nucléique marquées par code-barres, ou des produits de celles-ci ;
(c) la pluralité de molécules d'acide nucléique marquées par code-barres comprend des molécules d'acide désoxyribonucléique (ADN) marquées par code-barres et/ou des molécules d'acide ribonucléique (ARN) marquées par code-barres ; et/ou
(d) la cible d'acide nucléique comprend une molécule d'acide nucléique, facultativement un acide ribonucléique (ARN), un ARN messager (ARNm), un microARN, un petit ARN interférent (pARNi), un produit de dégradation d'ARN, un ARN comprenant une queue poly(A), ou une combinaison quelconque de ceux-ci, et en outre, facultativement, l'ARNm code pour un récepteur immunitaire.

14. Procédé selon l'une quelconque des revendications 1 à 13, dans lequel :
(a) au moins 10 de la première pluralité de codes-barres oligonucléotidiques comprennent des premières séquences de marqueurs moléculaires différentes ;
(b) chaque premier marqueur moléculaire de la pluralité de codes-barres oligonucléotidiques comprend au moins 6 nucléotides ;
(c) la pluralité de codes-barres oligonucléotidiques sont associés à un support solide, et facultativement, la pluralité de codes-barres oligonucléotidiques associés au même support solide comprennent chacun un marqueur d'échantillon identique, et en outre, facultativement, chaque marqueur d'échantillon de la pluralité de codes-barres oligonucléotidiques comprend au moins 6 nucléotides ; et/ou
(d) la pluralité de codes-barres oligonucléotidiques comprennent chacun un marqueur cellulaire, et facultativement, chaque marqueur cellulaire de la pluralité de codes-barres oligonucléotidiques comprend au moins 6 nucléotides, facultativement, les codes-barres oligonucléotidiques associés au même support solide comprenant :
(i) le même marqueur cellulaire ; ou
(ii) différents marqueurs cellulaires ; et/ou (e) la première pluralité de cellules et la deuxième pluralité de cellules sont issues du même échantillon.

15. Procédé selon l'une quelconque des revendications 1 à 14, le procédé comprenant l'association d'une particule synthétique comprenant la pluralité de codes-barres oligonucléotidiques à une cellule individuelle dans la première et/ou deuxième pluralité de cellules individuelles, facultativement dans lequel :
(a) le procédé comprend la lyse de la cellule individuelle après l'association de la particule synthétique à la cellule individuelle, et facultativement, la lyse de la cellule comprend le chauffage de l'échantillon, la mise en contact de l'échantillon avec un détergent, la modification du pH de l'échantillon, ou une combinaison quelconque de ceux-ci ;
(b) la particule synthétique et la cellule sont dans le même puits ;
(c) la particule synthétique et la cellule sont dans la même gouttelette ;
(d) au moins l'un des codes-barres oligonucléotidiques est immobilisé ou partiellement immobilisé sur la particule synthétique, ou le ou les codes-barres oligonucléotidiques sont encapsulés ou partiellement encapsulés dans la particule synthétique ;
(e) la particule synthétique est dégradable ; et/ou
(f) la particule synthétique comprend une bille, et facultativement, la bille comprend :
(i) une bille de Sepharose, une bille de streptavidine, une bille d'agarose, une bille magnétique, une bille conjuguée, une bille conjuguée à la protéine A, une bille conjuguée à la protéine G, une bille conjuguée à la protéine A/G, une bille conjuguée à la protéine L, une bille conjuguée à un oligo(dT), une bille de silice, une bille d'analogue de silice, une microbille anti-biotine, une microbille anti-fluorochrome ou une combinaison quelconque de celles-ci ;
(ii) un matériau choisi dans le groupe constitué par le polydiméthylsiloxane (PDMS), le polystyrène, le verre, le polypropylène, l'agarose, la gélatine, un hydrogel, un paramagnétique, une céramique, une matière plastique, le verre, le méthylstyrène, un polymère acrylique, le titane, le latex, la Sepharose, la cellulose, le nylon, la silicone et une combinaison quelconque de ceux-ci ; ou
(iii) une particule d'hydrogel dégradable ; et/ou
(g) chacun de la pluralité de codes-barres oligonucléotidiques comprend un groupe fonctionnel de lieur, la particule synthétique comprenant un groupe fonctionnel de support solide, et/ou le groupe fonctionnel de support solide et le groupe fonctionnel de lieur étant associés l'un à l'autre ; et facultativement, le groupe fonctionnel de lieur et le groupe fonctionnel de support sont choisis individuellement dans le groupe constitué par C6, la biotine, la streptavidine, une ou plusieurs amines primaires, un ou plusieurs aldéhydes, une ou plusieurs cétones, et une combinaison quelconque de ceux-ci.
